# EUROPÄISCHE PATENTSCHRIFT

(11) **EP 0 840 721 B1**
(45) Veröffentlichungstag und Bekanntmachung des Hinweises auf die Patenterteilung: **04.10.2001**
(21) Anmeldenummer: 96922790.9
(22) Anmeldetag: 10.06.1996
(51) Int. Cl.: C07C 251/48, C07C 251/60, C07D 333/22, C07D 213/53, C07C 257/20, A01N 37/50, A01N 43/10, A01N 43/40

(54) **CARBONSÄUREAMIDDERIVATE UND IHRE VERWENDUNG ALS SCHÄDLINGSBEKÄMPFUNGSMITTEL**
CARBOXYLIC ACID AMIDE DERIVATIVES AND THEIR USE AS PESTICIDES
DERIVES D'AMIDE D'ACIDE CARBOXYLIQUE ET LEUR UTILISATION COMME PESTICIDES

(30) Priorität: 22.06.1995 DE 19522702
(43) Veröffentlichungstag der Anmeldung: 13.05.1998
(73) Patentinhaber: BAYER AG, 51368 Leverkusen (DE)
(72) Erfinder: HEINEMANN, Ulrich, D-42799 Leichlingen (DE); GERDES, Peter, D-52080 Aachen (DE); GAYER, Herbert, D-40789 Monheim (DE); SEITZ, Thomas, D-40764 Langenfeld (DE); TIEMANN, Ralf, D-51375 Leverkusen (DE); STENZEL, Klaus, D-40595 Düsseldorf (DE); DUTZMANN, Stefan, D-40721 Hilden (DE)
(86) Internationale Anmeldenummer: EP9602511
(87) Internationale Veröffentlichungsnummer: WO9700856

(56) Entgegenhaltungen:
- EP-A- 0 398 692
- EP-A- 0 585 751
- DE-A- 2 124 090
- DATABASE CROSSFIRE Beilstein Informationsgesellschaft GmbH XP002016096 & TETRAHEDRON LETT., Bd. 23, Nr. 11, 1982, Seiten 1131-34,
- DATABASE CROSSFIRE Beilstein Informationsgesellschaft GmbH XP002016097 & JP,A,46 091 328 (TIEMARU ISAO)
- DATABASE CROSSFIRE Beilstein Informationsgesellschaft GmbH XP002016098 & J. INDIAN CHEM. SOC., Bd. 26, 1949, Seite 249
- DATABASE CROSSFIRE Beilstein Informationsgesellschaft GmbH XP002016099 & COLLECT. CZECH. CHEM. COMMUN., Bd. 42, 1977, Seiten 2672-79,

## Beschreibung

Die Erfindung betrifft neue Carbonsäureamidderivate, mehrere Verfahren zu ihrer Herstellung und ihre Verwendung als Fungizide.

Es ist bekannt, daß bestimmte Carbonsäureamide fungizide Eigenschaften aufweisen (vgl. z. B. EP-A 398692 und EP-A 528681). Die Wirkung dieser Verbindungen ist jedoch insbesondere bei niedrigen Aufwandmengen nicht in allen Anwendungsgebieten völlig zufriedenstellend. In DE-A-2 124 090 werden neue Phenylthiocarbanate und ihre Herstellung beschrieben. EP-A1-585 751 beschreibt N-Methylamide, Verfahren und Zwischenprodukte zu ihrer Herstellung sowie Verfahren zur Bekämpfung von Schädlingen. In EP-A2-398 692 werden Alkoxyiminoacetamidderivate und ihre Verwendung als Fungizide beschrieben.

Es wurden nun die neuen substituierten Heterocycloalkene der allgemeinen Formel (I) gefunden, 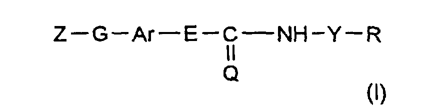 in welcher
- Ar: für gegebenenfalls substituiertes Arylen oder Heteroarylen steht,
- E: für eine 1-Alken-1,1-diyl-Gruppierung steht, die in 2-Position einen Rest R¹ enthält, oder für eine 2-Aza-1-alken-1,1-diyl-Gruppierung steht, die in 2-Position einen Rest R² enthält, wobei
R¹ für Wasserstoff, Halogen, Cyano oder für jeweils gegebenenfalls substituiertes Alkyl, Alkoxy, Alkylthio, Alkylamino oder Dialkylamino steht,
R² für Wasserstoff, Amino, Hydroxy, Cyano oder für jeweils gegebenenfalls substituiertes Alkyl, Alkoxy, Alkylamino oder Dialkylamino steht, und
- G: für eine Einfachbindung, für Sauerstoff, Schwefel oder für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- oder -T-Ar¹-Q¹- steht, wobei
Ar¹ für gegebenenfalls substituiertes Arylen, Heteroarylen, Cycloalkylen oder Heterocycloalkylen (d. h. ein zweifach verknüpfter aliphatischer Ring, in dem ein oder mehrere Kohlenstoffatome durch Heteroatome, d.h. von Kohlenstoff verschiedene Atome ersetzt sind) steht,
n für die Zahlen 0, 1 oder 2 steht,
Q¹ für Sauerstoff oder Schwefel steht,
R⁴ für Wasserstoff, Cyano oder jeweils gegebenenfalls substituieries Alkyl, Alkoxy, Alkylthio, Alkylamino, Dialkylamino oder Cycloalkyl steht,
R⁵ für Wasserstoff, Hydroxy, Cyano oder jeweils gegebenenfalls substituiertes Alkyl, Alkoxy oder Cycloalkyl steht,
L für Wasserstoff oder Alkyl steht und
T für eine Einfachbindung, für Sauerstoff, Schwefel, -CH₂-O-, -CH₂-S- oder für gegebenenfalls substituiertes Alkandiyl steht,
- Q: für Sauerstoff oder Schwefel steht,
- R: für Wasserstoff, Amino oder jeweils gegebenenfalls substituiertes Alkyl, Alkoxy, Alkylamino oder Dialkylamino steht,
- Y: für -CO-, -CS- oder -SO₂- steht und
- Z: für jeweils gegebenenfalls substituiertes Alkyl, Alkenyl, Alkinyl, Cycloalkyl, Aryl oder Heterocyclyl steht,
ausgenommen die Verbindung N-(Dimethyl-hydrazono)-(4-methoxy-phenyl)-acetyl-methansulfonamid.

Aryl steht im folgenden vorzugsweise für aromatische, mono- oder polycyclische Kohlenwasserstoffringe, wie z. B. Phenyl, Naphthyl, Anthranyl, Phenanthryl, vorzugsweise Phenyl oder Naphthyl, insbesondere Phenyl.

Heterocyclyl steht im folgenden vorzugsweise für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält der Ring mehr als ein Sauerstoffatom, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Weiterhin wurde gefunden, daß man die neuen substituierten Heterocycloalkene der allgemeinen Formel (I) erhält, wenn man
Carbonsäureamide der allgemeinen Formel (II), 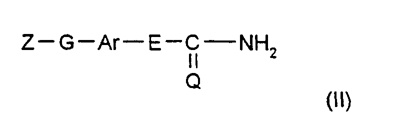 in welcher
- Ar, E, G, Q und Z: die oben angegebenen Bedeutungen haben,
entweder
a) mit einem Carbonsäurehalogenid der Formel (III), 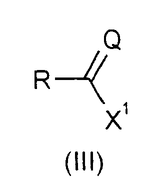 in welcher
   - Q und R: die oben angegebenen Bedeutungen haben, und
   - X¹: für Halogen steht, oder
b) mit einem Carbonsäureanhydrid der Formel (IV), 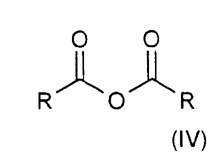 in welcher
   - R: die oben angegebene Bedeutung hat, oder
c) mit einem Sulfonsäurehalogenid der Formel (V), 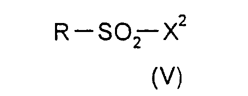 in welcher
   - R: die oben angegebene Bedeutung hat und
   - X²: für Halogen steht,
   jeweils gegebenenfalls in Gegenwart eines Verdünnungsmittels und jeweils gegebenenfalls in Gegenwart eines Säurebindemittels, umsetzt,
   oder wenn man
d) Carbonsäureamide der allgemeinen Formel (II)
   mit einem Isocyanat der Formel (VI), 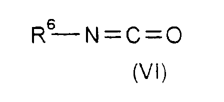 in welcher
   - R⁶: für Alkyl steht,
   gegebenenfalls in Gegenwart eines Verdünnungsmittels und gegebenenfalls in Gegenwart eines Reaktionshilfsmittels, umsetzt,
   oder wenn man
e) Amidine der allgemeinen Formel (VII), 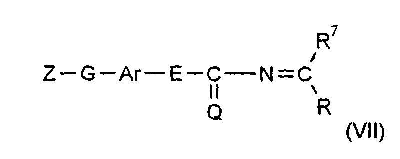 in welcher
   - Ar, E, G, Q, R, und Z: die oben angegebenen Bedeutungen haben und
   - R⁷: für Dialkylamino steht,
   gegebenenfalls in Gegenwart eines Verdünnungsmittels und gegebenenfalls in Gegenwart einer Säure, hydrolysiert.

Schließlich wurde gefunden, daß die neuen Carbonsäureamidderivate der allgemeinen Formel (I) sehr starke fungizide Wirkung zeigen.

Die erfindungsgemäßen Verbindungen Gegen gegebenenfalls als Mischungen verschiedener möglicher isomerer Formen, insbesondere von Stereoisomeren, wie z. B. E- und Z-Isomeren, gegebenenfalls aber auch von Tautomeren vor. Es werden sowohl die E- als auch die Z-Isomeren, beliebige Mischungen dieser Isomeren, sowie die möglichen tautomeren Formen beansprucht.

Die erfindungsgemäßen substituierten Carbonsäureamidderivate sind durch die Formel (I) allgemein definiert. Von besonderem Interesse sind Verbindungen der Formel (I), in denen Z, G, Ar, E, Q, R und Y jeweils unabhängig voneinander wie folgt definiert sind: Wobei vorzugsweise
- Ar: für jeweils gegebenenfalls substituiertes Phenylen oder Naphthylen steht, oder, für mono- oder bicyclisches Heteroarylen mit jeweils 5 oder 6 Ringgliedern oder für benzokondensiertes Heteroarylen mit 5 oder 6 Ringgliedern steht, von denen jeweils mindestens eines für Sauerstoff, Schwefel oder Stickstoff steht und gegebenenfalls ein oder zwei weitere für Stickstoff stehen, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
- E: für eine der nachstehenden Gruppierungen steht: 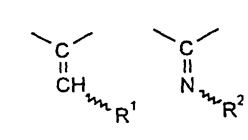 worin
R¹ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R² für Wasserstoff, Amino, Hydroxy, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht,
- G: für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen steht oder für eine der nachstehenden Gruppierungen
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- oder -T-Ar¹-Q¹- steht, wobei
n für die Zahlen 0, 1 oder 2 steht,
Q' für Sauerstoff oder Schwefel steht,
R⁴ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxycarbonyl substituiertes Cycloalkyl mit 3 bis 6 Kohlenstoffatomen steht,
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
L für Wasserstoff oder Alkyl mit 1 bis 4 Kohlenstoffatomen steht und
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen:
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie Cycloalkyl mit 3 bis 6 Kohlenstoffatomen und

T für eine Einfachbindung, für Sauerstoff, Schwefel, -CH₂-O-, -CH₂-S- oder für Alkandiyl mit 1 bis 3 Kohlenstoffatomen steht,
- Q: für Sauerstoff steht;
- R: für Wasserstoff, Amino, Alkyl, Halogenalkyl, Alkoxy Halogenalkoxy, Alkylamino oder Dialkylamino mit jeweils 1 bis 4 Kohlenstoffatomen in der jeweiligen Alkylkette steht;
- Y: für -CO- oder -SO₂- steht;
- Z: für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen steht;
für jeweils gegebenenfalls durch Halogen substituiertes Alkenyl oder Alkinyl mit jeweils bis zu 8 Kohlenstoffatomen steht;
für jeweils gegebenenfalls einfach oder mehrfach, gleich oder verschieden durch Halogen, Cyano, Carboxy. Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substiuiertes Cycloalkyl mit 3 bis 6 Kohlenstoffatomen steht;
für jeweils gegebenenfalls einfach oder mehrfach, gleich oder verschieden substituiertes Phenyl, Naphthyl oder für Heterocyclyl mit 3 bis 7 Ringgliedern steht, von denen mindestens eines für Sauerstoff, Schwefel oder Stickstoff und gegebenenfalls ein oder zwei weitere für Stickstoff stehen, wobei die möglichen Substituenten vorzugsweise aus der nachstehenden Aufzählung ausgewählt sind:
   Halogen, Cyano, Nitro, Amino, Hydroxy, Formyl, Carboxy, Carbamoyl, Thiocarbamoyl;
   jeweils geradkettiges oder verzweigtes Alkyl, Alkoxy, Alkylthio, Alkylsulfinyl oder Alkylsulfonyl mit jeweils 1 bis 6 Kohlenstoffatomen;
   jeweils geradkettiges oder verzweigtes Alkenyl oder Alkenyloxy mit jeweils 2 bis 6 Kohlenstoffatomen;
   jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
   jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
   jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
   jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
   Cycloalkyl mit 3 bis 6 Kohlenstoffatomen;
   Heterocyclyl oder Heterocyclyl-methyl mit jeweils 3 bis 7 Ringgliedern, von denen jeweils 1 bis 3 gleiche oder verschiedene Heteroatome sind insbesondere Stickstoff, Sauerstoff und/oder Schwefel
      oder eine Gruppierung 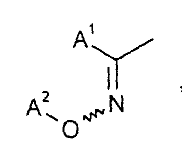 worin
      A¹ für Alkyl mit 1 bis 4 Kohlenstoffatomen oder Cycloalkyl mit 1 bis 6 Kohlenstoffatomen steht und
      A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht;
wobei insbesondere

- Ar: für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Cyclopropyl, Trifluormethyl, Methoxy. Ethoxy, Methylthio, Methylsulfinyl oder Methylsulfonyl;
- E: für eine der nachstehenden Gruppierungen steht: 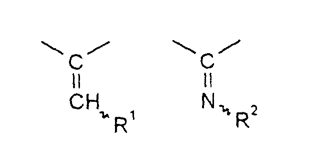 worin
R¹ für jeweils gegebenenfalls durch Fluor, Chlor, Cyano, Methoxy oder Ethoxy substituiertes Methyl, Ethyl oder Methoxy steht,
R² für jeweils gegebenenfalls durch Fluor, Chlor, Cyano, Methoxy oder Ethoxy substituiertes Methyl, Ethyl oder Methoxy steht;
- G: für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen steht
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- oder -T-Ar¹-Q¹-, wobei
n für die Zahlen 0, 1 oder 2 steht,
Q' für Sauerstoff oder Schwefel steht,
R⁴ für Wasserstoff, Cyano, Methyl, Ethyl oder Cyclopropyl steht und
R⁵ für Wasserstoff, Methyl, Ethyl oder Cyclopropyl steht,
L für Wasserstoff oder Methyl steht und
Ar¹ für jeweils gegebenenfalls einfach bis dreifach, gleich oder verschieden substituiertes Phenylen oder Pyridindiyl, für jeweils gegebenenfalls einfach substituiertes Pyrimidindiyl, Pyridazindiyl, Pyrazindiyl, 1,2,3-Triazindiyl, 1,2,4-Triazindiyl oder 1,3,5-Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
T für eine Einfachbindung, für Sauerstoff, Schwefel, -CH₂-O-, -CH₂-S-, Methylen, Ethylen oder Propylen steht;
- Q: für Sauerstoff steht;
- R: für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht;
- Y: für -CO- oder -SO₂- steht;
- Z: für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl. Trifluorethyl, Difluormethoxy, Trifluormethoxy, Difluorchlormethoxy, Trifluorethoxy, Difluormethvlthio, Trifluormethylthio. Difluorchlormethylthio, Trifluormethylsulfinyl oder Trifluormethylsulfonyl, Methoxycarbonyl, Ethoxycarbonyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy
oder eine Gruppierung 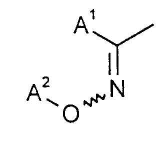 wobei
A¹ für Methyl, Ethyl, n- oder i-Propyl, n-, i-, s- oder t-Butyl, Cyclopropyl oder Cyclobutyl steht;
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

Eine besonders bevorzugte Gruppe erfindungsgemäßer Verbindungen sind diejenigen Verbindungen der Formel (I),
in welcher
- Ar: für ortho-Phenylen, Pyridin-2,3-diyl oder Thiophen-2,3-diyl steht,
- E: für eine der nachstehenden Gruppierungen steht, 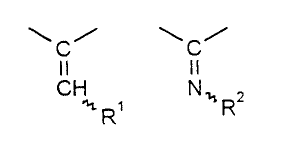 worin
- R¹ und R²: jeweils für Methoxy stehen,
- G: für -O-CH₂ steht,
- Q: für Sauerstoff steht,
- R: für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht,
- Y: für -CO- oder -SO₂- steht und
- Z: für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy
oder eine Gruppierung 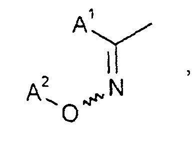 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyh n-, i-, s- oder t-Butyl, Allyl, Propargyl, But-2-en-1-yl, 2-Methyl-prop-1-en-3-yl, Cyanmethyl, Methoxymethyl Ethoxymethyl, Methoxyethyl, Ethoxyethyl, Methylthiomethyl, Ethylthiomethyl. Methylthioethyl, Ethylthioethyl, Dimethylaminomethyl, Dimethylaminoethyl, Methylaminomethyl, Methylaminoethyl oder Benzyl steht

Eine ebenfalls besonders bevorzugte Gruppe erfindungsgemäßer Verbindungen sind diejenigen Verbindungen der Formel (I),
in welcher
- Ar: für ortho-Phenylen, Pyridin-2,3-diyl oder Thiophen-2,3-diyl steht,
- E: für eine der nachstehenden Gruppierungen steht, 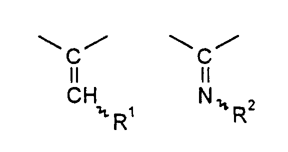 worin
- R¹ und R²: jeweils für Methoxy stehen,
- G: für -C(R⁴)=N-O-CH₂- steht, wobei
- R⁴: für Wasserstoff, Cyano, Methyl, Ethyl oder Cyclopropyl steht,
- Q: für Sauerstoff steht,
- R: für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht,
- Y: für -CO- oder -SO₂- steht und
- Z: für jeweils gegebenenfalls einfach bis dreifach, gleich oder verschieden substituiertes Phenyl, Pyridyl oder Pyrimidyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy.

Eine weiterhin besonders bevorzugte Gruppe erfindungsgemäßer Verbindungen sind diejenigen Verbindungen der Formel (I),
in welcher
- Ar: für ortho-Phenylen steht,
- E: für eine der nachstehenden Gruppierungen steht: 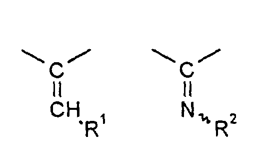 worin
R¹ und R² jeweils für Methoxy stehen,
G für -T-Ar¹-Q- steht, wobei
Q für Sauerstoff oder Schwefel steht,
Ar¹ für 1,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio. Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, -CH₂-S-, Methylen, Ethylen oder Propylen steht,
Q für Sauerstoff steht,
R für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht,
Y für -CO- oder -SO₂- steht und
Z für jeweils gegebenenfalls einfach bis dreifach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy, Difluorchlormethoxy, Trifluorethoxy, Trifluormethoxy oder jeweils gegebenenfalls einfach oder mehrfach, gleich oder verschieden durch Fluor, Chlor, Methyl, Trifluormethyl oder Ethyl substituiertes, jeweils zweifach verknüpftes Methylendioxy oder Ethylendioxy, substituiertes Phenyl, Pyridyl oder Thienyl steht.

Die oben aufgeführten allgemeinen oder bevorzugten Restedefinitionen gelten sowohl für die Endprodukte der Formel (I) als auch entsprechend für die jeweils zur Herstellung benötigten Ausgangsstoffe bzw. Zwischenprodukte.

Beispiele für die erfindungsgemäßen Verbindungen sind in den Tabellen 1 bis 266 aufgeführt:

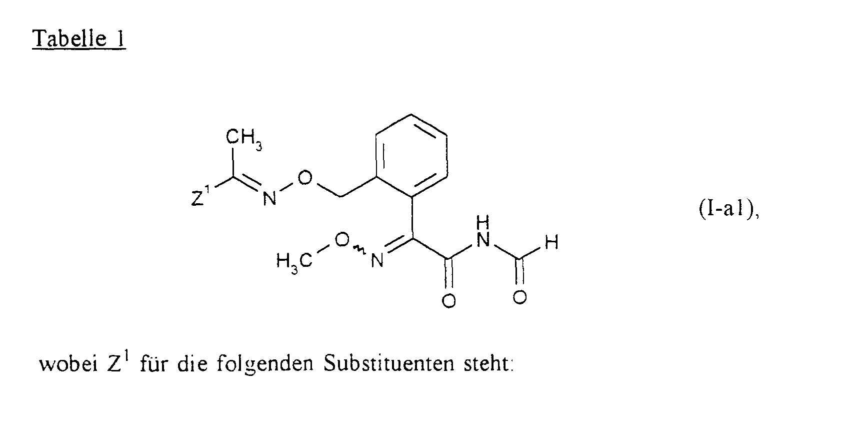

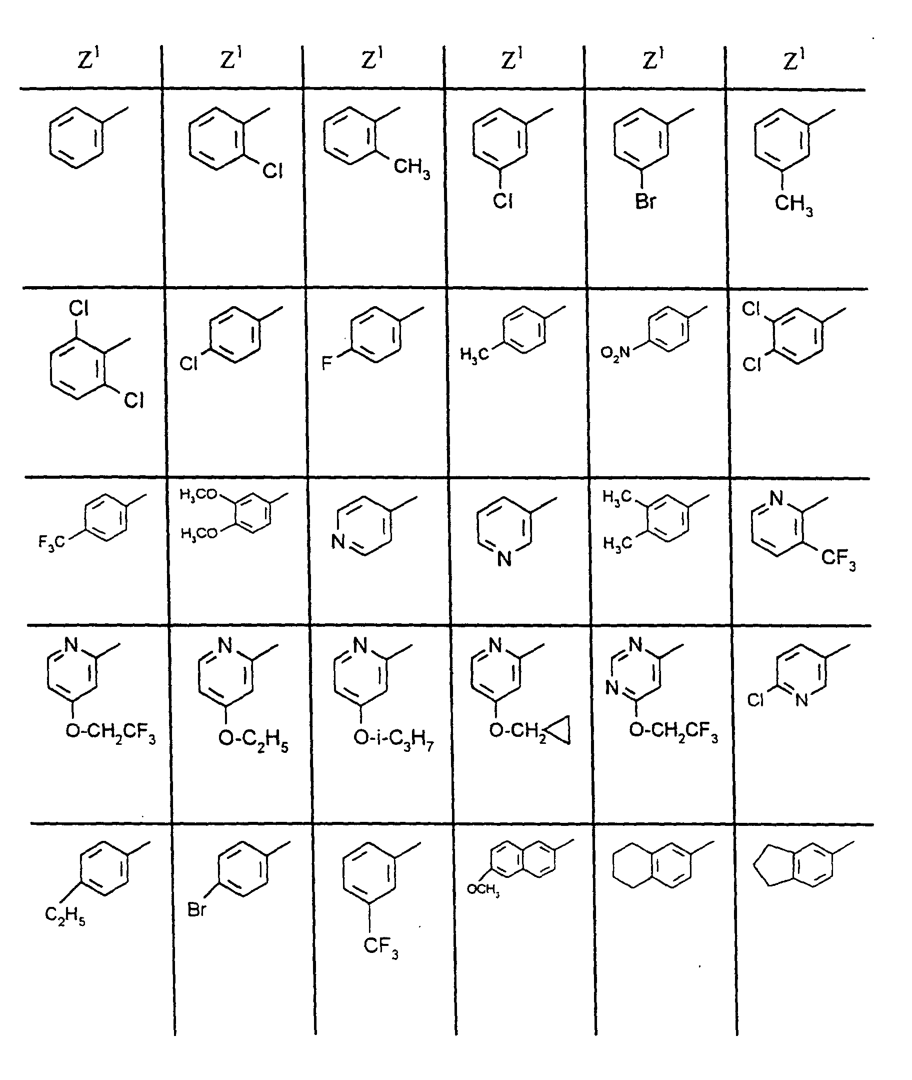

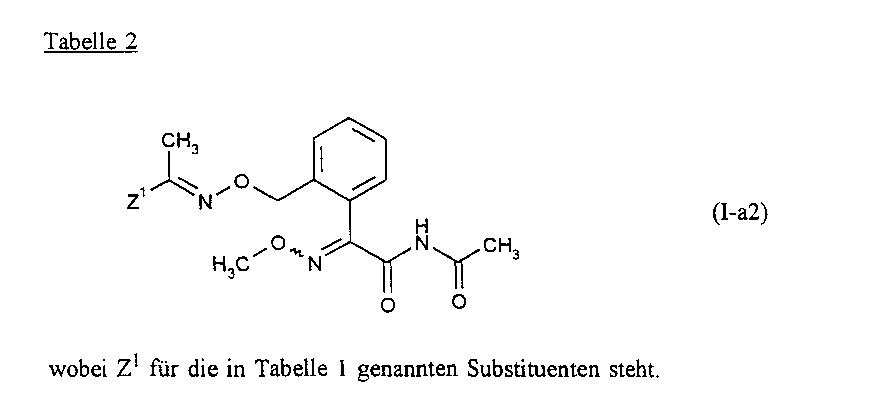

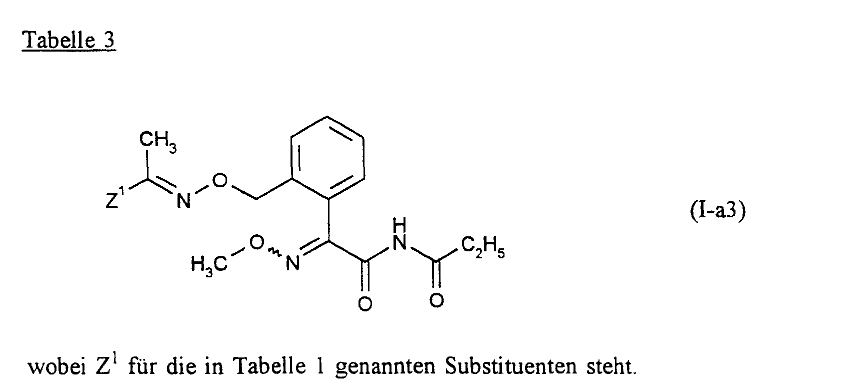

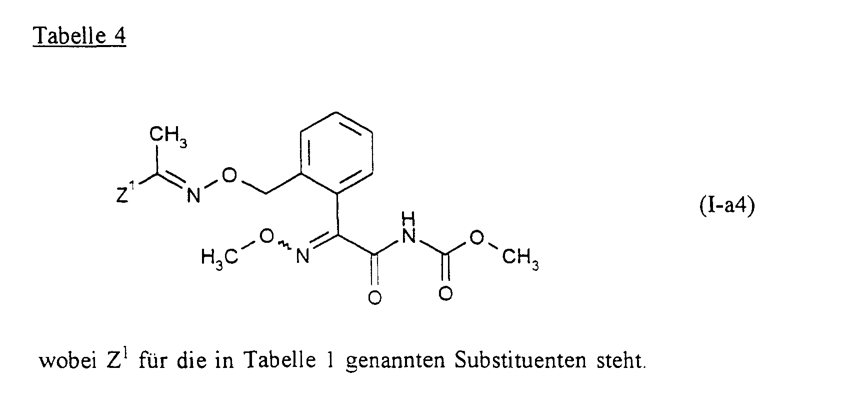

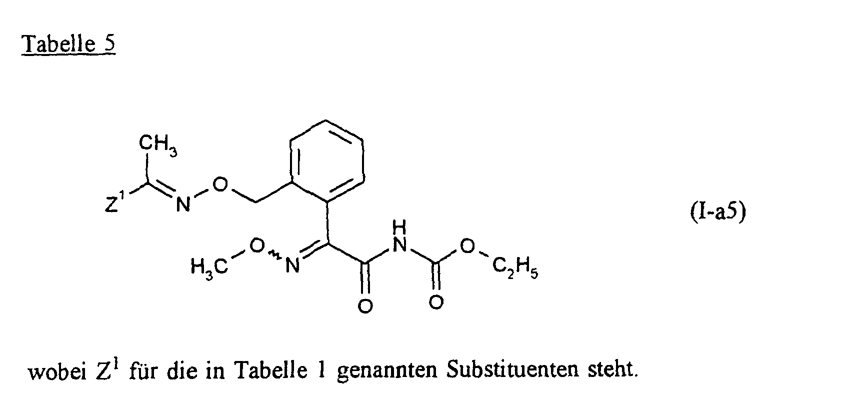

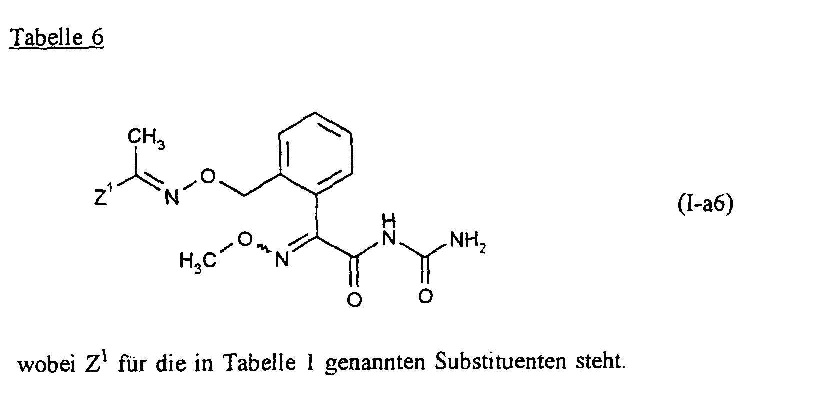

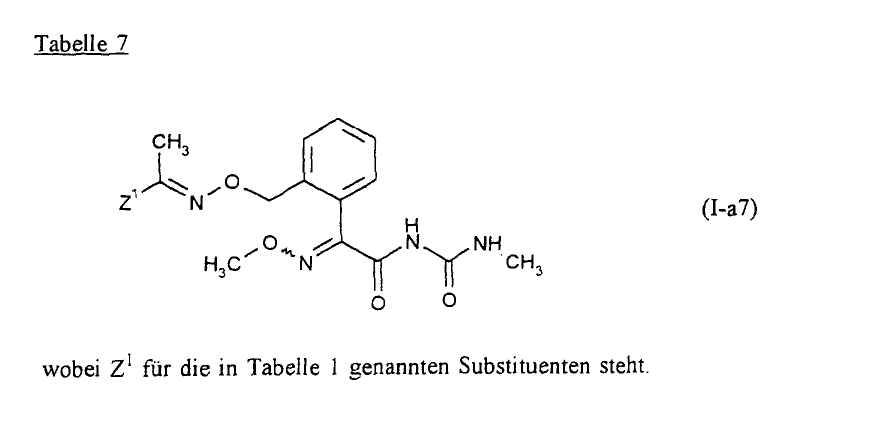

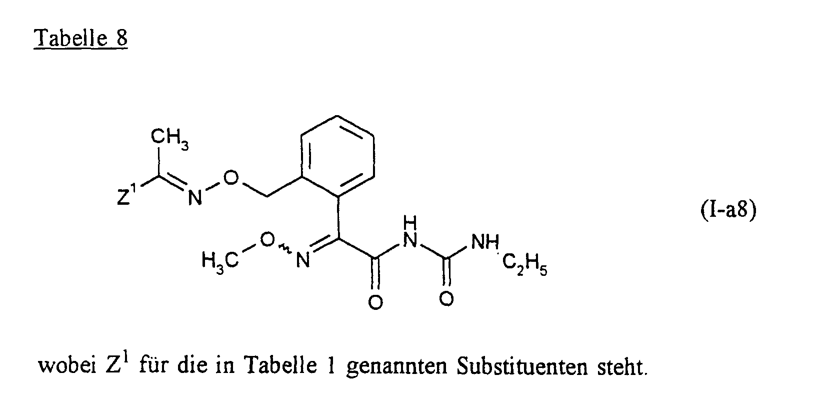

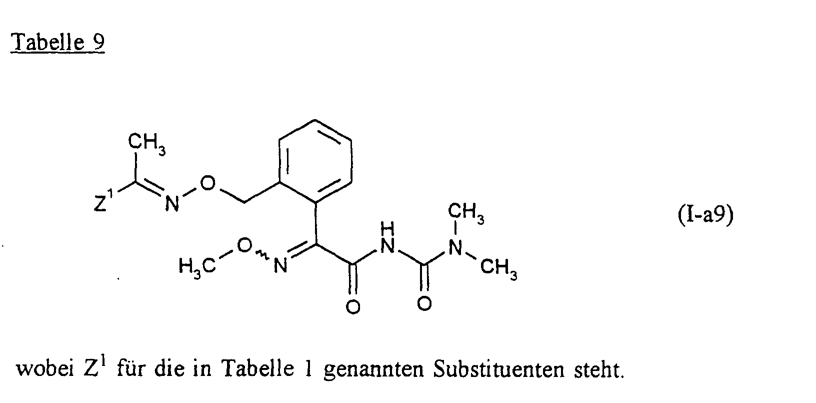

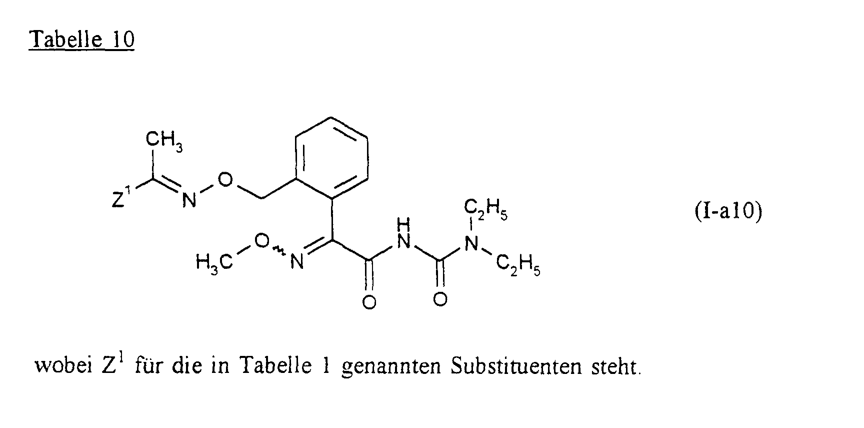

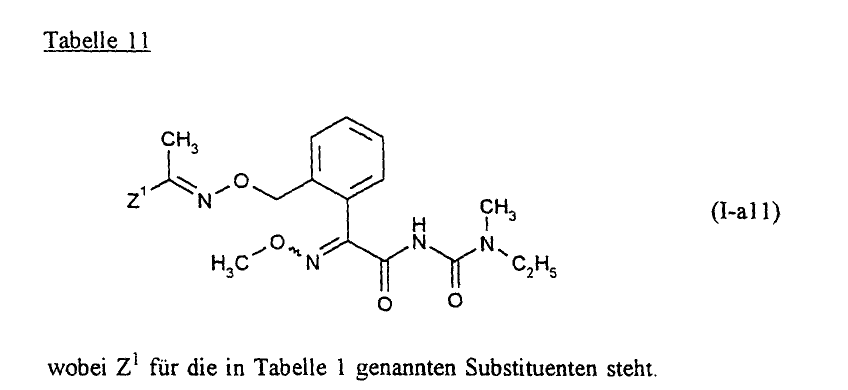

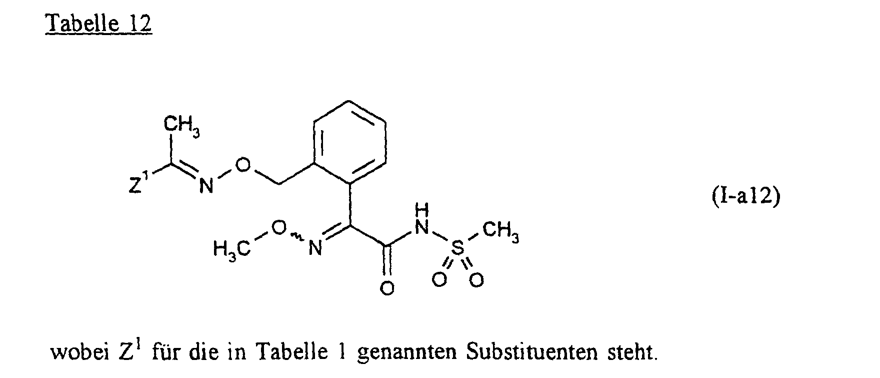

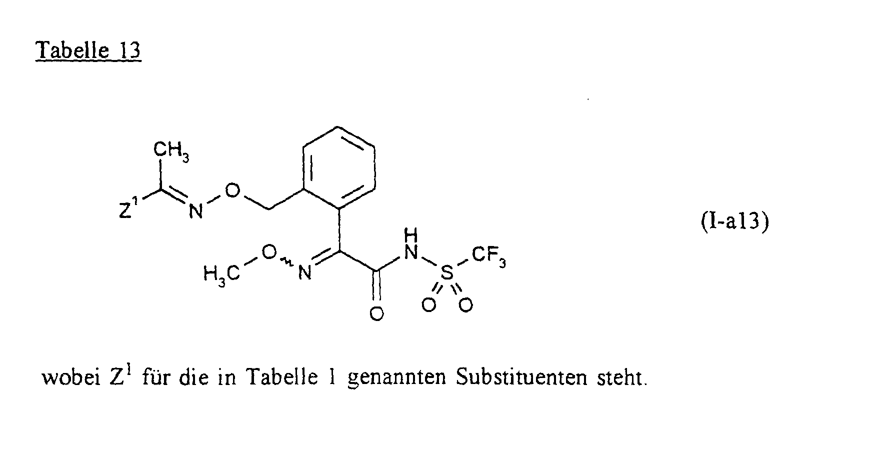

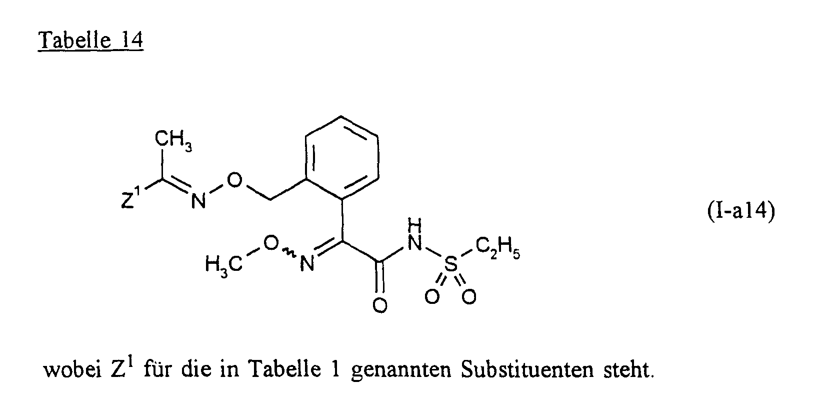

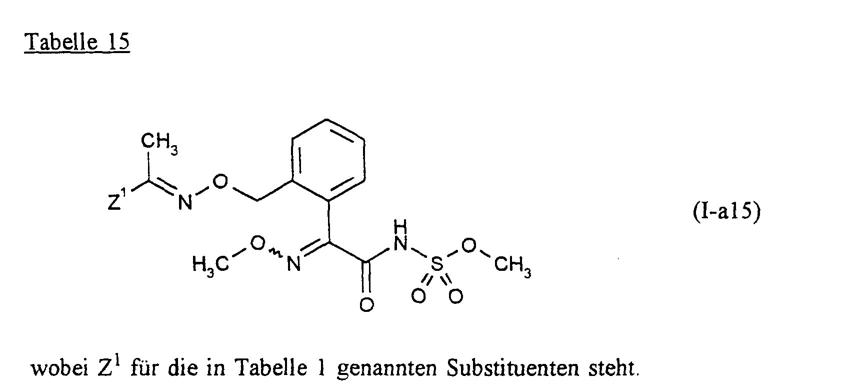

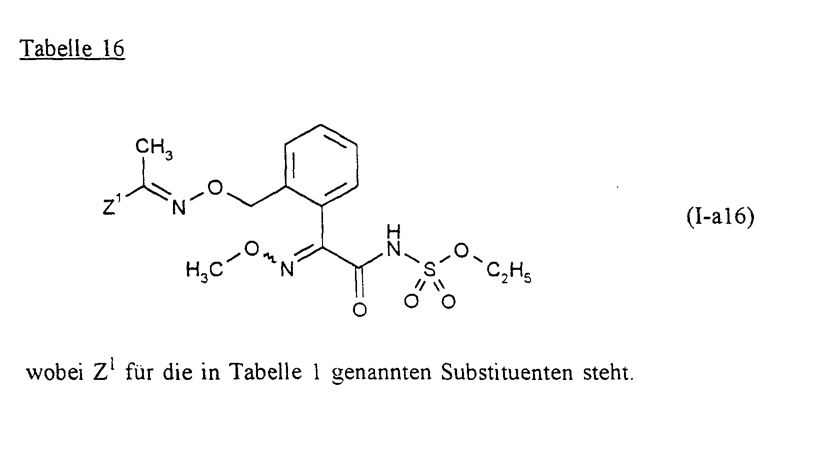

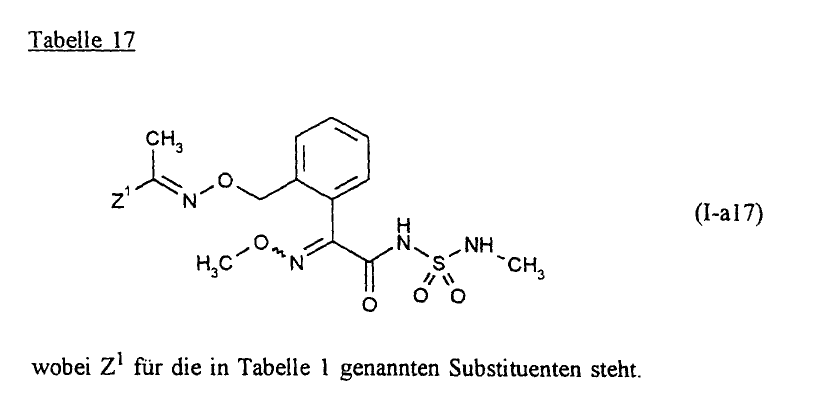

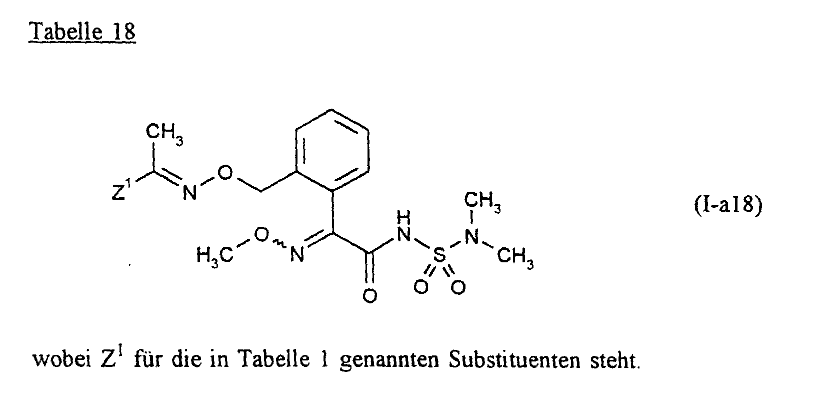

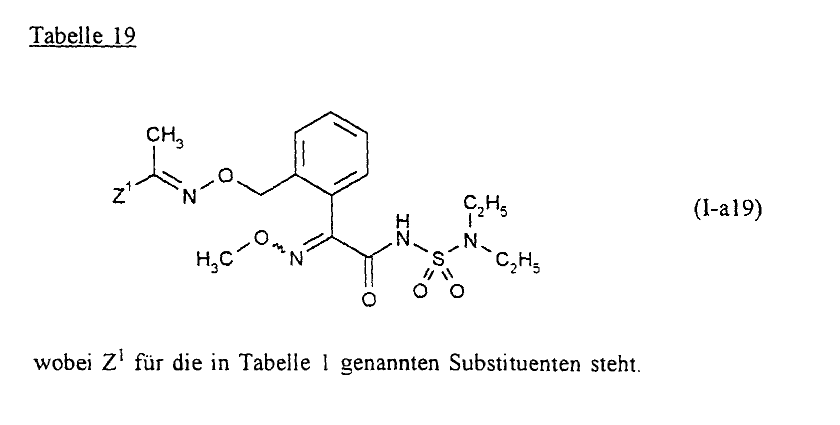

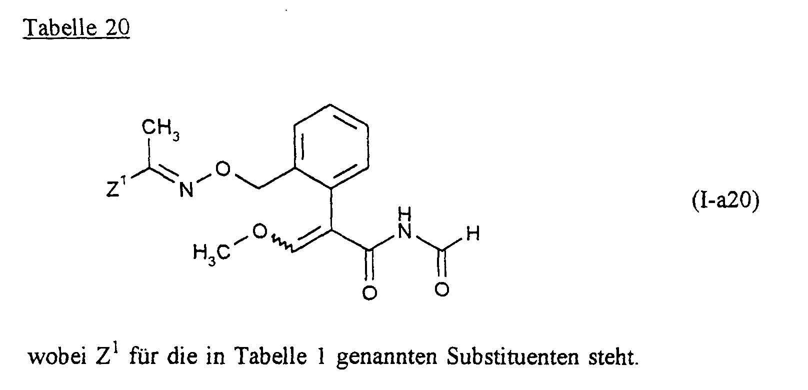

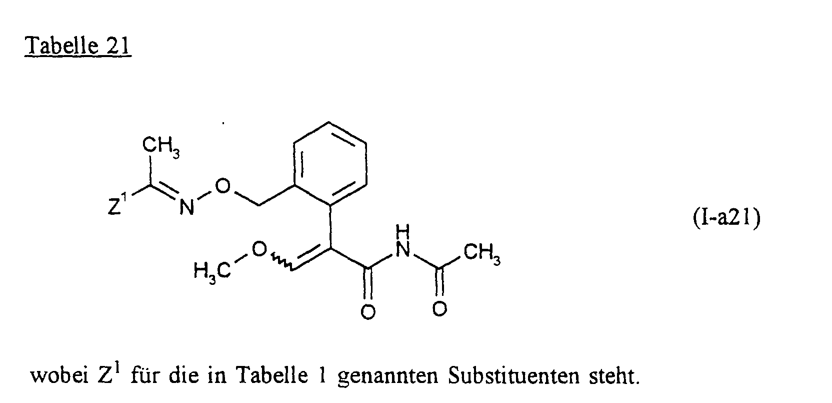

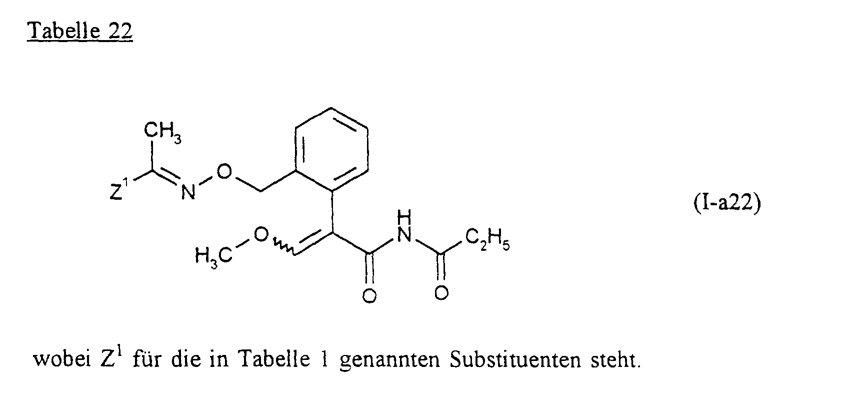

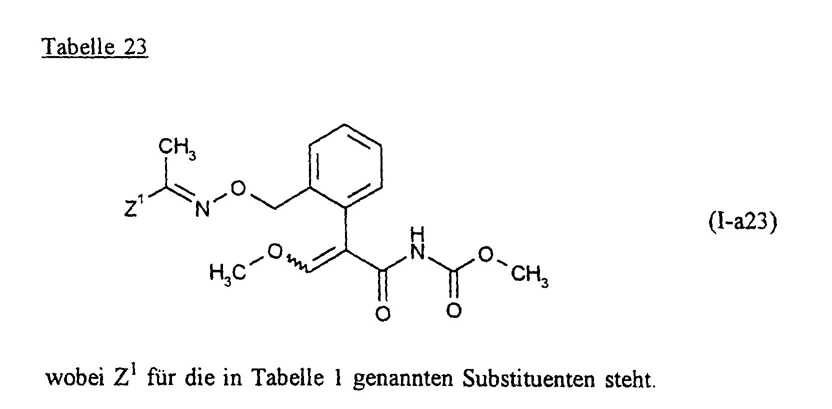

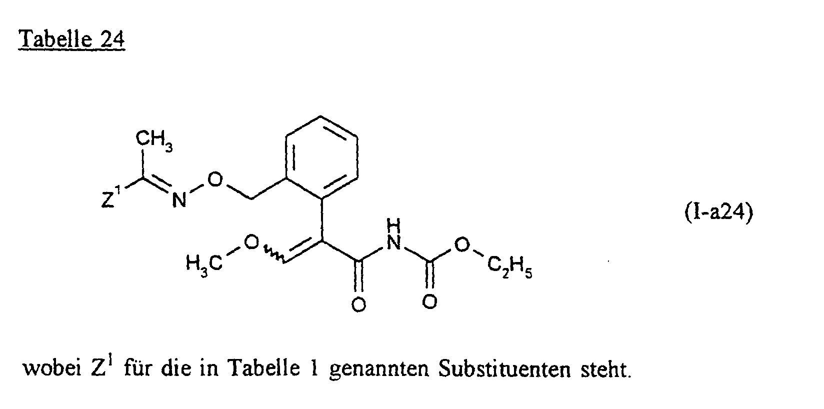

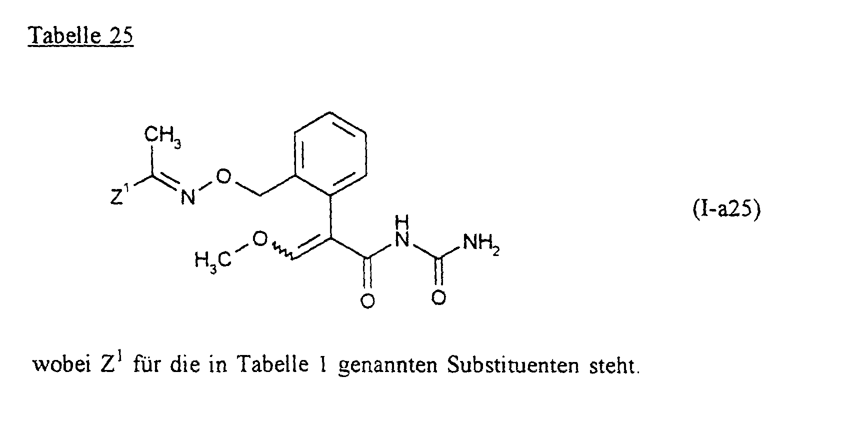

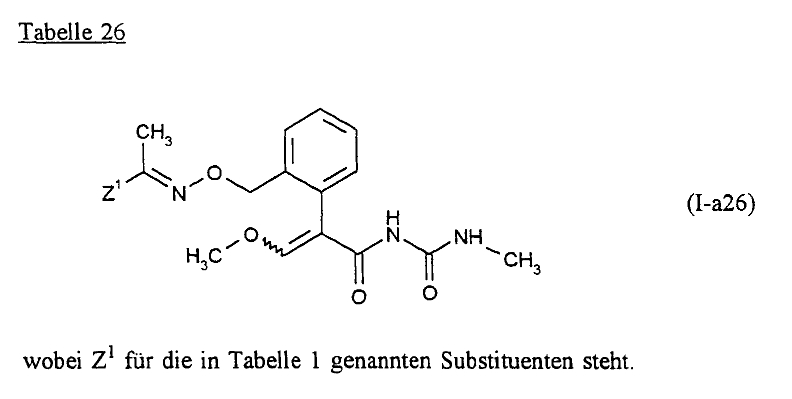

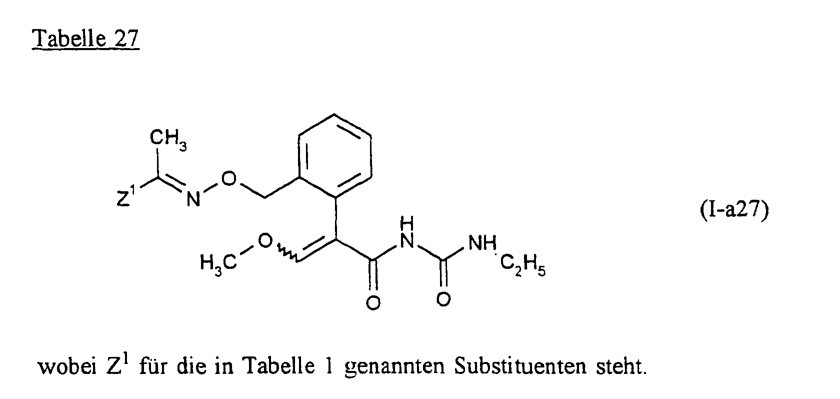

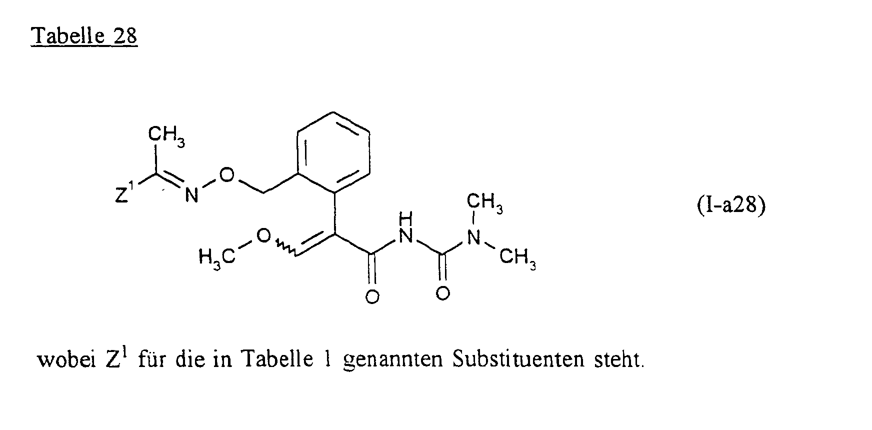

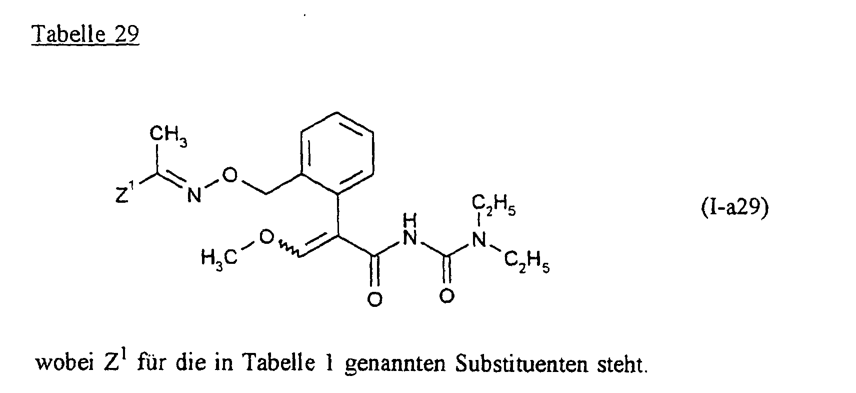

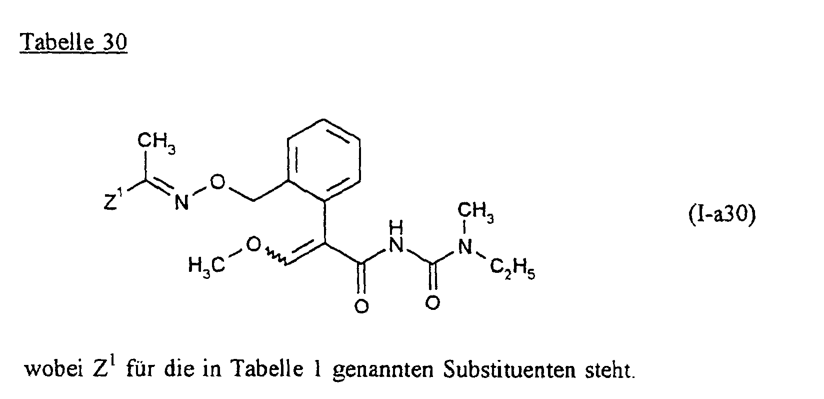

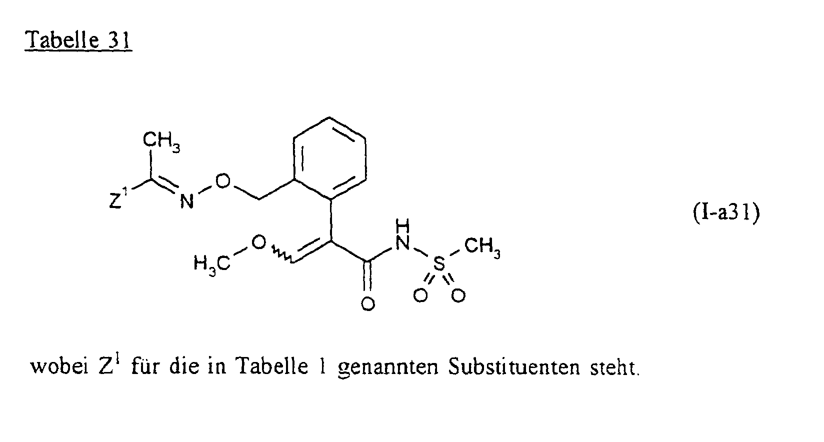

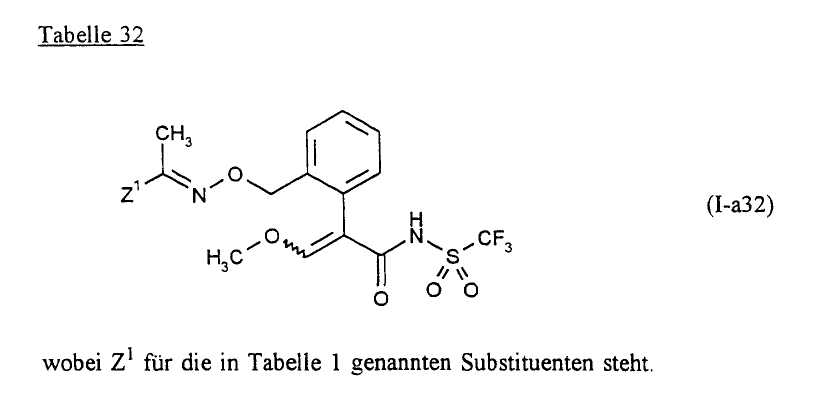

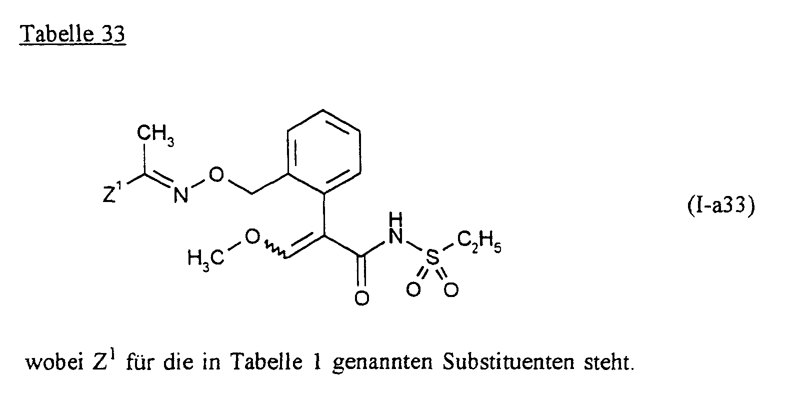

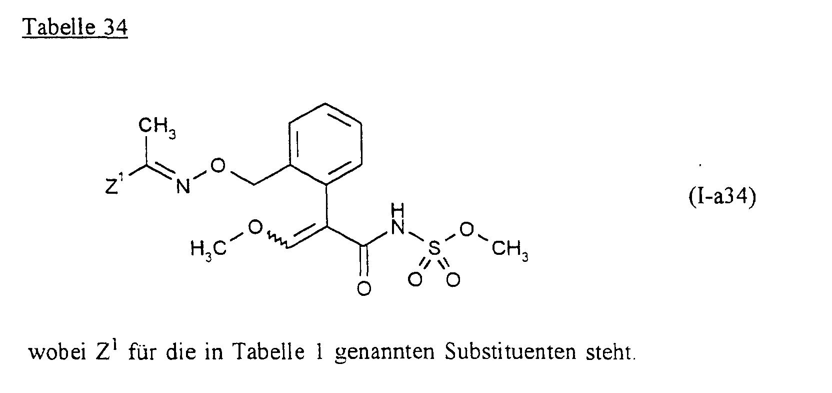

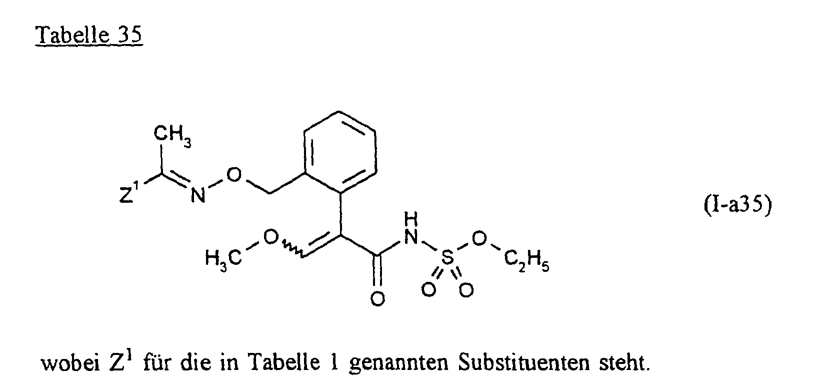

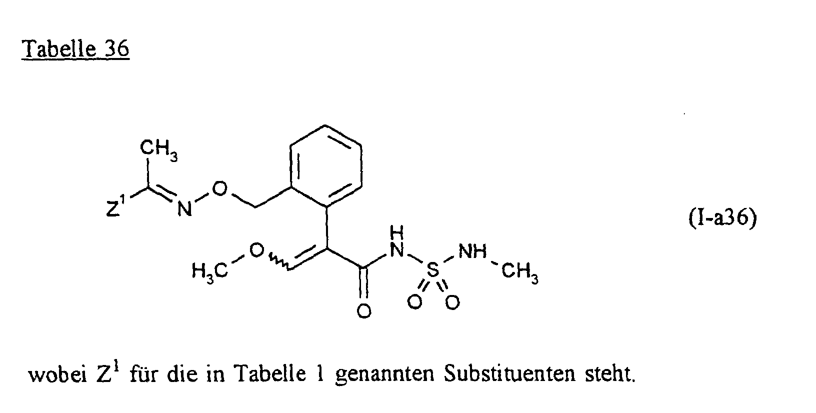

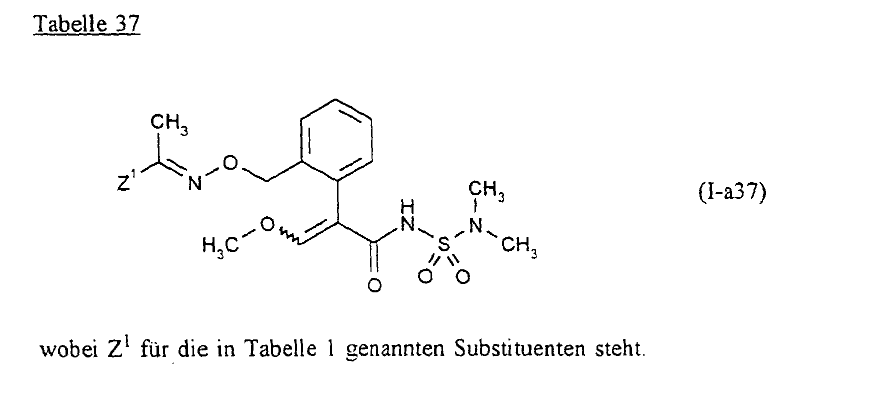

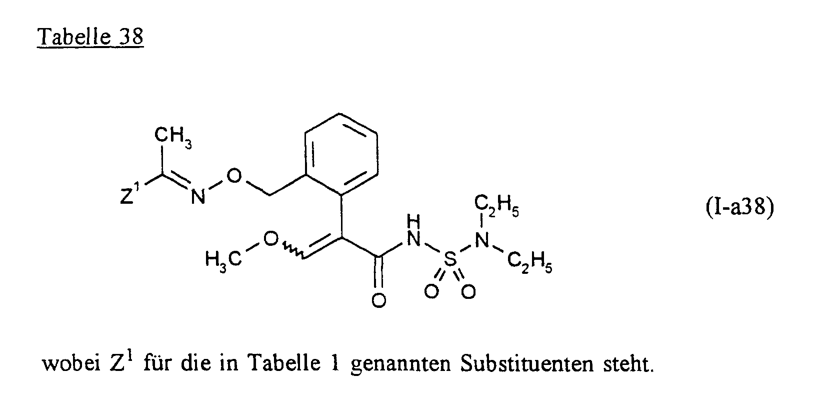

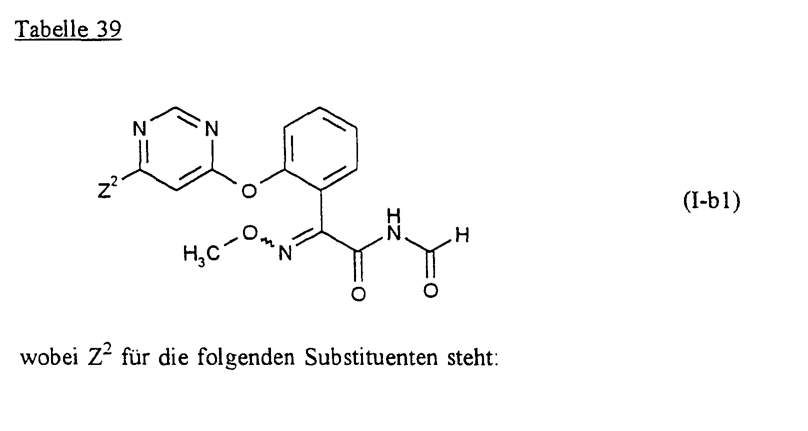

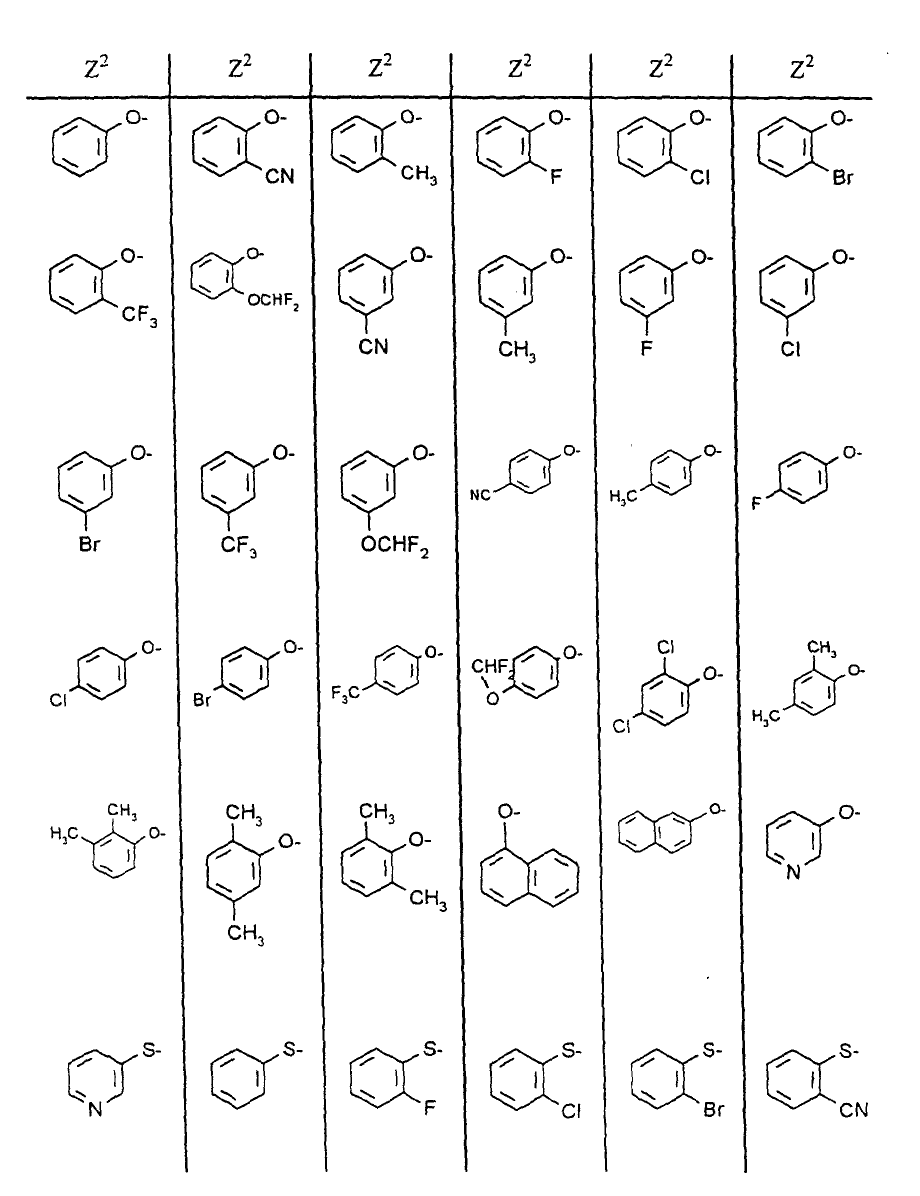

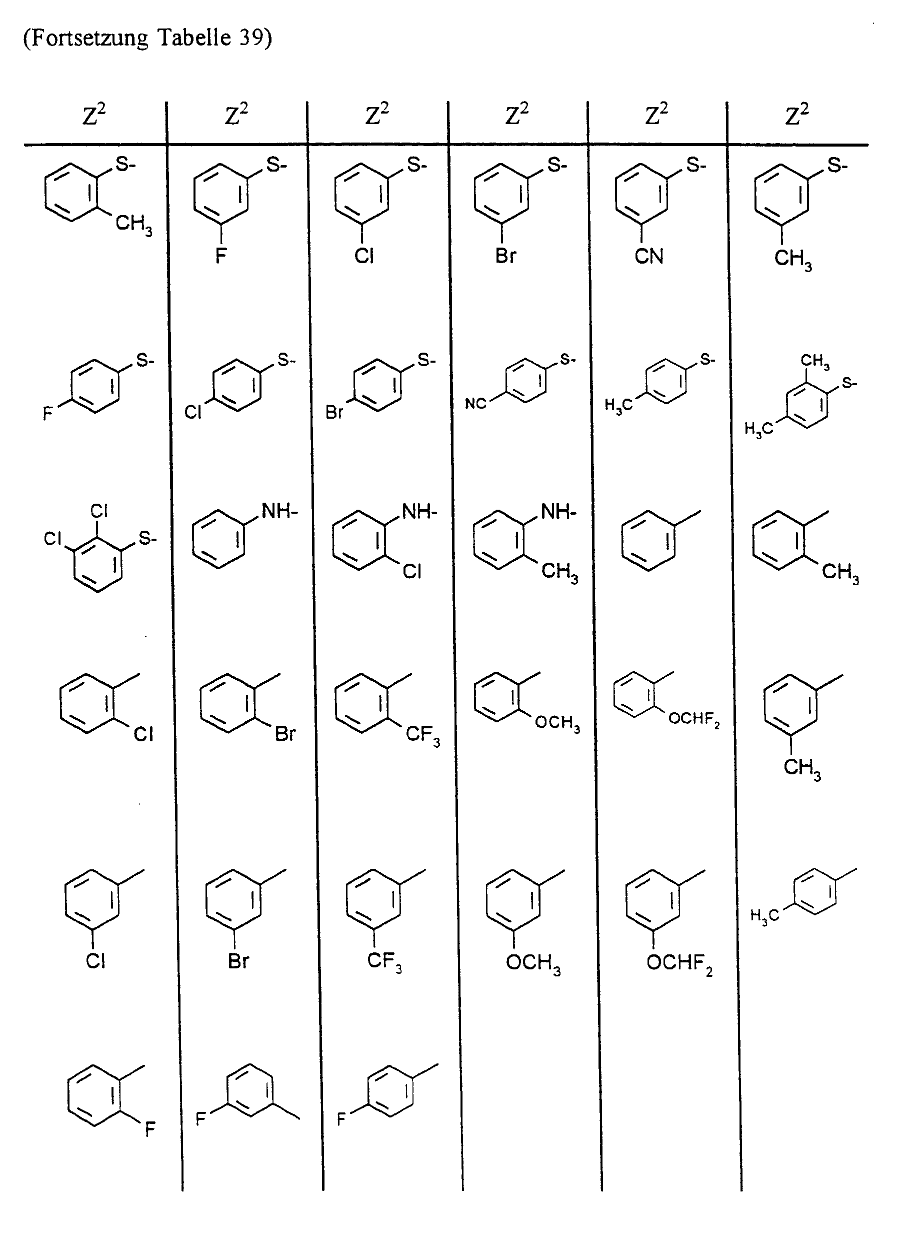

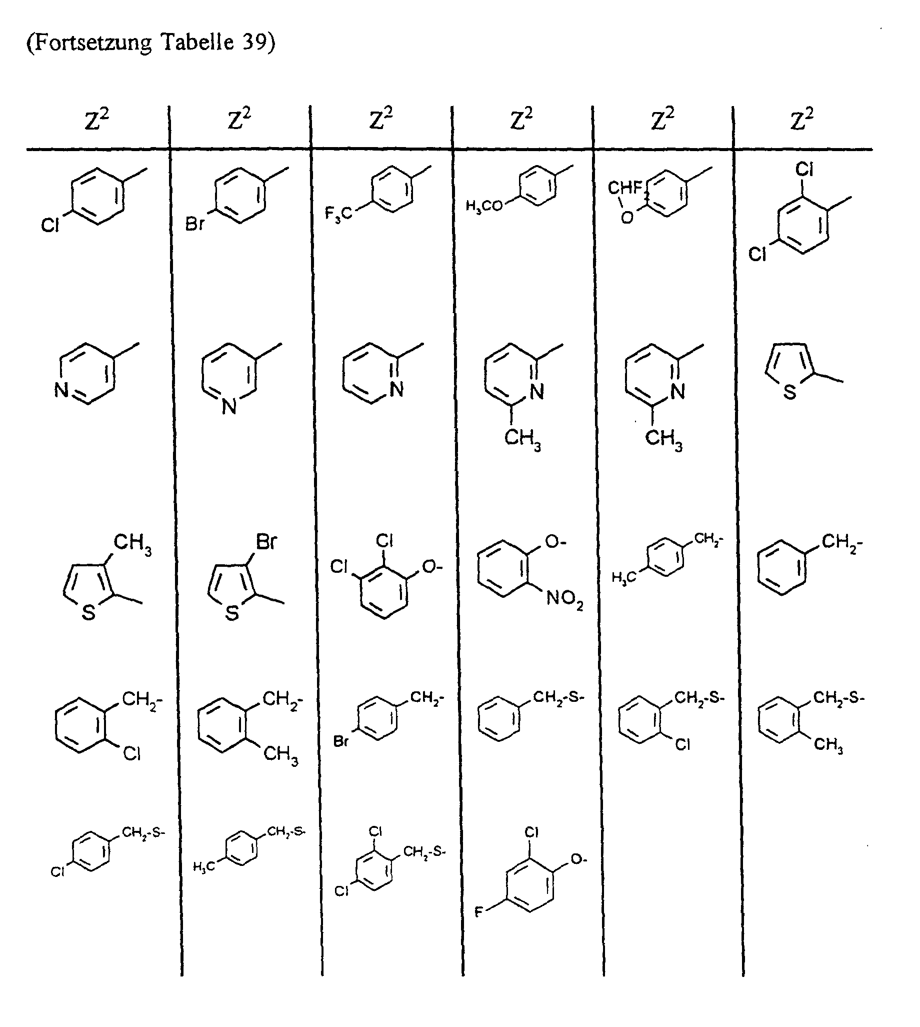

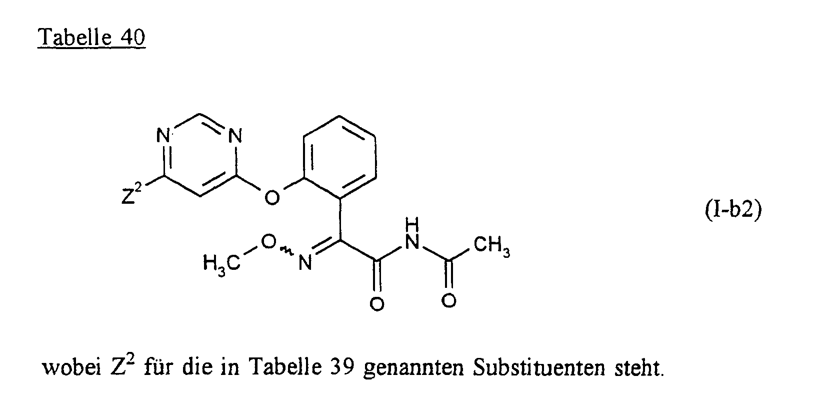

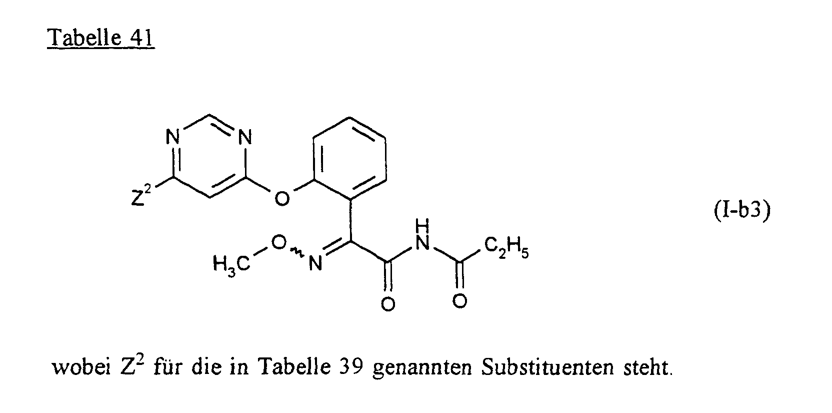

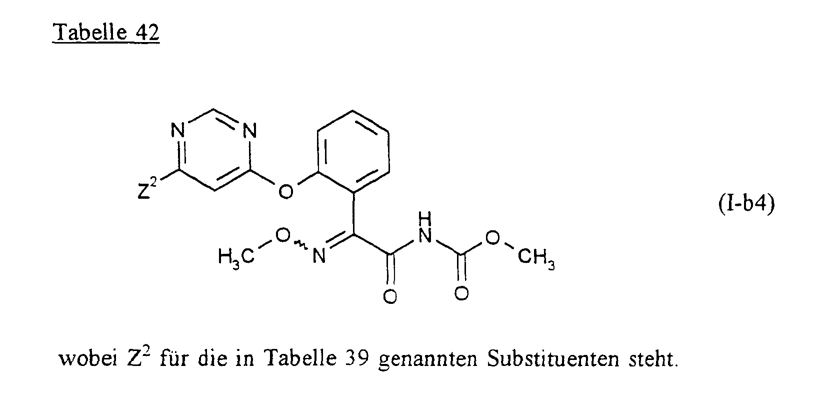

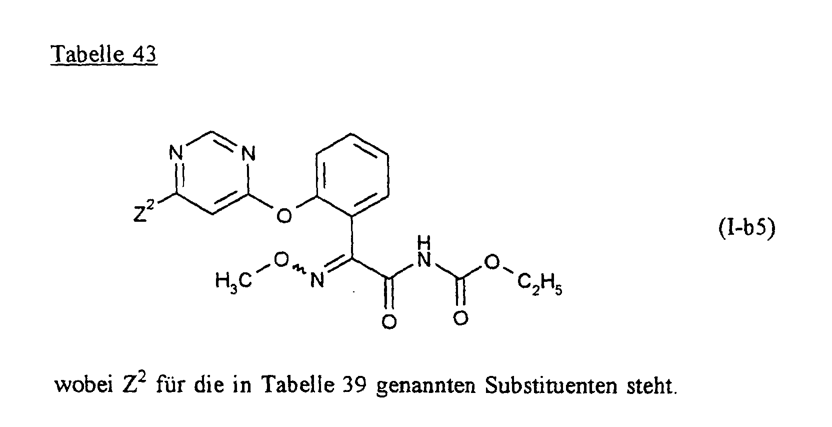

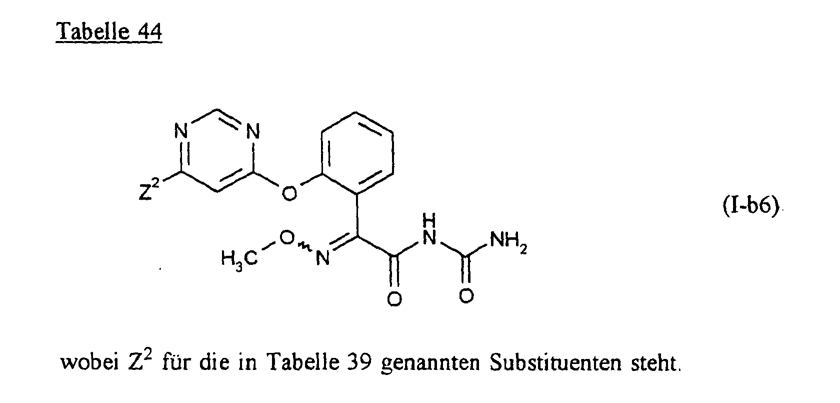

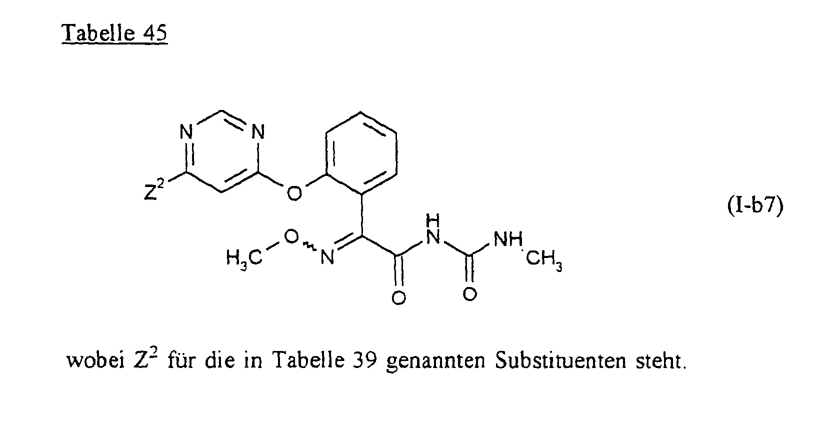

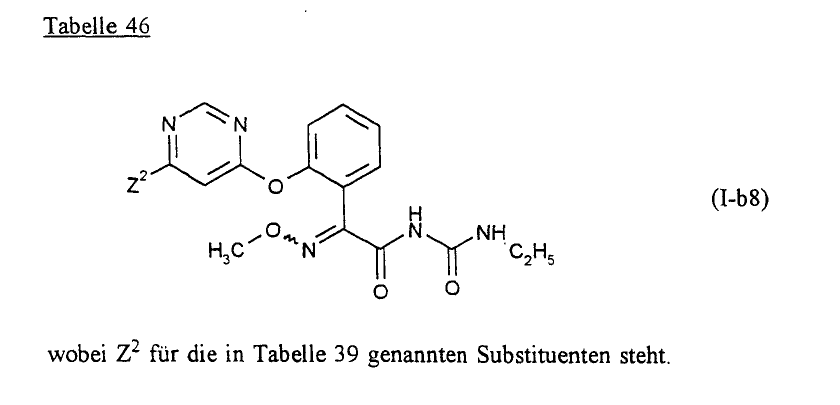

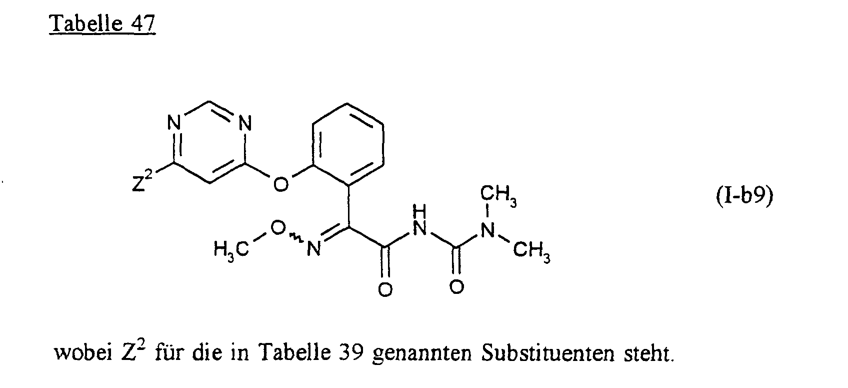

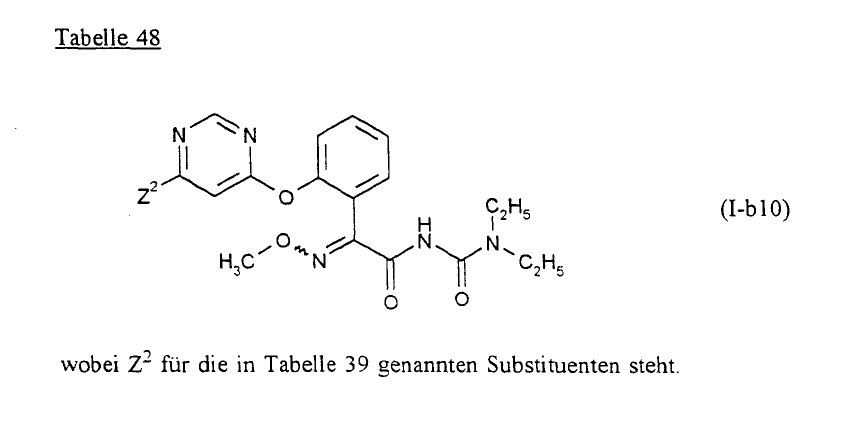

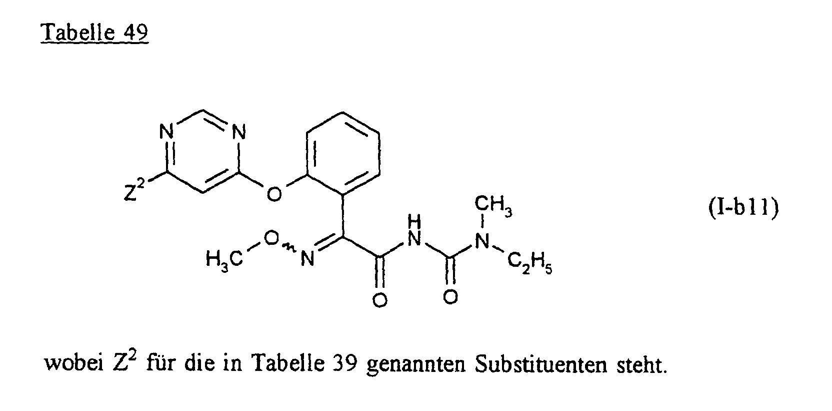

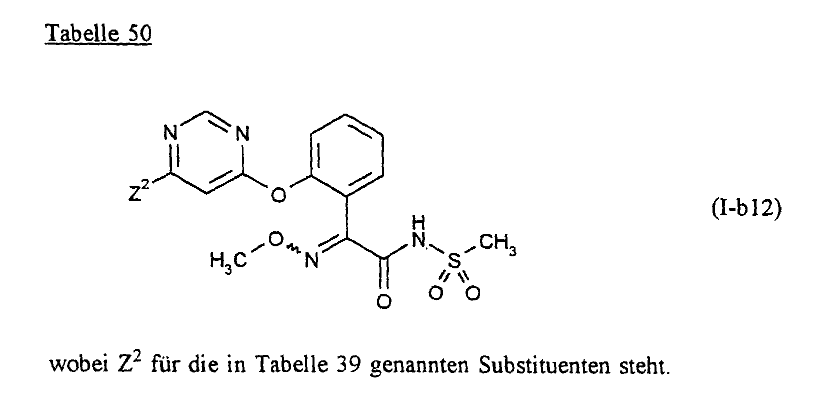

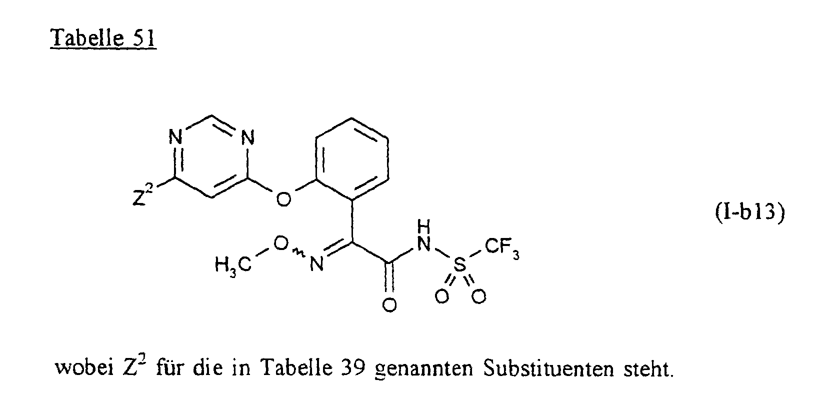

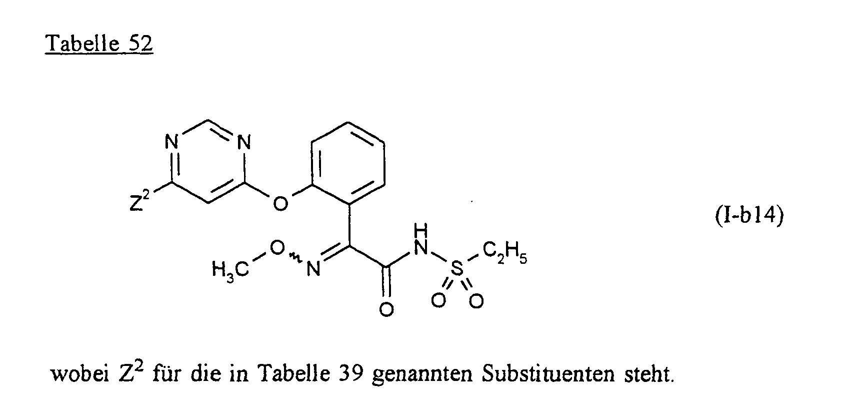

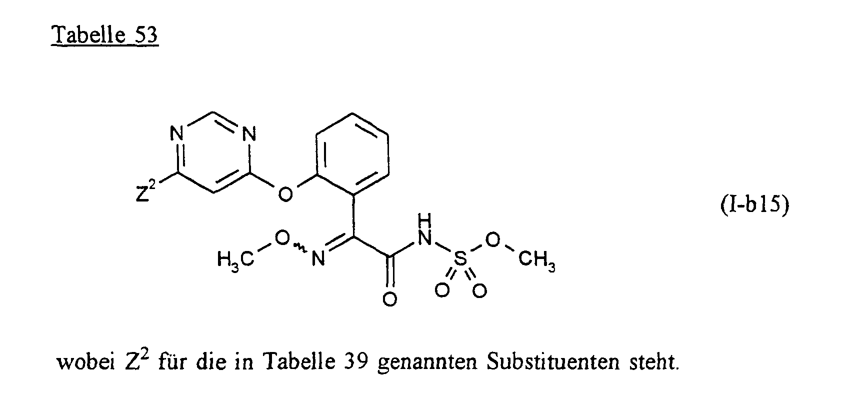

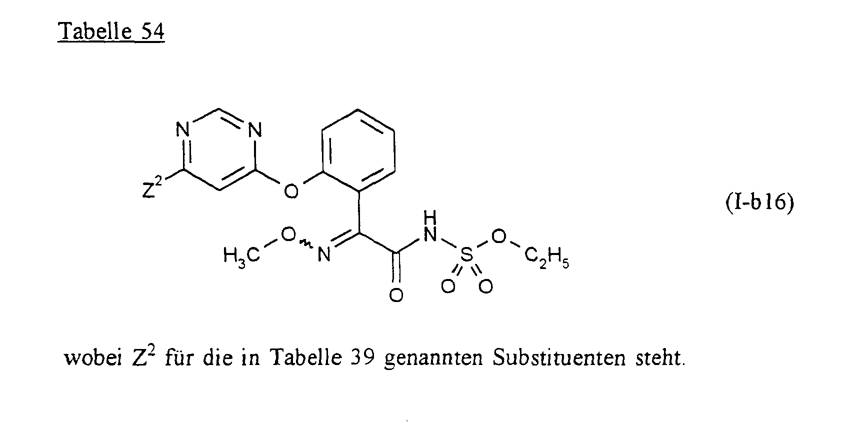

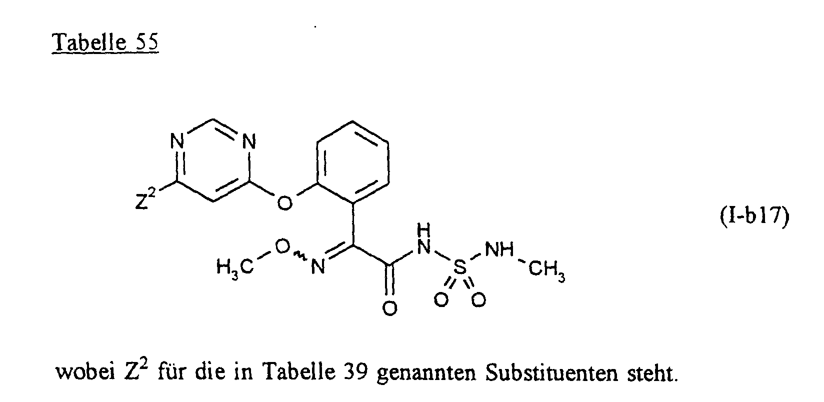

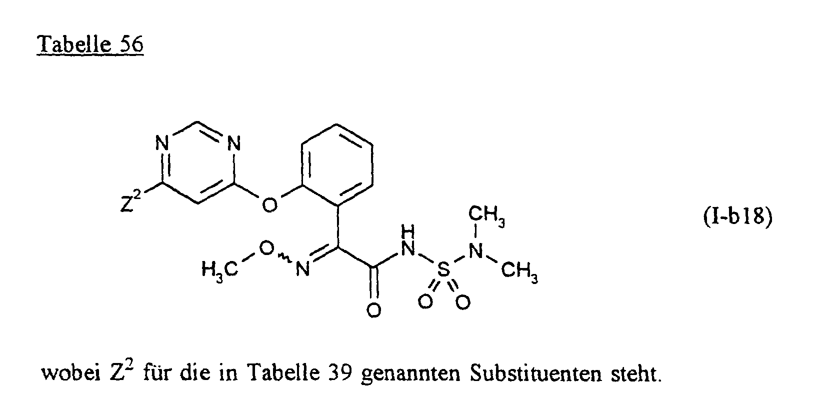

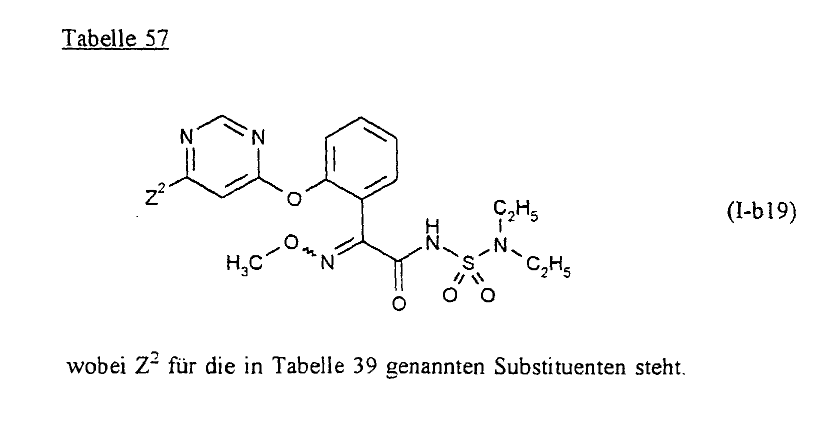

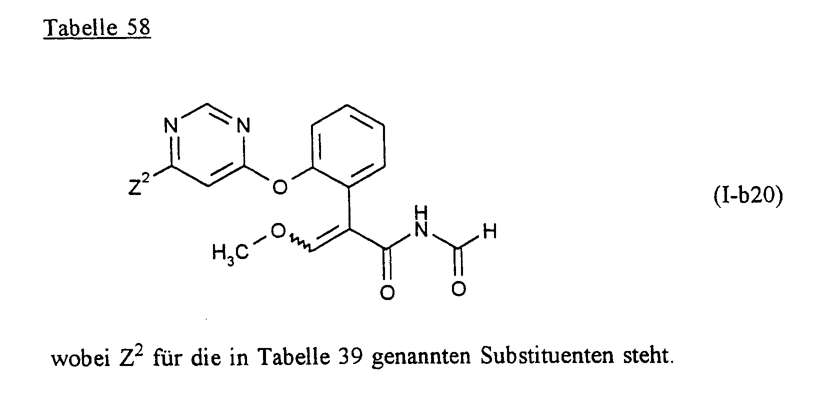

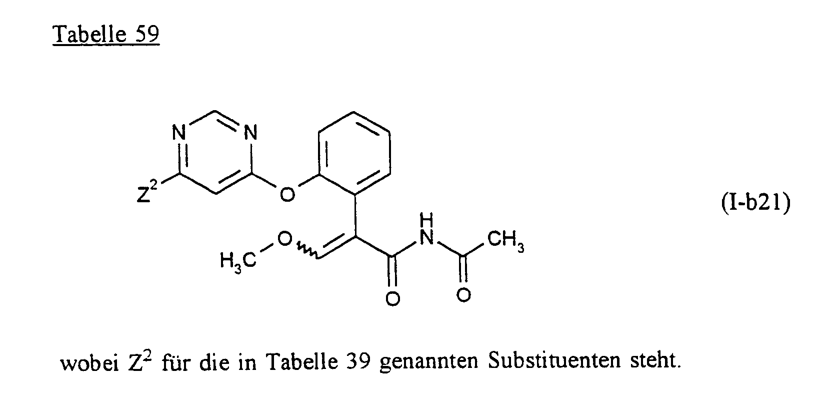

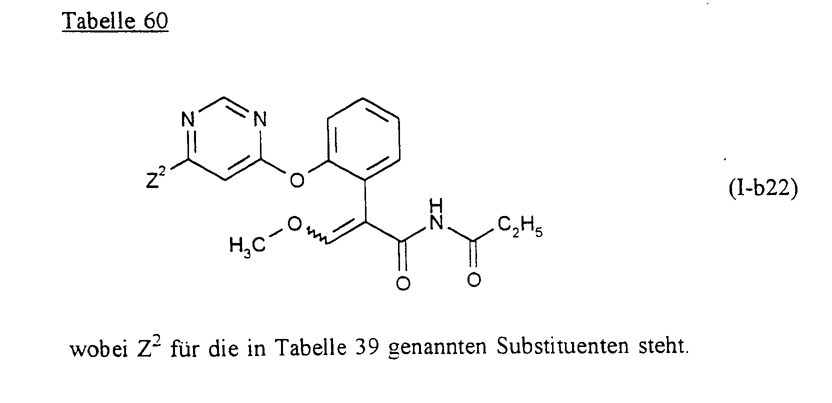

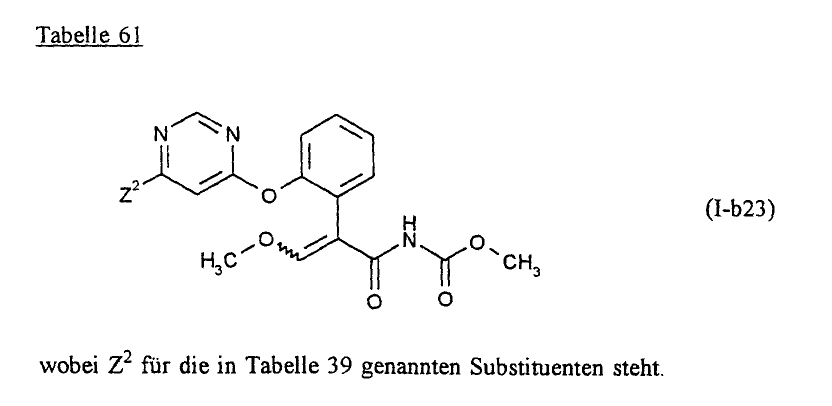

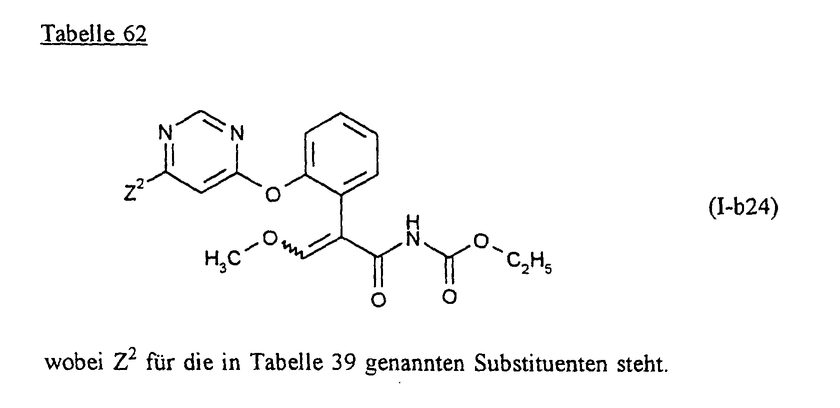

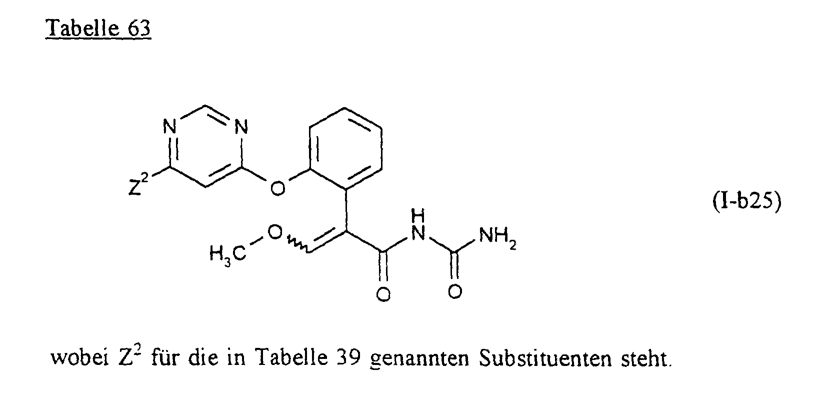

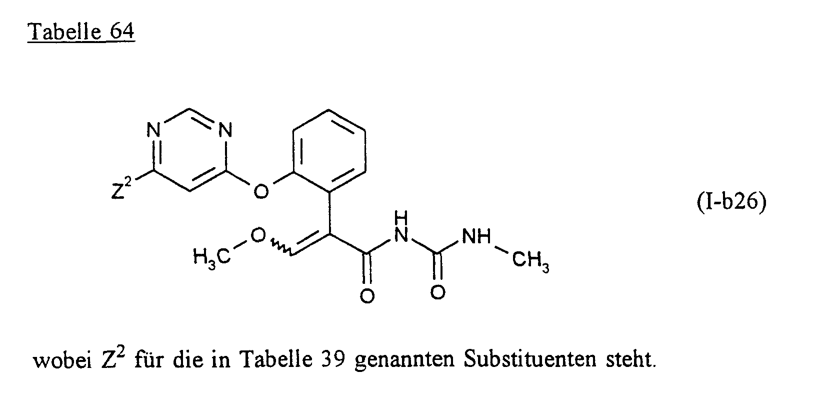

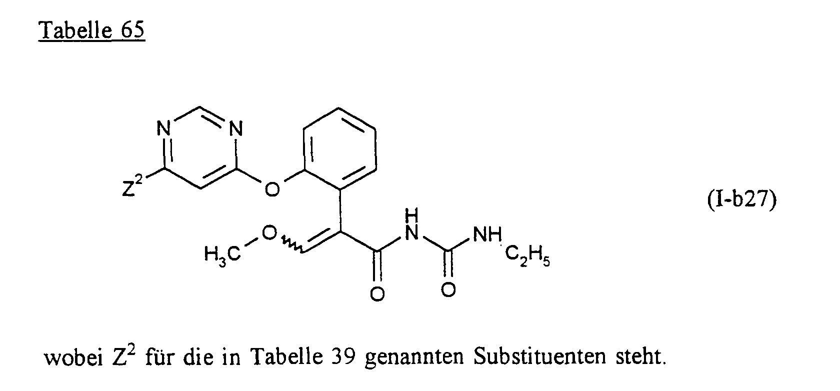

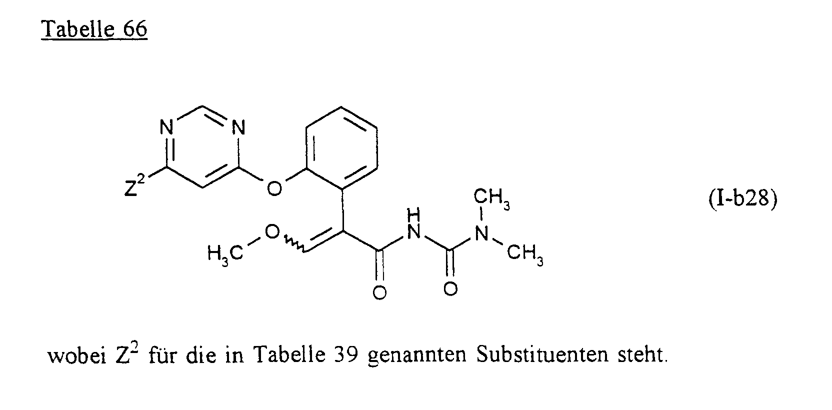

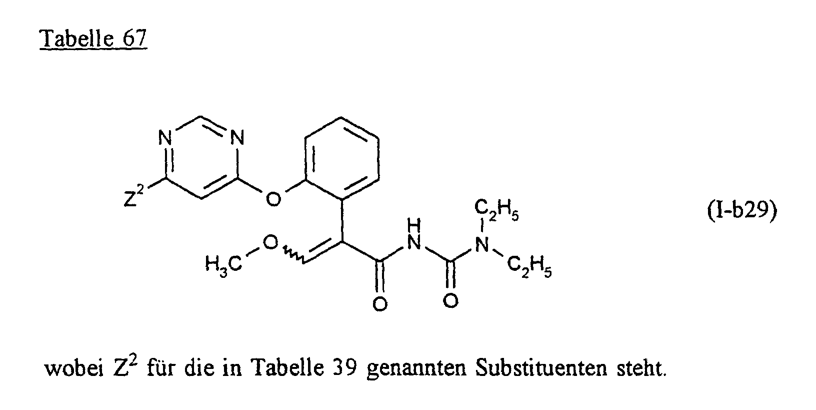

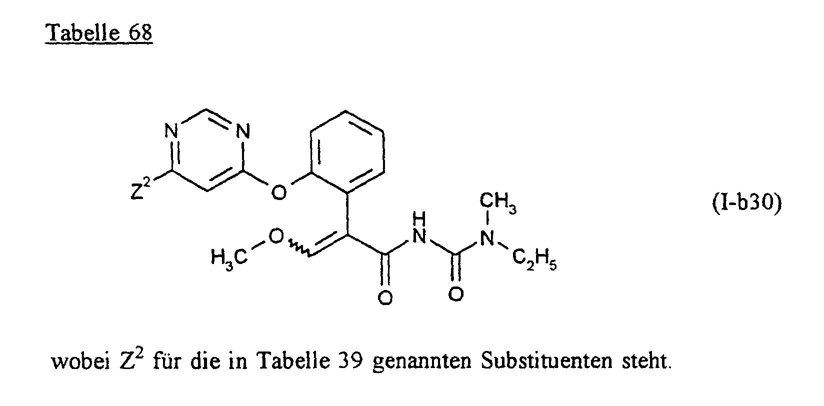

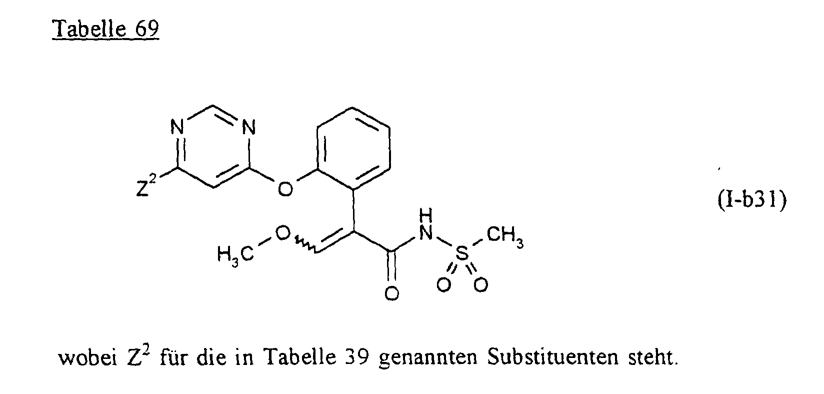

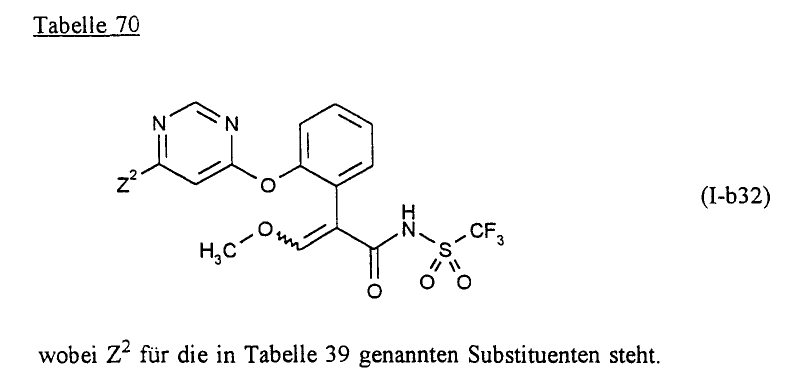

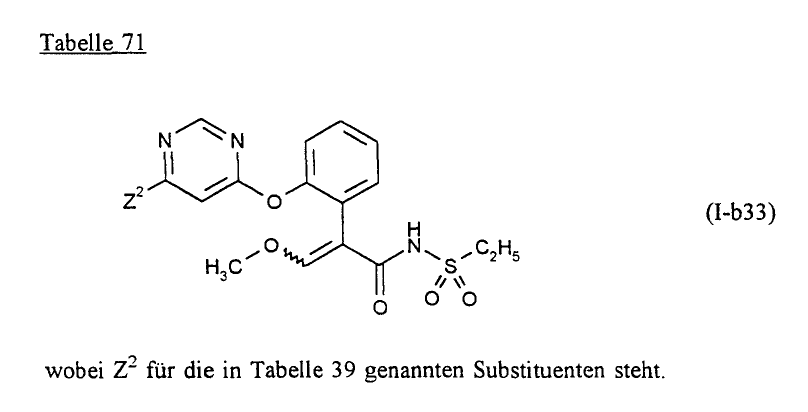

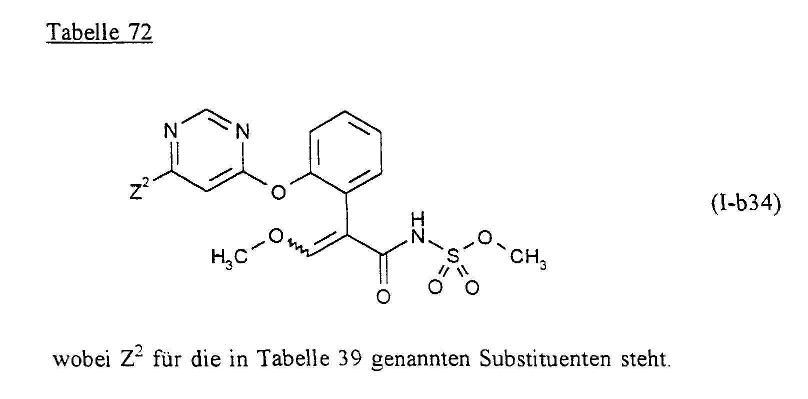

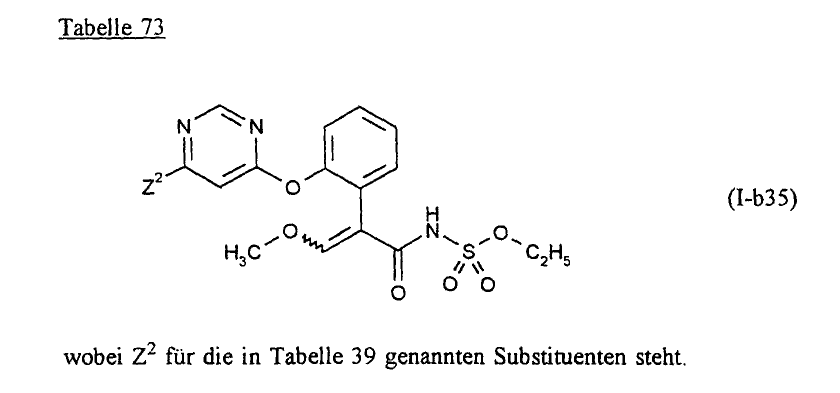

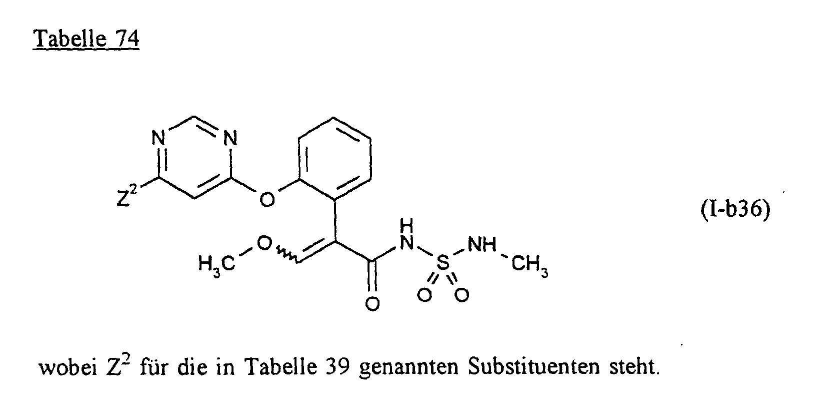

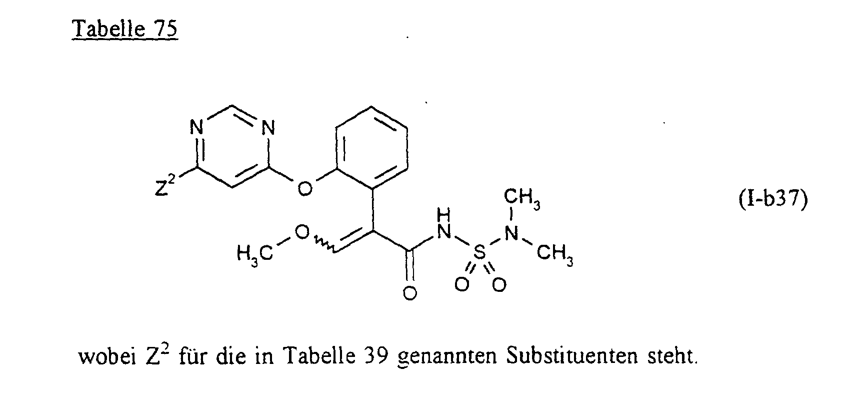

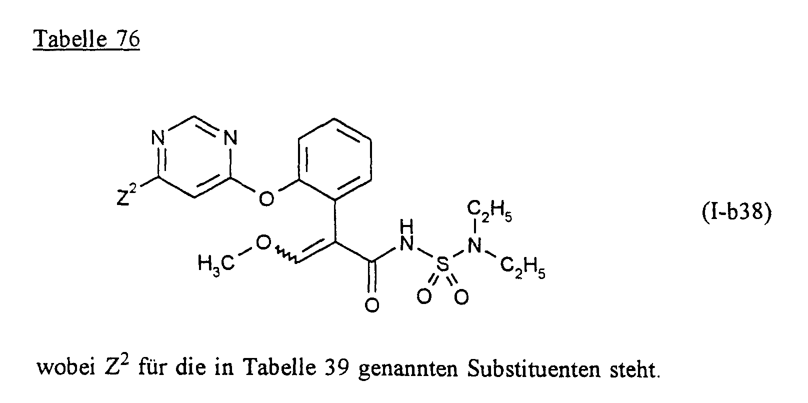

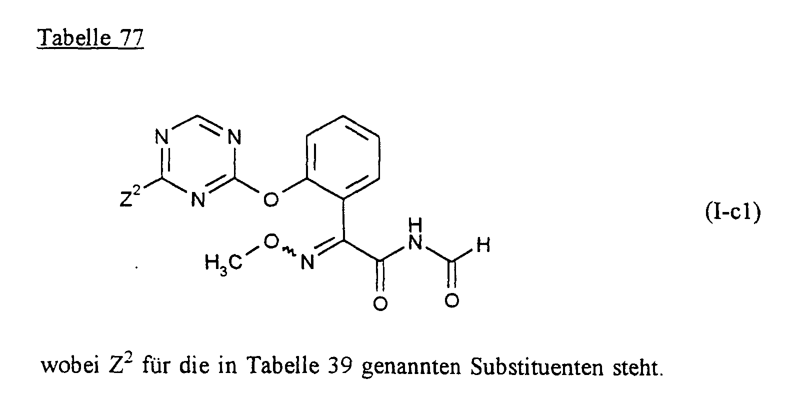

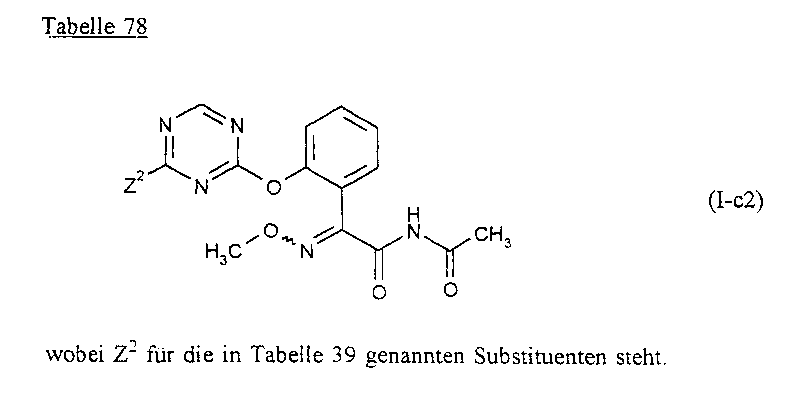

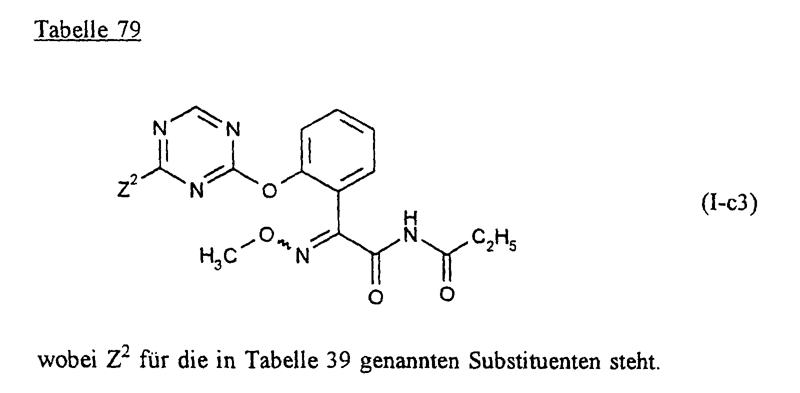

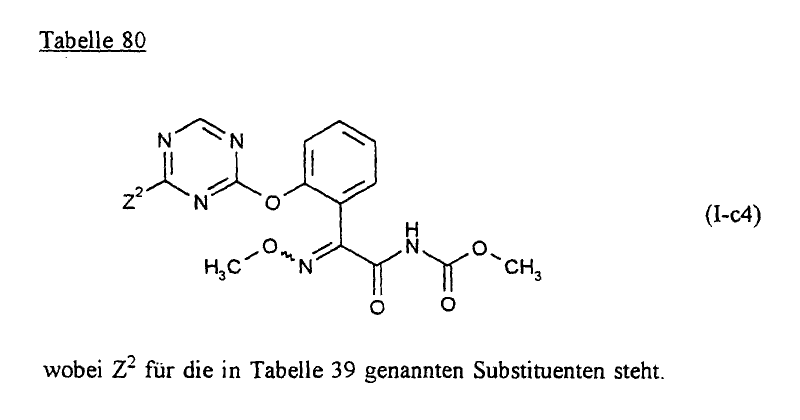

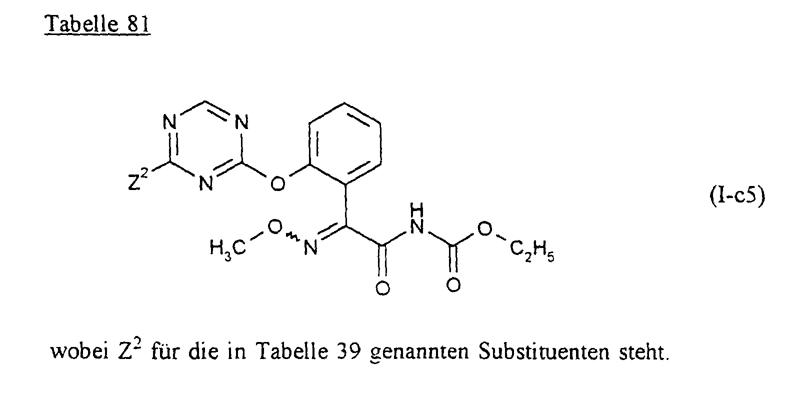

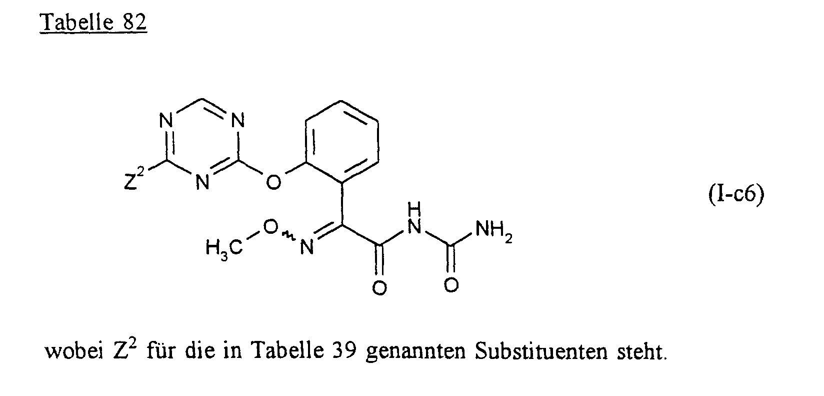

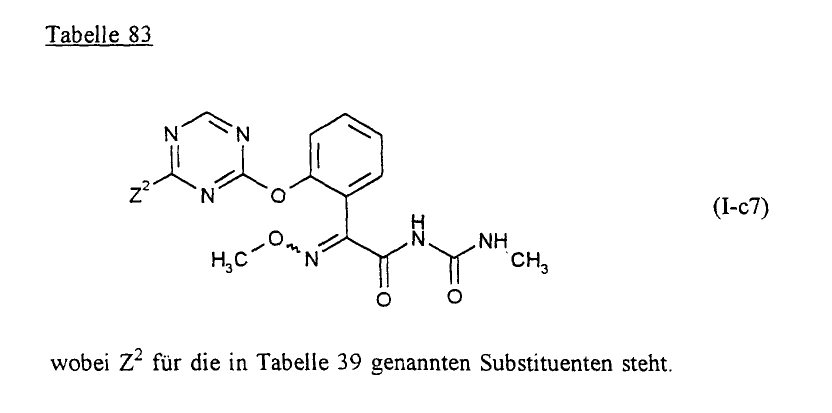

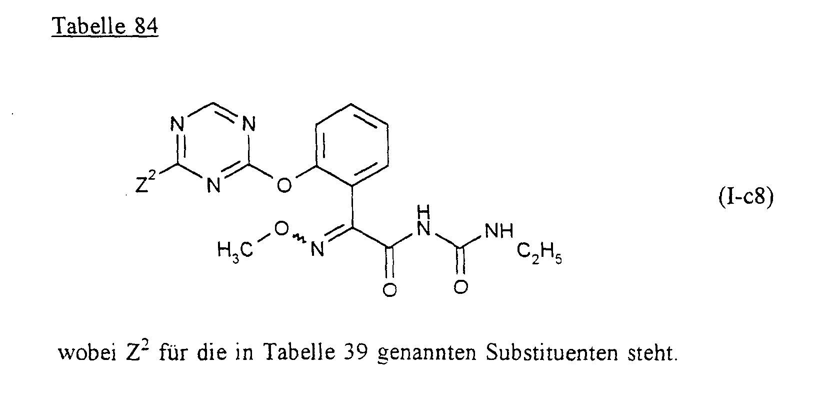

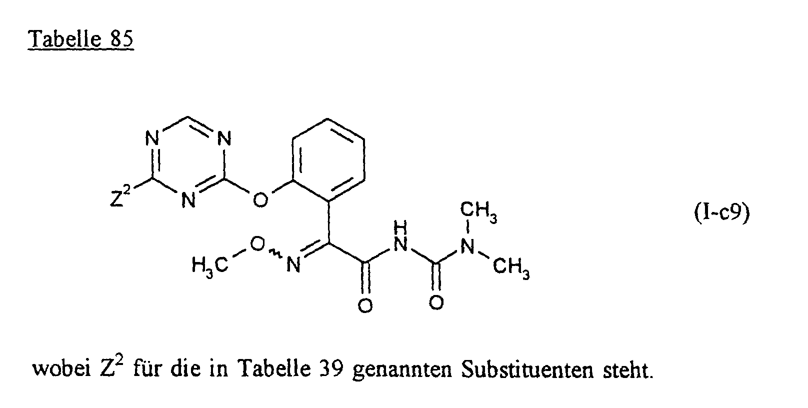

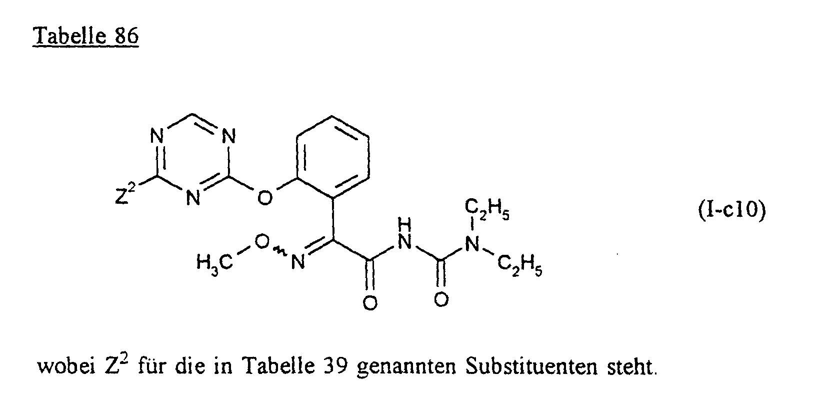

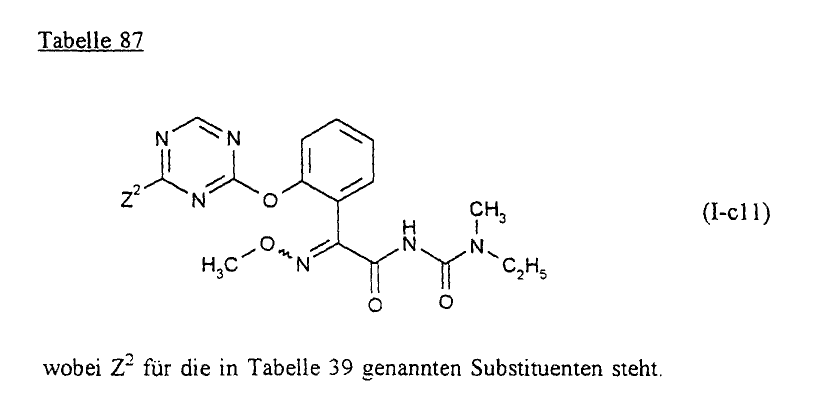

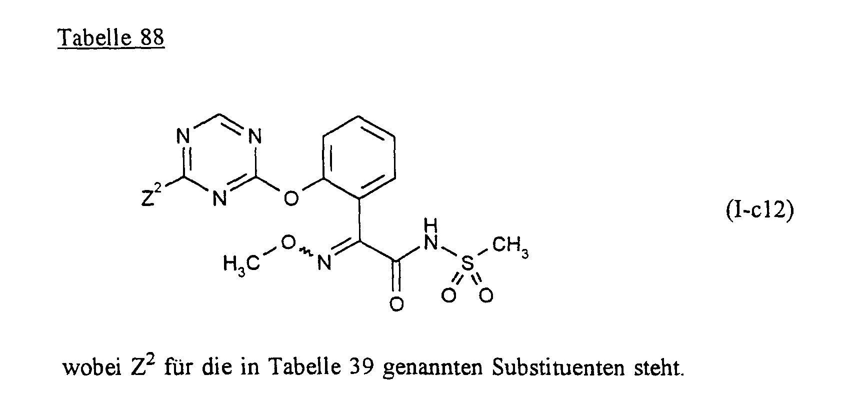

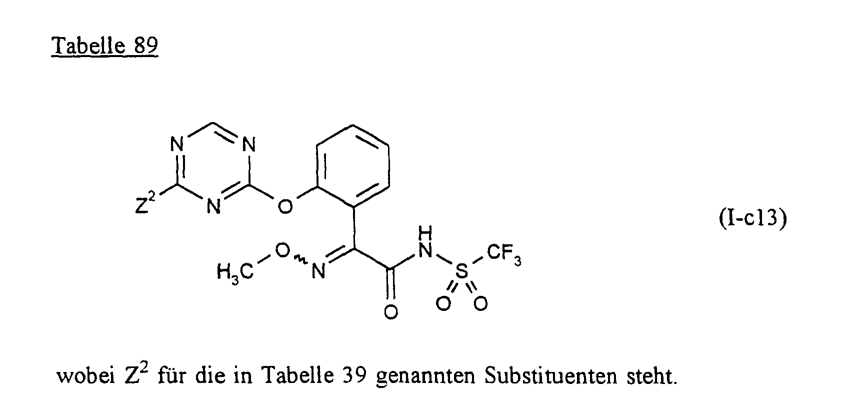

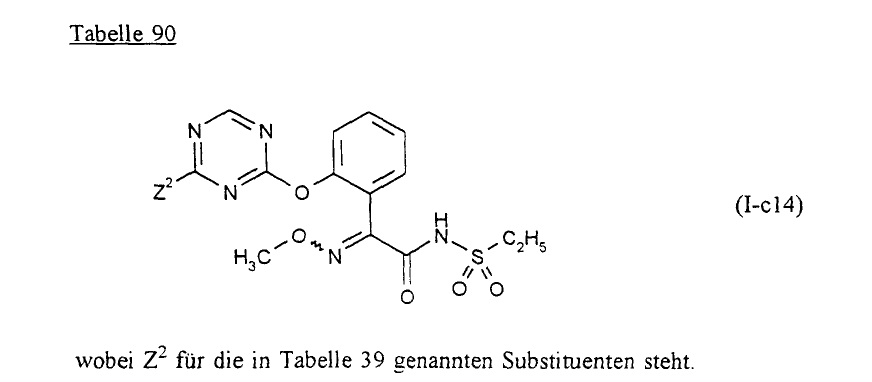

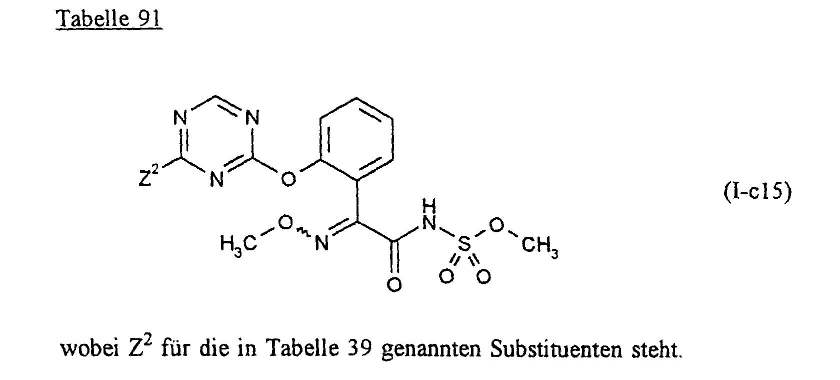

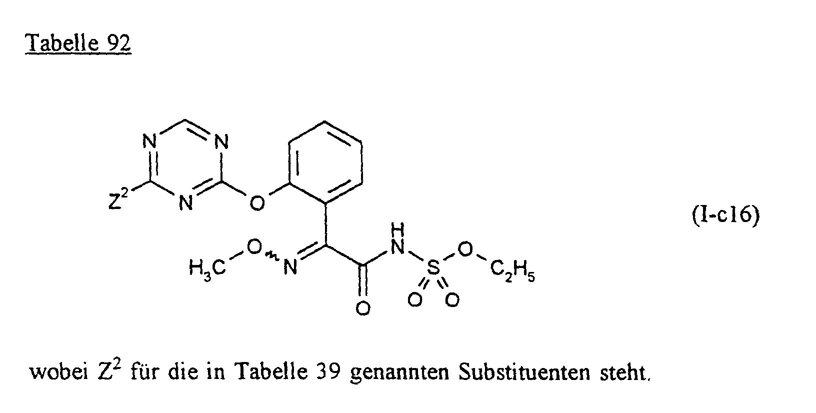

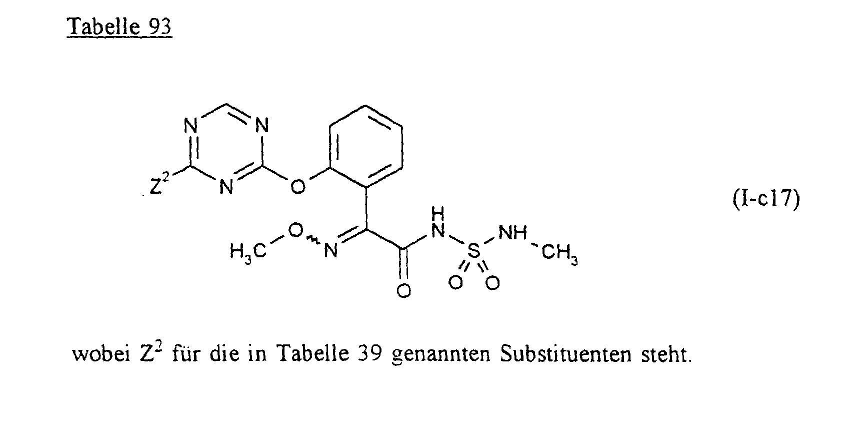

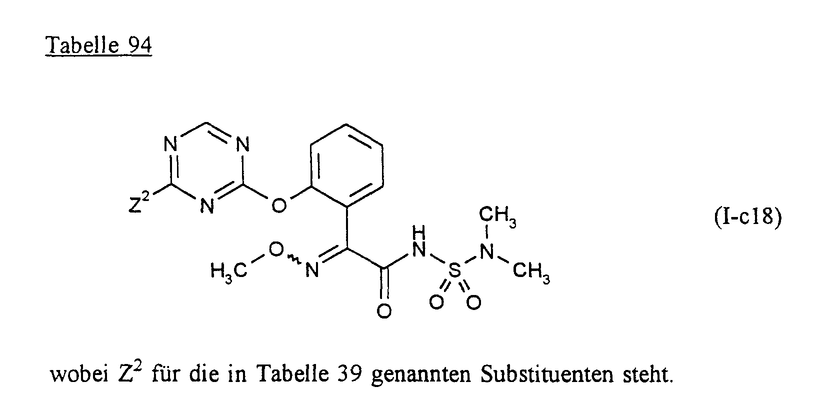

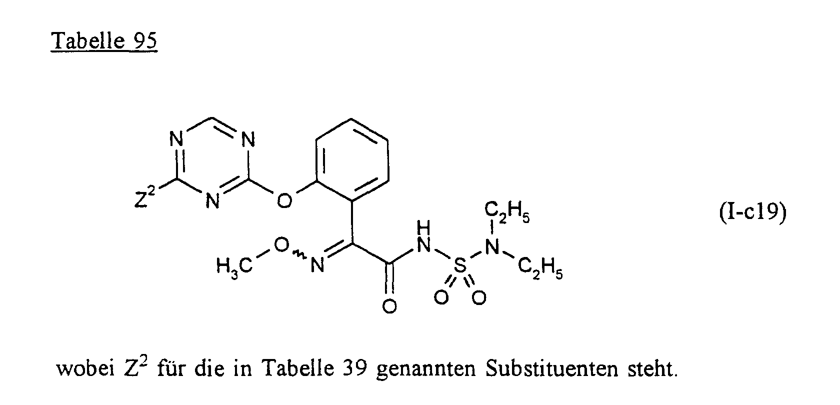

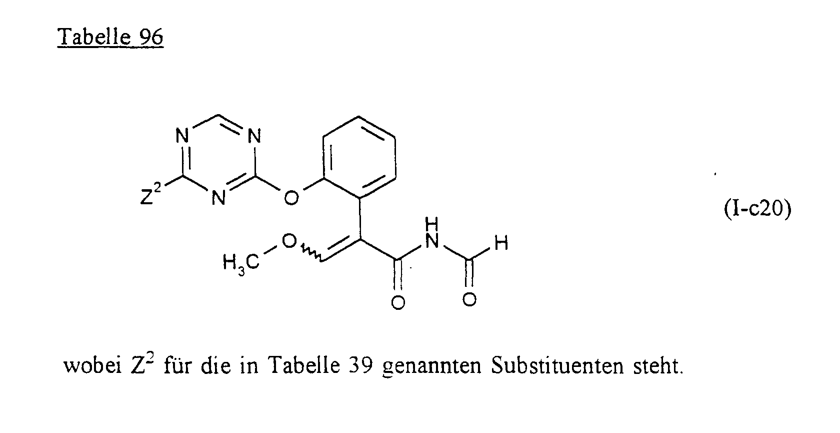

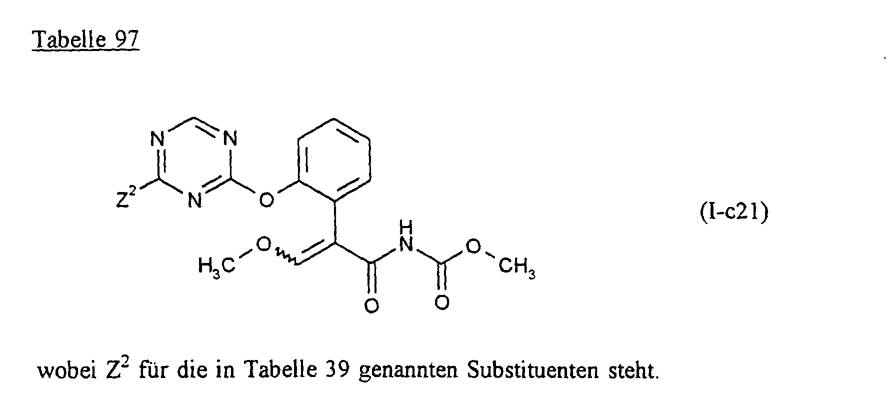

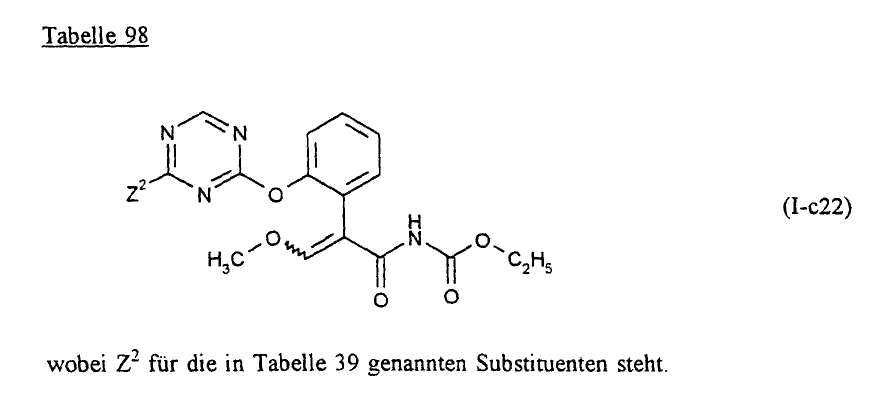

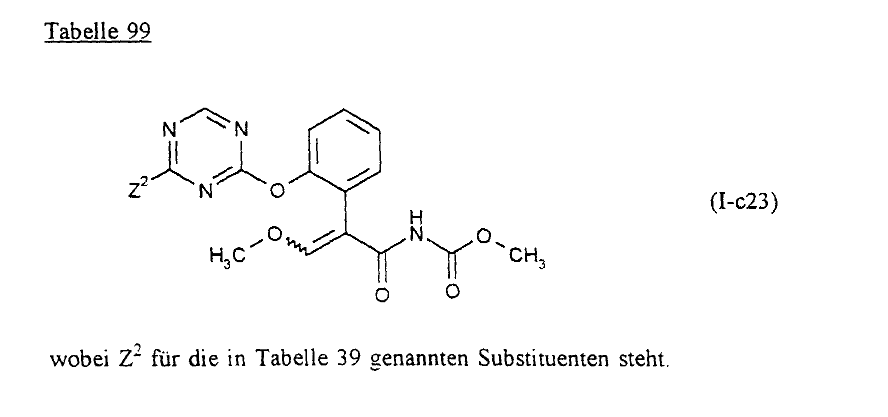

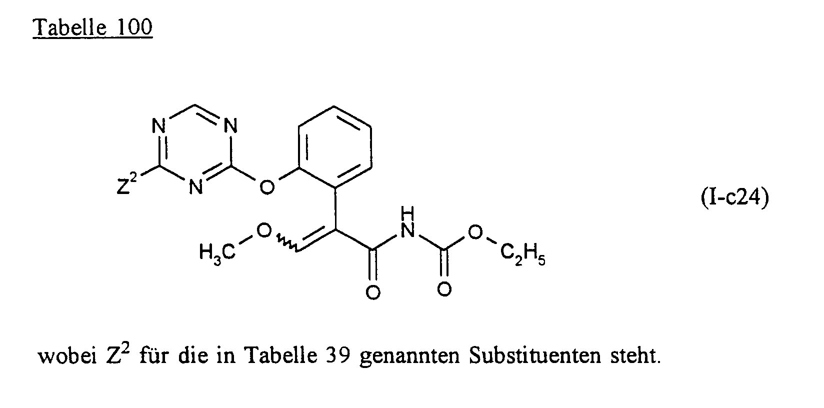

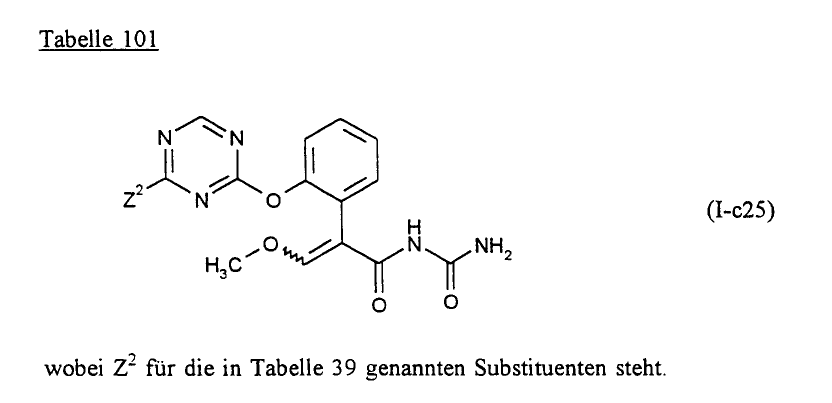

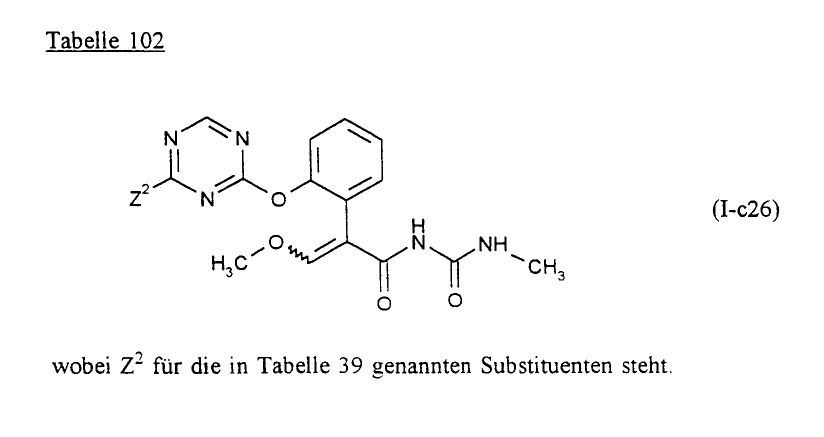

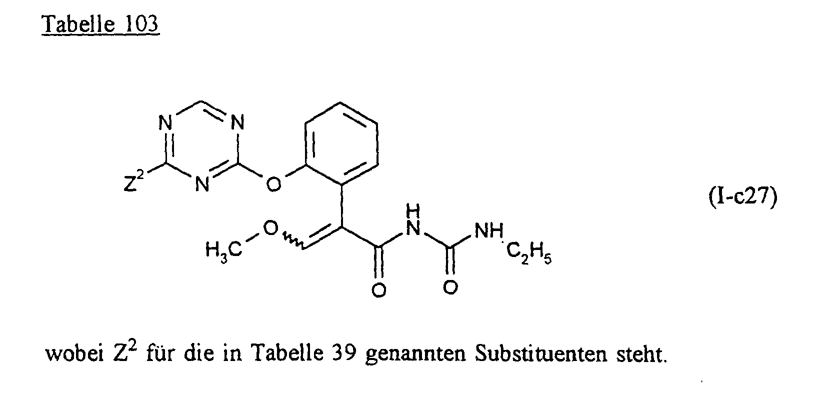

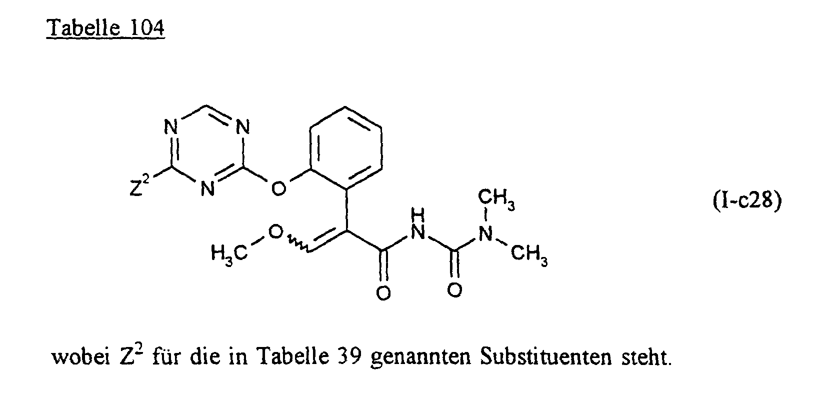

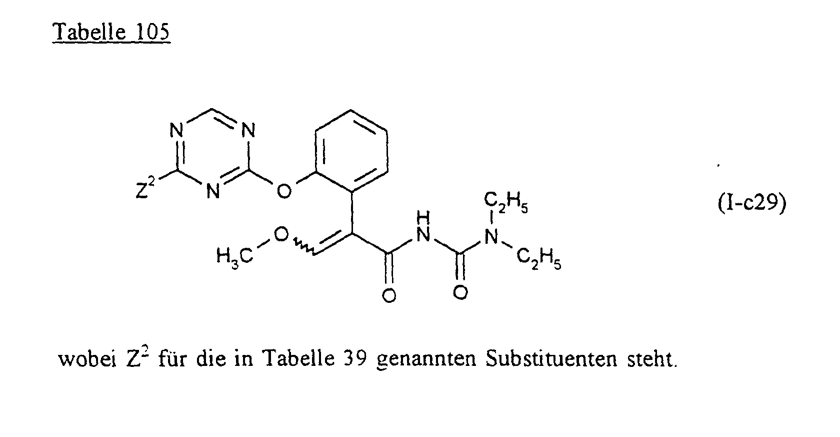

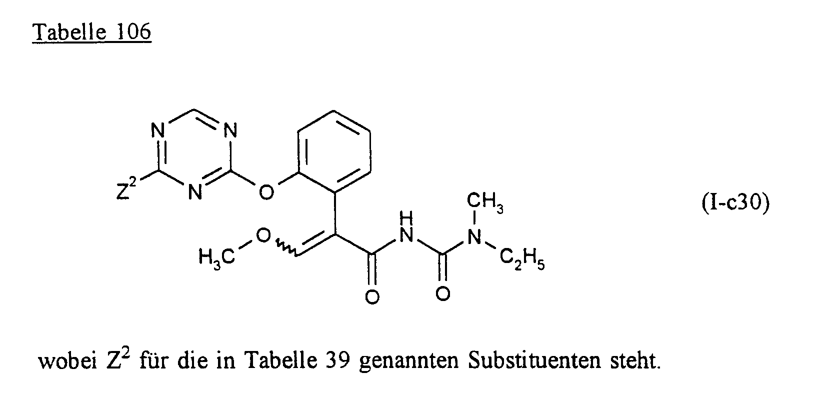

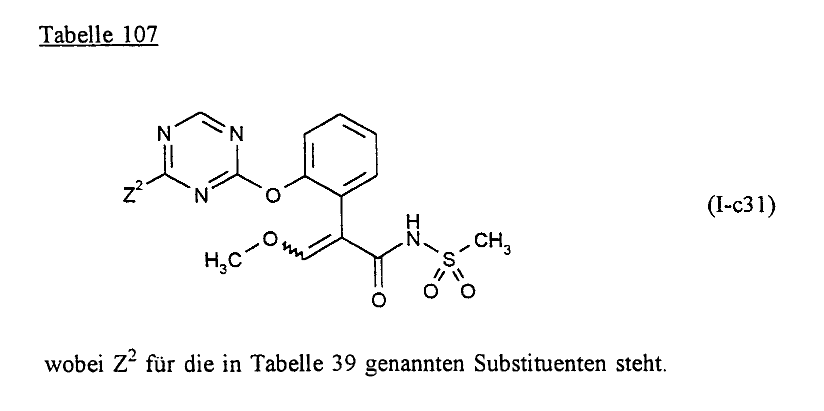

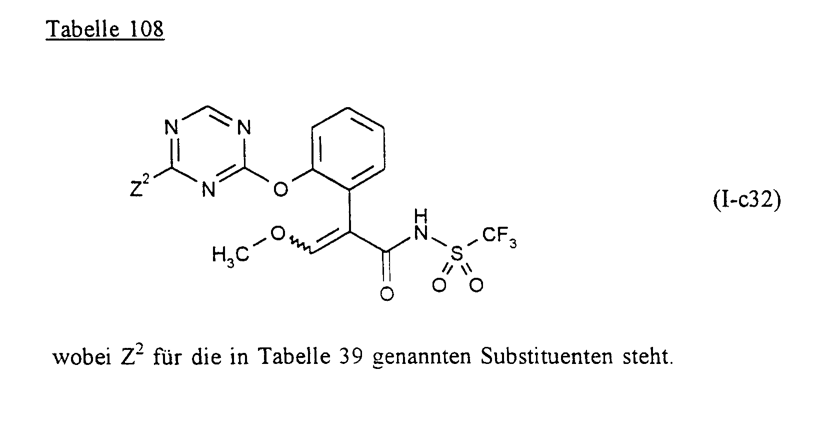

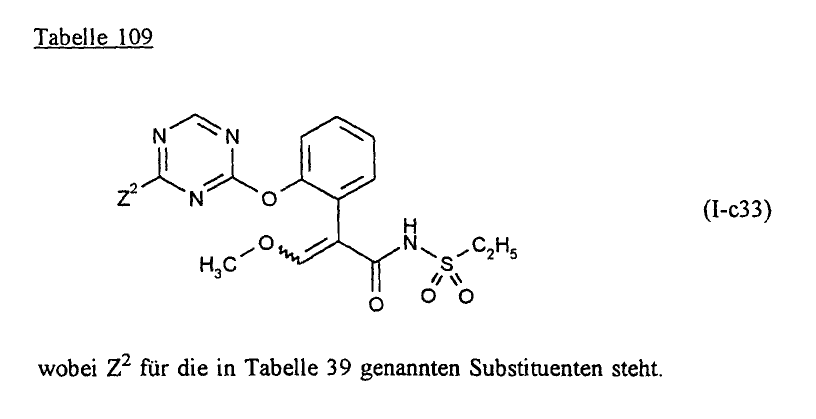

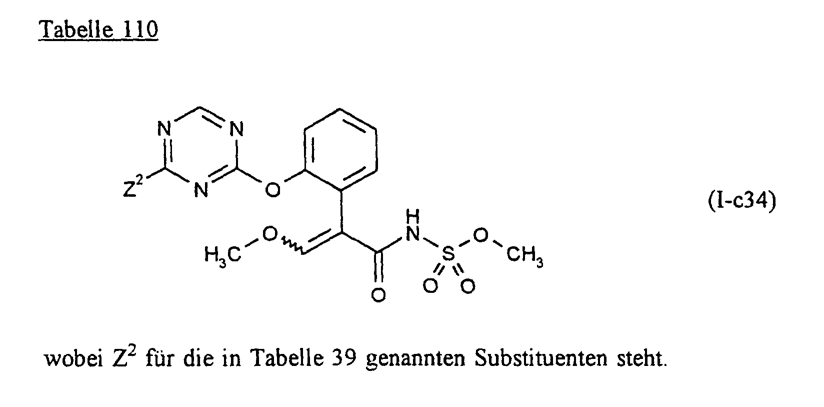

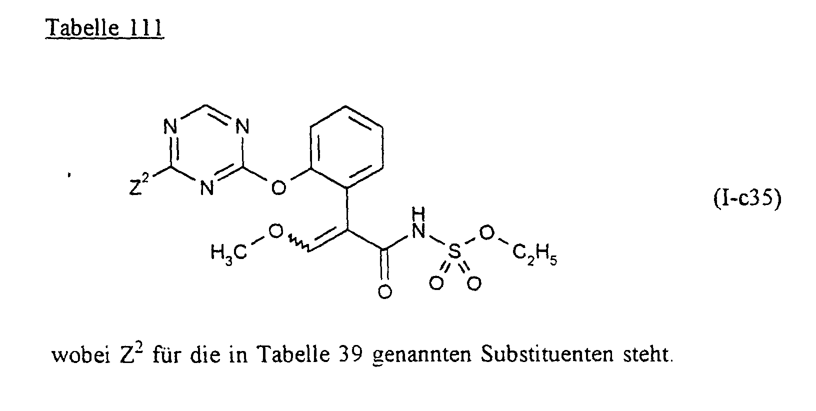

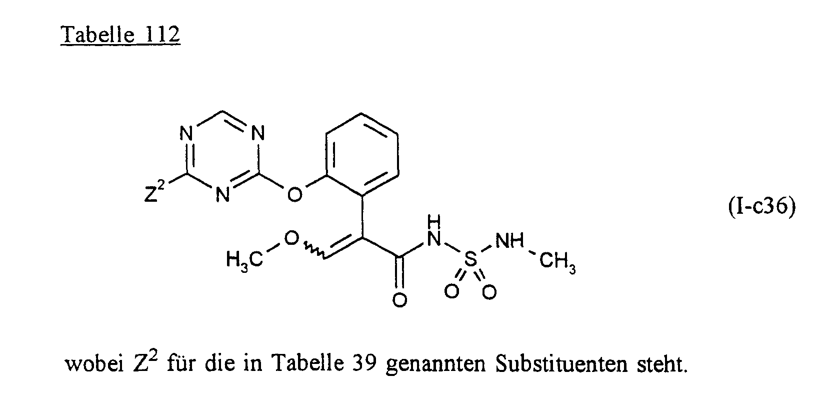

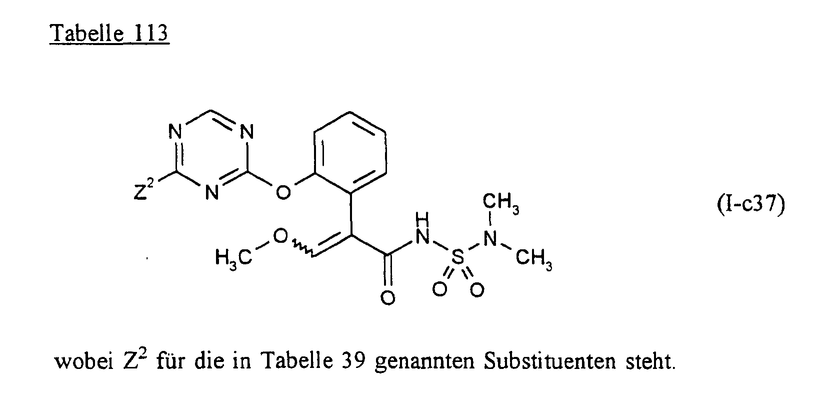

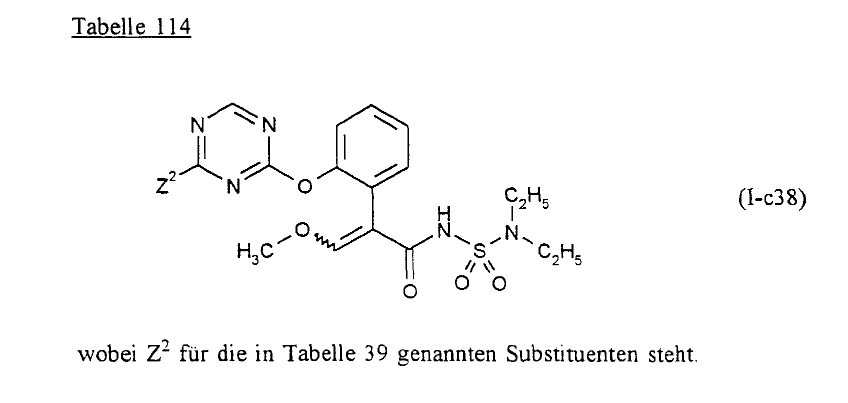

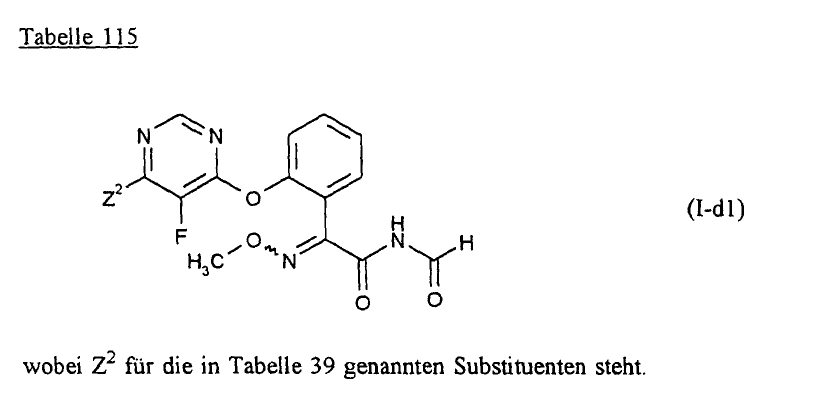

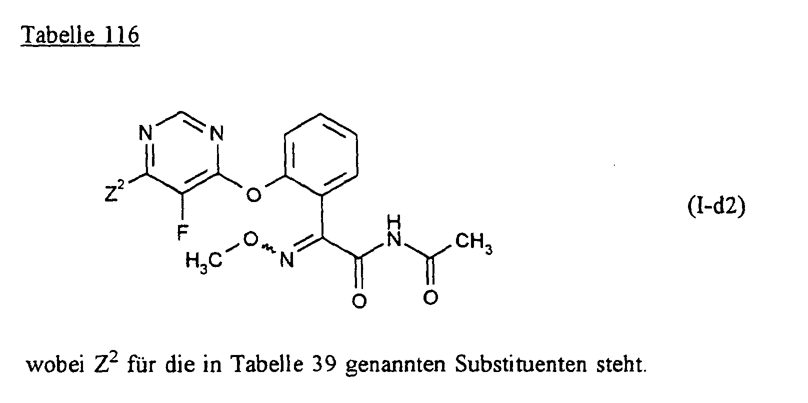

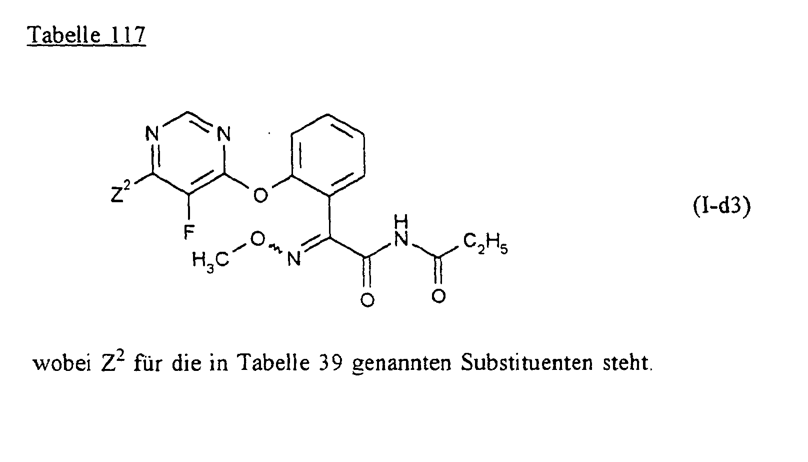

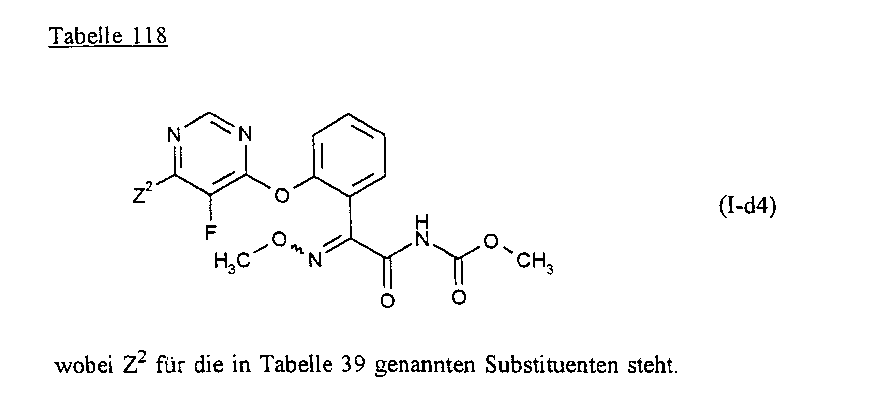

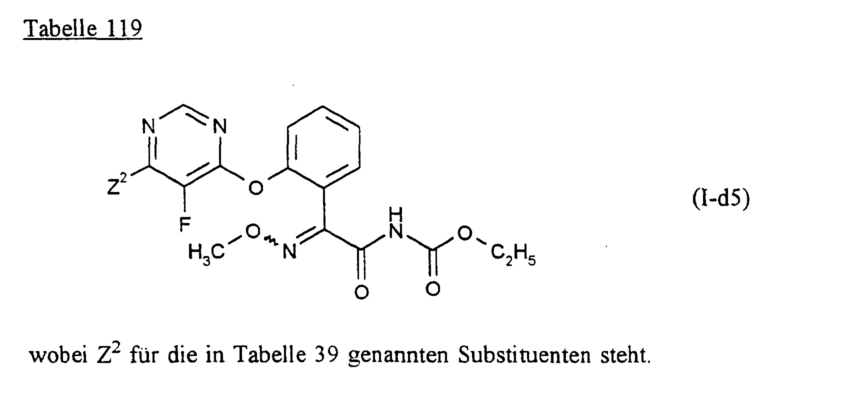

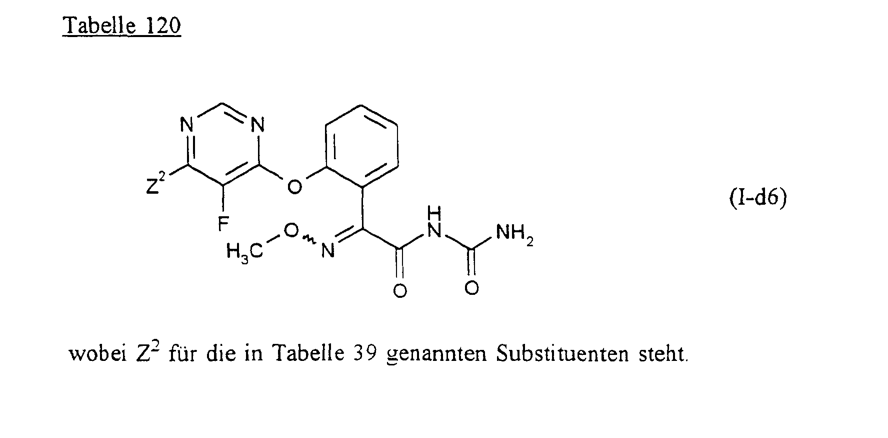

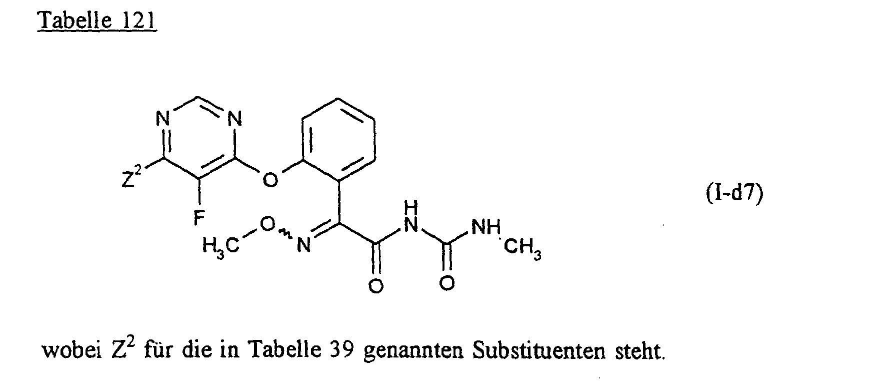

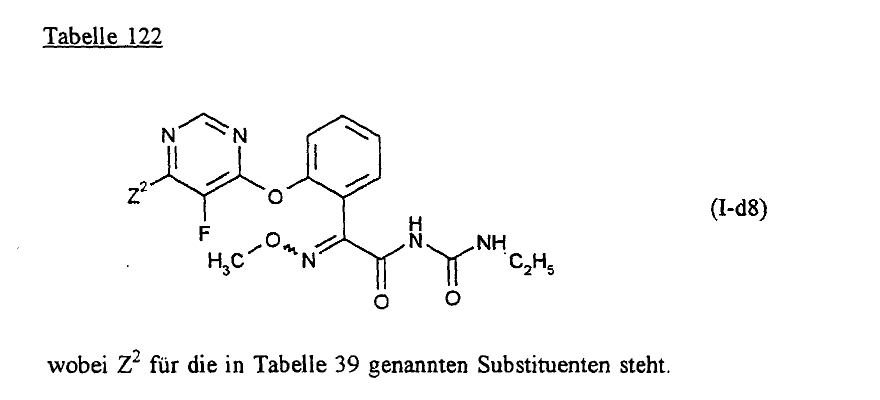

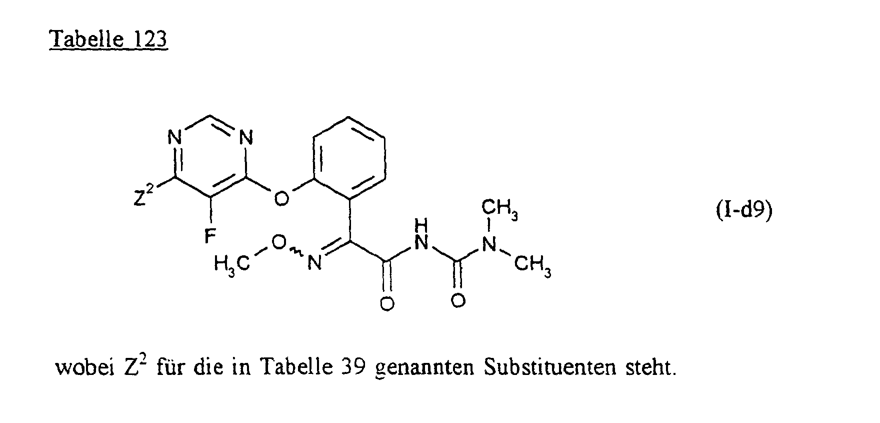

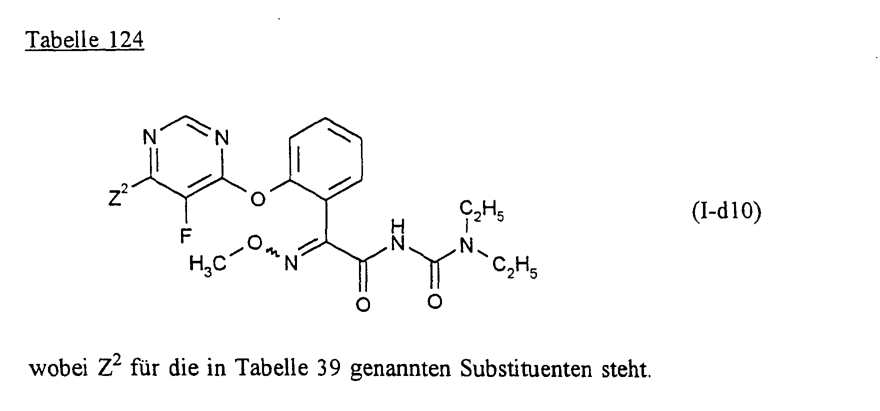

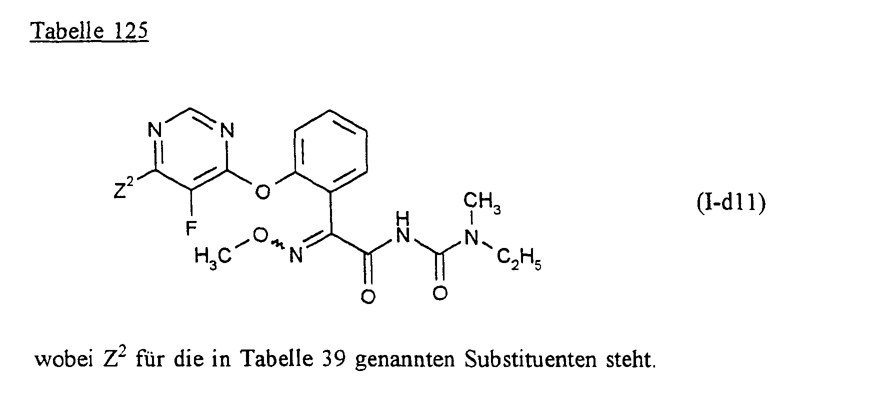

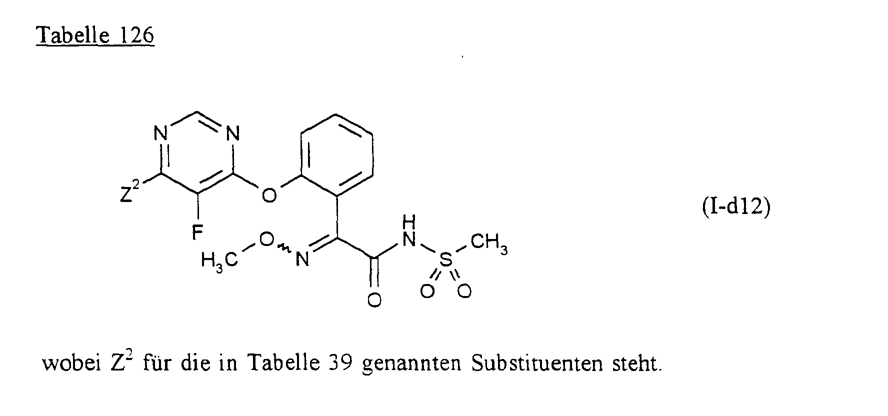

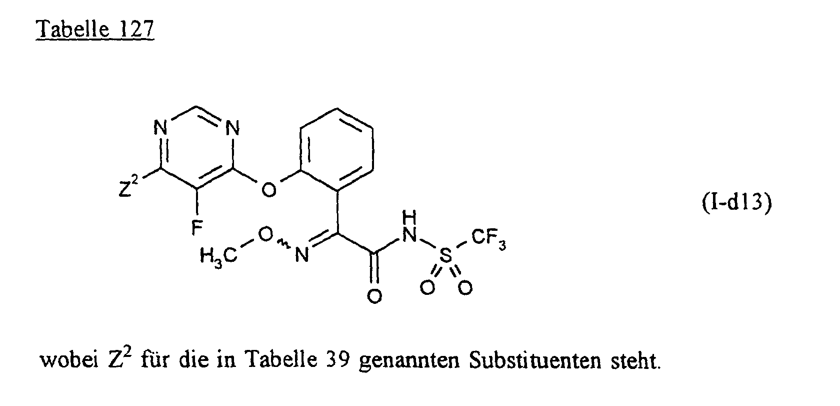

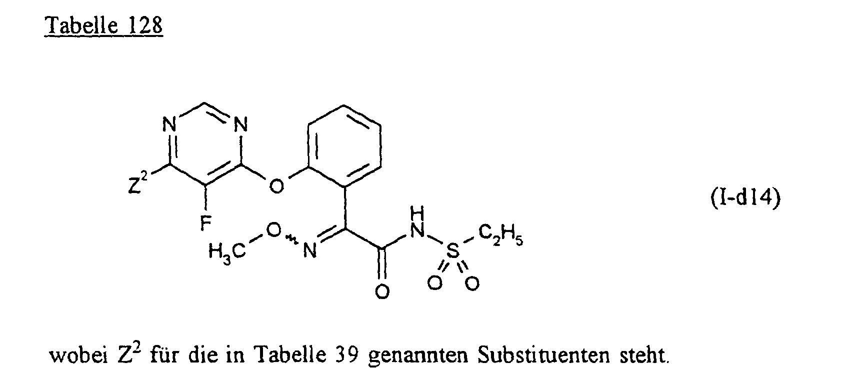

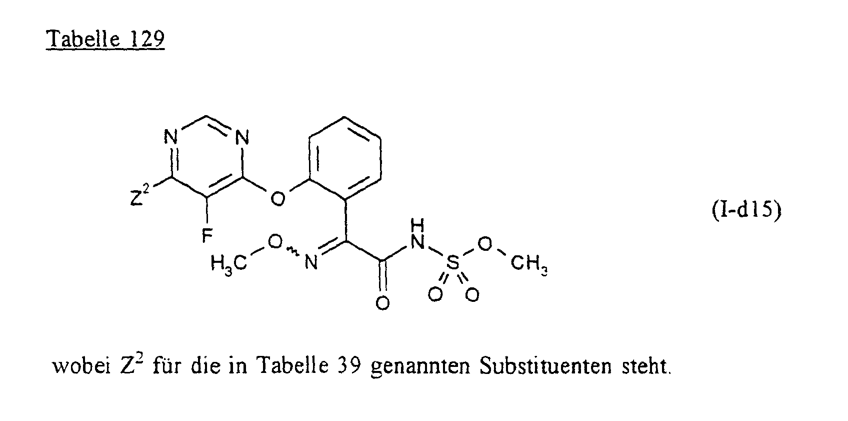

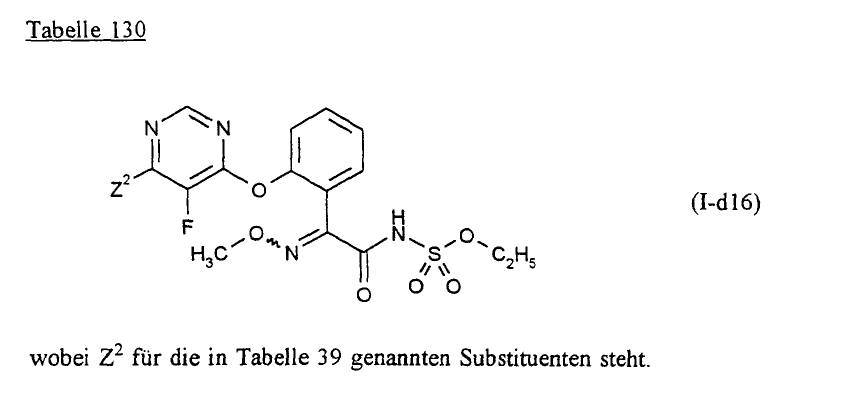

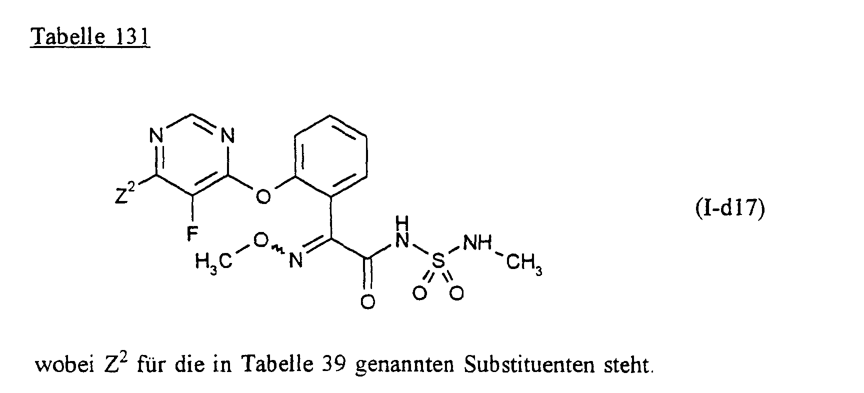

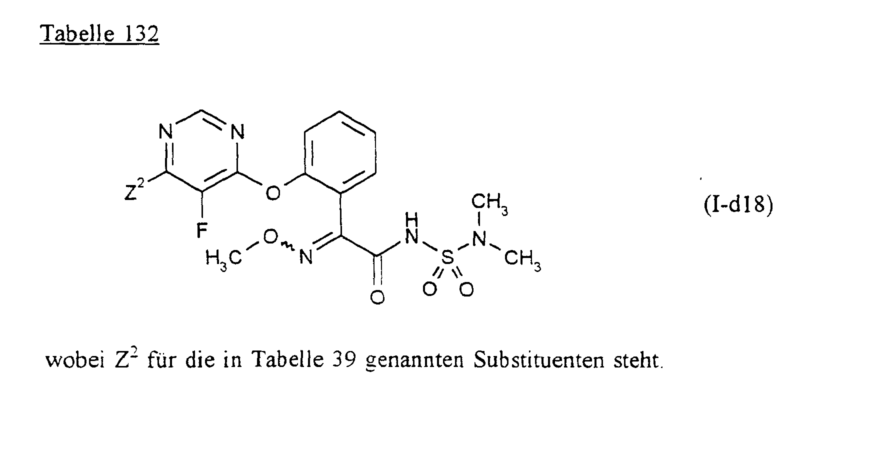

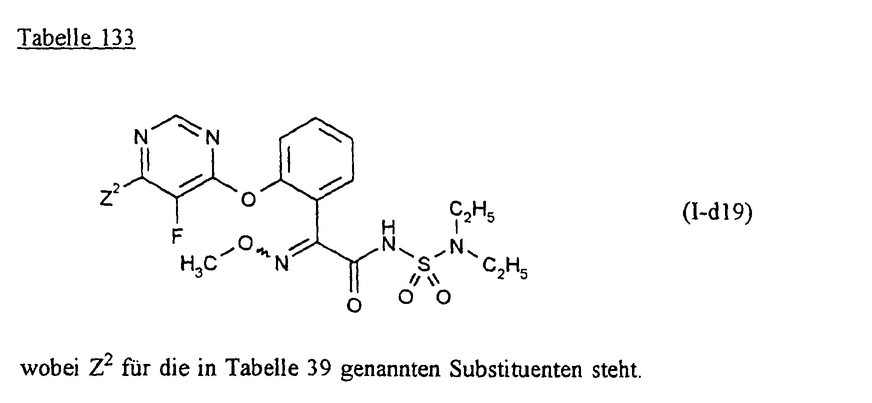

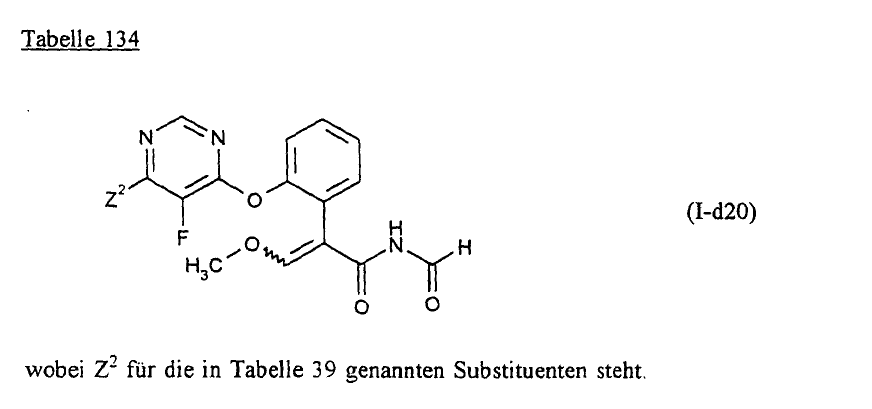

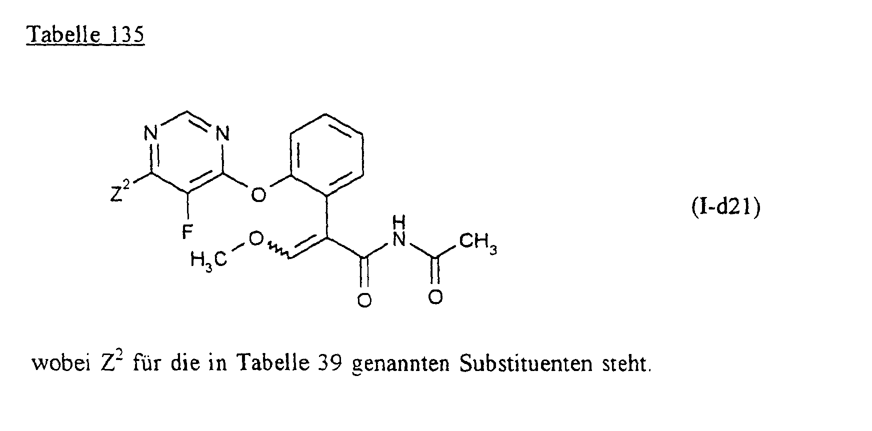

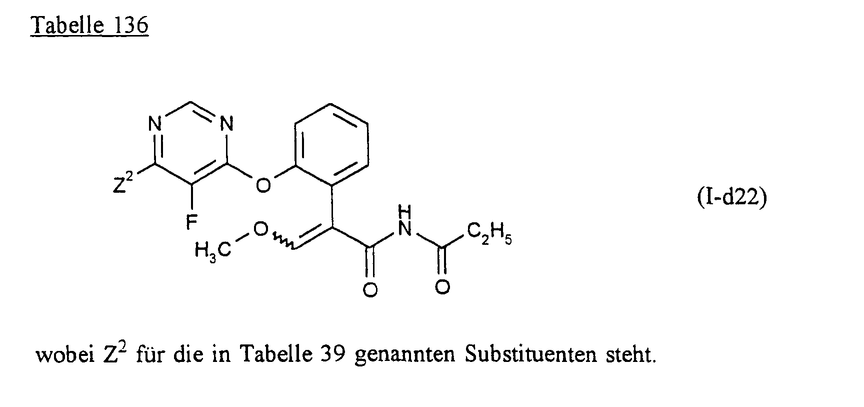

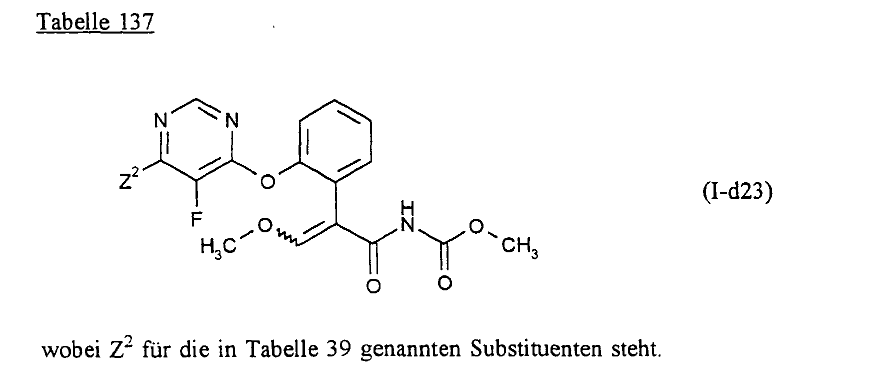

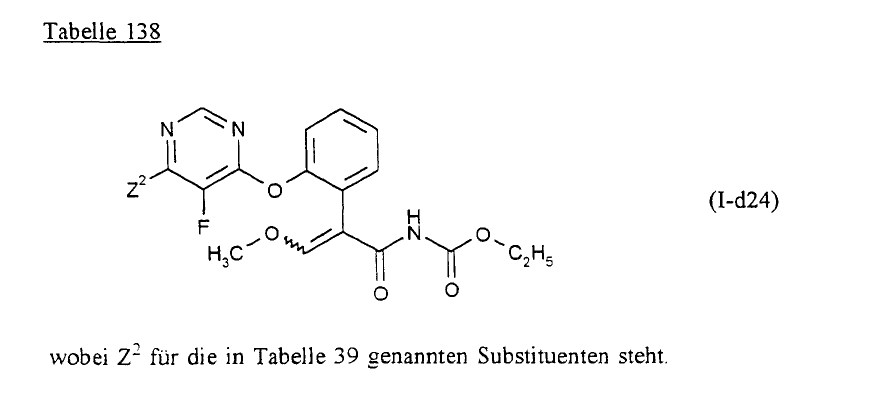

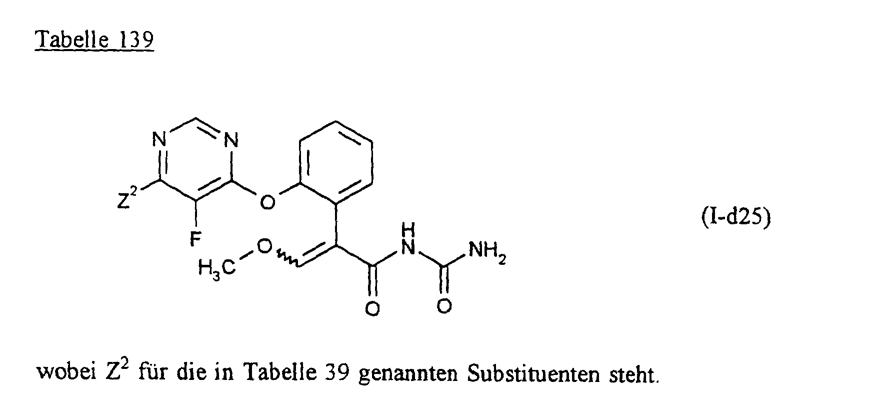

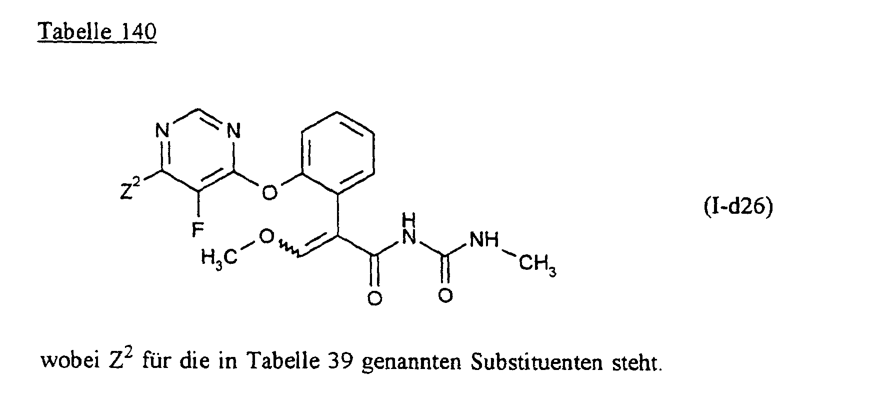

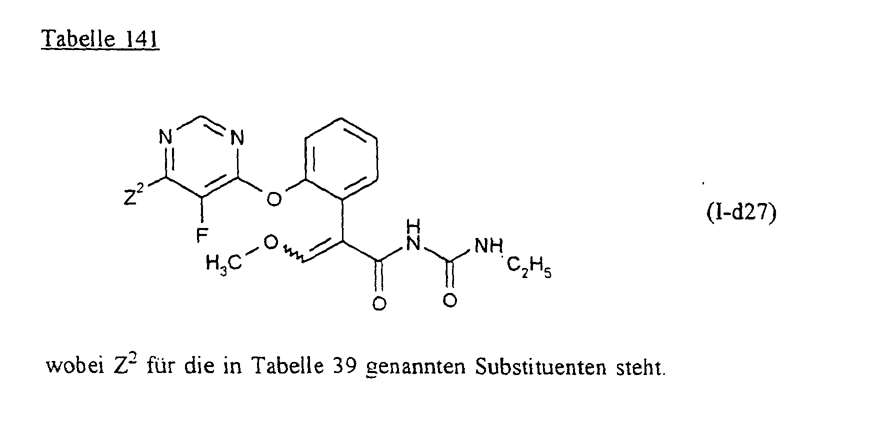

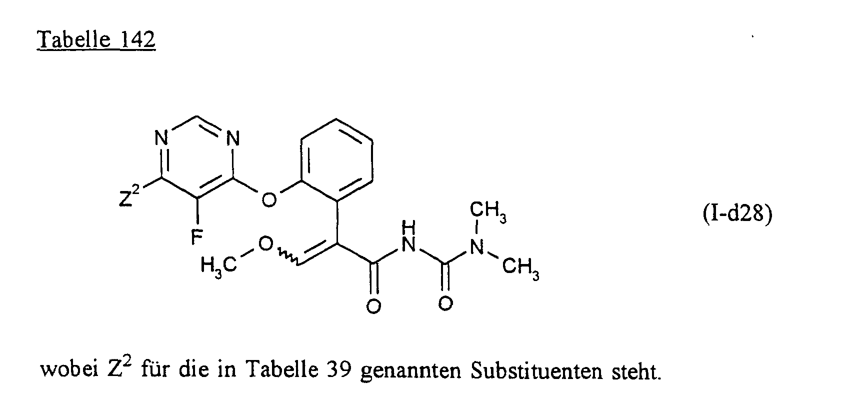

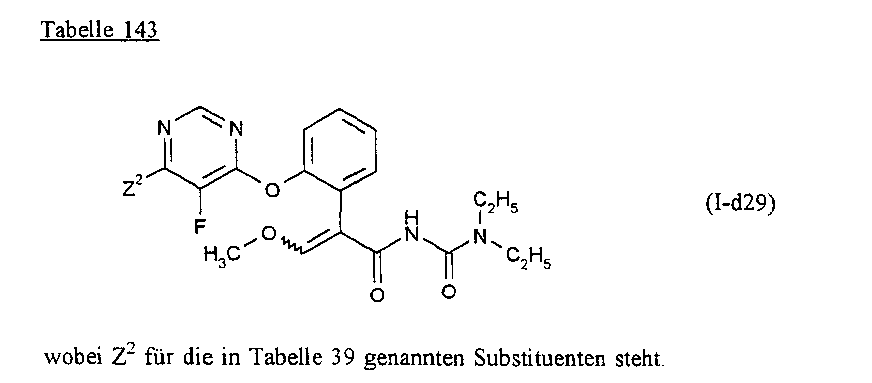

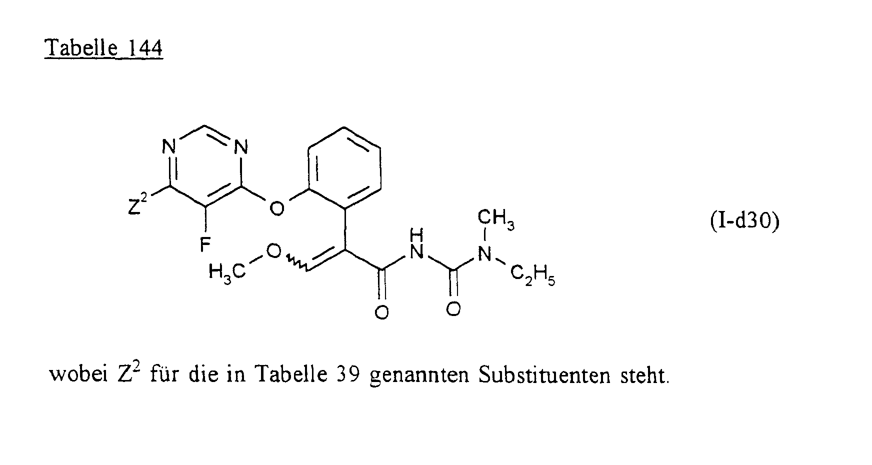

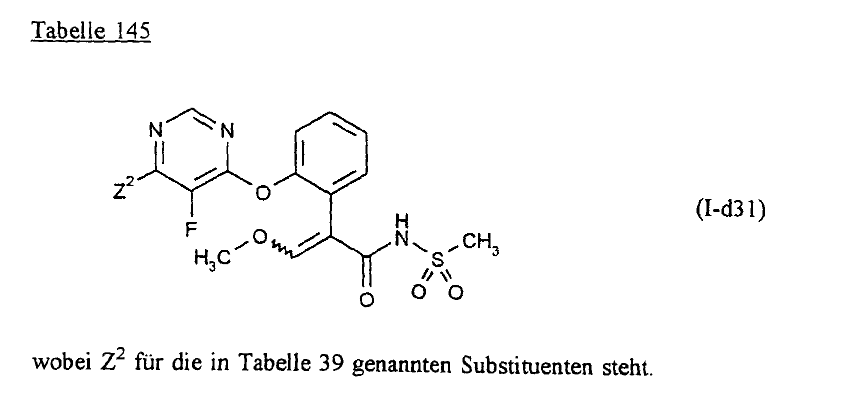

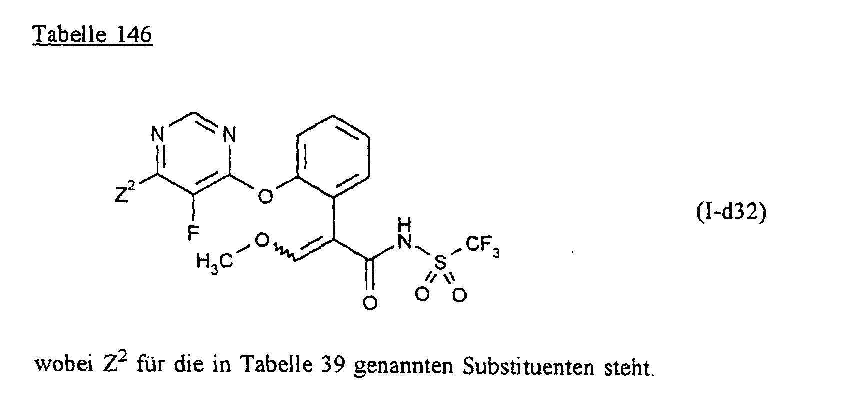

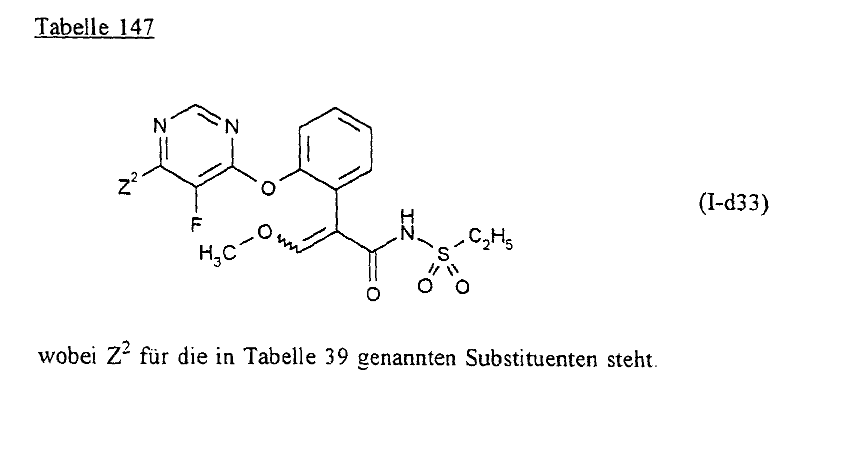

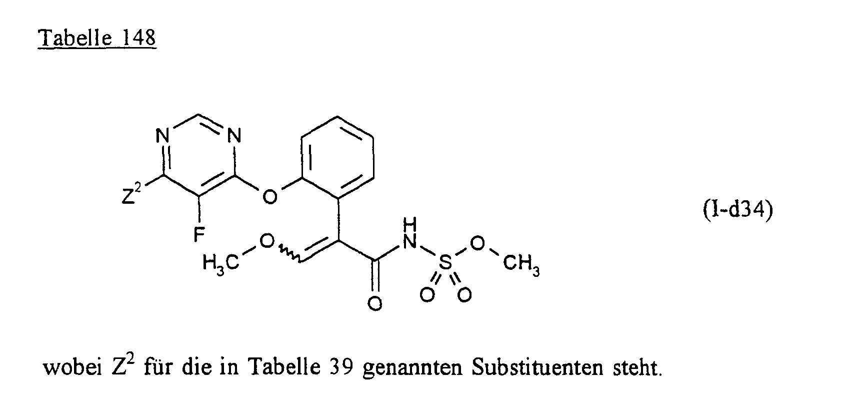

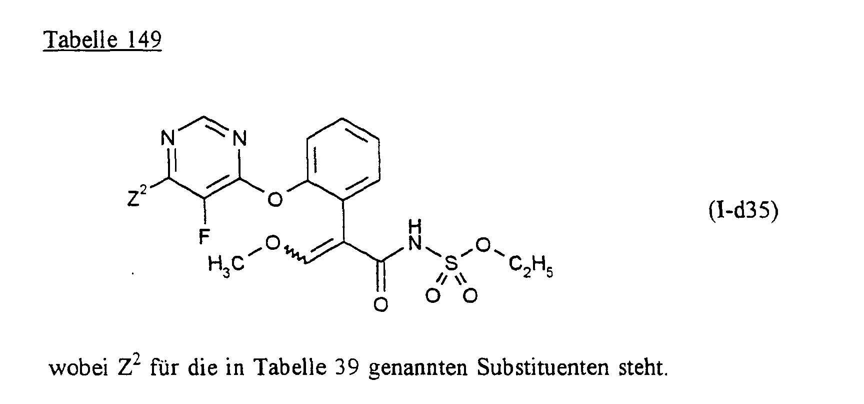

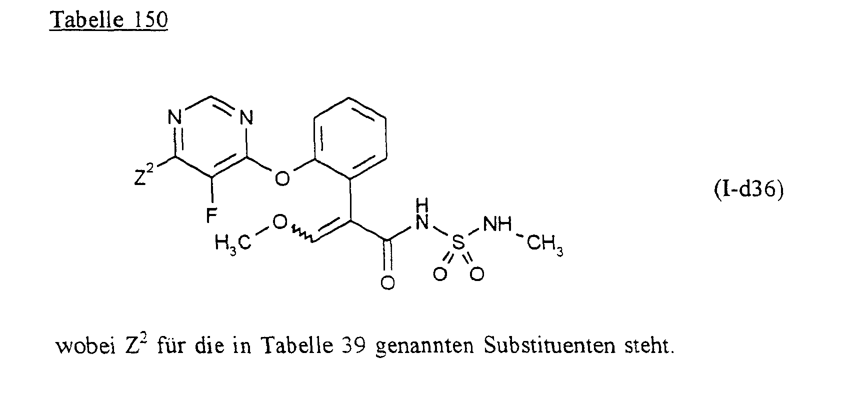

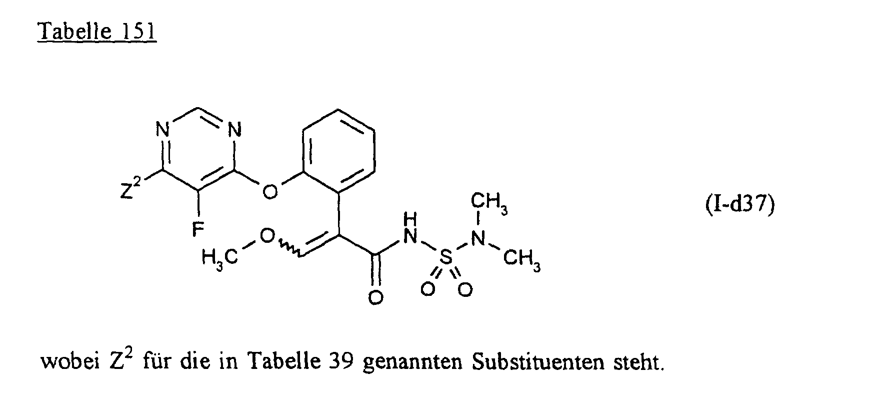

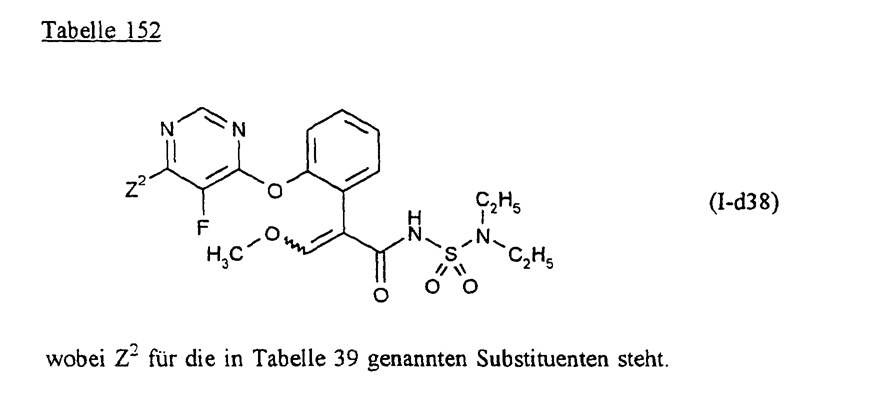

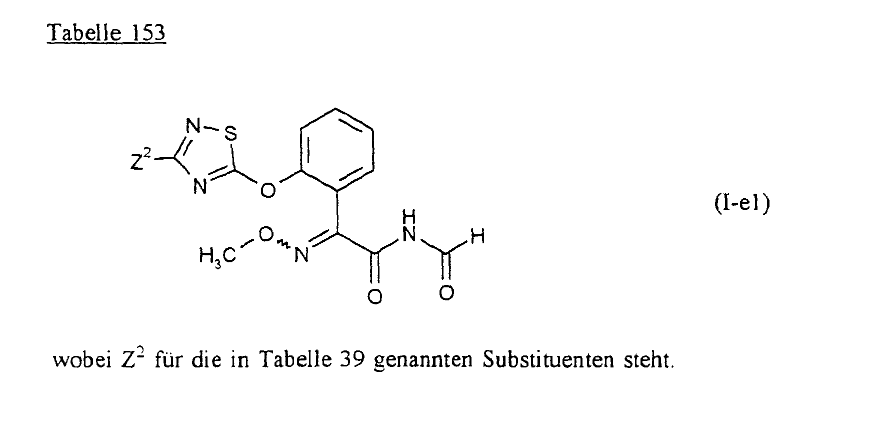

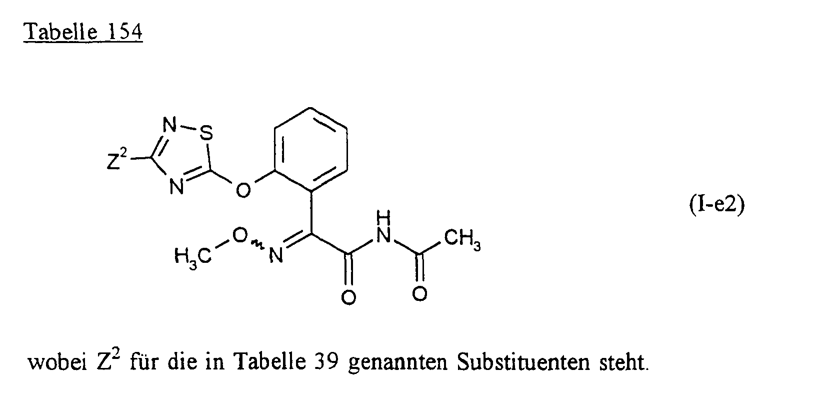

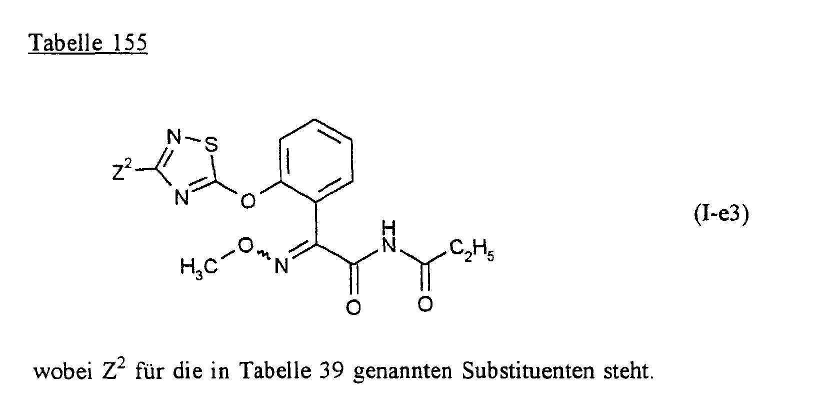

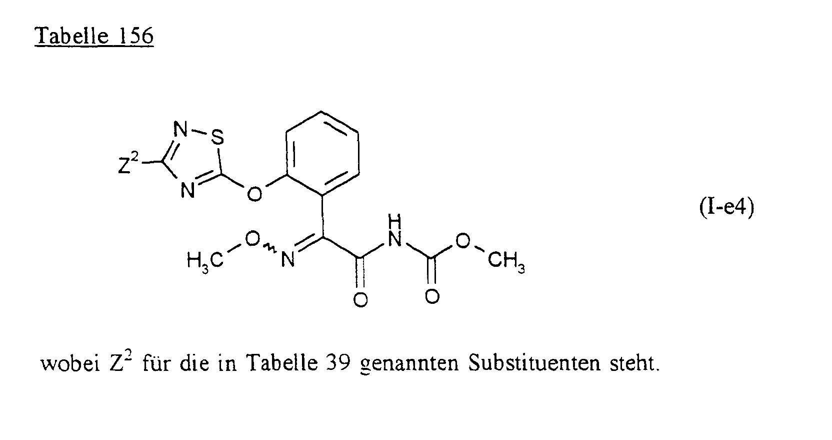

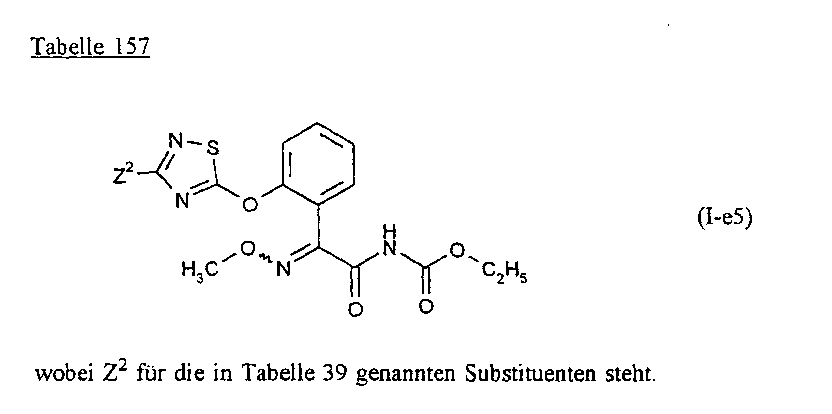

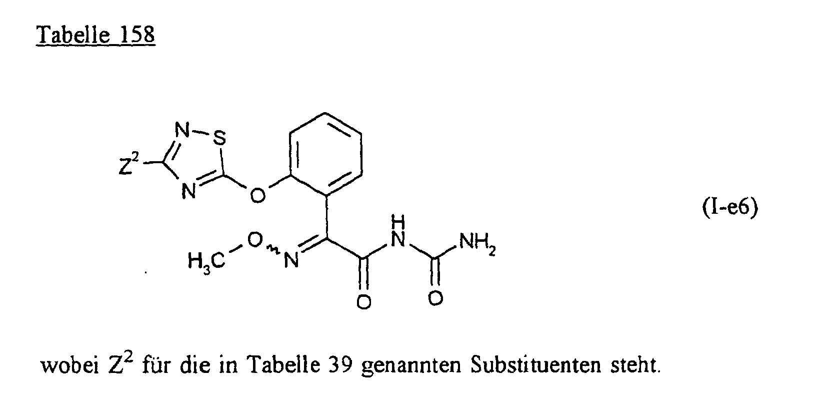

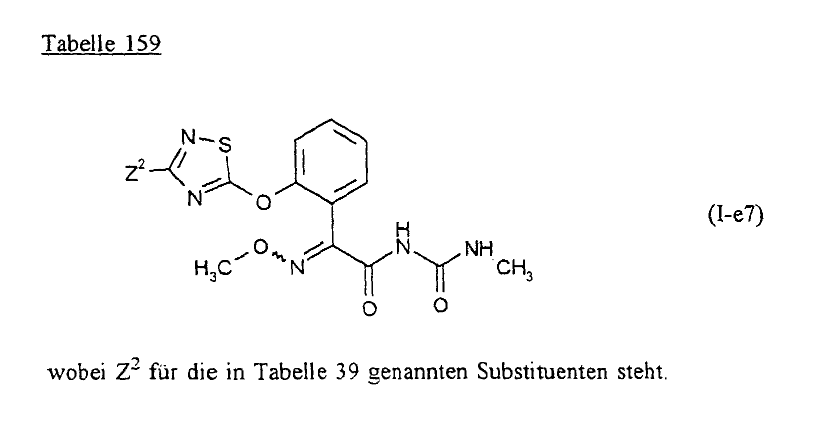

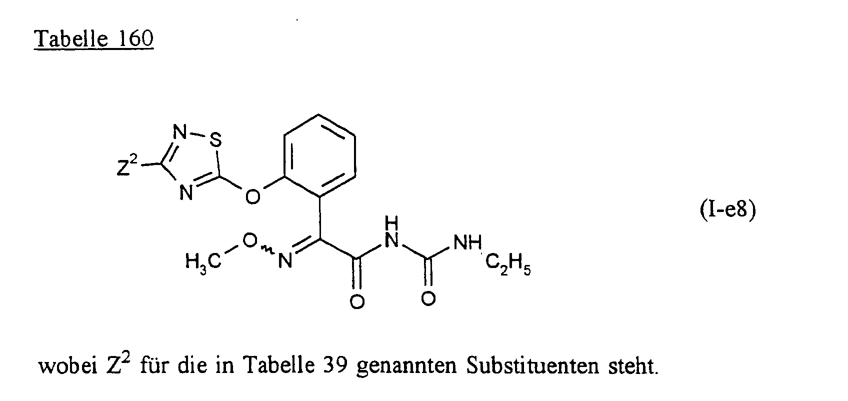

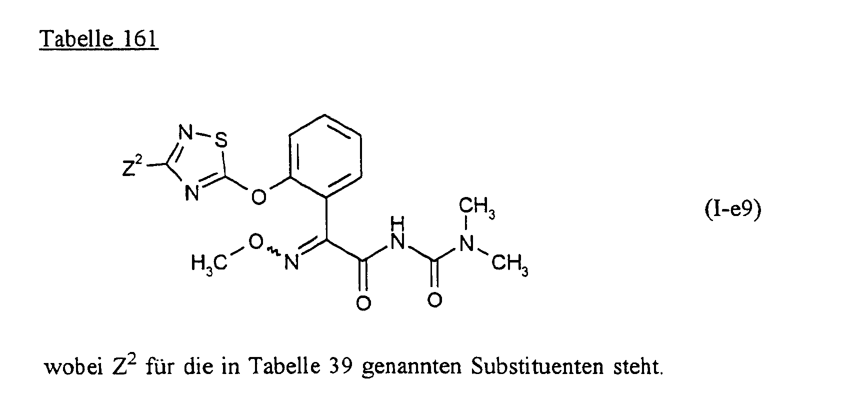

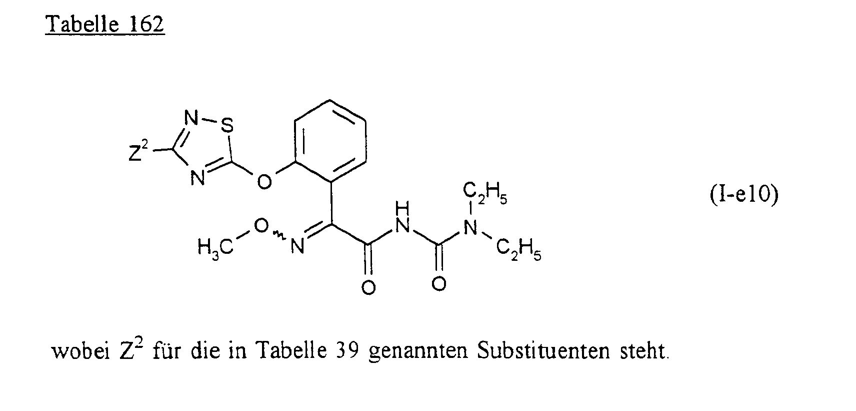

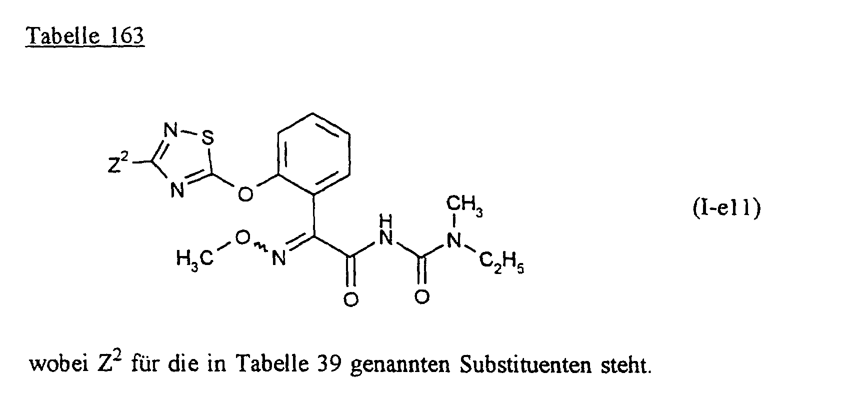

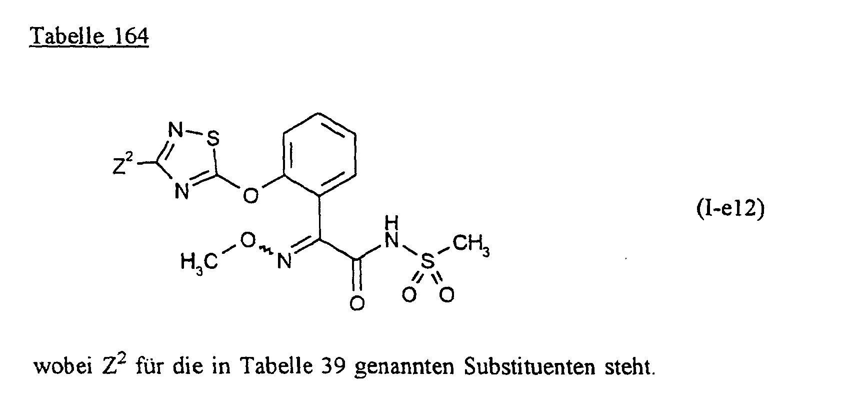

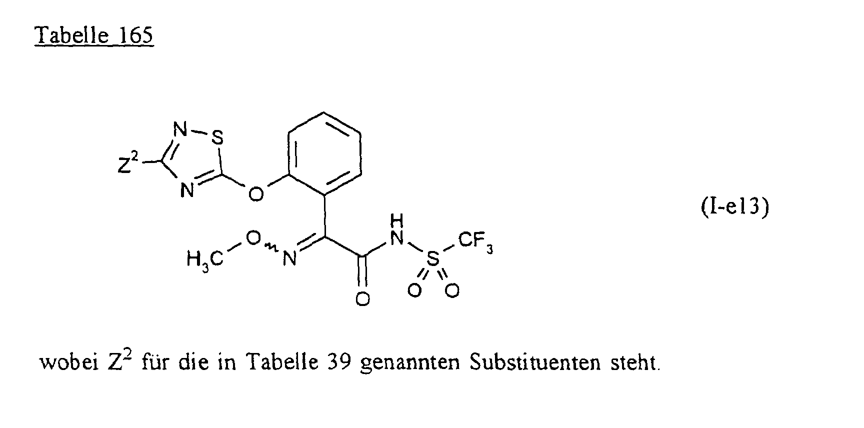

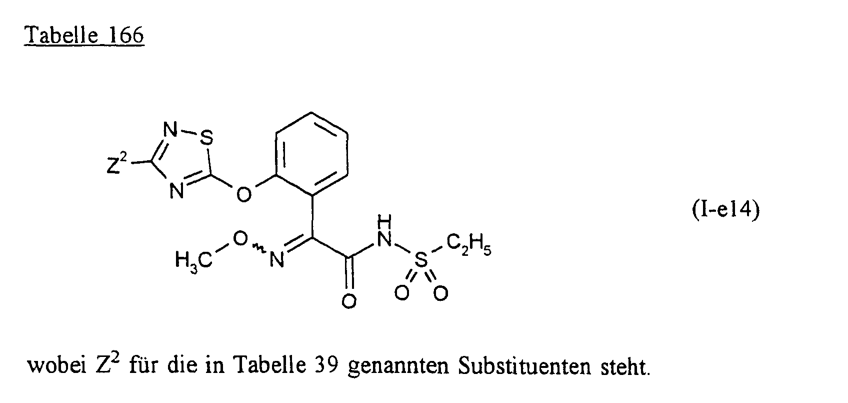

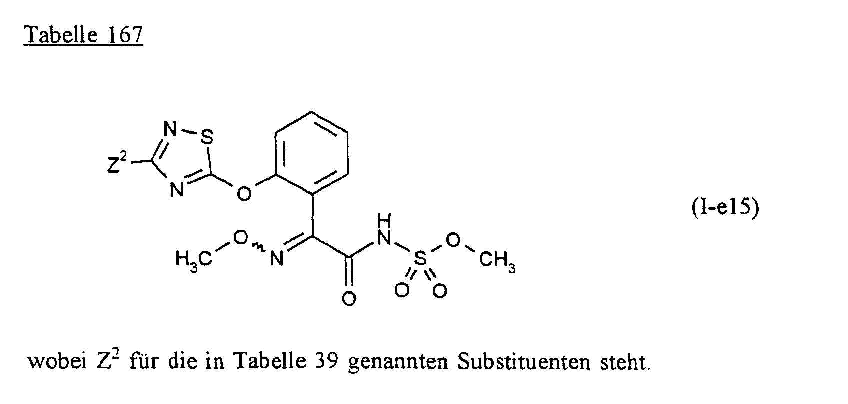

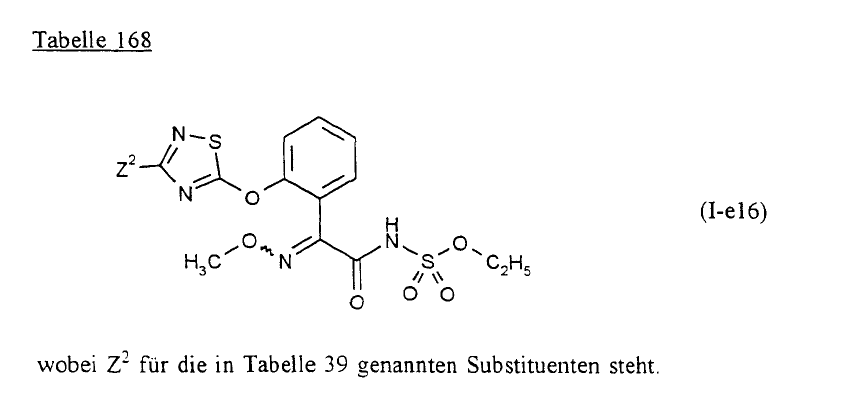

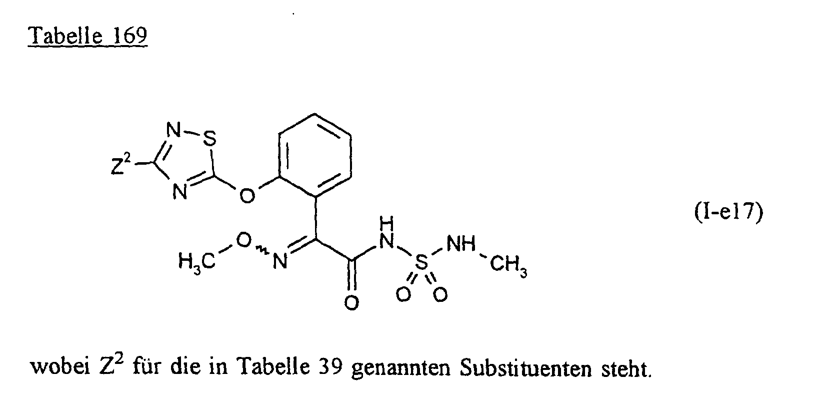

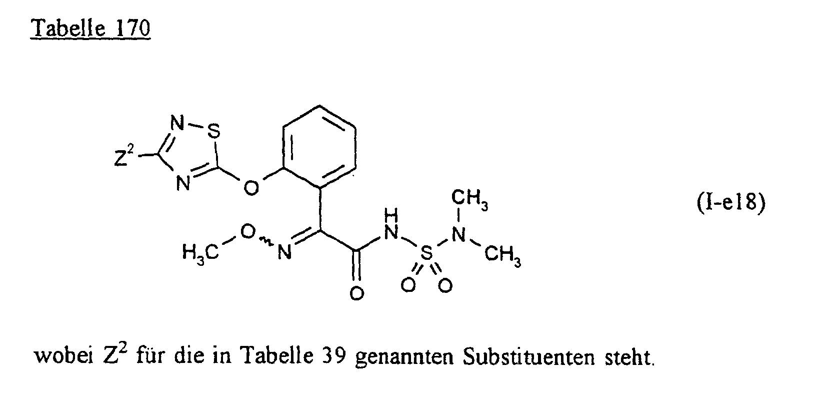

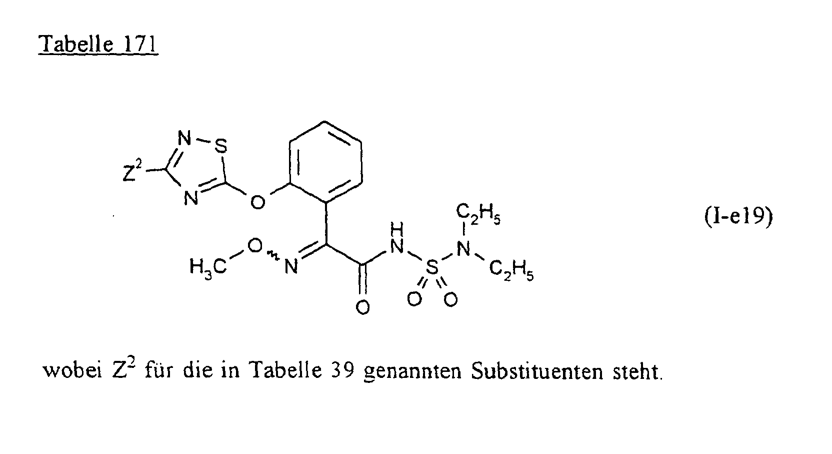

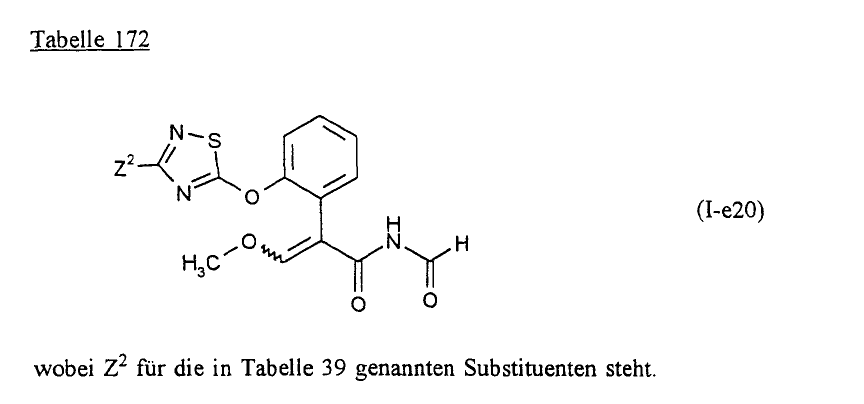

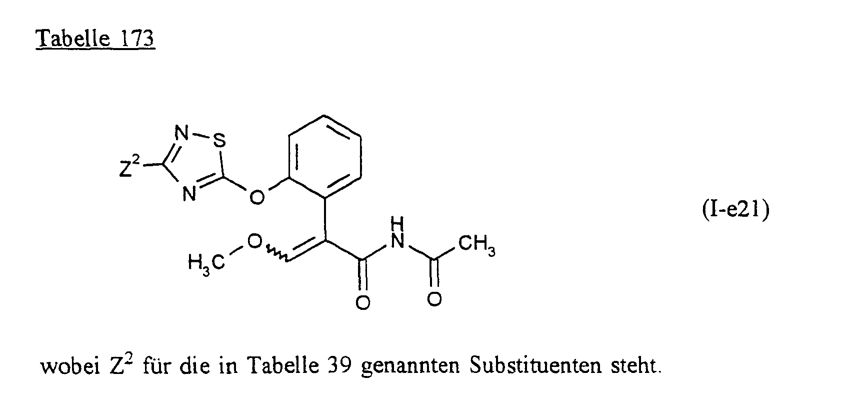

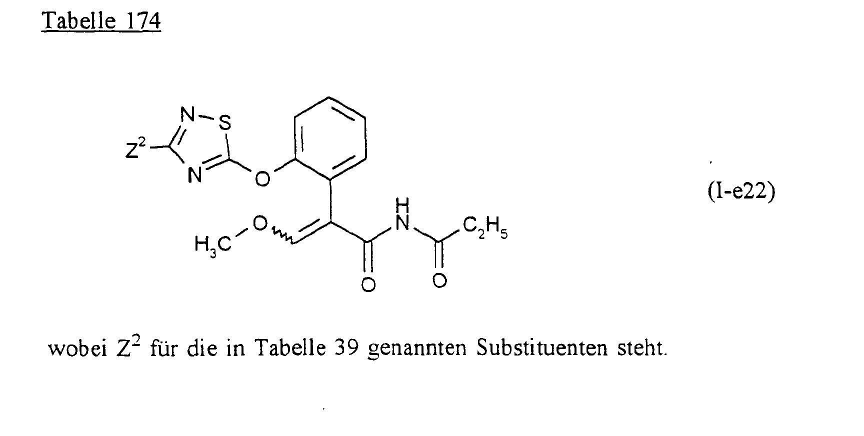

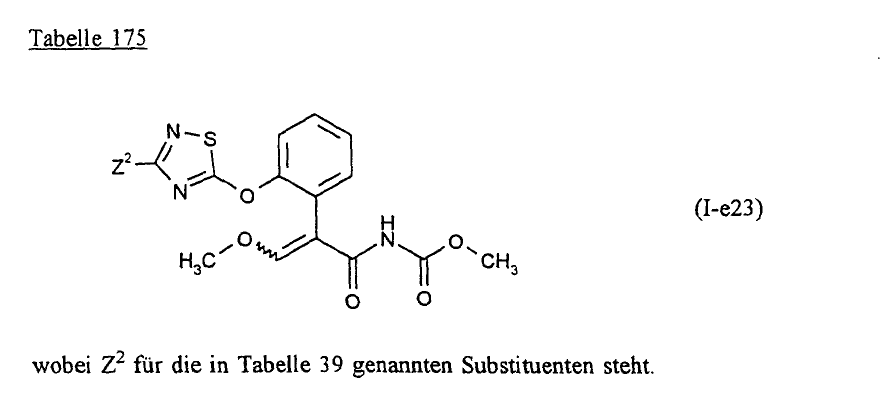

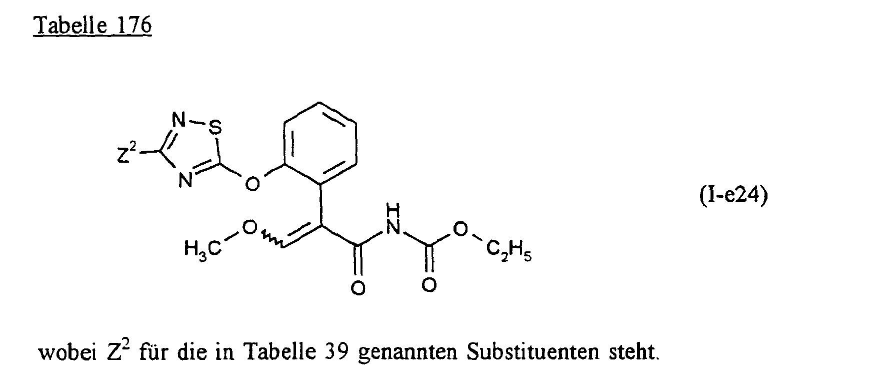

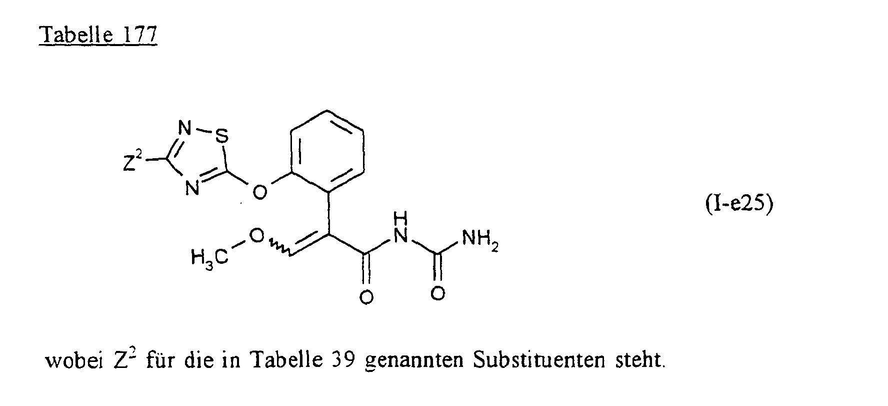

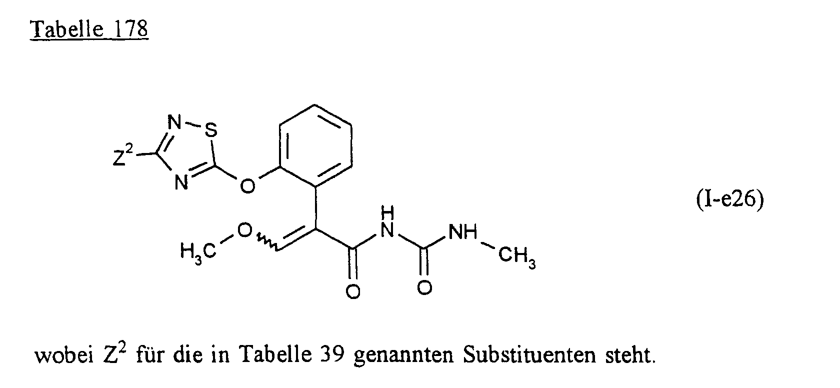

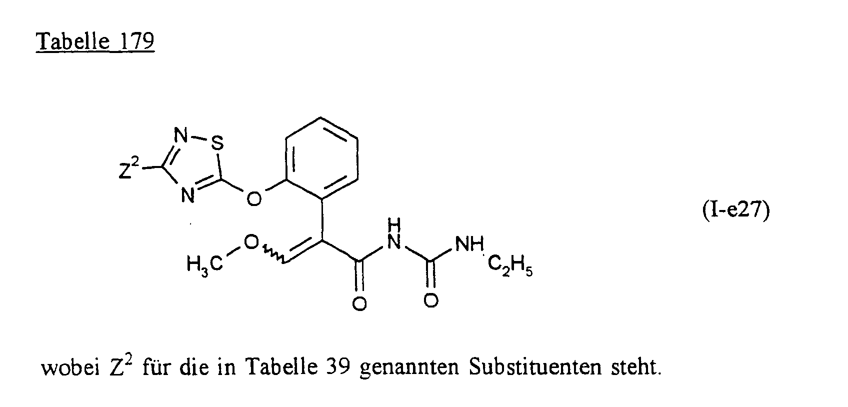

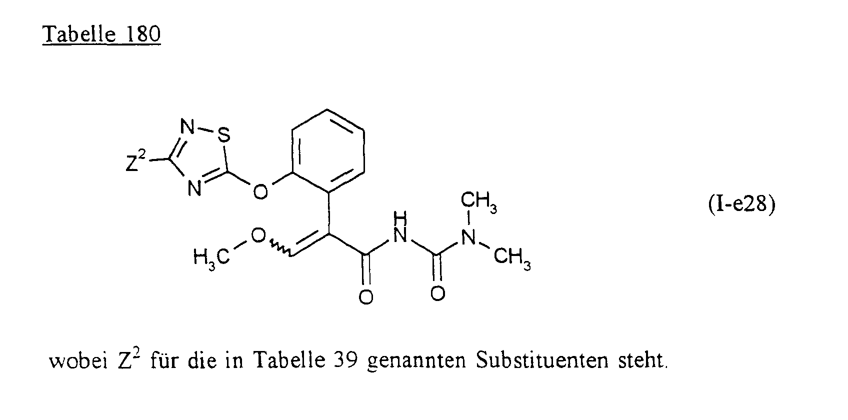

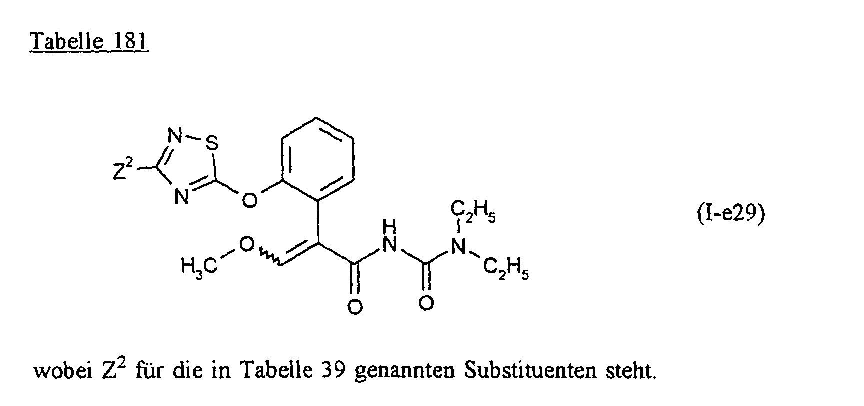

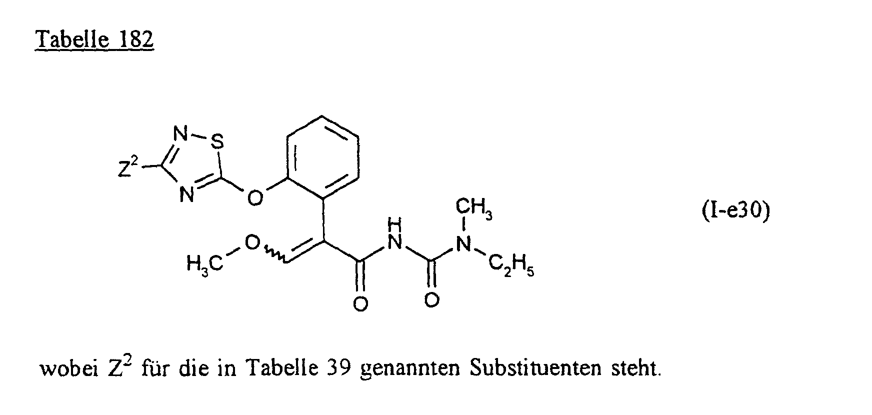

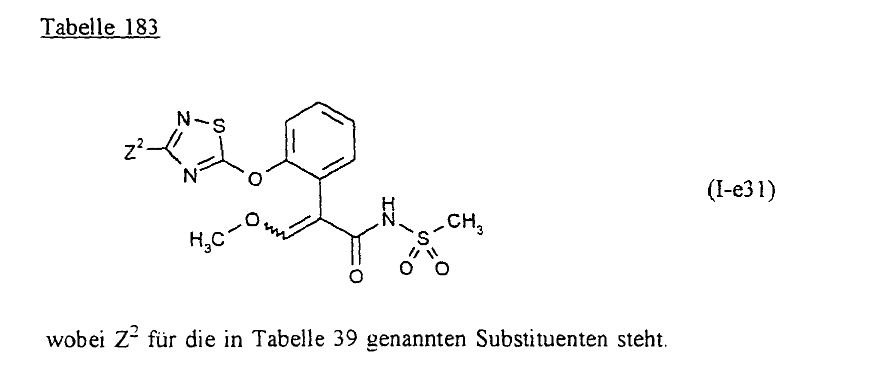

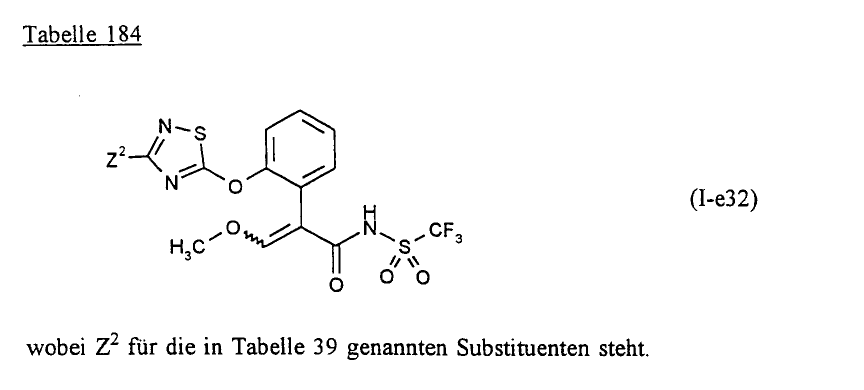

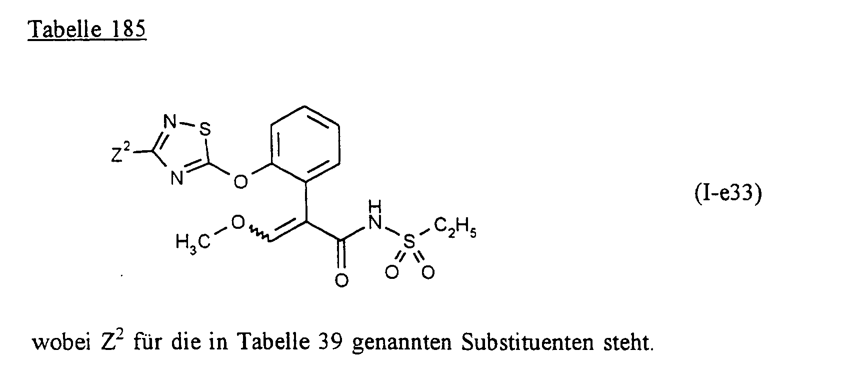

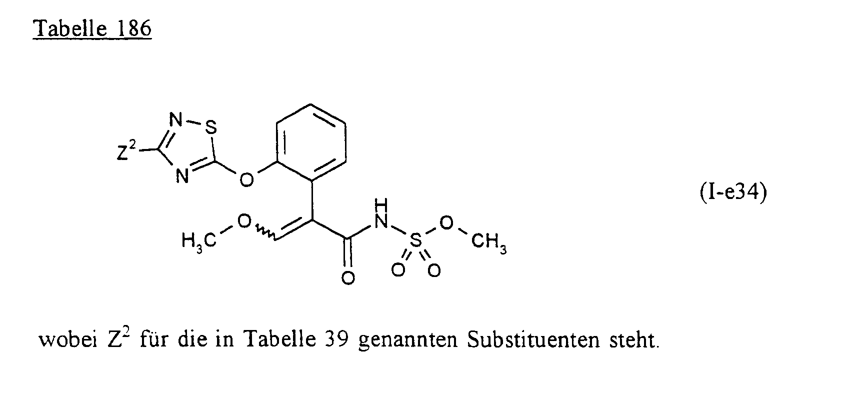

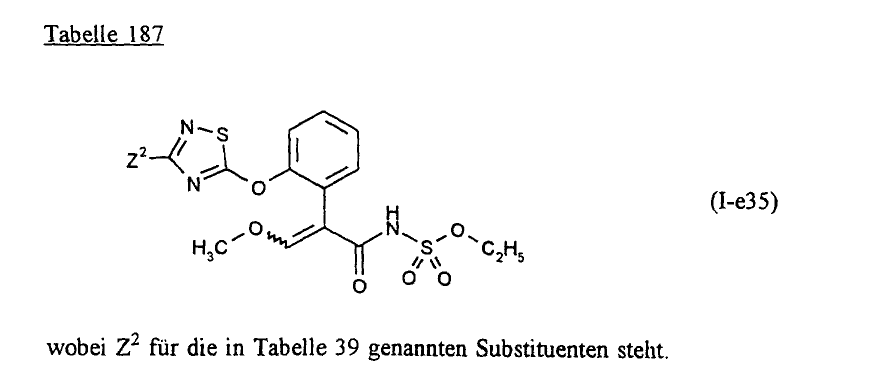

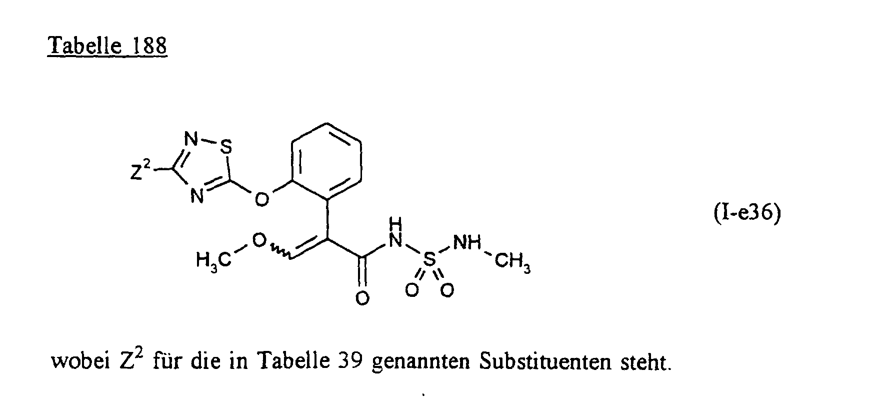

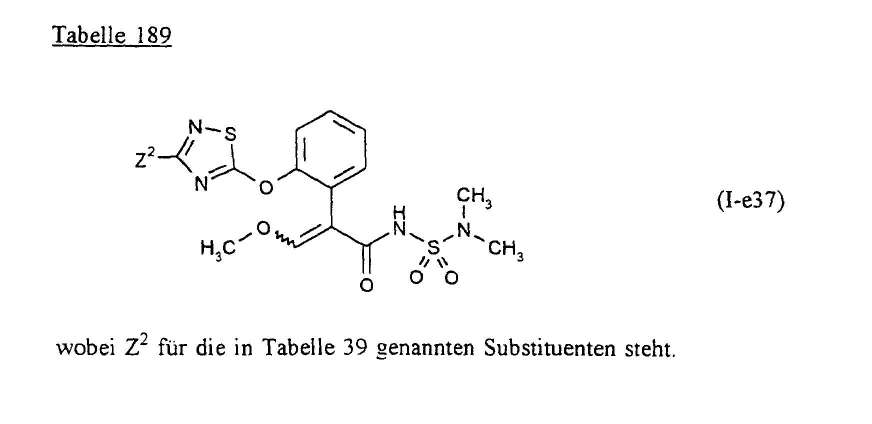

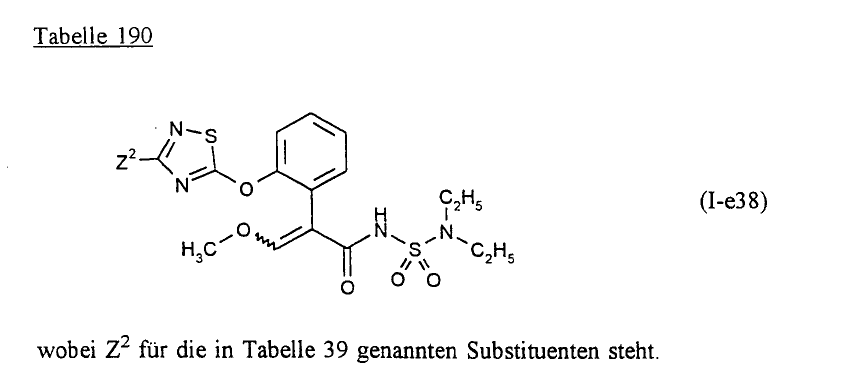

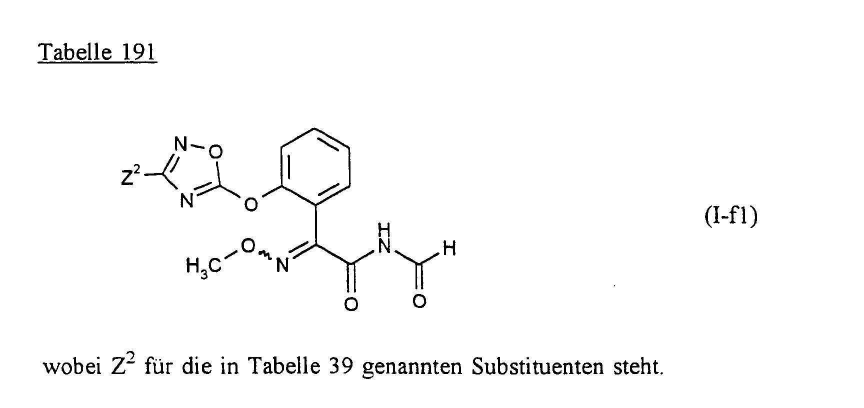

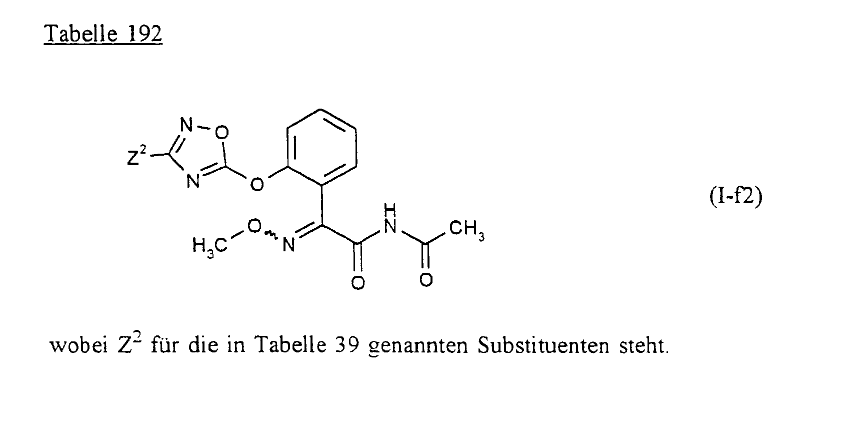

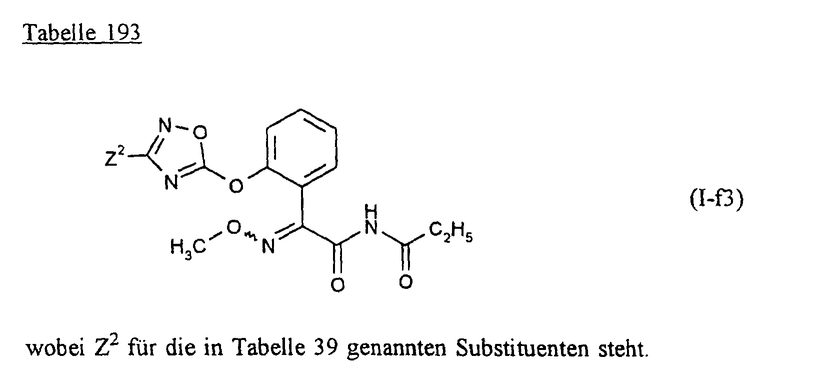

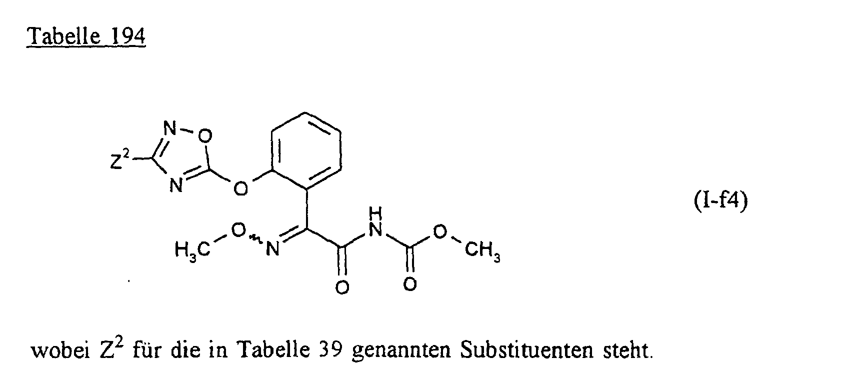

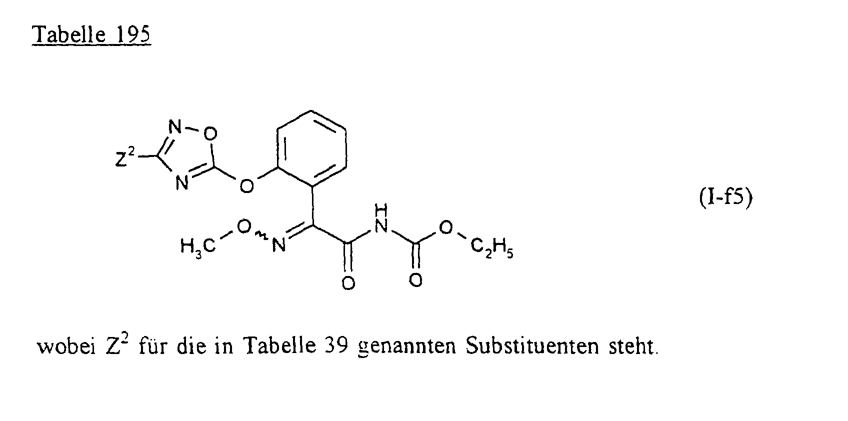

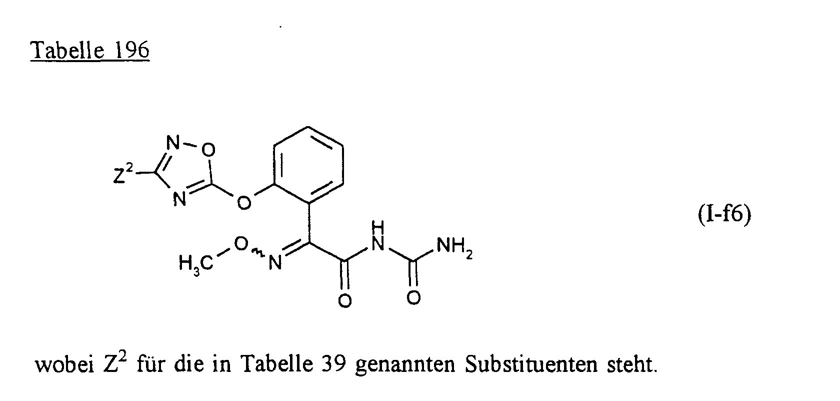

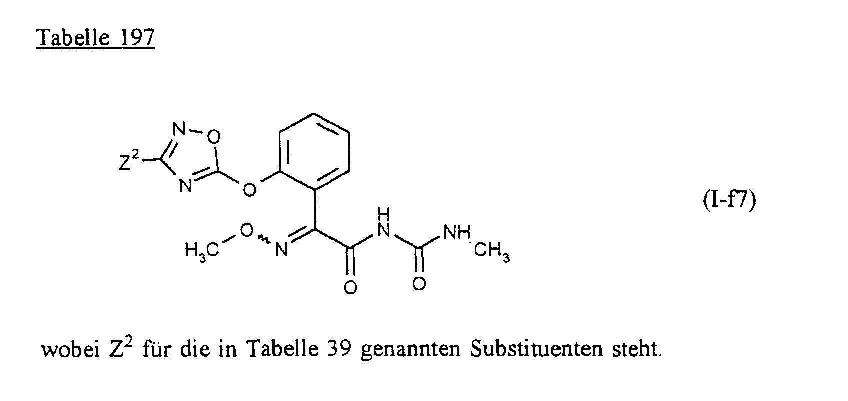

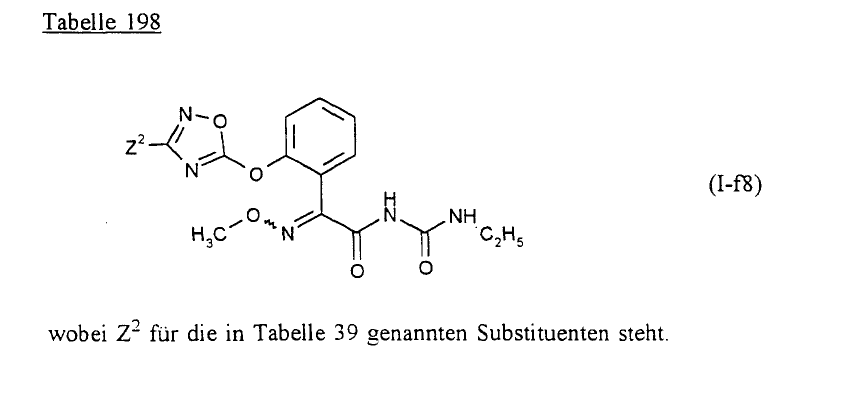

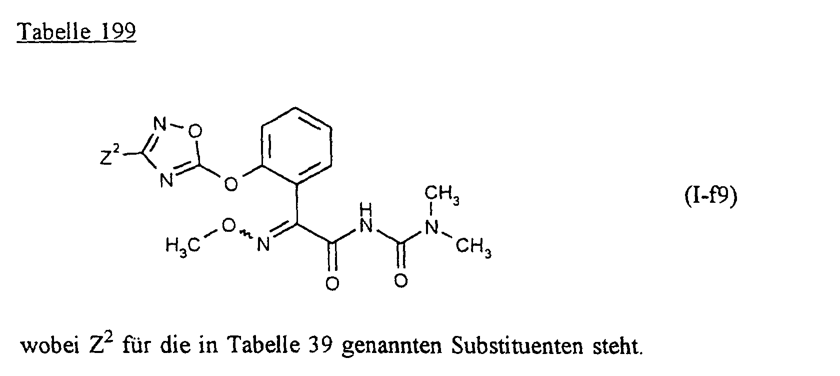

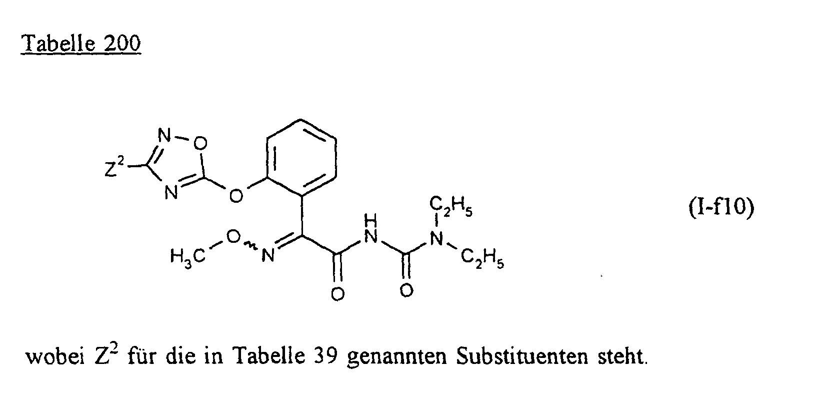

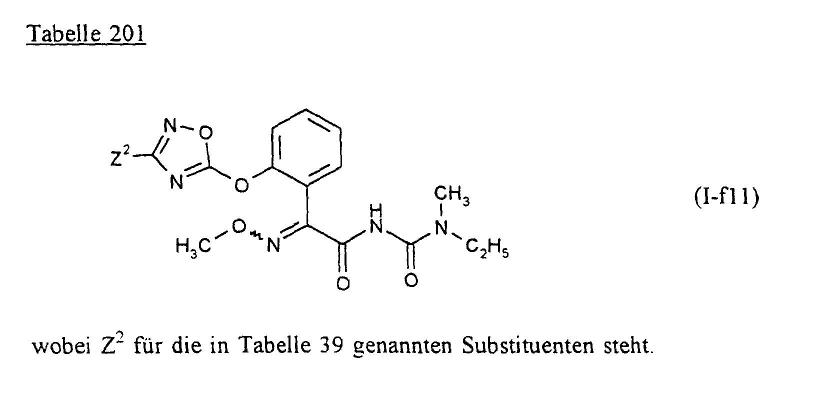

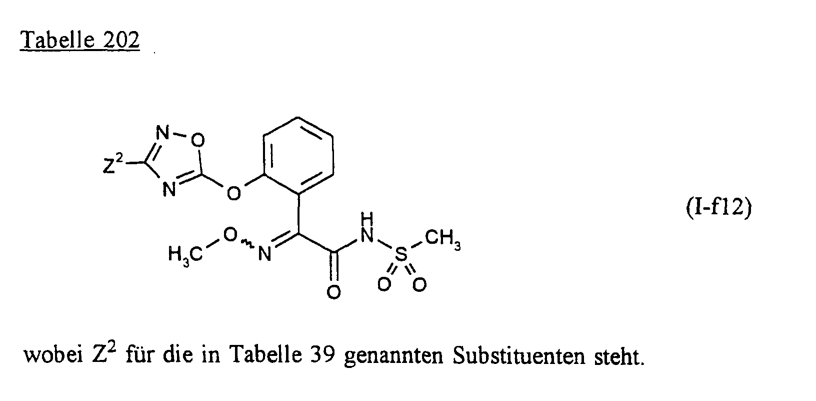

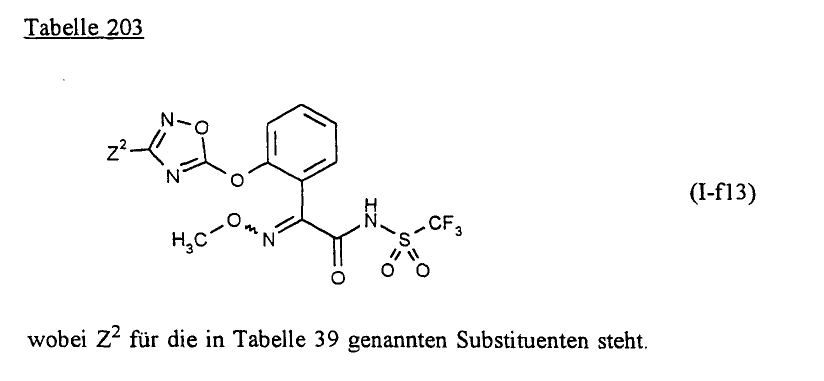

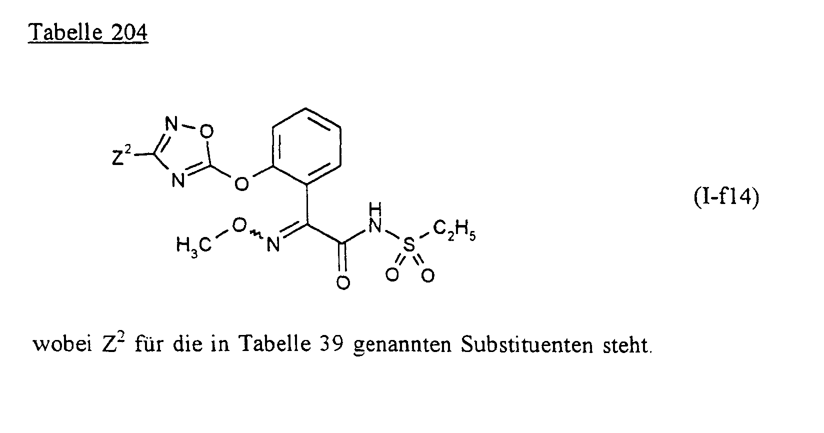

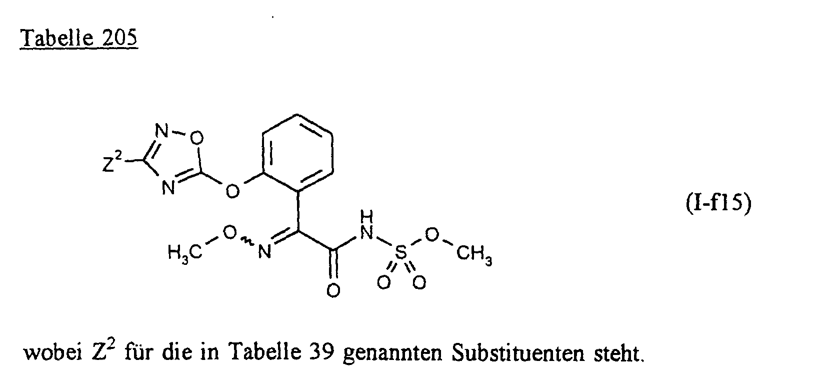

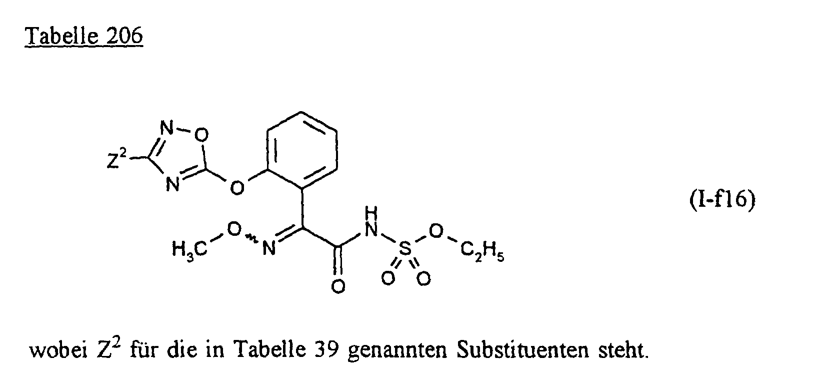

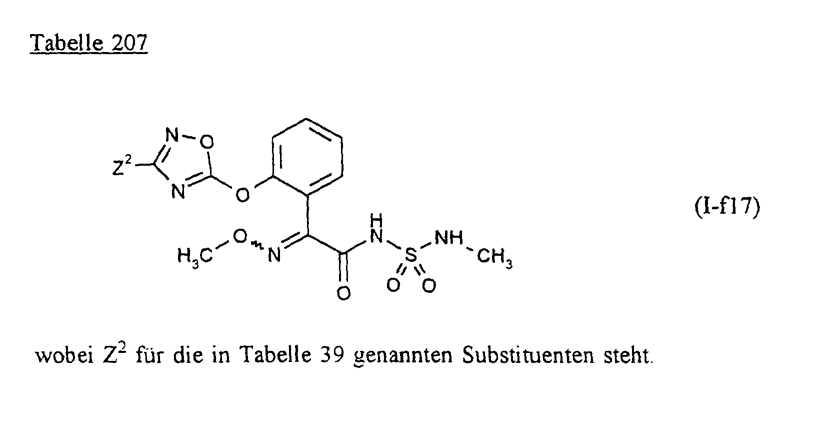

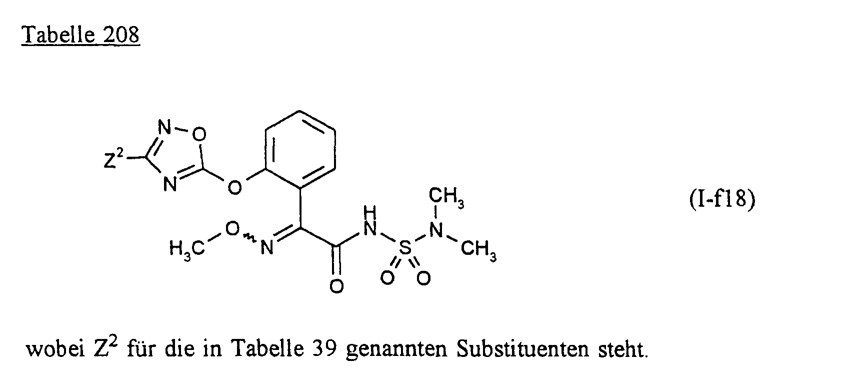

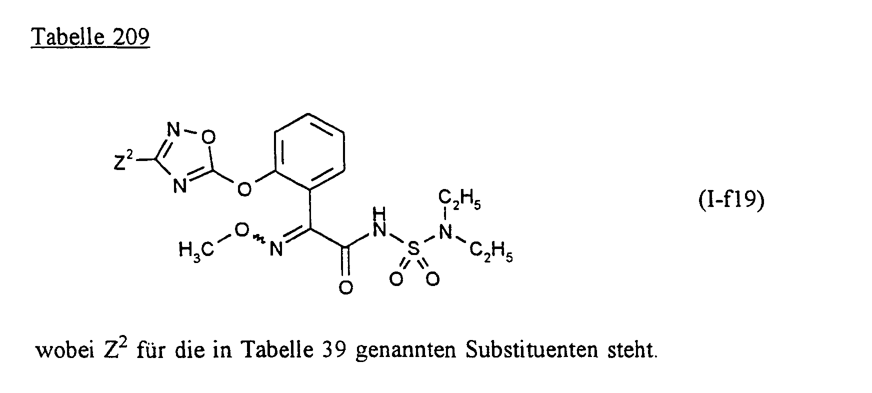

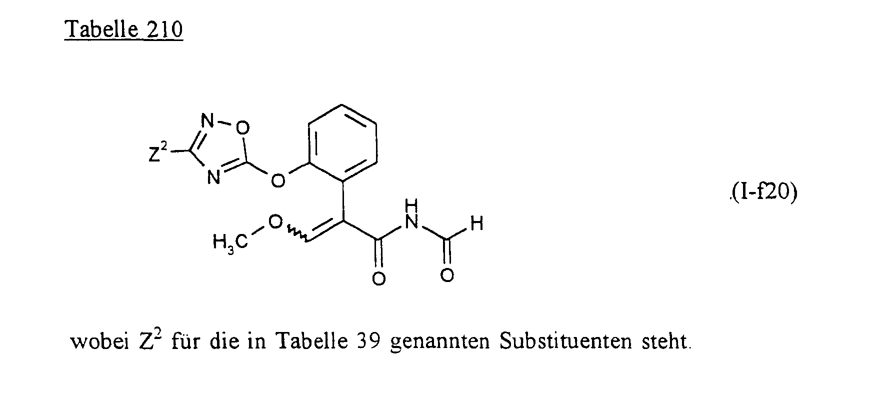

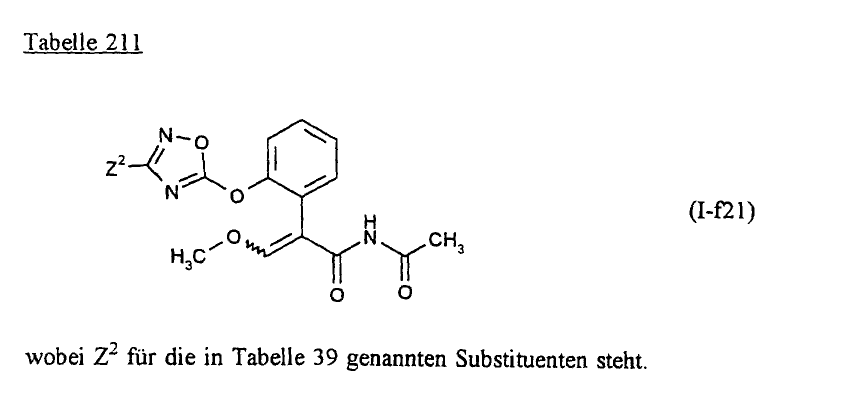

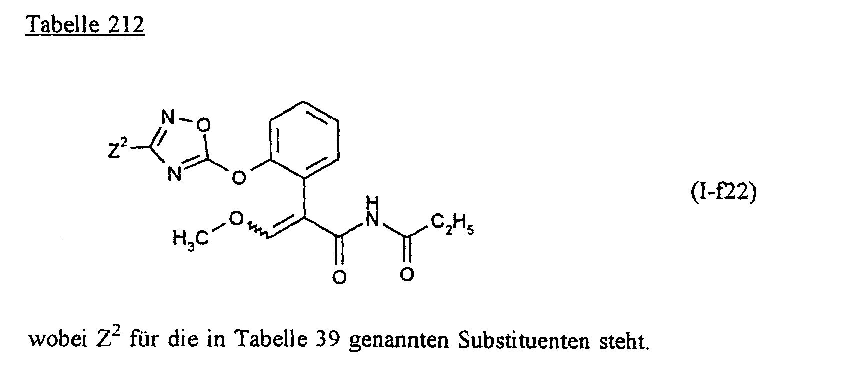

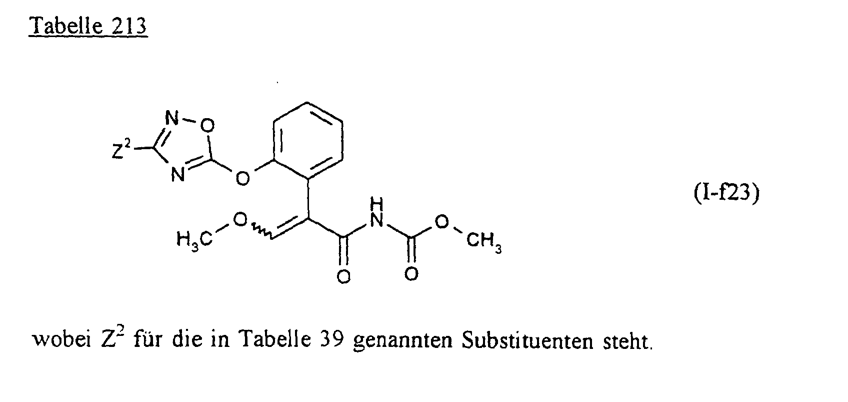

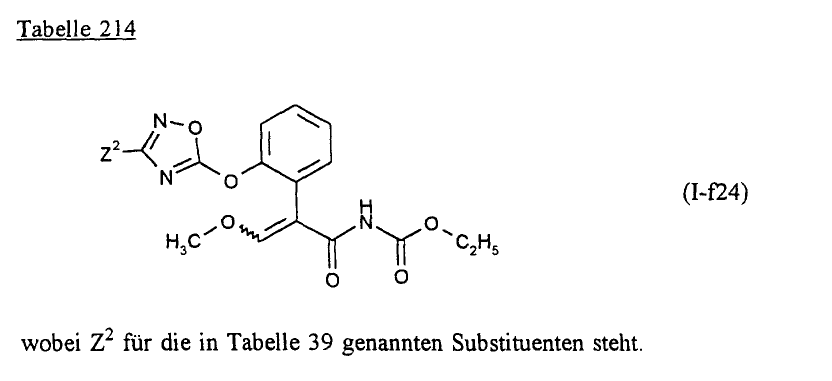

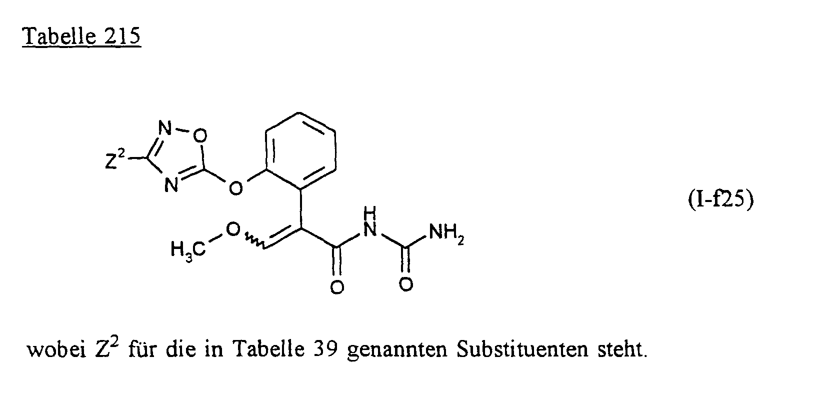

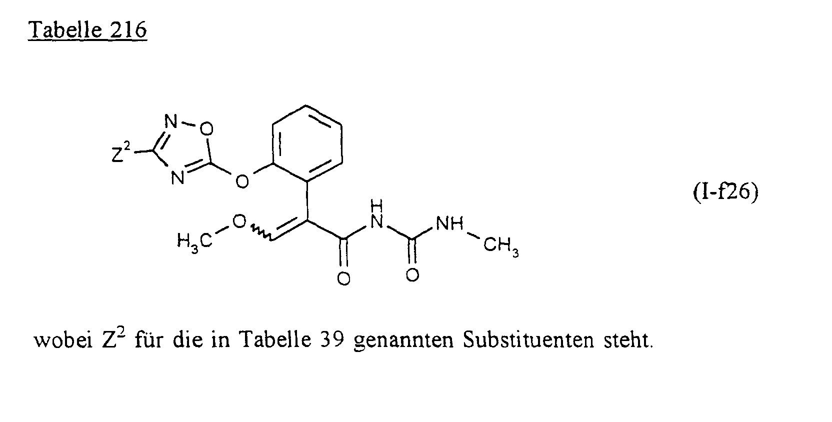

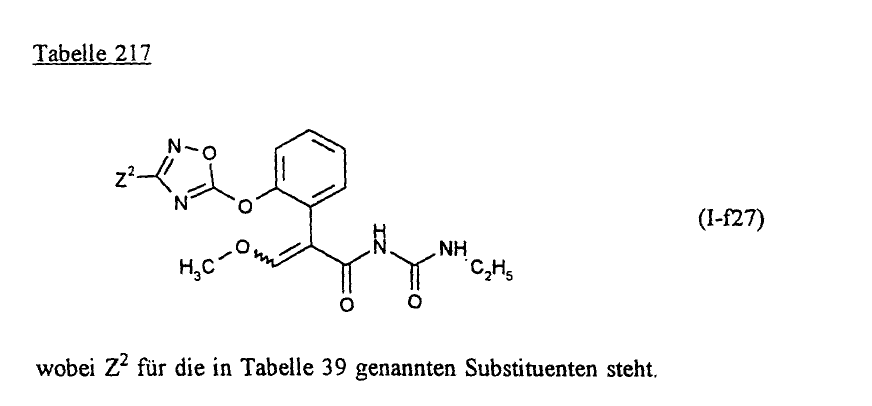

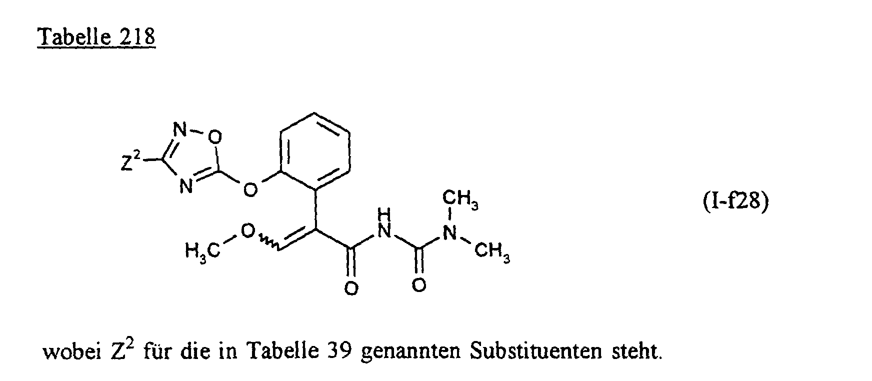

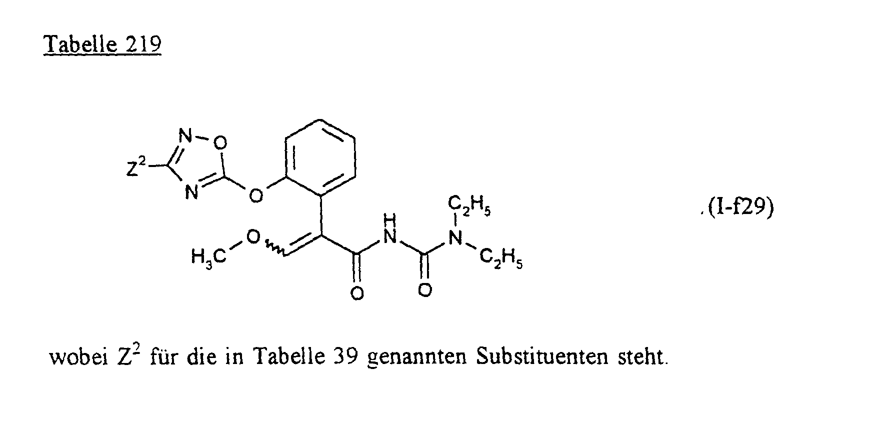

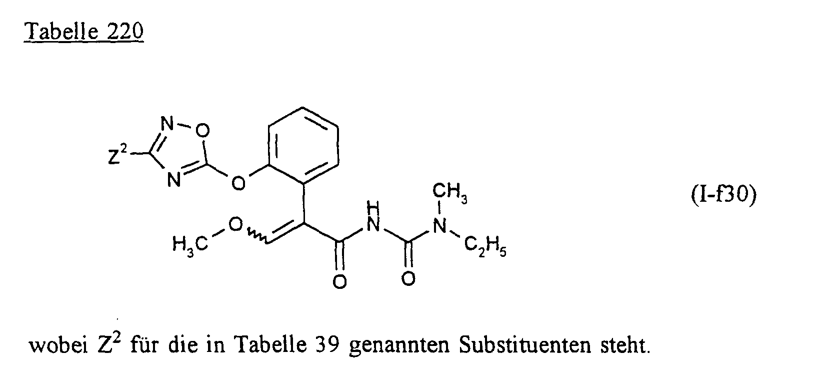

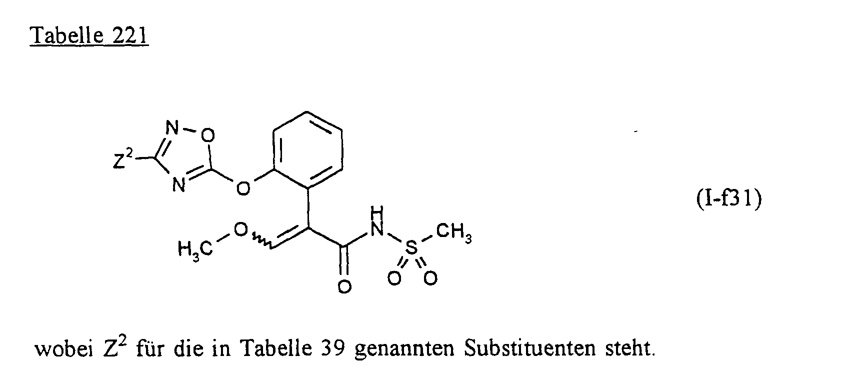

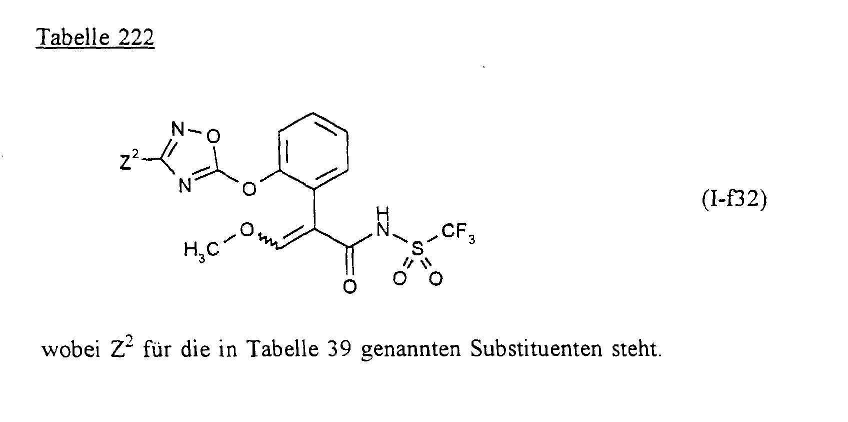

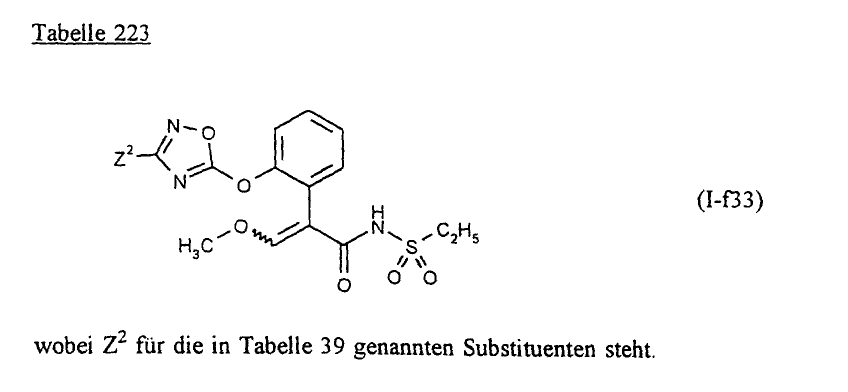

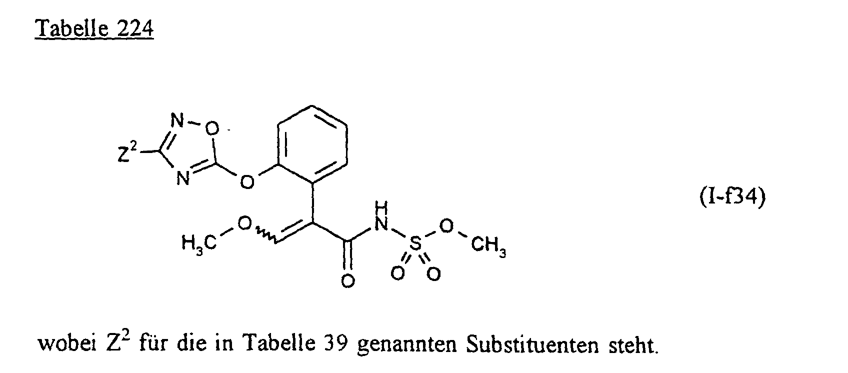

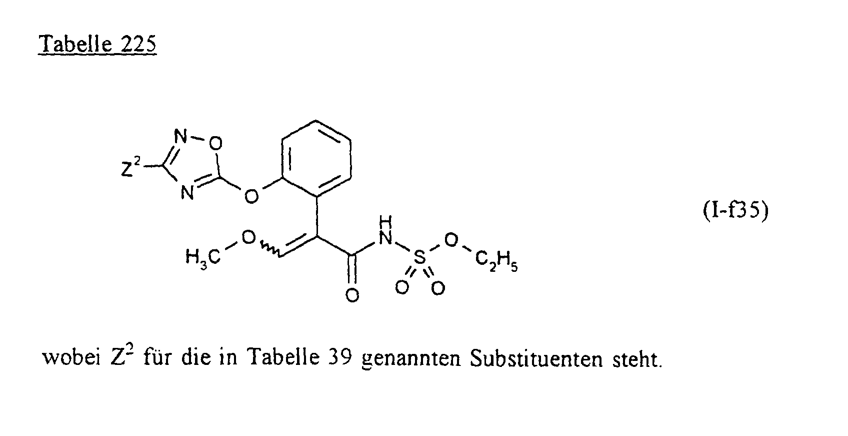

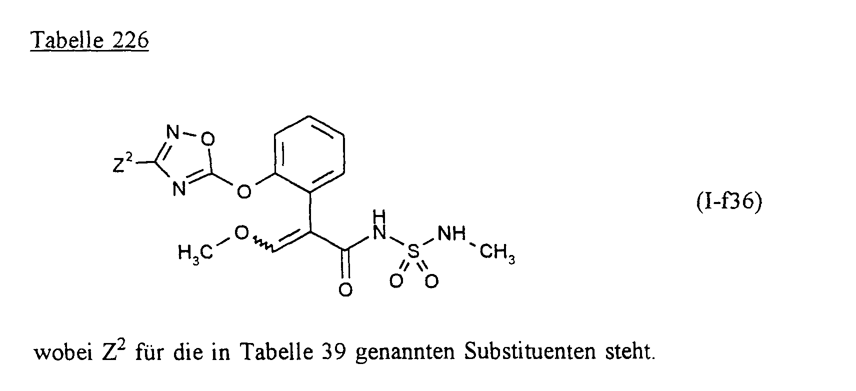

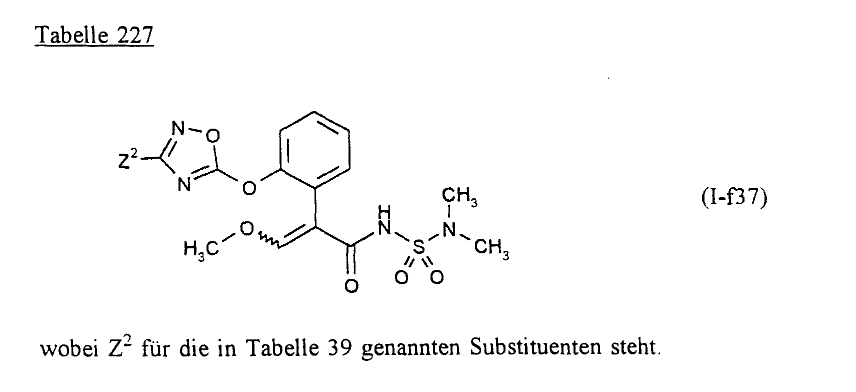

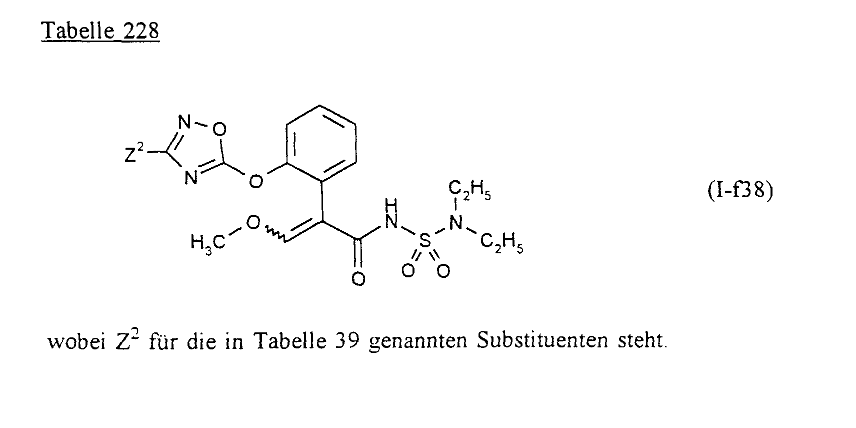

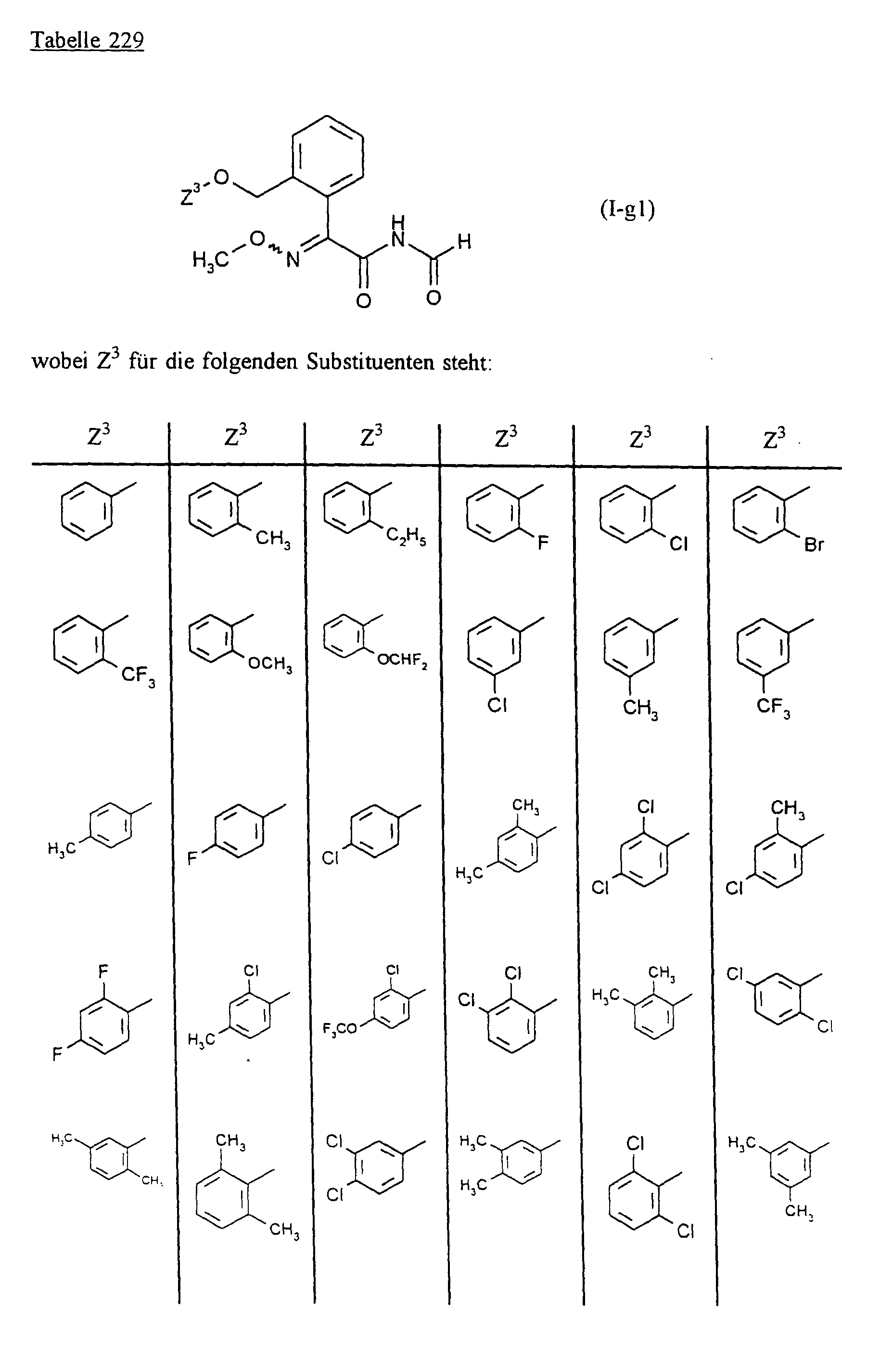

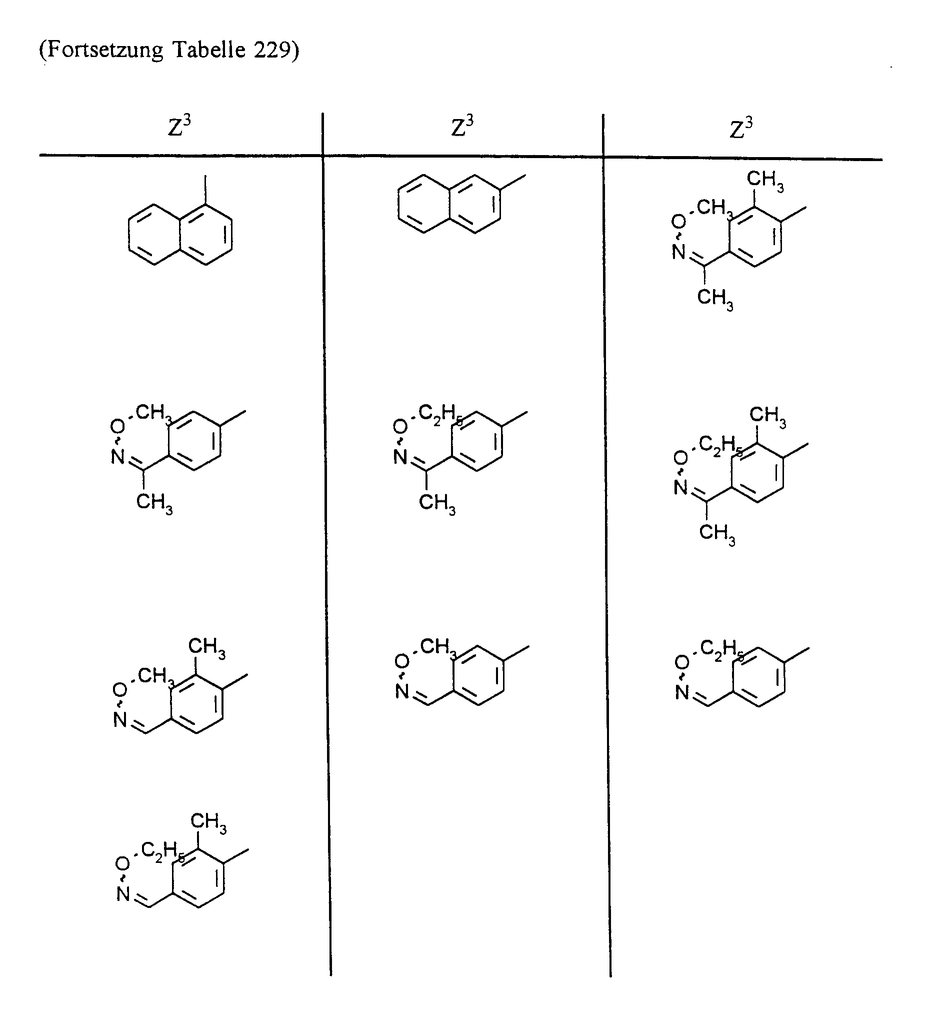

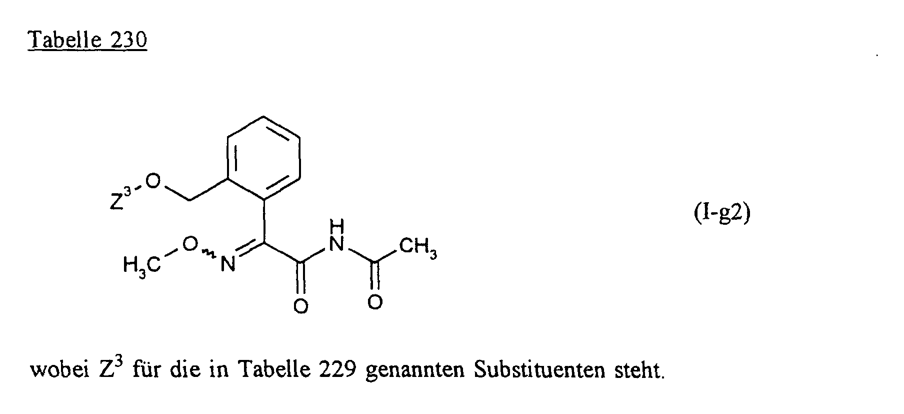

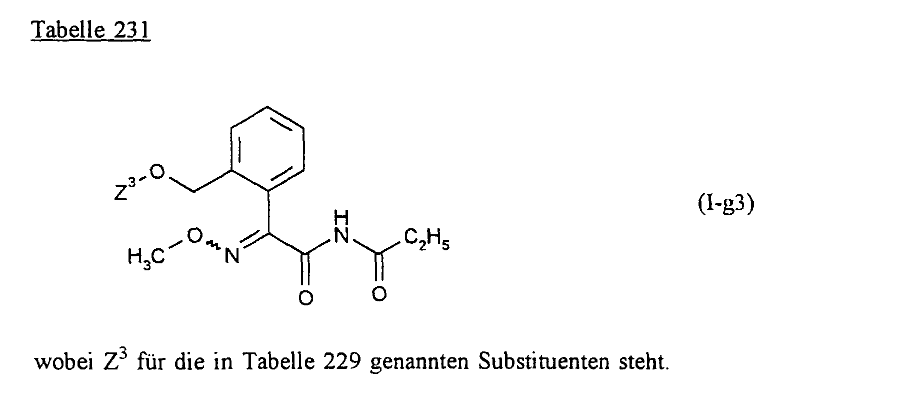

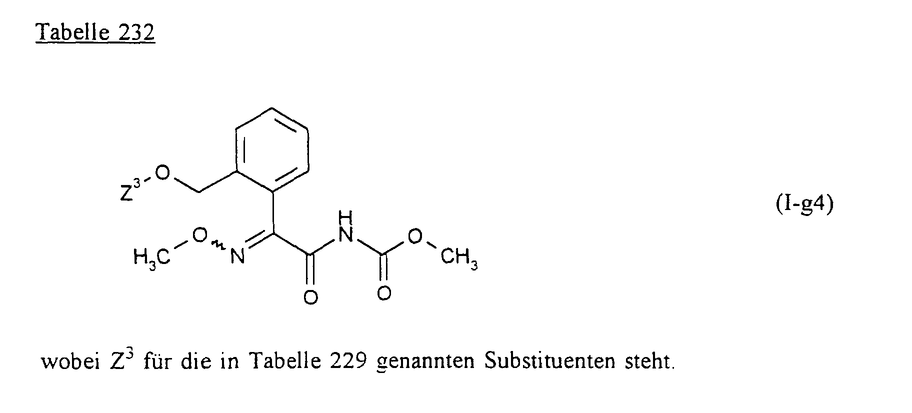

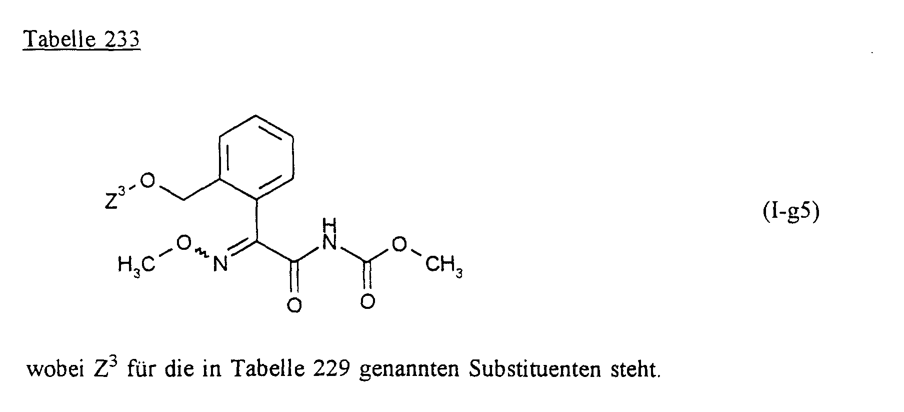

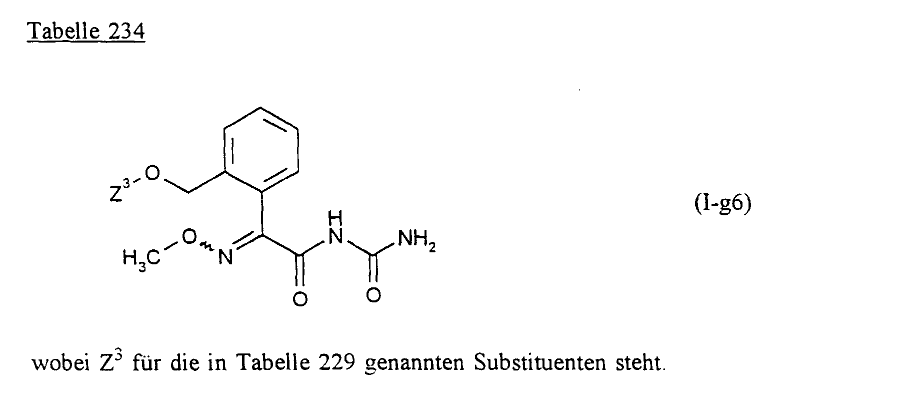

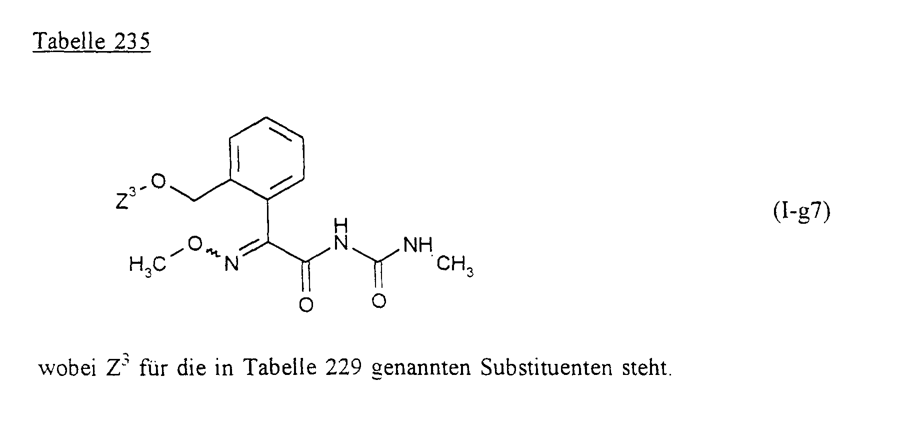

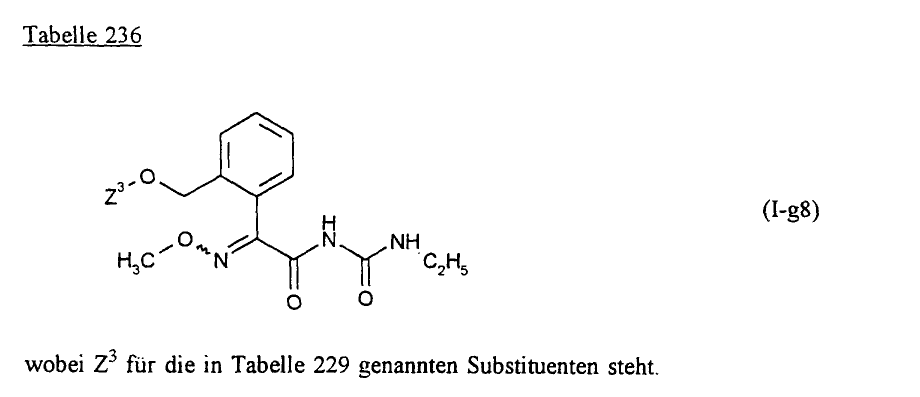

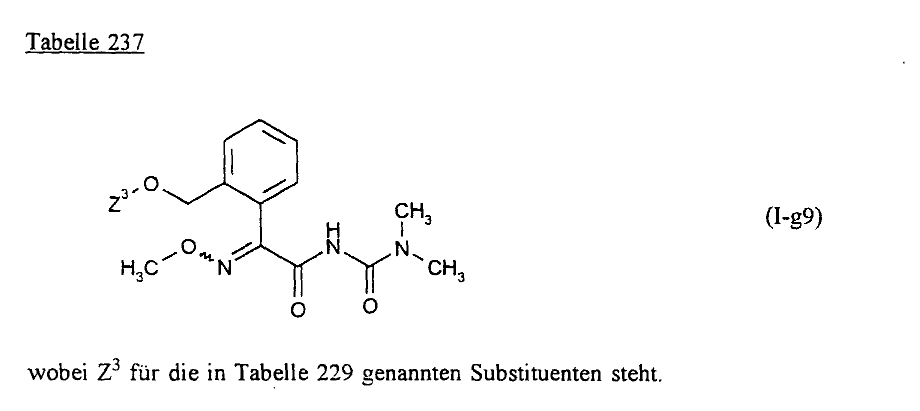

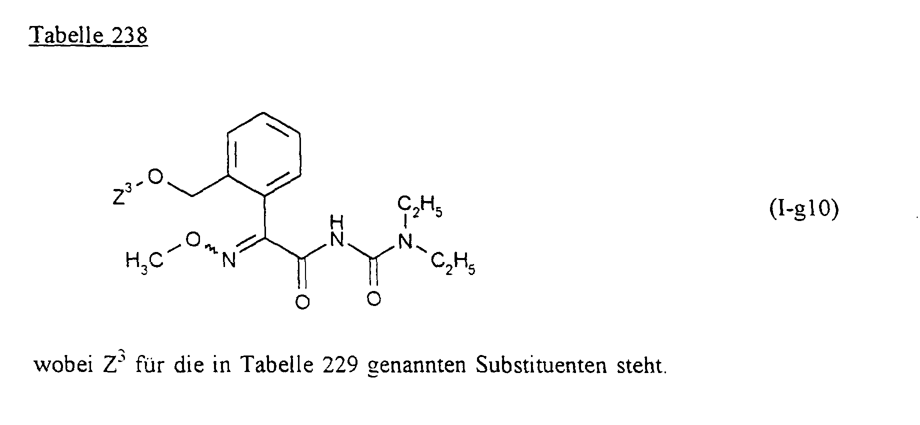

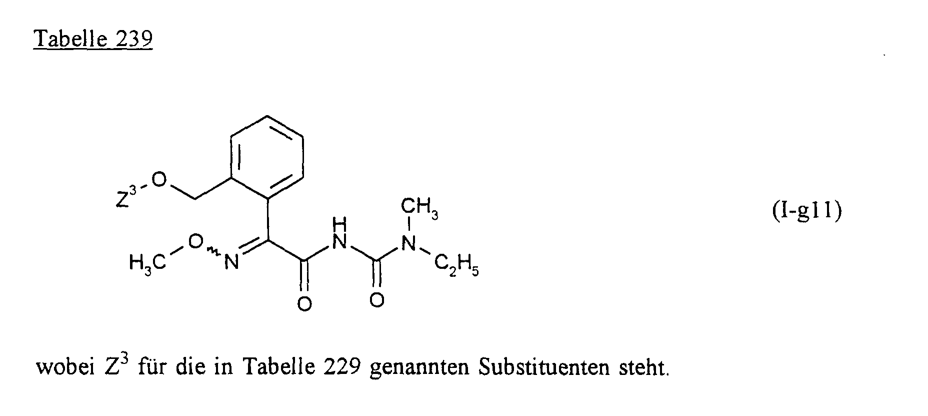

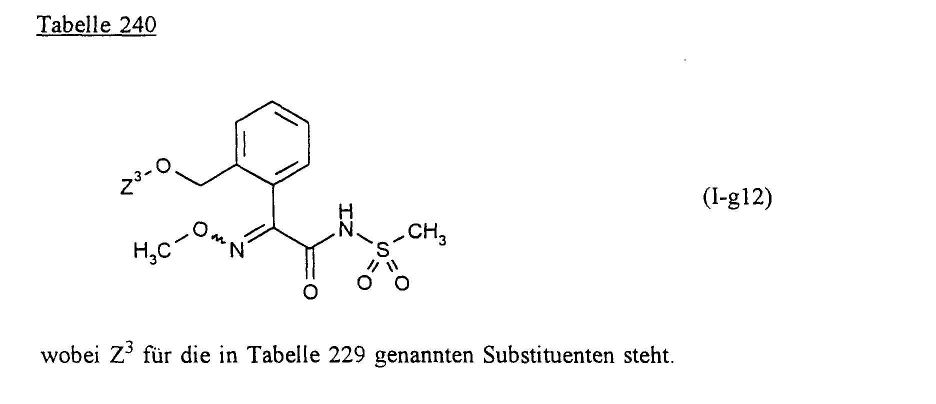

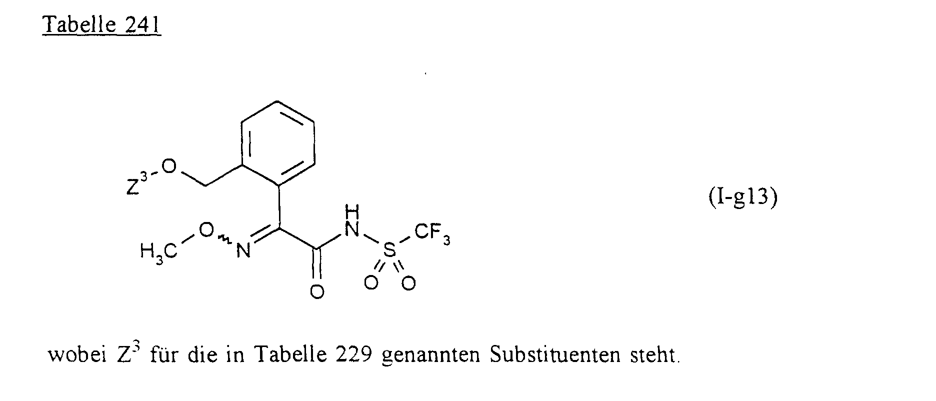

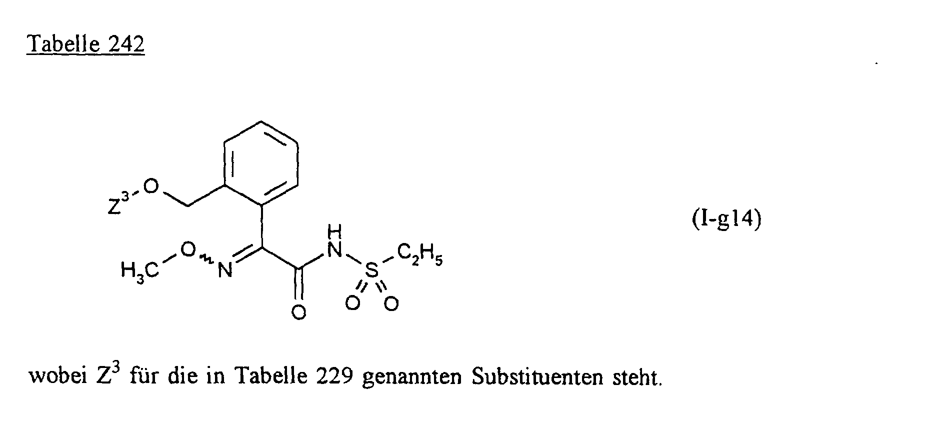

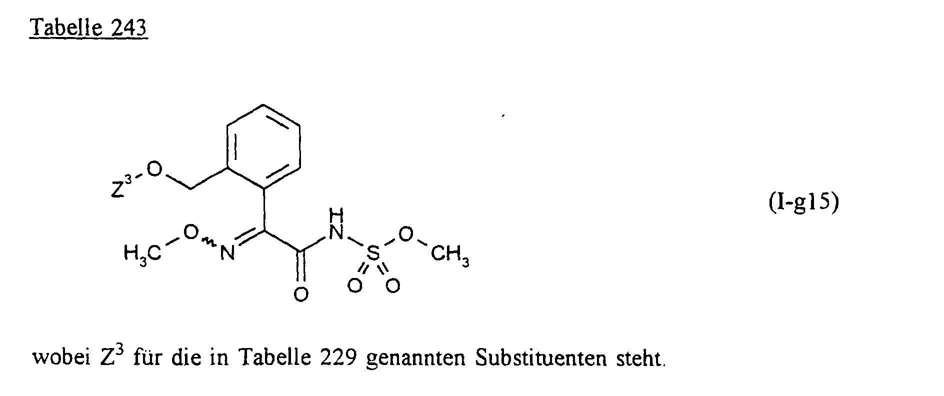

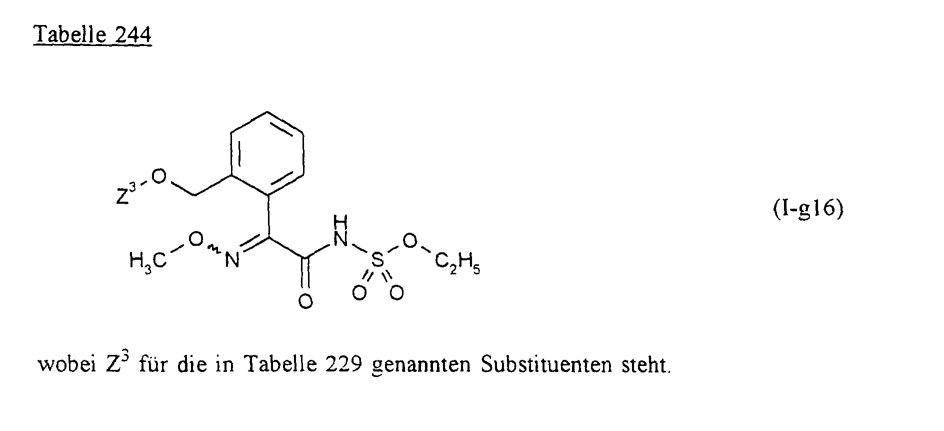

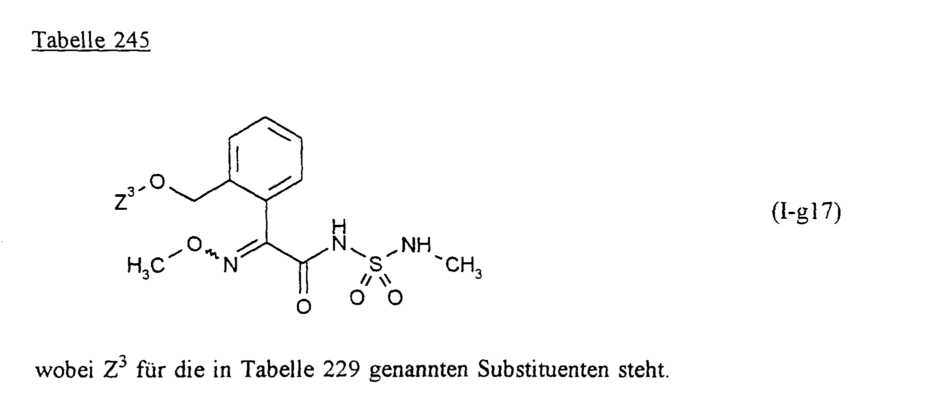

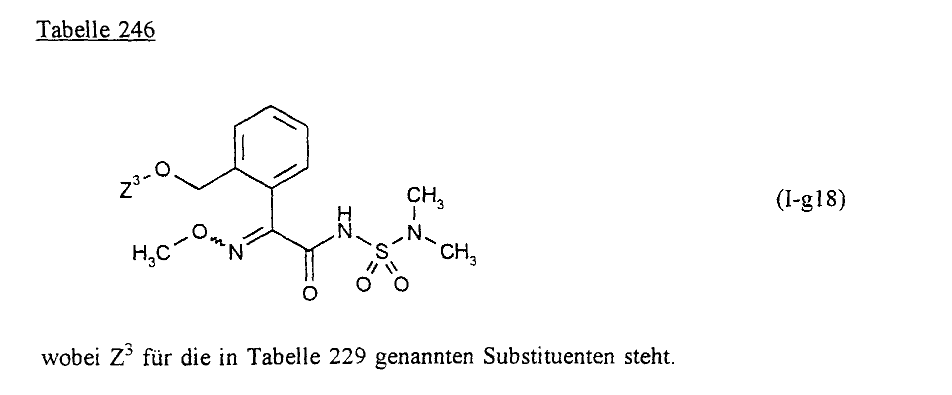

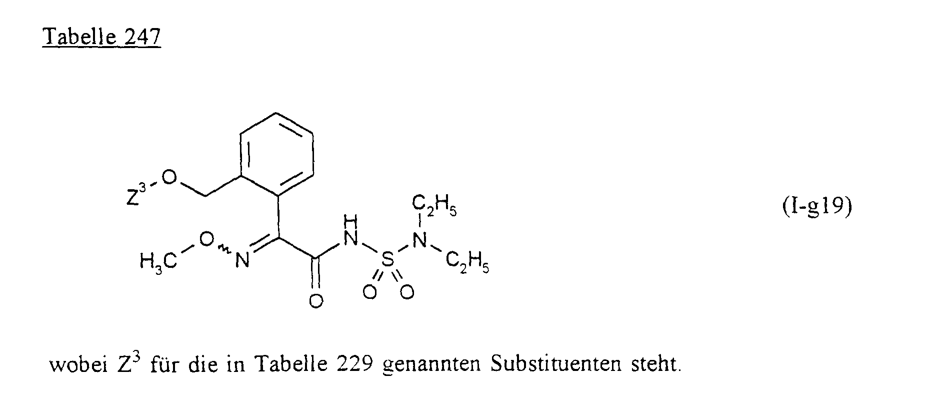

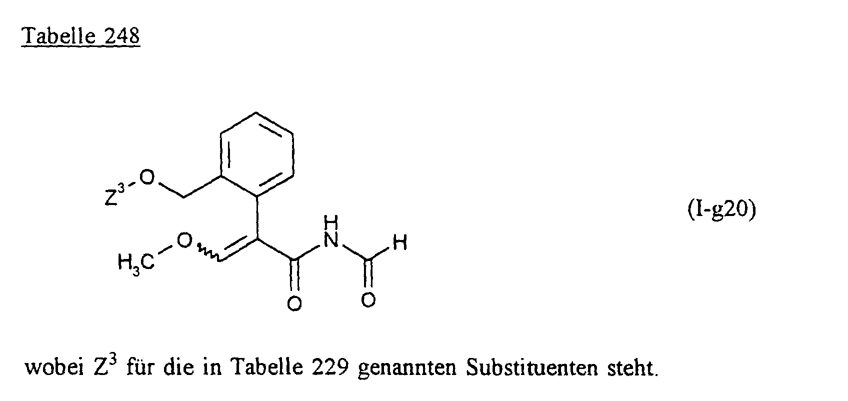

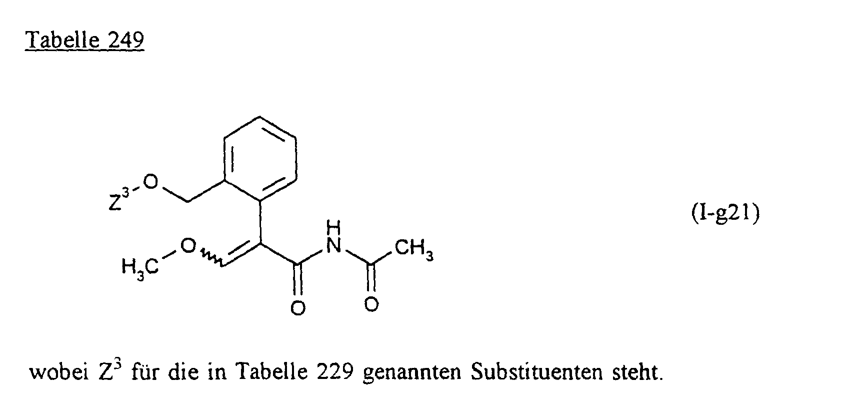

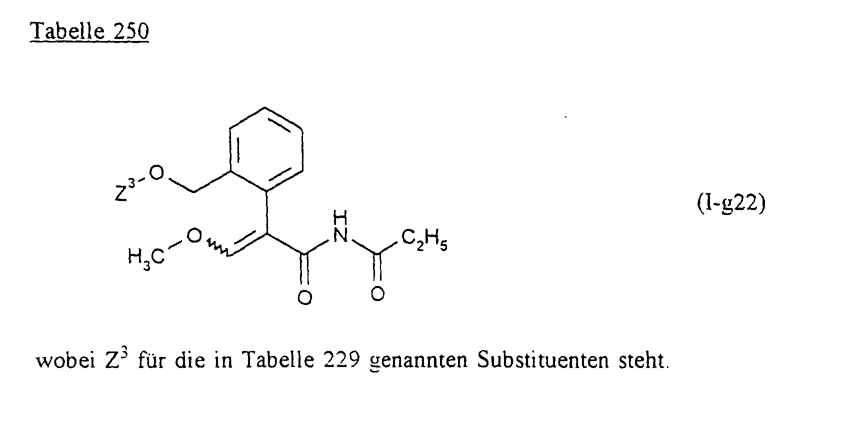

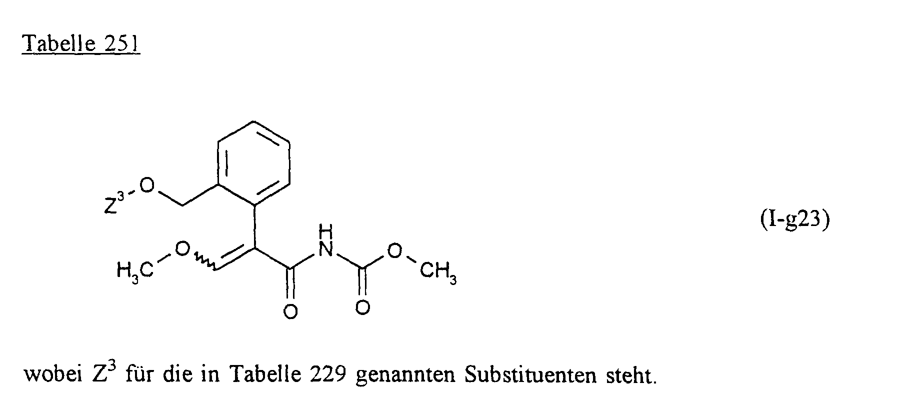

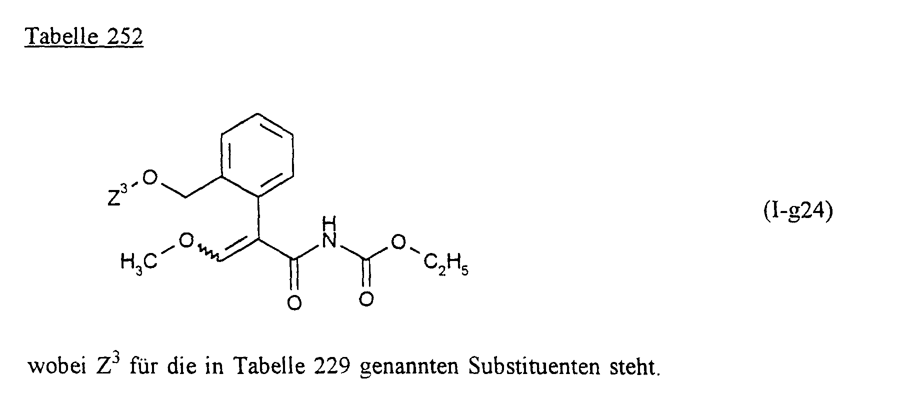

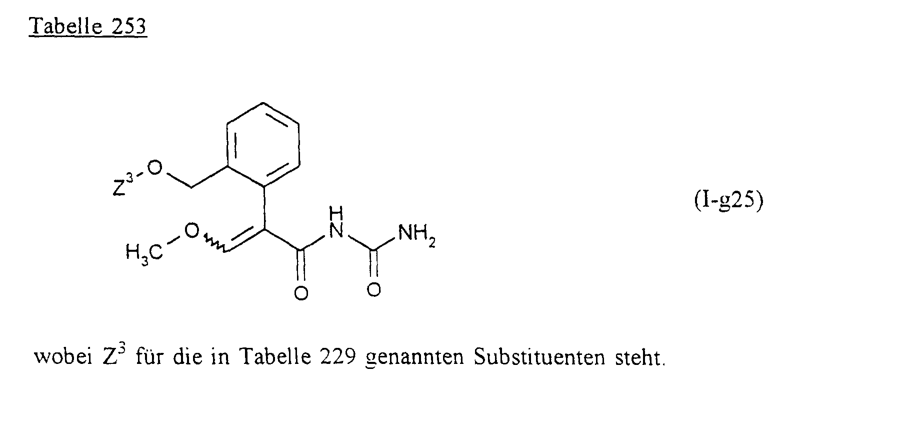

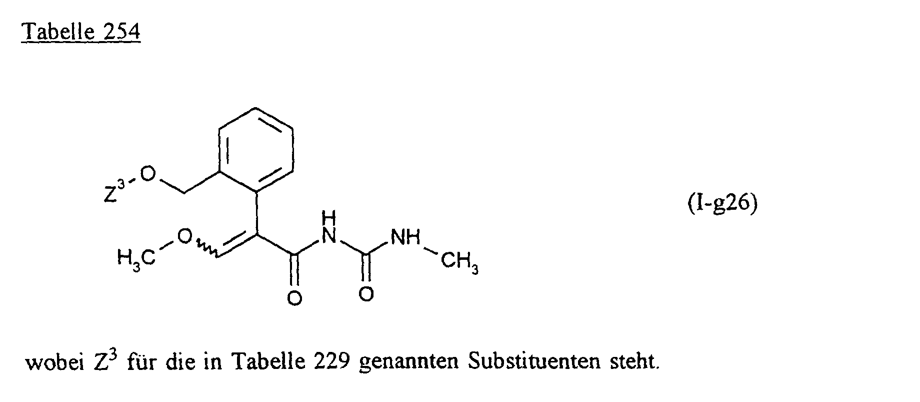

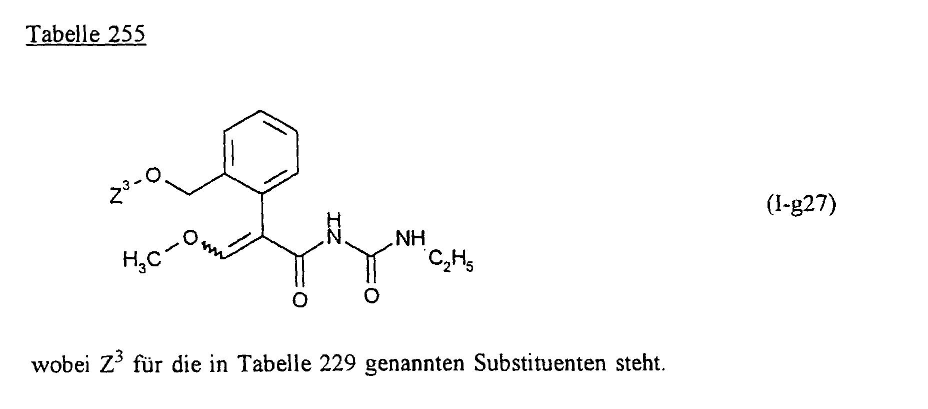

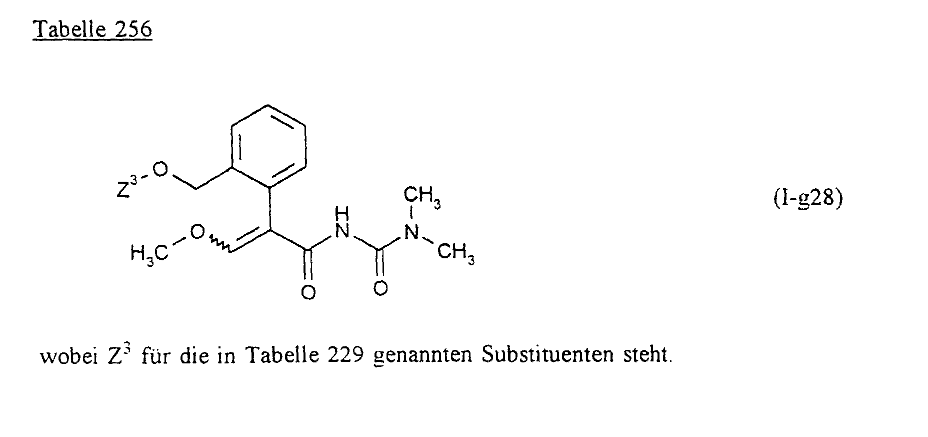

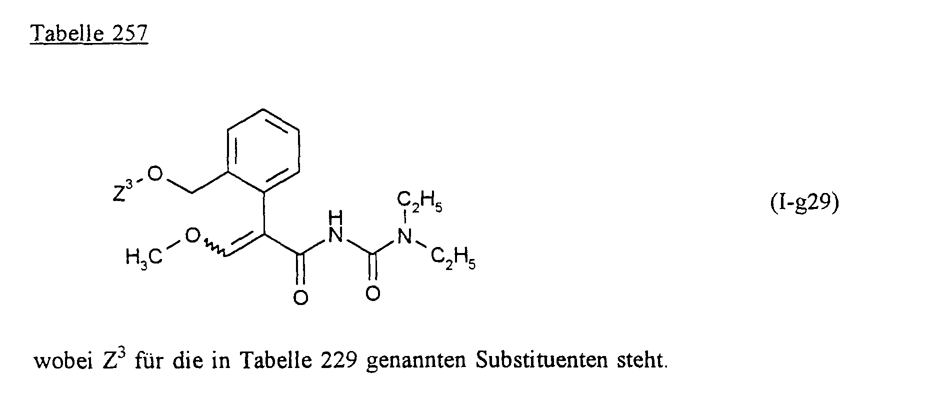

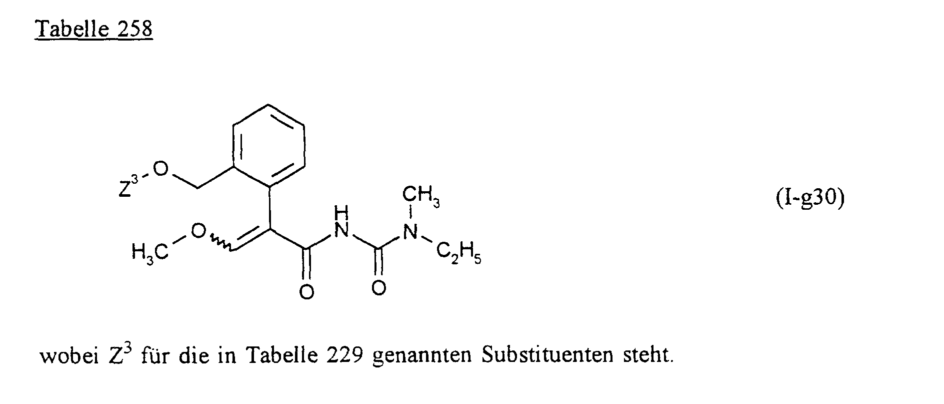

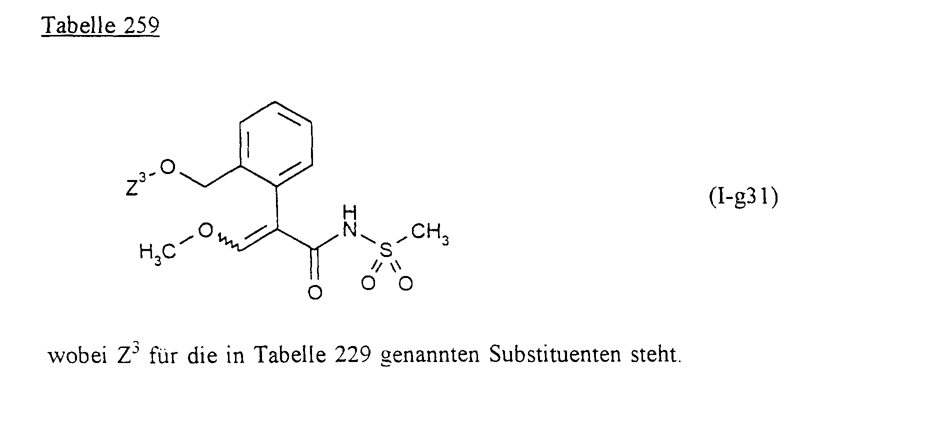

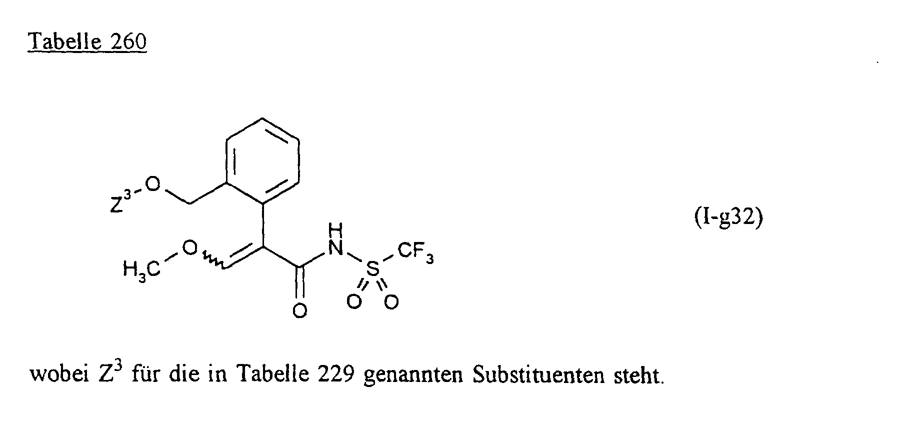

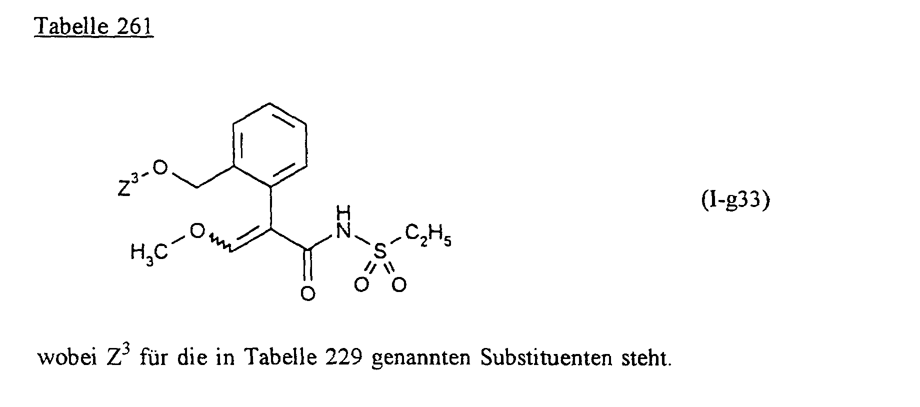

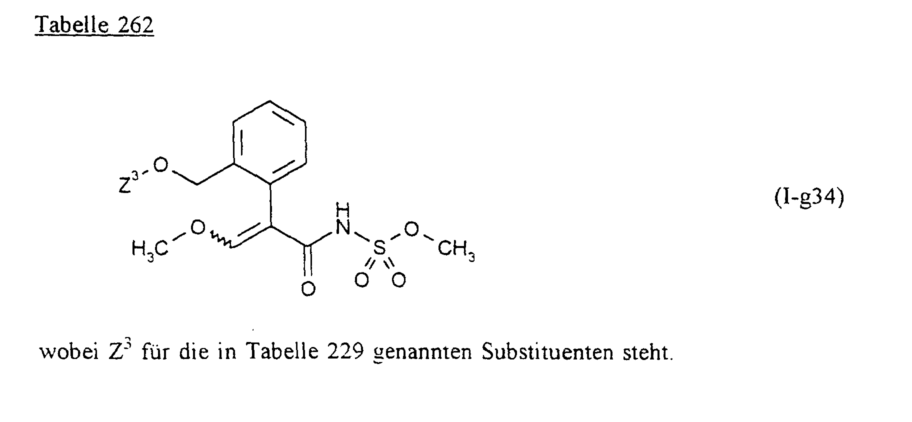

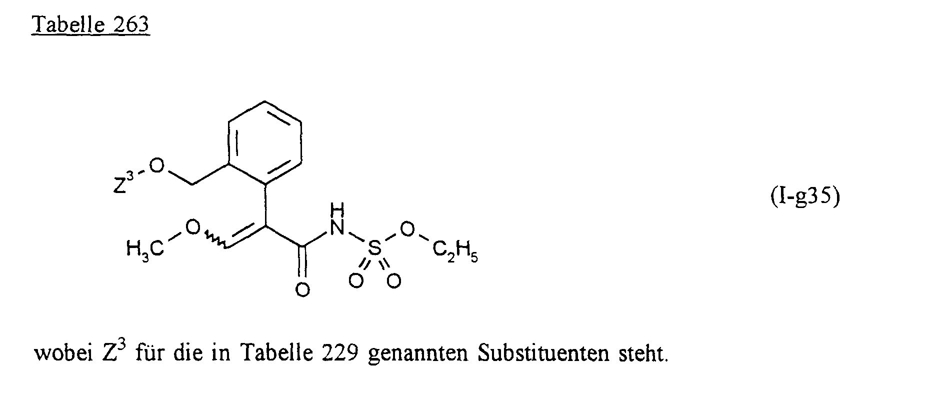

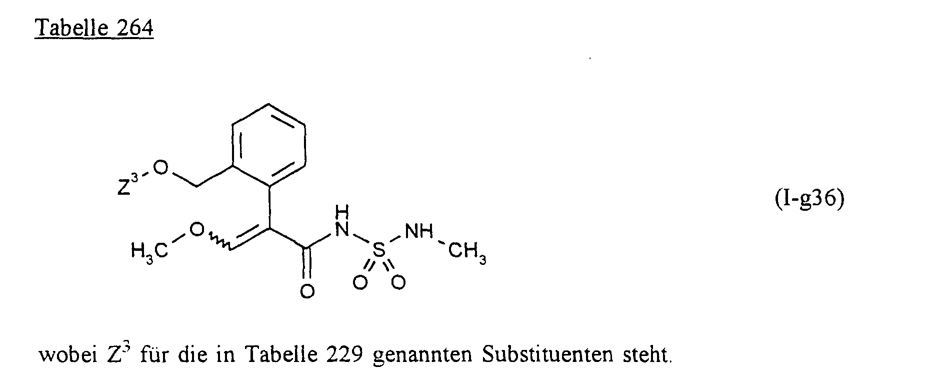

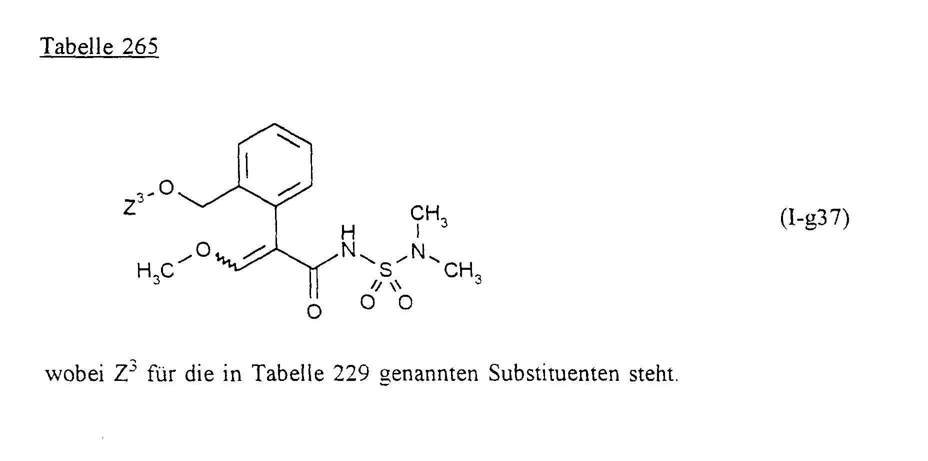

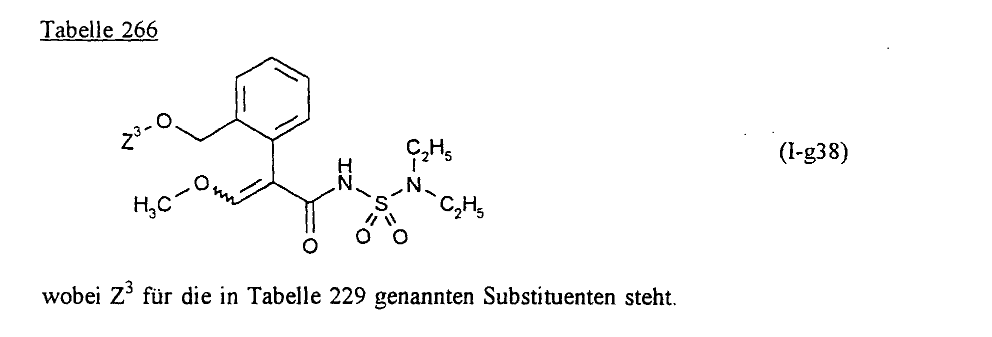

Die zur Durchführung der erfindungsgemäßen Verfahren a), b), c), und d) als Ausgangsstoffe benötigten Carbonsäureamide sind durch die Formel (II) allgemein definiert. In dieser Formel (II) haben Ar, E, G, Q und Z vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für Ar, E, G, Q und Z angegeben wurde.

Die Carbonsäurederivate der Formel (II) sind bekannt und/oder können nach an sich bekannten Verfahren hergestellt werden (vgl. z. B. EP-A 398692 und EP-A 528681).

Die zur Durchführung der erfindungsgemäßen Verfahren a) weiterhin als Ausgangsstoffe benötigten Carbonsäurehalogenide sind durch die Formel (III) allgemein definiert. In dieser Formel (III) hat R vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R angegeben wurde. X¹ steht für Halogen, vorzugsweise Chlor.

Die Carbonsäurehalogenide der Formel (III) sind bekannte Synthesechemikalien.

Die zur Durchführung der erfindungsgemäßen Verfahren b) weiterhin als Ausgangsstoffe benötigten Carbonsäureanhydride sind durch die Formel (IV) allgemein definiert. In dieser Formel (IV) hat R vorzugsweise bzw insbesondere diejenige Bedeutung, die bereits im Zusammenhang mit der Beschreibung der erfindungsgemäßen Verbindungen der Formel (I) als bevorzugt bzw. als insbesondere bevorzugt für R angegeben wurde.

Die Carbonsäureanhydride der Formel (IV) sind bekannte Synthesechemikalien.

Die zur Durchführung des erfindungsgemäßen Verfahrens c) weiterhin als Ausgangsstoffe benötigten Sulfonsäurehalogenide sind durch die Formel (V) allgemein definiert. In dieser Formel (V) hat R vorzugsweise bzw insbesondere diejenige Bedeutung, die bereits im Zusammenhang mit der Beschreibung der erfindungsgemäßen Verbindungen der Formel (I) als bevorzugt bzw. als insbesondere bevorzugt für R angegeben wurde. X² steht für Halogen, vorzugsweise Chlor.

Die Sulfonsäurehalogenide der Formel (III) sind bekannte Synthesechemikalien.

Die zur Durchführung des erfindungsgemäßen Verfahrens d) weiterhin als Ausgangsstoffe benötigten Isocyanate sind durch die Formel (VI) allgemein definiert. R⁶ steht für Alkyl, vorzugsweise für Alkyl mit 1 bis 4 Kohlenstoffatomen, insbesondere für Methyl oder Ethyl.

Die Isocyanate der Formel (VI) sind bekannte Synthesechemikalien.

Die zur Durchführung des erfindungsgemäßen Verfahrens e) als Ausgangsstoffe benötigten Amidine sind durch die Formel (VII) allgemein definiert. In dieser Formel (VII) haben Ar, E, G, Q, R und Z vorzugsweise bzw. insbesondere diejenige Bedeutung, die bereits im Zusammenhang mit der Beschreibung der erfindungsgemäßen Verbindungen der Formel (I) als bevorzugt bzw als insbesondere bevorzugt für Ar, E, G, Q, R und Z angegeben wurde. R⁷ steht für Dialkylamino, vorzugsweise für Dimethylamino.

Die Amidine der Formel (VII) sind noch nicht bekannt, sie werden erhalten, wenn man Carbonsäureamide der Formel (II) mit einem Orthocarbonsäurederivat der Formel (VIII), 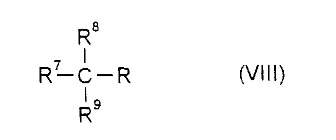 in welcher
- R und R⁷: die oben angegebenen Bedeutungen haben und
- R⁸ und R⁹: gleich oder verschieden sind und für Dialkylamino, vorzugsweise Dimethylamino, oder Alkoxy, vorzugsweise Methoxy, Ethoxy, i-Propoxy oder t-Butoxy stehen,
bevorzugt ohne, gegebenenfalls aber auch in Gegenwart eines Verdünnungsmittels, vorzugsweise eines Kohlenwasserstoffes, wie beispielsweise Toluol, oder eines Amides, wie N,N-Dimethylformamid, oder eines Alkoholes, wie Methanol oder Ethanol, bei Temperaturen zwischen 0°C und +200°C, vorzugsweise bei Temperaturen zwischen 20°C und 150°C, umsetzt.

In einer bevorzugten Ausführungsform werden die Amidine der Formel (VII) ohne Aufarbeitung gleich weiter, gemäß Verfahren e), umgesetzt.

Die Orthocarbonsäurederivate der Formel (VIII) sind bekannte Synthesechemikalien.

Als Verdünnungsmittel zur Durchführung der erfindungsgemäßen Verfahren a), b), c), d) und e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid und Sulfone, wie Sulfolan.

Als Verdünnungsmittel zur Durchführung des erfindungsgemäßen Verfahrens e) kommen außerdem auch Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser infrage.

Die erfindungsgemäßen Verfahren a), b), und c) werden gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren d) wird gegebenenfalls in Gegenwart eines geeigneten Katalysators durchgeführt. Als solche kommen beispielsweise alle üblichen organischen Basen infrage. Hierzu gehören beispielsweise tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren e) wird gegebenenfalls in Gegenwart einer Säure durchgeführt. Als solche kommen alle anorganischen und organischen, sowie auch alle polymeren Säuren infrage. Hierzu gehören beispielsweise Chlorwasserstoff, Schwefelsäure Phosphorsäure, Ameisensäure, Essigsäure, Methansulfonsäure, Trifluoressigsäure, Trifluormethansulfonsäure, Toluolsulfonsäure, saure Ionenaustauscher und saures Kieselgel.

Die Reaktionstemperaturen können bei der Durchführung der erfindungsgemäßen Verfahren a), b), c), und d) in einem größeren Bereich variiert werden. Im allgemeinen arbeitet man bei Temperaturen zwischen -20°C und 200°C, vorzugsweise bei Temperaturen zwischen 20°C und 180°C

Das erfindungsgemäße Verfahren e) wird im allgemeinen bei Temperaturen zwischen -20°C und +180°C, vorzugsweise bei Temperaturen zwischen 0°C und 150°C durchgeführt.

Zur Durchführung des erfindungsgemäßen Verfahrens a) zur Herstellung der Verbindungen der Formel (I) setzt man pro Mol des Carbonsäureamids der Formel (II) im allgemeinen 1 bis 15 Mol, vorzugsweise 1 bis 8 Mol an Carbonsäurehalogenid der Formel (III) ein.

Zur Durchführung des erfindungsgemäßen Verfahrens b) zur Herstellung der Verbindungen der Formel (I) setzt man pro Mol des Carbonsäureamids der Formel (II) im allgemeinen 1 bis 15 Mol, vorzugsweise 1 bis 8 Mol an Carbonsäureanhydrid der Formel (IV) ein.

Zur Durchführung des erfindungsgemäßen Verfahrens c) zur Herstellung der Verbindungen der Formel (I) setzt man pro Mol des Carbonsäureamids der Formel (II) im allgemeinen 1 bis 15 Mol, vorzugsweise 1 bis 8 Mol an Sulfonsäurehalogenid der Formel (V) ein.

Zur Durchführung des erfindungsgemäßen Verfahrens d) zur Herstellung der Verbindungen der Formel (I) setzt man pro Mol des Carbonsäureamids der Formel (II) im allgemeinen 1 bis 15 Mol, vorzugsweise 1 bis 8 Mol an Isocyanat der Formel (VI) ein.

Zur Durchführung des erfindungsgemäßen Verfahrens e) zur Herstellung der Verbindungen der Formel (I) setzt man pro Mol des Amidins der Formel (VII) im allgemeinen 1 bis 50 Mol, vorzugsweise 1 bis 20 Mol an Säure ein.

Die erfindungsgemäßen Verfahren a), b), c), d) und e) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. auch die Herstellungsbeispiele).

Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Getreidekrankheiten, wie beispielsweise gegen Erysiphe-Arten oder von Krankheiten im Wein-, Obst- und Gemüseanbau, wie beispielsweise gegen Venturia-, Podospherea- und Plasmopara-Arten eingesetzt. Mit gutem Erfolg werden mit den erfindungsgemäßen Wirkstoffen auch Reiskrankheiten, wie beispielsweise Pyricularia-Arten, und weitere Getreidekrankheiten, wie beispielsweise Leptosphaeria-, Cochliobolus- und Pyrenophora-Arten bekämpft.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor. Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157
419, CGA 184699, Chloethocarb, Chlorethoxyfos, Chloretoxyfos, Chlorfenvinphos.
Chlorfluazuron, Chlormephos, Chlorpyrifos, Chlorpyrifos M, Cis-Resmethrin,
Clocythrin, Clofentezin, Cyanophos; Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb,
Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos,
Methamidophos, Methidathion, Methiocarb, Methomyl, Metolcarb, Milbemectin,
Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet,
Phosphamdon, Phoxim, Pirimicarb, Pirimiphos M, ,Primiphos A, Profenofos,
Profenophos, Promecarb, Propaphos, Propoxur, Prothiofos, Prothiophos, Prothoat.
Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0.0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel (1)

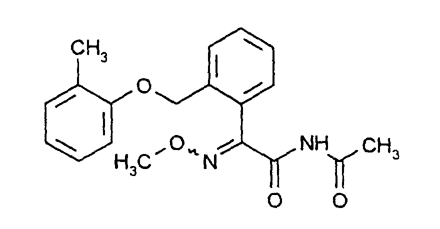

### (Verfahren a))

0,2 g (0,00195 Mol) Acetanhydrid und 0,4 g (0,0013 Mol) 2-Methoximino-2-[2-(2-methylphenoxymethyl)-phenyl]-acetamid werden in 10 ml Dioxan 18 Stunden unter Rückfluß erhitzt. Nach dem Abkühlen gießt man die Mischung auf Eiswasser und extrahiert mit Essigsäureethylester. Die organische Phase wird über Natriumsulfat getrocknet und im Vakuum eingeengt, wobei ein hochviskoses Öl zurückbleibt. Man erhält 0,3 g (68% der Theorie) N-Acetyl-2-methoximino-2-[2-(2-methylphenoxymethyl)-phenyl]-acetainid.
1H-NMR (CDCl3, TMS): δ = 4,0 (s, 3H) ppm.

### Beispiel (2)

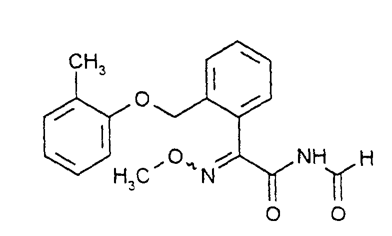

### (Verfahren e))

1 g (0,006 Mol) t-Butyloxy-bis-(dimethylamino)-methan und 0,89 g (0,003 Mol) 2-Methoximino-2-[2-(2-methylphenoxymethyl)-phenyl]-acetamid werden 2 Stunden unter Rückfluß erhitzt. Nach dem Abkühlen versetzt man die Mischung mit 30 ml 10%iger Salzsäure und extrahiert mit Essigsäureethylester. Die organische Phase wird über Natriumsulfat getrocknet und im Vakuum eingeengt. Man erhält 0,8 g (82,5% der Theorie) N-Formyl-2-methoximino-2-[2-(2-methylphenoxymethyl)-phenyl]-acetamid.
¹H-NMR(CDCl₃, TMS): δ = 2,19 (s, 3H); 3,99 (s, 3H); 4,96 (s, 2H)

Analog den Beispielen (1) und (2), sowie entsprechend der allgemeinen Beschreibung der erfindungsgemäßen Herstellungsverfahren, erhält man auch die in der nachstehenden Tabelle 267 aufgeführten erfindungsgemäßen Verbindungen der Formel (I-h):

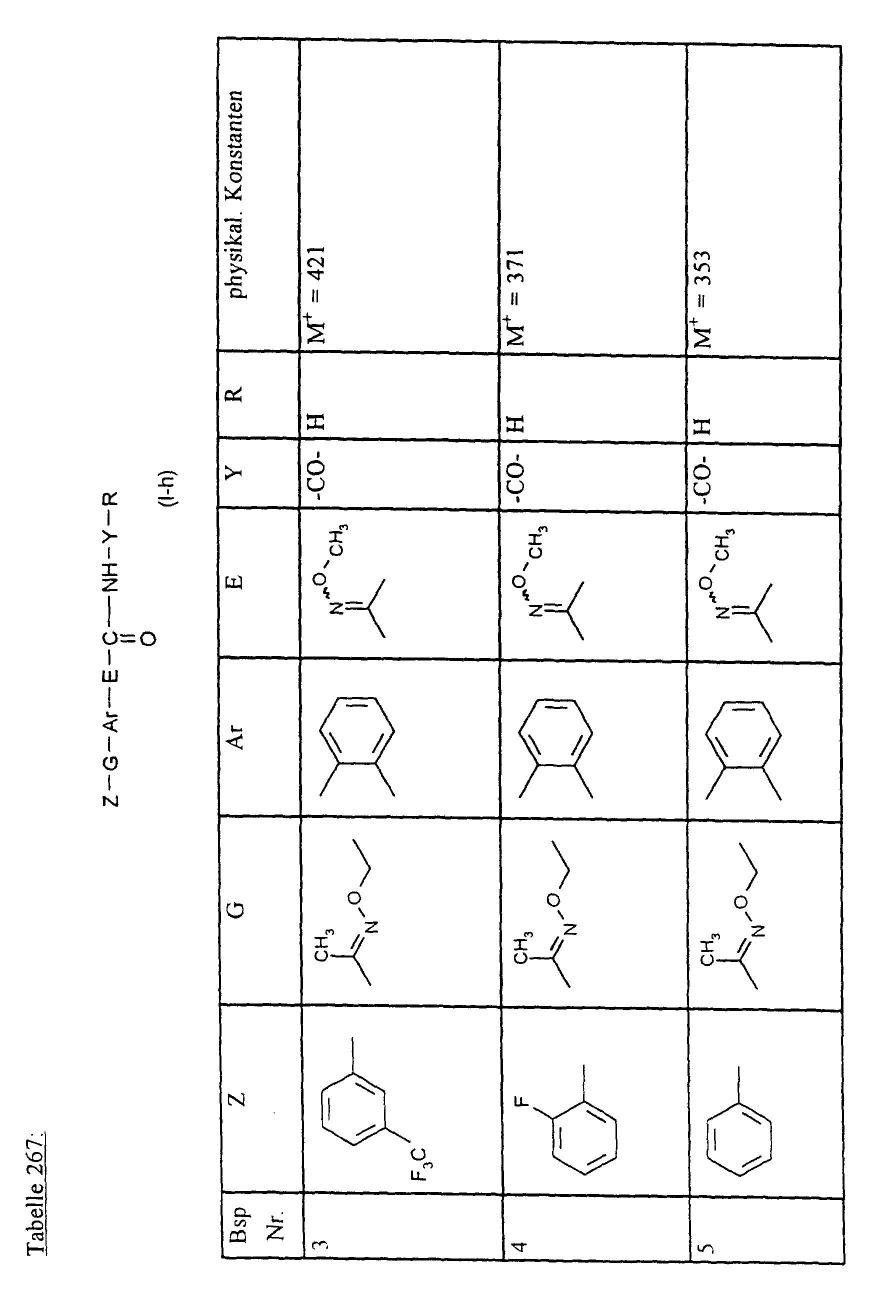

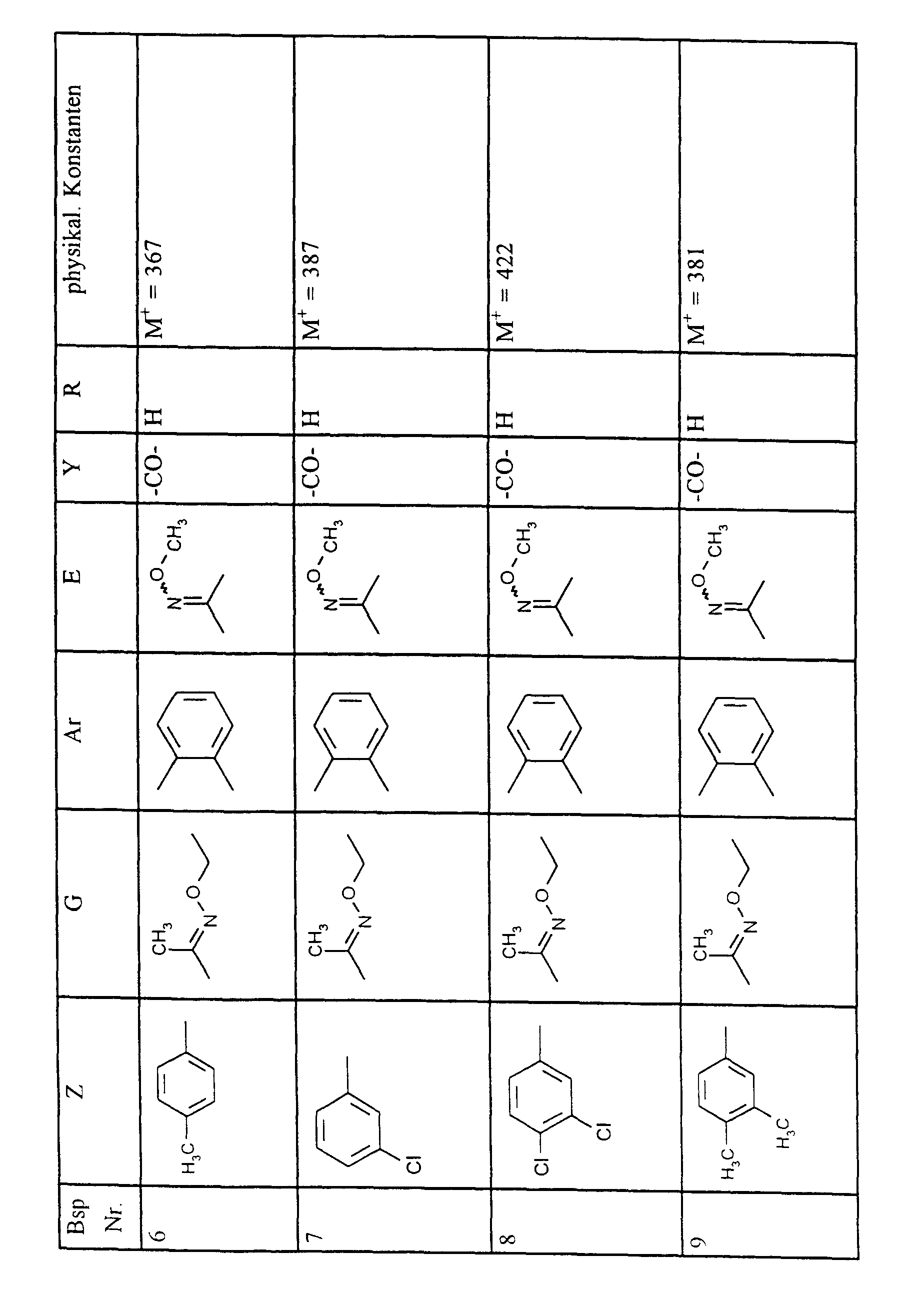

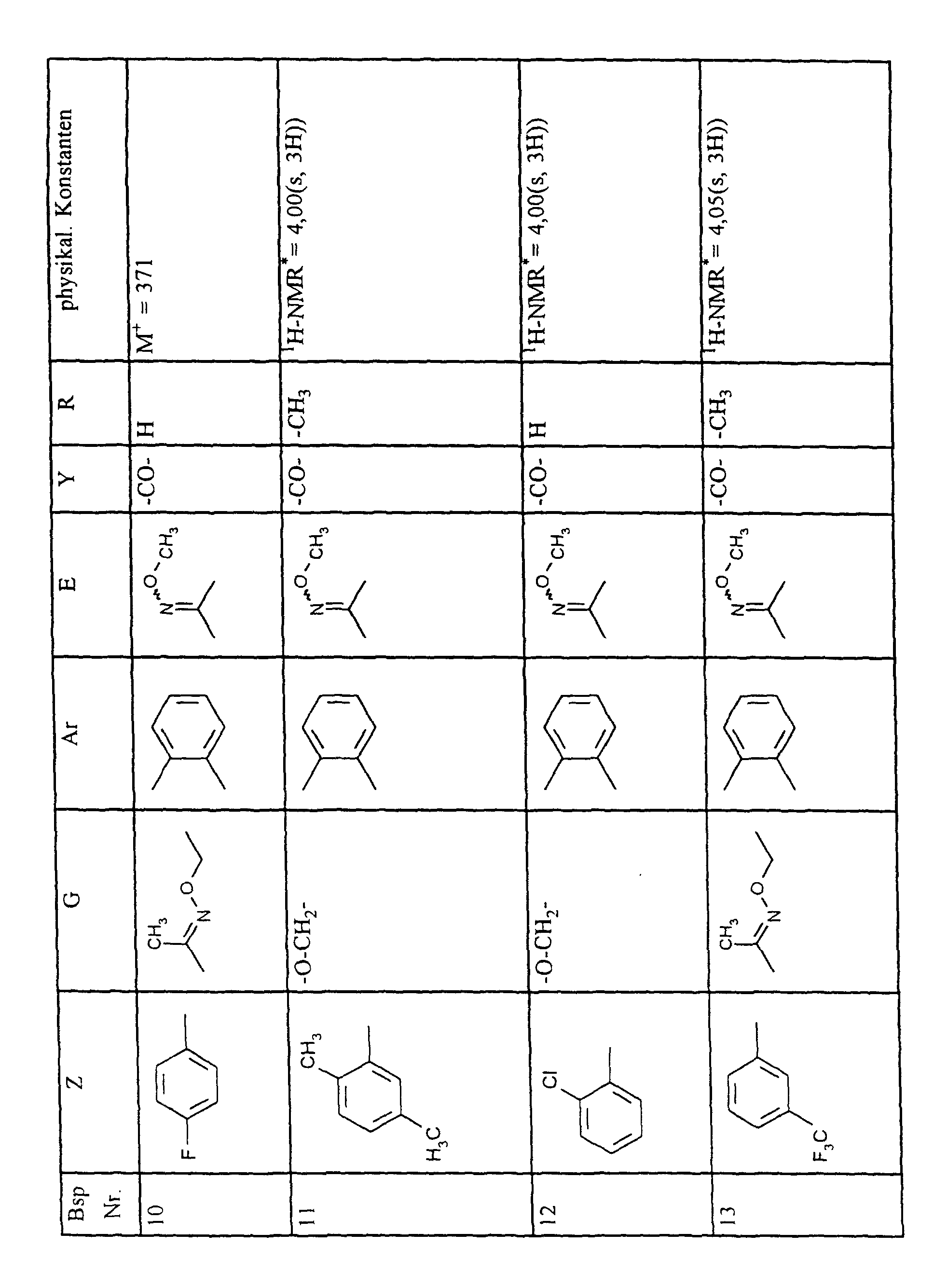

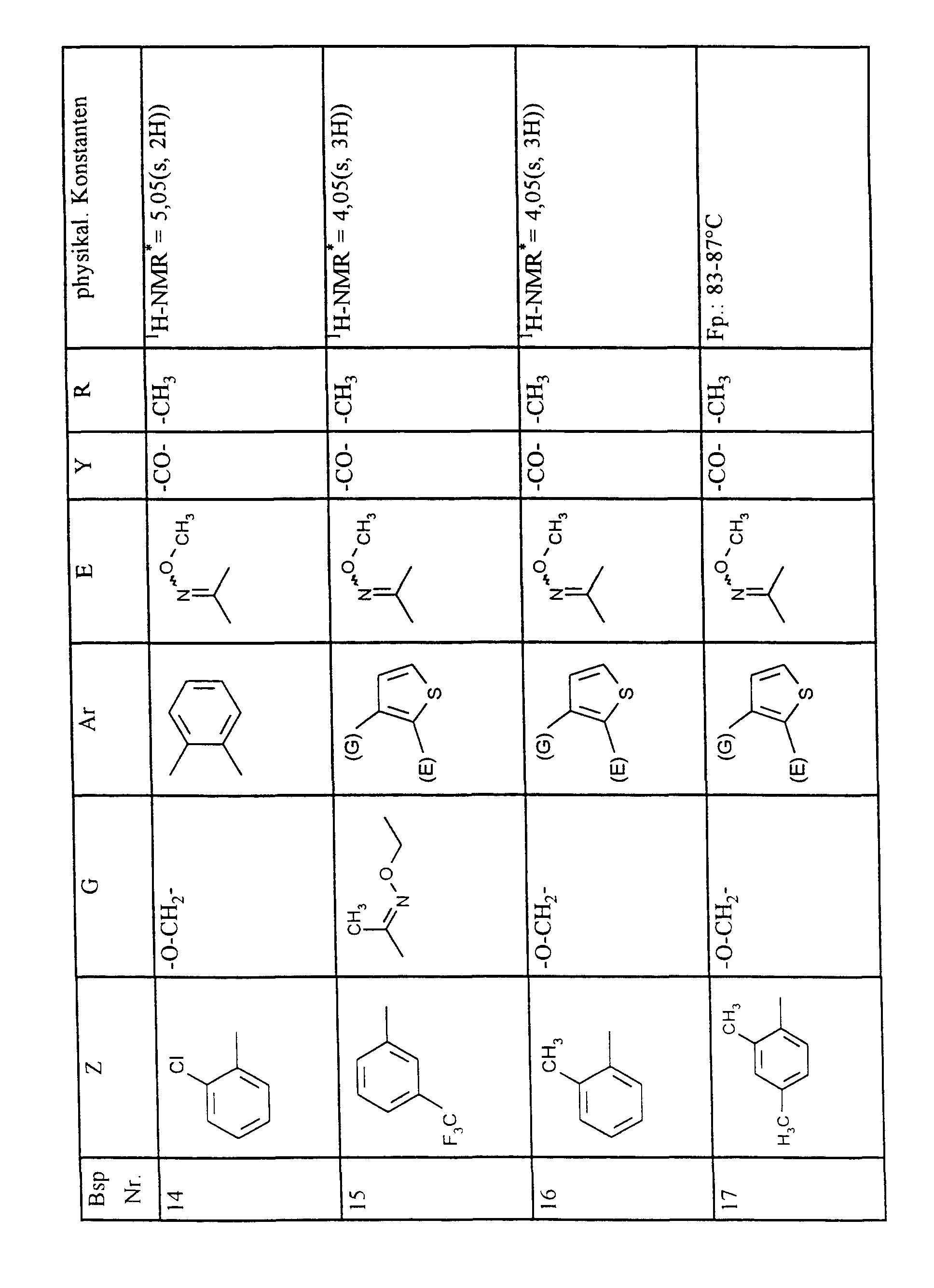

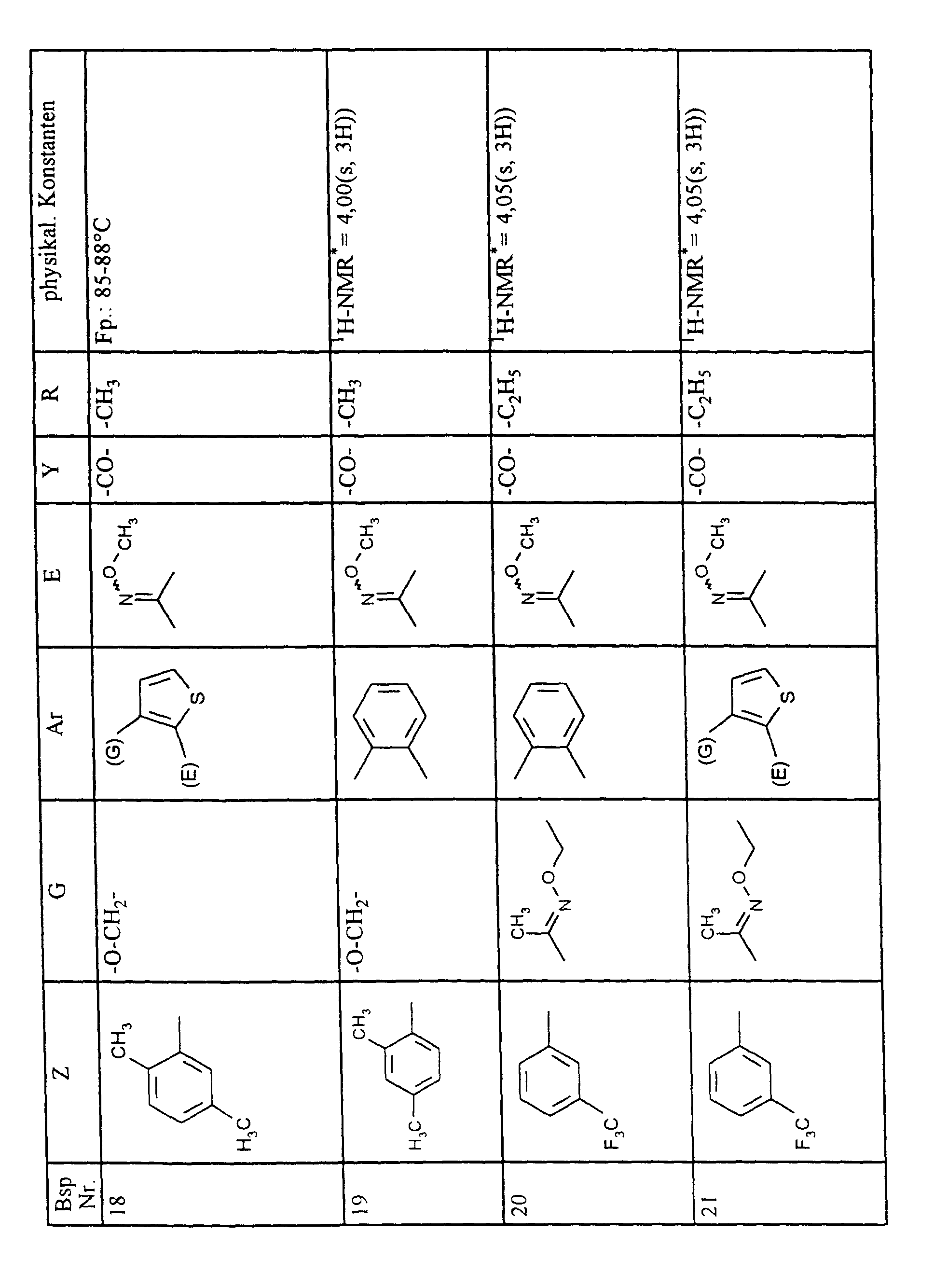

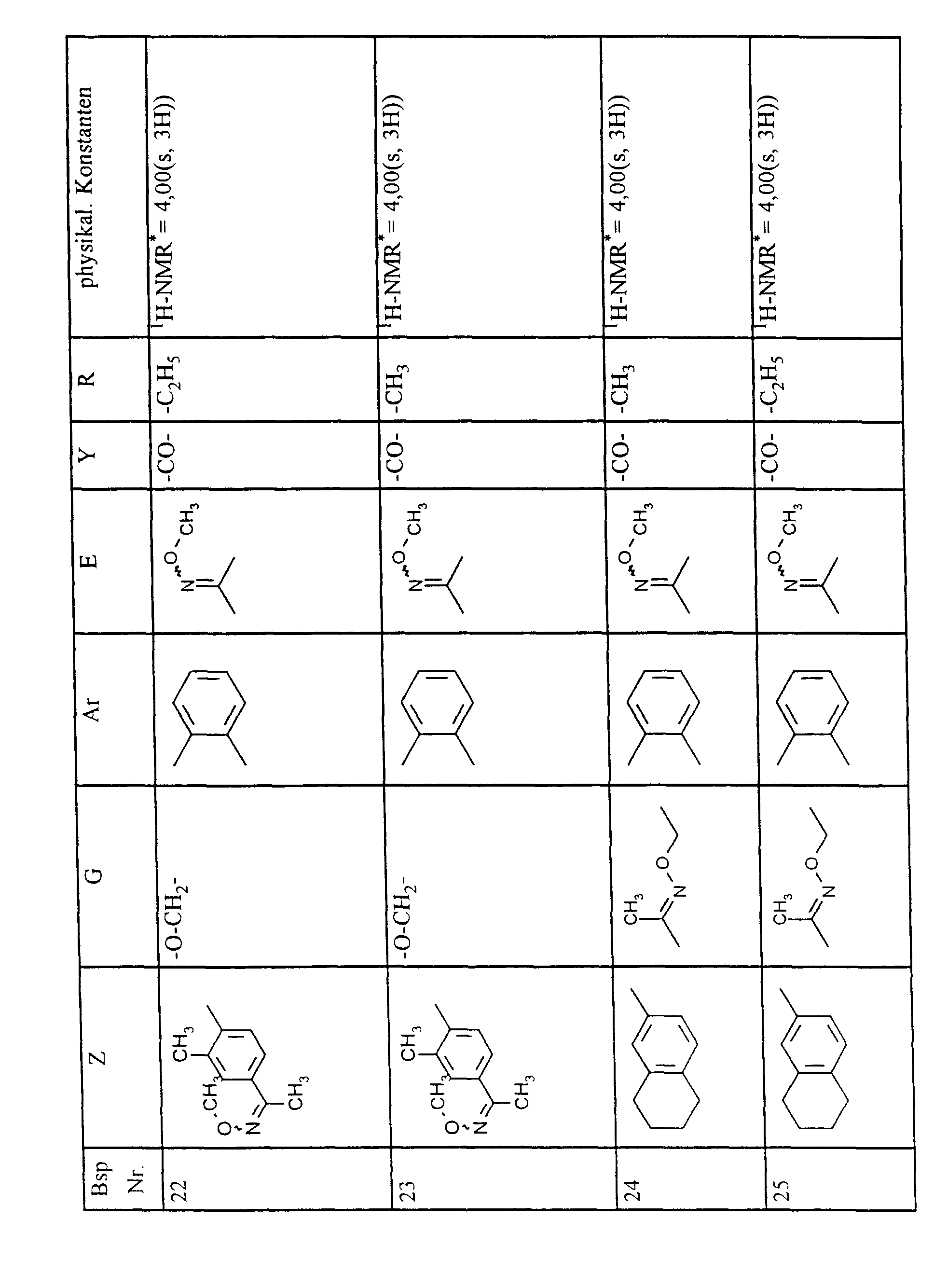

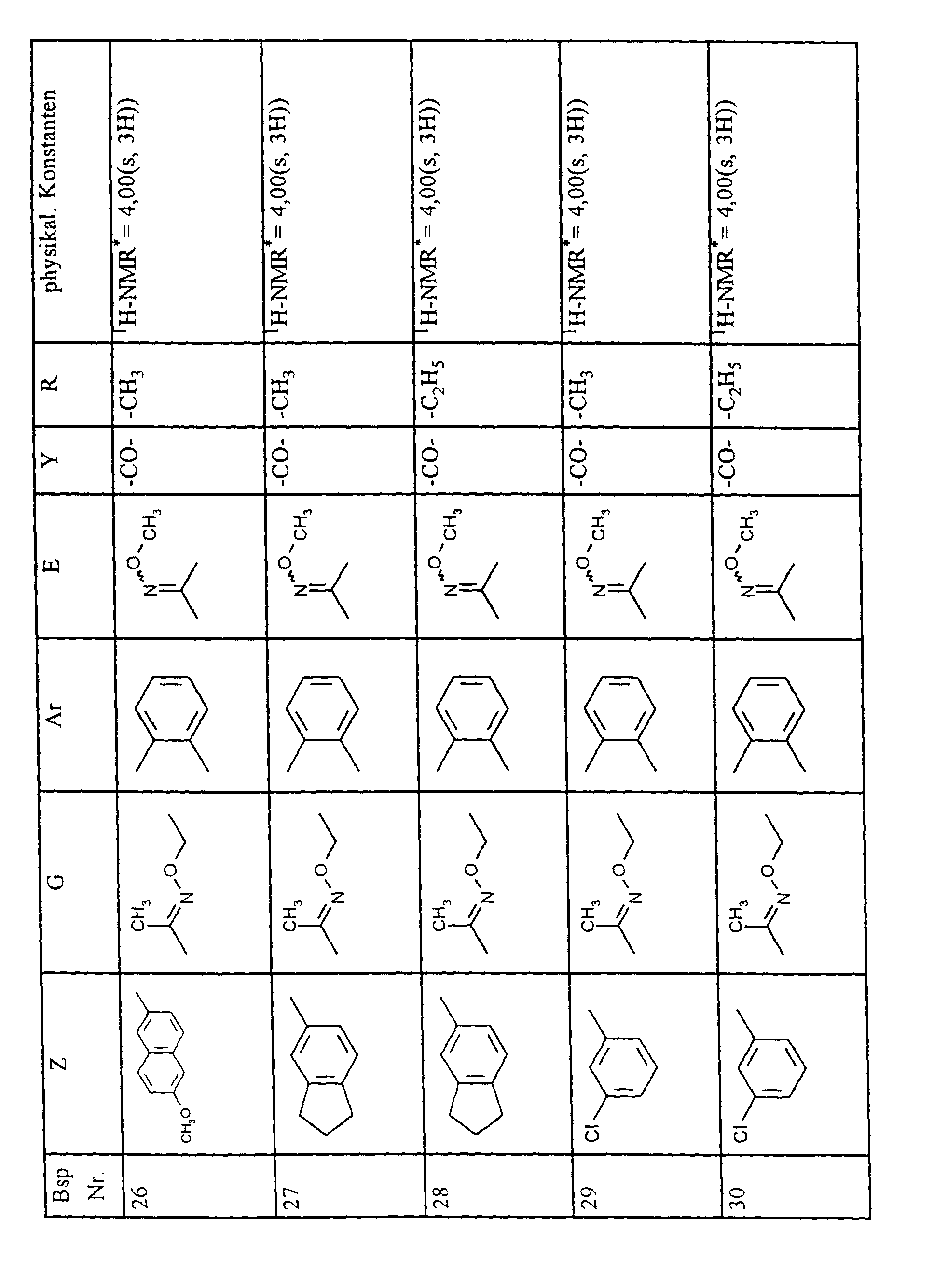

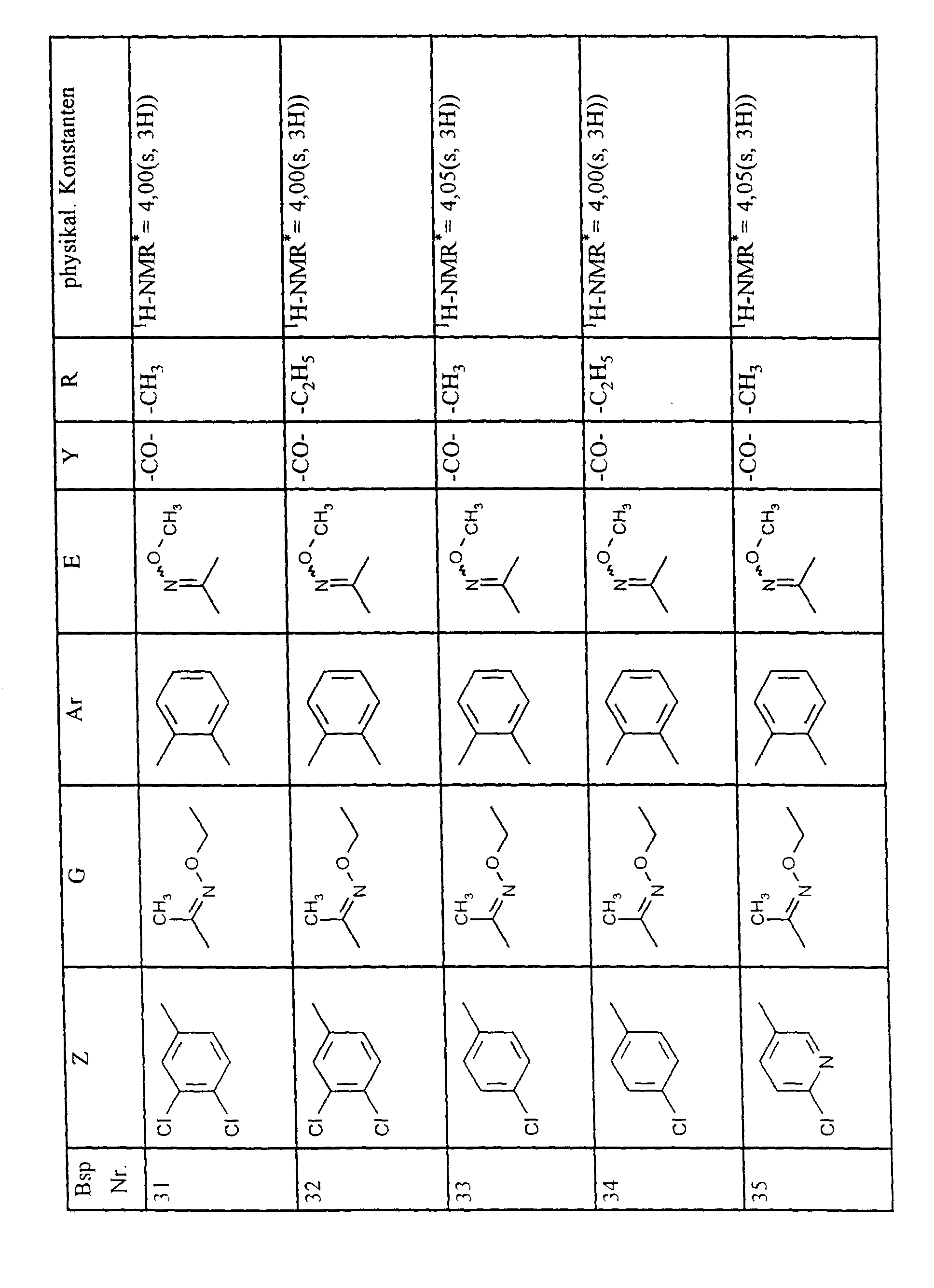

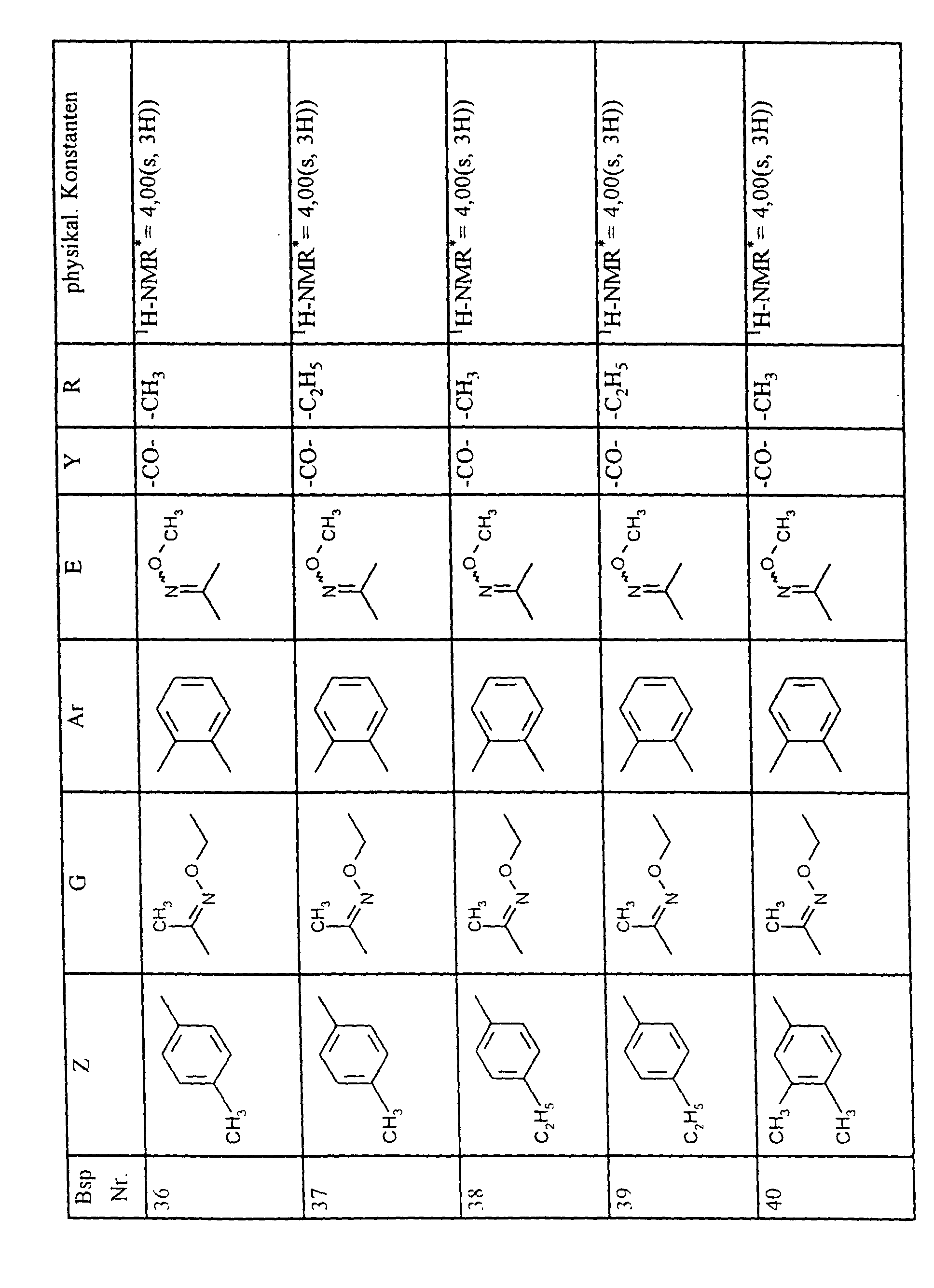

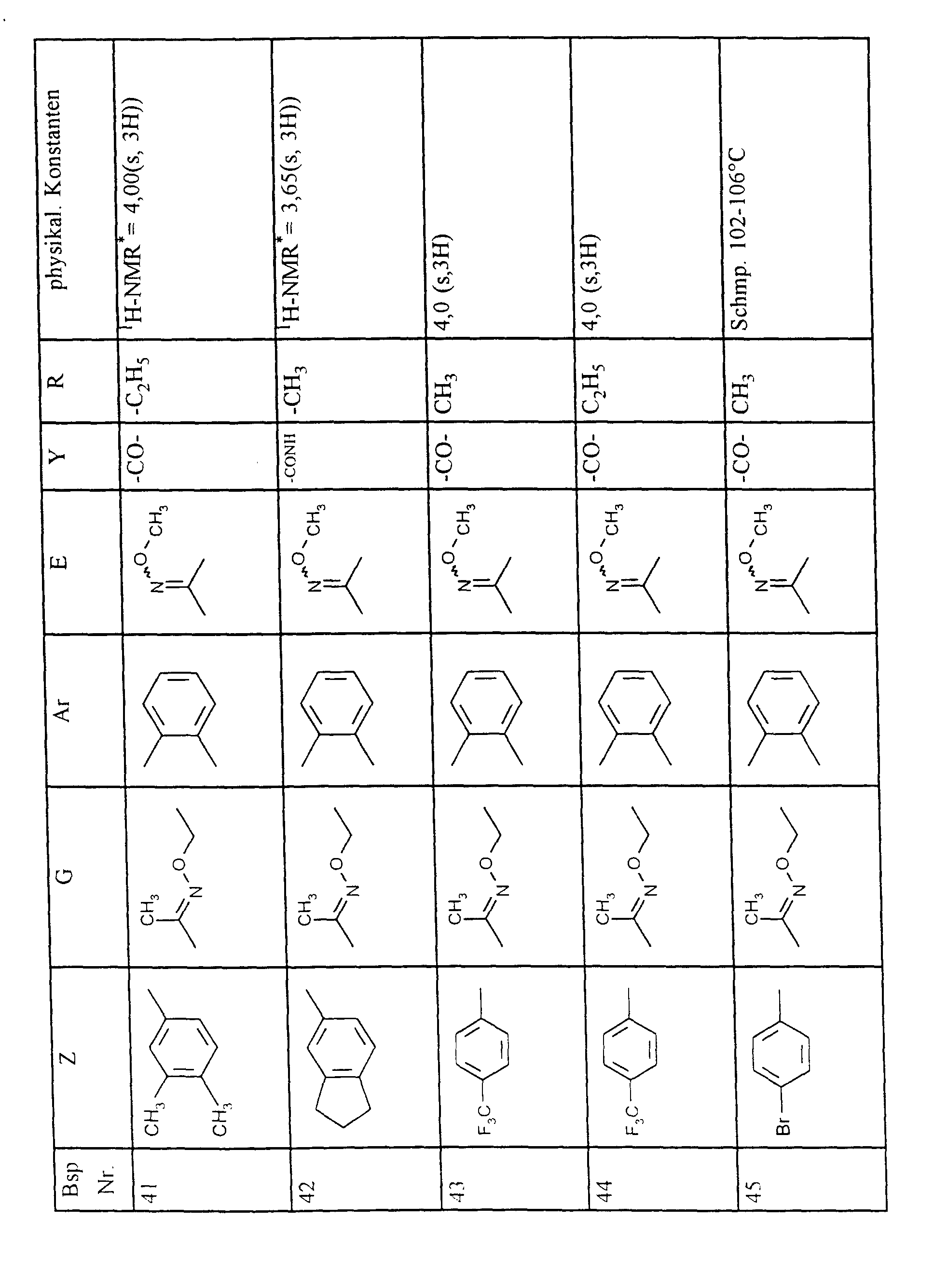

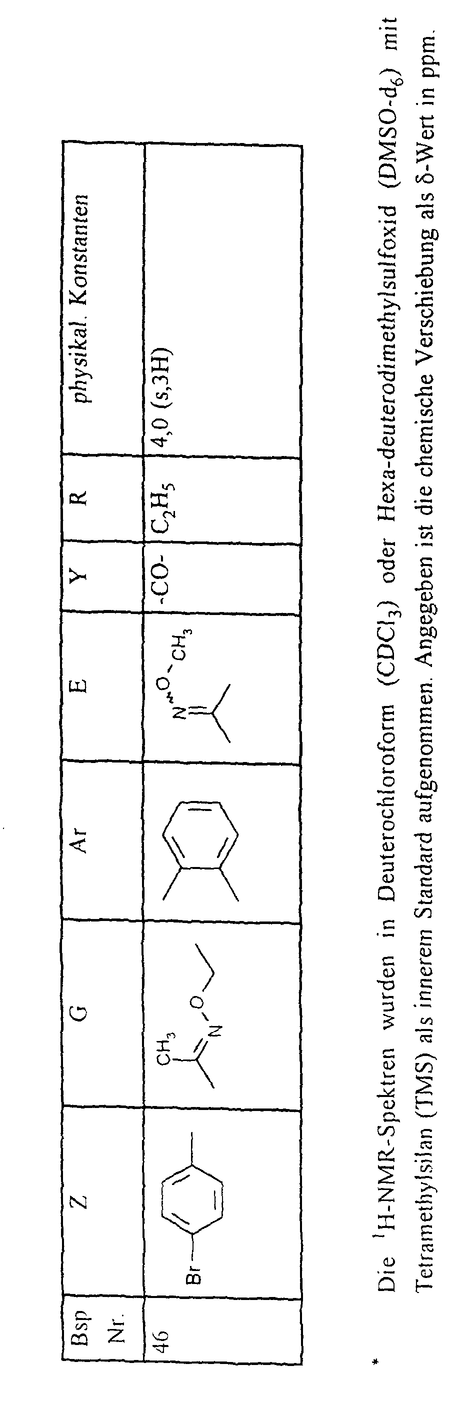

### Anwendungsbeispiele:

### Beispiel A

| **Plasmopara-Test** (Reben) / protektiv | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung.

In diesem Test zeigen die erfindungsgemäßen Verbindungen 11, 13, 24, 25, 26, 31, 35 und 36 bei einer Wirkstoffkonzentration von 100 ppm in der Spritzbrühe einen Wirkungsgrad von mehr als 90%.

### Beispiel B

| **Podosphaera-Test (Apfel)** / protektiv | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung.

In diesem Test zeigt die erfindungsgemäße Verbindung 11 bei einer Wirkstoffkonzentration von 100 ppm in der Spritzbrühe einen Wirkungsgrad von mehr als 90%.

### Beispiel C

| **Venturia-Test (Apfel)** / protektiv | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigen die erfindungsgemäßen Verbindungen 11, 13, 29, 31, 33, 36, 38 und 42 bei einer Wirkstoffkonzentration von 1 ppm in der Spritzbrühe einen Wirkungsgrad von mehr als 90%.

### Beispiel D

| **Erysiphe-Test (Gerste)** / protektiv | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen 11, 12, 13, 20 und 22 bei einer Aufwandmenge von 250 g/ha einen Wirkungsgrad von mehr als 90%.

### Beispiel E

| **Erysiphe-Test (Gerste)** / kurativ | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen 2 und 12 bei einer Aufwandmenge von 250 g/ha einen Wirkungsgrad von mehr als 90%.

### Beispiel F

| **Erysiphe-Test (Gerste)** / protektiv | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigen die Verbindungen 1 und 12 bei einer Aufwandmenge von 250 g/ha einen Wirkungsgrad von 100 %.

### Beispiel G

| **Erysiphe-Test (Weizen)** / kurativ | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. tritici bestäubt. 48 Stunden nach der Inokulation werden Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Bei diesem Test zeigt die Verbindung 12 bei einer Aufwandmenge von 250 g/ha einen Wirkungsgrad von 100 %.

### Beispiel H

| **Leptosphaeria nodorum-Test (Weizen)** / protekttiv | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigt die Verbindung 22 bei einer Aufwandmenge von 250 g/ha einen Wirkungsgrad von 100 %.

## Patentansprüche

1. Verbindungen der allgemeinen Formel (I), 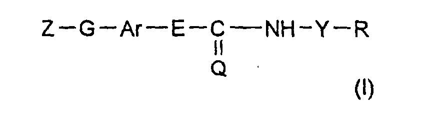 in welcher
Ar für gegebenenfalls substituiertes Arylen oder Heteroarylen steht,
E für eine 1-Alken-1,1-diyl-Gruppierung steht, die in 2-Position einen Rest R¹ enthält, oder für eine 2-Aza-1-alken-1,1-diyl-Gruppierung steht, die in 2-Position einen Rest R² enthält, wobei
R¹ für Wasserstoff, Halogen, Cyano oder für jeweils gegebenenfalls substituiertes Alkyl, Alkoxy, Alkylthio, Alkylamino oder Dialkylamino steht,
R² für Wasserstoff, Amino, Hydroxy, Cyano oder für jeweils gegebenenfalls substituiertes Alkyl, Alkoxy, Alkylamino oder Dialkylamino steht, und
G für eine Einfachbindung, für Sauerstoff, Schwefel oder für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- oder -T-Ar¹-Q¹- steht, wobei
Ar¹ für gegebenenfalls substituiertes Arylen, Heteroarylen, Cycloalkylen oder Heterocycloalkylen (d. h. ein zweifach verknüpfter aliphatischer Ring, in dem ein oder mehrere Kohlenstoffatome durch Heteroatome, d.h. von Kohlenstoff verschiedene Atome ersetzt sind) steht,
n für die Zahlen 0, 1 oder 2 steht,
Q¹ für Sauerstoff oder Schwefel steht,
R⁴ für Wasserstoff, Cyano oder jeweils gegebenenfalls substituiertes Alkyl, Alkoxy, Alkylthio, Alkylamino, Dialkylamino oder Cycloalkyl steht, und
R⁵ für Wasserstoff, Hydroxy, Cyano oder jeweils gegebenenfalls substituiertes Alkyl, Alkoxy oder Cycloalkyl steht und
L für Wasserstoff oder Alkyl steht und
T für eine Einfachbindung, für Sauerstoff, Schwefel, -CH₂-O-, -CH₂-S- oder für gegebenenfalls substituiertes Alkandiyl steht,
Q für Sauerstoff oder Schwefel steht,
R für Wasserstoff, Amino oder jeweils gegebenenfalls substituiertes Alkyl, Alkoxy, Alkylamino oder Dialkylamino steht,
Y für -CO-, -CS- oder -SO₂- steht und
Z für jeweils gegebenenfalls substituiertes Alkyl, Alkenyl, Alkinyl, Cycloalkyl, Aryl oder Heterocyclyl steht,
ausgenommen die Verbindung N-(Dimethyl-hydrazono)-(4-methoxy-phenyl)-acetyl-methansulfonamid.

2. Verbindungen der Formel (I) gemäß Anspruch 1, in denen
Ar steht für jeweils gegebenenfalls substituiertes Phenylen oder Naphthylen steht, oder, für mono- oder bicyclisches Heteroarylen mit jeweils 5 oder 6 Ringgliedern oder für benzokondensiertes Heteroarylen mit 5 oder 6 Ringgliedern steht, von denen jeweils mindestens eines für Sauerstoff, Schwefel oder Stickstoff steht und gegebenenfalls ein oder zwei weitere für Stickstoff stehen, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
E für eine der nachstehenden Gruppierungen steht: 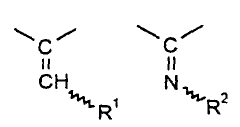 worin
R¹ für Wasserstoff, Halogen, Cyano oder für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylresten steht,
R² für Wasserstoff, Amino, Hydroxy, Cyano oder für jeweils gegebenenfalls durch Halogen, Cyano oder C₁-C₄-Alkoxy substituiertes Alkyl, Alkoxy, Alkylamino oder Dialkylamino mit jeweils 1 bis 6 Kohlenstoffatomen in den Alkylresten steht,
gegebenenfalls durch Halogen, Cyano, C₁-C₄-Alkyl oder C₁-C₄-Alkoxy- substituiertes Cycloalkyl oder Cycloalkylalkyl mit 3 bis 6 Kohlenstoffatomen in den Cycloalkylteilen und gegebenenfalls 1 bis 4 Kohlenstoffatomen im Alkylteil steht;
G für eine Einfachbindung, für Sauerstoff, Schwefel oder für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen steht oder für eine der nachstehenden Gruppierungen
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- oder -T-Ar¹-Q¹- steht, wobei
n für die Zahlen 0, 1 oder 2 steht,
Q¹ für Sauerstoff oder Schwefel steht,
R⁴ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht und
L für Wasserstoff oder Alkyl mit 1 bis 4 Kohlenstoffatomen steht,
Ar¹ für jeweils gegebenenfalls einfach oder mehrfach, gleich oder verschieden substituiertes Phenylen, Naphthylen, Cycloalkylen oder für Heteroarylen oder Heterocycloalkylen mit 3 bis 7 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, sowie;
Cycloalkyl mit 3 bis 6 Kohlenstoffatomen und
T für eine Einfachbindung, für Sauerstoff, Schwefel, -CH₂-O-, -CH₂-S- oder für Alkandiyl mit 1 bis 3 Kohlenstoffatomen steht;
Q für Sauerstoff steht;
R für Wasserstoff, Amino, Alkyl, Halogenalkyl, Alkoxy Halogenalkoxy, Alkylamino oder Dialkylamino mit jeweils 1 bis 4 Kohlenstoffatomen in der jeweiligen Alkylkette steht;
Y für -CO- oder -SO₂- steht;
Z für gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 8 Kohlenstoffatomen steht;
für jeweils gegebenenfalls durch Halogen substituiertes Alkenyl oder Alkinyl mit jeweils bis zu 8 Kohlenstoffatomen steht;
für jeweils gegebenenfalls einfach oder mehrfach, gleich oder verschieden durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen steht;
für jeweils gegebenenfalls einfach oder mehrfach, gleich oder verschieden substituiertes Phenyl, Naphthyl oder für Heierocyclyl mit 3 bis 7 Ringgliedern, von denen mindestens eines für Sauerstoff, Schwefel oder Stickstoff und gegebenenfalls ein oder zwei weitere für Stickstoff stehen, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl oder Alkylsulfonyloxy, mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -
oder eine Gruppierung 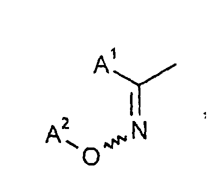 worin
A¹ für Alkyl mit 1 bis 4 Kohlenstoffatomen oder Cycloalkyl mit 1 bis 6 Kohlenstoffatomen steht und
A² für gegebenenfalls durch Cyano, Alkoxy, Alkylthio, Alkylamino, Dialkylamino oder Phenyl substituiertes Alkyl mit 1 bis 4 Kohlenstoffatomen, Alkenyl oder Alkinyl mit jeweils 2 bis 4 Kohlenstoffatomen steht.

3. Verbindungen der Formel (I) gemäß Anspruch 1, in denen
Ar für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Cyclopropyl, Trifluormethyl, Methoxy, Ethoxy, Methylthio, Methylsulfinyl oder Methylsulfonyl;
E für eine der nachstehenden Gruppierungen steht: 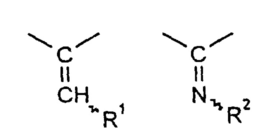 worin
R¹ für jeweils gegebenenfalls durch Fluor, Chlor, Cyano, Methoxy oder Ethoxy substituiertes Methyl, Ethyl oder Methoxy steht,
R² für jeweils gegebenenfalls durch Fluor, Chlor, Cyano, Methoxy oder Ethoxy substituiertes Methyl, Ethyl oder Methoxy steht;
G für Sauerstoff oder für jeweils gegebenenfalls durch Fluor, Chlor oder Brom substituiertes Dimethylen (Ethan-1,2-diyl), Ethen-1,2-diyl oder eine der nachstehenden Gruppierungen steht
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)-O-CH₂)-, -C(=N-O-L)-C(R⁴)-O-N=CH-, -C(-O-L)-C(R⁴)-O-N=C(CH₃)-, -T-Ar¹- oder -T-Ar¹-Q¹-, wobei
n für die Zahlen 0, 1 oder 2 steht,
Q¹ für Sauerstoff oder Schwefel steht,
R⁴ für Wasserstoff, Cyano, Methyl, Ethyl oder Cyclopropyl steht und
R⁵ für Wasserstoff, Methyl, Ethyl oder Cyclopropyl steht,
L für Wasserstoff oder Methyl steht und
Ar¹ für jeweils gegebenenfalls einfach bis dreifach, gleich oder verschieden substituiertes Phenylen oder Pyridindiyl, für jeweils gegebenenfalls einfach substituiertes Pyrimidindiyl, Pyridazindiyl, Pyrazindiyl, 1,2,3-Triazindiyl, 1,2,4-Triazindiyl oder 1,3,5-Triazindiyl oder für 1,2,4-Thiadiazoldiyl, 1,3,4-Thiadiazoldiyl, 1,2,4-Oxadiazoldiyl, 1,3,4-Oxadiazoldiyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, Cyclopropyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl und
T für eine Einfachbindung, für Sauerstoff, Schwefel, -CH₂-O-, -CH₂-S-, Methylen, Ethylen oder Propylen steht;
Q für Sauerstoff steht;
R für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht;
Y für -CO- oder -SO₂- steht;
Z für jeweils gegebenenfalls einfach bis dreifach, gleich oder verschieden substituiertes Phenyl, 1,2,4-Thiadiazolyl, 1,3,4-Thiadiazolyl, 1,2,4-Oxadiazolyl, 1,3,4-Oxadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy, Ethylendioxy oder eine Gruppierung 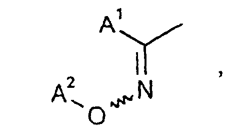 worin
A¹ für Methyl, Ethyl, n- oder i-Propyl, n-, i-, s- oder t-Butyl, Cyclopropyl oder Cyclobutyl steht;
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

4. Verbindungen der Formel (I) gemäß Anspruch 1,
in welcher
Ar für ortho-Phenylen, Pyridin-2,3-diyl oder Thiophen-2,3-diyl steht,
E für eine der nachstehenden Gruppierungen steht, 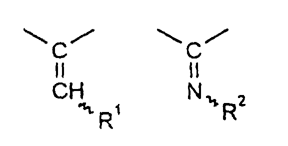 worin
R¹ und R² jeweils für Methoxy stehen,
G für -O-CH, steht,
Q für Sauerstoff steht,
R für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht,
Y für -CO- oder -SO₂- steht und
Z für jeweils gegebenenfalls einfach bis dreifach, gleich oder verschieden substituiertes Phenyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl,
jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy
oder eine Gruppierung 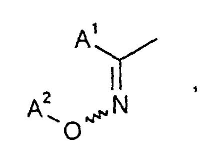 worin
A¹ für Methyl, Ethyl, n- oder i-Propyl, n-, i-, s- oder t-Butyl, Cyclopropyl oder Cyclobutyl steht und
A² für Methyl, Ethyl, n- oder i-Propyl, n-, i-, s- oder t-Butyl, Allyl, Propargyl, But-2-en-1-yl, 2-Methyl-prop-1-en-3-yl, Cyanmethyl, Methoxymethyl, Ethoxymethyl, Methoxyethyl, Ethoxyethyl, Methylthiomethyl, Ethylthiomethyl, Methylthioethyl, Ethylthioethyl, Dimethylaminomethyl, Dimethylaminoethyl, Methylaminomethyl, Methylaminoethyl oder Benzyl steht.

5. Verbindungen der Formel (I) gemäß Anspruch 1,
in welcher
Ar für ortho-Phenylen, Pyridin-2,3-diyl oder Thiophen-2,3-diyl steht,
E für eine der nachstehenden Gruppierungen steht, 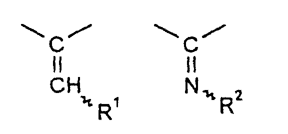 worin
R¹ und R² jeweils für Methoxy stehen,
G für -C(R⁴)=N-O-CH₂- steht, wobei
R⁴ für Wasserstoff, Cyano, Methyl, Ethyl oder Cyclopropyl steht,
Q für Sauerstoff steht,
R für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht,
Y für -CO- oder -SO₂- steht und
Z für jeweils gegebenenfalls einfach bis dreifach, gleich oder verschieden substituiertes Phenyl, Pyridyl oder Pyrimidyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Trifluormethylthio, Difluorchlormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach bis vierfach, gleich oder verschieden durch Fluor, Chlor, Methyl, Trifluormethyl oder Ethyl substituiertes, jeweils zweifach verknüpftes Methylendioxy oder Ethylendioxy.

6. Verbindungen der Formel (I) gemäß Anspruch 1,
in welcher
Ar für ortho-Phenylen steht,
E für eine der nachstehenden Gruppierungen steht: 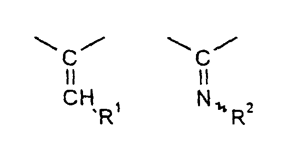 worin
R¹ und R² jeweils für Methoxy stehen,
G für -T-Ar¹-Q- steht, wobei
Q für Sauerstoff oder Schwefel steht,
Ar¹ für 1 ,2,4-Thiadiazoldiyl, 1,3,4-Thiadiazoldiyl, 1,2,4-Oxadiazoldiyl, 1,3,4-Oxadiazoldiyl oder für jeweils gegebenenfalls einfach oder zweifach, gleich oder verschieden durch Fluor, Chlor, Cyano, Methyl, Cyclopropyl, Methoxy, Methylthio, Trifluormethyl, Difluormethoxy, Trifluormethoxy, Difluorchlormethoxy substituiertes, Pyridindiyl, Pyrimidindiyl oder 1,3,5-Triazindiyl steht,
T für eine Einfachbindung, für Sauerstoff, Schwefel, -CH₂-O-, CH₂-S-, Methylen, Ethylen oder Propylen steht,
Q für Sauerstoff steht,
R für Wasserstoff, Methyl, Ethyl, Trifluormethyl, Methoxy, Ethoxy, Amino, Methylamino, Ethylamino, Dimethylamino, Diethylamino oder N-Methyl-N-ethylamino steht,
Y für -CO- oder -SO₂- steht und
Z für jeweils gegebenenfalls einfach bis dreifach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n-oder i-Propoxy, Difluormethoxy, Difluorchlormethoxy, Trifluorethoxy, Trifluormethoxy oder jeweils gegebenenfalls einfach oder mehrfach, gleich oder verschieden durch Fluor, Chlor, Methyl, Trifluormethyl oder Ethyl substituiertes, jeweils zweifach verknüpftes Methylendioxy oder Ethylendioxy, substituiertes Phenyl, Pyridyl oder Thienyl steht.

7. Schädlingsbekämpfungsmittel, **gekennzeichnet durch** einen Gehalt an mindestens einer Verbindung der Formel (I) nach Anspruch 1.

8. Verfahren zur Bekämpfung von Schädlingen, **dadurch gekennzeichnet, daß daß** man Verbindungen der Formel (I) nach Anspruch 1 auf Schädlinge und/oder ihren Lebensraum einwirken läßt.

9. Verfahren zur Herstellung von Verbindungen der Formel (I) nach Anspruch 1, **dadurch gekennzeichnet, daß** man
Carbonsäureamide der allgemeinen Formel (II) 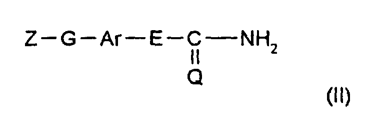 in welcher
Ar, E, G, Q und Z die in Anspruch 1 angegebenen Bedeutungen haben,
entweder
a) mit einem Carbonsäurehalogenid der Formel (III), 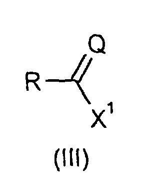 in welcher
Q und R die in Anspruch 1 angegebenen Bedeutungen haben, und
X¹ für Halogen steht, oder
b) mit einem Carbonsäureanhydrid der Formel (IV), 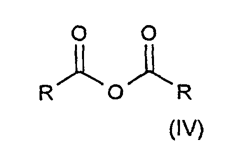 in welcher
R die in Anspruch 1 angegebene Bedeutung hat, oder
c) mit einem Sulfonsäurehalogenid der Formel (V), 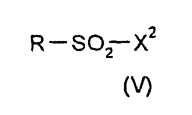 in welcher
R die in Anspruch 1 angegebene Bedeutung hat und
X² für Halogen steht,
jeweils gegebenenfalls in Gegenwart eines Verdünnungsmittels und jeweils gegebenenfalls in Gegenwart eines Säurebindemittels, umsetzt,
oder
d) Carbonsäureamide der allgemeinen Formel (II)
mit einem Isocyanat der Formel (VI), 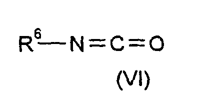 in welcher
R⁶ für Alkyl steht,
gegebenenfalls in Gegenwart eines Verdünnungsmittels und gegebenenfalls in Gegenwart eines Reaktionshilfsmittels, umsetzt,
oder
e) Amidine der allgemeinen Formel (VII), 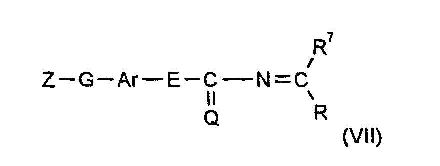 in welcher
Ar, E, G, Q, R und Z die in Anspruch 1 angegebenen Bedeutungen haben und
R⁷ für Dialkylamino steht,
gegebenenfalls in Gegenwart eines Verdünnungsmittels und gegebenenfalls in Gegenwart einer Säure, hydrolysiert.

10. Verwendung von Verbindungen der Formel (I) nach den Ansprüchen 1 bis 6 zur Bekämpfung von Schädlingen.

11. Verfahren zur Herstellung von Schädlingsbekämpfungsmitteln, **dadurch gekennzeichnet, daß** man Verbindungen der Formel (I) nach den Ansprüchen 1 bis 6 mit Streckmitteln und/oder oberflächenaktiven Mitteln vermischt.

12. Verbindungen der Formel (VII) 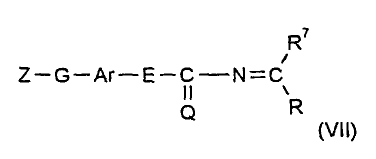 in welcher
Ar, E, G, Q, R und Z die in Anspruch 1 angegebenen Bedeutungen haben und
R⁷ für Dialkylamino steht.

## Claims

1. Compounds of the general formula (I), 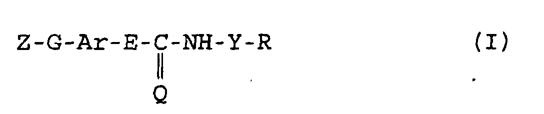 in which
Ar represents an optionally substituted arylene or heteroarylene,
E represents a 1-alkene-1,1-diyl grouping which contains a group R¹ in the 2-position, or represents a 2-aza-1-alkene-1,1-diyl grouping which contains a group R² in the 2-position, wherein
R¹ represents hydrogen, halogen, cyano or, each optionally substituted, alkyl, alkoxy, alkylthio, alkylamino or dialkylamino,
R² represents hydrogen, amino, hydroxy, cyano or, each optionally substituted, alkyl, alkoxy, alkylamino or dialkylamino, and
G represents a single bond, oxygen, sulfur, or, each optionally substituted by halogen, hydroxy, alkyl, haloalky or cycloalkyl substituted alkanediyl, alkenediyl, alkyndiyl or one of the following groupings
-Q¹-CQ¹-, -CQ¹-Q¹-, -CH₂-Q¹-, -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- or -T-Ar¹-Q¹-, wherein
Ar¹ represents optionally substituted arylene, heteroarylene, cycloalkylene or heterocycloalkylene (i.e. a doubly-linked aliphatic ring in which one or more carbon atoms are replaced by heteroatoms, i.e. atoms which differ from carbon),
n represents the numbers 0, 1 or 2,
Q¹ represents oxygen or sulfur,
R⁴ represents hydrogen, cyano or, each optionally substituted, alkyl, alkoxy, alkylthio, alkylamino, dialkylamino or cycloalkyl, and
R⁵ represents hydrogen, hydroxy, cyano or, each optionally substituted, alkyl, alkoxy or cycloalkyl and
L represents hydrogen or alkyl and
T represents a single bond, oxygen, sulfur, -CH₂-O-, -CH₂-S- or optionally substituted alkanediyl,
Q represents oxygen or sulfur,
R represents hydrogen, amino or, each optionally substituted, alkyl, alkoxy, alkylamino or dialkylamino,
Y represents -CO-, -CS-, or -SO₂- and
z represents, each optionally substituted, alkyl, alkenyl, alkynyl, cycloalkyl, aryl or heterocyclyl,
except for the compound (dimethyl-hydrazono)-(4-methoxy-phenyl) -acetyl-methanesulfonamide.

2. Compounds of the formula (I) according to Claim 1, in which
Ar represents, each optionally substituted, phenylene or naphthylene, or monocyclic or bicyclic heteroaryls each with 5 or 6 ring members or benzo-condensed heteroaryls with 5 or 6 ring members, of which at least one is oxygen, sulfur or nitrogen and optionally one or two more are nitrogen atoms, wherein the possible substituents are preferably chosen from the following list:
halogen, cyano, nitro, amino, hydroxy, formyl, carboxy, carbamoyl, thiocarbamoyl, straight-chain or branched alkyl, alkoxy, alkylthio, alkylsulfinyl or alkylsulfonyl, each with 1 to 6 carbon atoms, straight-chain or branched alkenyl, alkenyloxy or alkynyloxy, each with 2 to 6 carbon atoms, straight-chain or branched haloalkyl, haloalkoxy, haloalkylthio, haloalkylsulfinyl or haloalkylsulfonyl, each with 1 to 6 carbon atoms and 1 to 13 identical or different halogen atoms, straight-chain or branched haloalkenyl or haloalkenyloxy each with 2 to 6 carbon atoms and 1 to 11 identical or different halogen atoms, straight-chain or branched alkylamino, dialkylamino, alkylcarbonyl, alkylcarbonyloxy, alkoxycarbonyl, alkylsulfonyloxy, hydroxyiminoalkyl or alkoximinoalkyl each with 1 to 6 carbon atoms in the individual alkyl sections, doubly linked alkylene or dioxyalkylene each with 1 to 6 carbon atoms and substituted with 1 to 9 identical or different halogen atoms and optionally monosubstituted or polysubstituted, identically or differently, by halogen and/or straight-chain or branched alkyl with 1 to 4 carbon atoms and/or straight-chain or branched haloalkyl with 1 to 4 carbon atoms,
E represents one of the groupings given below: 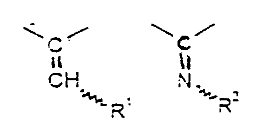 in which
R¹ represents hydrogen, halogen, cyano or alkyl, alkoxy, alkylthio, alkylamino or dialkylamino each with 1 to 6 carbon atoms in the alkyl group and, each optionally substituted by halogen, cyano or C₁-C₄-alkoxy,
R² represents hydrogen, amino, hydroxy, cyano or alkyl, alkoxy, alkylamino or dialkylamino each with 1 to 6 carbon atoms in the alkyl group and each optionally substituted by halogen, cyano or C₁-C₄-alkoxy,
G represents a single bond, oxygen sulfur or alkanediyl, alkenediyl, alkyndiyl, each with up to 4 carbon atoms and optionally substituted by halogen, hydroxy, C₁-C₄-alkyl, C₁-C₄-haloalkyl or C₃-C₆-cycloalkyl, or for one of the following groupings
-Q¹-CQ¹-, -CQ¹-Q¹⁻, -CH₂-Q¹-, -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C (R⁴) =N-O-CH₂-, -C (=N-O-L)-C(R⁴)-O-N=CH-, -C(=N-O-L)-C(R⁴)-O-N=C(CH₃)-, -T-Ar¹- or -T-Ar¹-Q¹-, wherein
n represents the numbers 0, 1 or 2,
Q¹ represents oxygen or sulfur,
R⁴ represents hydrogen, cyano or alkyl, alkoxy, alkylthio, alkylamino or dialkylamino, each with 1 to 6 carbon atoms in the alkyl group and each optionally substituted by halogen, cyano or C₁-C₄-alkoxy, or cycloalkyl with 3 to 6 carbon atoms, each optionally substituted by halogen, cyano, carboxy, C₁-C₄-alkyl or C₁-C₄-alkoxycrbonyl, and
R⁵ represents hydrogen, hydroxy, cyano, or alkyl with 1 to 6 carbon atoms optionally substituted by halogen, cyano or C₁-C₄-alkoxy, or cycloalkyl with 3 to 6 carbon atoms optionally substituted by halogen, cyano, carboxy, C₁-C₄-alkyl or C₁-C₄-alkoxycarbonyl, and
L represents hydrogen or alkyl with 1 to 4 carbon atoms and
Ar¹ represents, each optionally monosubstituted or polysubstituted, identically or differently, phenylene, naphthylene, cycloalkylene, or heteroarylene or heterocycloalkylene with 3 to 7 ring members, of which at least one is oxygen, sulfur or nitrogen and optionally one or two more are nitrogen, wherein the possible substituents, are preferably chosen from the following list:
halogen, cyano, nitro, amino, hydroxy, formyl, carboxy, carbamoyl, thiocarbamoyl;
straight-chain or branched alkyl, alkoxy, alkylthio, alkylsulfinyl, or alkylsulfonyl, each with 1 to 6 carbon atoms;
straight-chain or branched alkenyl or alkenyloxy, each with 2 to 6 carbon atoms;
straight-chain or branched haloalkyl, haloalkoxy, haloalkylthio, haloalkylsulfinyl or haloalkylsulfonyl, each with 1 to 6 carbon atoms and 1 to 13 identical or different halogen atoms;
straight-chain or branched haloalkenyl or haloalkenyloxy, each with 2 to 6 carbon atoms and 1 to 11 identical or different halogen atoms;
straight-chain or branched alkylamino, dialkylamino, alkylcarbonyl, alkylcarbonyloxy, alkoxycarbonyl, alkylsulfonyloxy, hydroximinoalkyl or alkoximinoalkyl, each with 1 to 6 carbon atoms in the individual alkyl sections, and;
cycloalkyl with 3 to 6 carbon atoms and
T represents a single bond, oxygen, sulfur, -CH₂-O-, -CH₂-S- or alkanediyl with 1 to 3 carbon atoms;
Q represents oxygen;
R represents hydrogen, amino, alkyl, haloalkyl, alkoxy, haloalkoxy, alkylamino or dialkylamino, each with 1 to 4 carbon atoms in the relevant alkyl chain;
Y represents -CO-, or -SO₂- and
Z represents alkyl with 1 to 8 carbon atoms optionally monosubstituted or polysubstituted, identically or differently, by halogen, cyano, hydroxy, amino, C₁-C₄-alkoxy, C₁-C₄-alkylthio, C₁-C₄-alkylsulfinyl or C₁-C₄-alkylsulfonyl (which may each optionally be substituted by halogen);
alkenyl or alkynyl, each with up to 8 carbon atoms and each optionally substituted by halogen;
cycloalkyl with 3 to 6 carbon atoms, optionally substituted, singly or several times, identically or differently, by halogen, cyano, carboxy, phenyl (which may optionally be substituted by halogen, cyano, C₁-C₄-alkyl, C₁-C₄-haloalkyl, C₁-C₄-alkoxy or C₁-C₄-haloalkoxy), C₁-C₄-alkyl or C₁-C₄-alkoxycarbonyl;
each optionally monosubstituted or polysubstituted, identically or differently, phenyl, naphthyl, or heterocyclyl with 3 to 7 ring members, of which at least one is oxygen, sulfur or nitrogen and optionally one or two more are nitrogen, wherein the possible substituents, are preferably chosen from the following list:
halogen, cyano, nitro, amino, hydroxy, formyl, carboxy, carbamoyl, thiocarbamoyl;
straight-chain or branched alkyl, alkoxy, alkylthio, alkylsulfinyl, or alkylsulfonyl, each with 1 to 6 carbon atoms;
straight-chain or branched alkenyl or alkenyloxy, each with 2 to 6 carbon atoms;
straight-chain or branched haloalkyl, haloalkoxy, haloalkylthio, haloalkylsulfinyl or haloalkylsulfonyl, each with 1 to 6 carbon atoms and 1 to 13 identical or different halogen atoms;
straight-chain or branched haloalkenyl or haloalkenyloxy, each with 2 to 6 carbon atoms and 1 to 11 identical or different halogen atoms;
straight-chain or branched alkylamino, dialkylamino, alkylcarbonyl, alkylcarbonyloxy, alkoxycarbonyl or alkylsulfonyloxy, each with 1 to 6 carbon atoms in the individual alkyl sections;
doubly linked alkylene or dioxyalkylene, each with 1 to 6 carbon atoms and substituted by 1 to 9 identical or different halogen atoms and each optionally monosubstituted or polysubstituted, identically or differently, by halogen and/or straight-chain or branched alkyl with 1 to 4 carbon atoms and/or straight-chain or branched haloalkyl with 1 to 4 carbon atoms;
cycloalkyl with 3 to 6 carbon atoms;
heterocyclyl or heterocyclyl-methyl, each with 3 to 7 ring members, of which 1 to 3 are identical or different heteroatoms, in particular nitrogen, oxygen and/or sulfur,
or a grouping 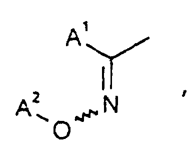 in which
A¹ represents alkyl with 1 to 4 carbon atoms or cycloalkyl with 1 to 6 carbon atoms and
A² represents alkyl with 1 to 4 carbon atoms, optionally substituted by cyano, alkoxy, alkylthio, alkylamino, dialkylamino or phenyl or alkenyl or alkynyl, each with 2 to 4 carbon atoms.

3. Compounds of the formula (I) according to Claim 1, in which
Ar represents, each optionally substituted, ortho-, meta- or para-phenylene, furandiyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl (in particular pyridine-2,3-diyl), pyrimidinediyl, pyridazinediyl, pyrazinediyl, 1,3,4-triazinediyl or 1,2,3-triazinediyl, wherein the possible substituents are chosen in particular from the following list:
fluorine, chlorine, cyano, methyl, ethyl, cyclopropyl, trifluoromethyl, methoxy, ethoxy, methylthio, methylsulfinyl or methylsulfonyl;
E represents one of the groupings given below: 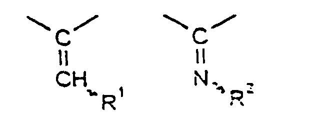 in which
R¹ represents methyl, ethyl or methoxy, each optionally substituted by fluorine, chlorine, cyano, methoxy or ethoxy,
R² represents methyl, ethyl or methoxy, each optionally substituted by fluorine, chlorine, cyano, methoxy or ethoxy,
G represents oxygen or dimethylene (ethane-1,2-diyl), ethene-1,2-diyl, each optionally substituted by fluorine, chlorine or bromine or for one of the following groupings
-Q¹-CQ¹-, -CQ¹-Q¹-, -CH₂-Q¹-, -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴) =N-O-, -C(R⁴) =N-O-CH₂-, -N(R⁵)-, CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵) -CQ¹-Q¹-, -N(R⁵)-CQ¹-Q¹-CH₂-, -Q¹-C(R⁴)=N-O-CH₂-, -N(R⁵) -C (R⁴) =N-O-CH₂-, -O-CH₂-C(R⁴) =N-O-CH₂-, -N=N-C (R⁴) =N-O-CH₂-, -C(CH₃)-O-N=C(R⁴)-, -C (=N-O-L) -C(R⁴)=N-O-CH₂-, -C(=N-O-L) -C(R⁴) -O-N=CH-, -C(=N-O-L)-C(R⁴)-O-N=C(CH₃)-, -T-Ar¹- or -T-Ar¹-Q¹-, wherein
n represents the numbers 0, 1 or 2,
Q¹ represents oxygen or sulfur,
R⁴ represents hydrogen, cyano, methyl, ethyl or cyclopropyl, and
R⁵ represents hydrogen, methyl, ethyl or cyclopropyl,
L represents hydrogen or methyl and
Ar¹ represents, each optionally monosubstituted to trisubstituted, identically or differently, phenylene or pyridinediyl, or optionally monosubstituted pyrimidinediyl, pyridazinediyl, pyrazinediyl, 1,2,3-triazinediyl, 1,2,4-triazinediyl or 1,3,5-triazinediyl or 1,2,4-thadiazolediyl, 1,3,4-thiadiazolediyl, 1,2,4-oxadiazolediyl, 1,3,4-oxadiazolediyl, wherein the possible substituents, are preferably chosen from the following list:
fluorine, chlorine, bromine, cyano, methyl, ethyl, n- or i-propyl, cyclopropyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulfinyl or trifluoromethylsulfonyl and
T represents a single bond, oxygen, sulfur, -CH₂-O-. -CH₂-S-, methylene, ethylene or propylene;
Q represents oxygen,
R represents hydrogen, methyl, ethyl, trifluoromethyl, methoxy, ethoxy, amino, methylamino, ethylamino, dimethylamino, diethylamino or N-methyl-N-ethylamino;
Y represents -CO-, or -SO₂- and
Z represents, each optionally monosubstituted to trisubstituted, identically or differently, phenyl, 1,2,4-thiadiazolyl, 1,3,4-thiadiazolyl, 1,2,4-oxadiazolyl, 1,3,4-oxadiazolyl, pyridinyl, pyrimidinyl, pyridinazinyl, pyrazinyl, 1,2,3-triazinyl, 1,2,4-triazinyl or 1,3,5-triazinyl, wherein the possible substituents, are preferably chosen from the following list:
fluorine, chlorine, bromine, cyano, methyl, ethyl, n- or i-propyl, n-. i-, s- or t-butyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulfinyl or trifluoromethylsulfonyl, methoxycarbonyl, ethoxycarbonyl,
doubly linked methylenedioxy or ethylenedioxy, each optionally monosubstituted to tetrasubstituted, identically or differently, by fluorine, chlorine, methyl, trifluoromethyl or ethyl,
or a grouping 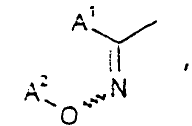 in which
A¹ represents methyl, ethyl, n- or i-propyl, n-, i-, s- or t-butyl, cyclopropyl or cyclobutyl;
A² represents methyl, ethyl, n- or i-propyl, n-, i-, s- or t-butyl, allyl, propargyl, but-2-ene-1-yl, 2-methyl-prop-1-ene-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylamino-ethyl or benzyl.

4. Compounds of the formula (I) according to Claim 1, in which
Ar represents ortho-phenylene, pyridine-2,3-diyl or thiophene-2,3-diyl,
E represents one of the groupings given below: 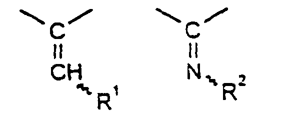 in which
R¹ and R² each represent methoxy,
G represents -O-CH₂,
Q represents oxygen,
R represents hydrogen, methyl, ethyl, trifluoromethyl, methoxy, ethoxy, amino, methylamino, ethylamino, dimethylamino, diethylamino or N-methyl-N-ethylamino;
Y represents -CO-, or -SO₂- and
Z represents optionally monosubstituted to trisubstituted, identically or differently, phenyl, wherein the possible substituents, are preferably chosen from the following list:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, difluoromethoxy, trifluoromethoxy, difluorochloromethoxy, trifluoroethoxy, difluoromethylthio, trifluoromethylthio, difluorochloromethylthio, trifluoromethylsulfinyl or trifluoromethylsulfonyl, methoxycarbonyl, ethoxycarbonyl,
doubly linked methylenedioxy or ethylenedioxy, each optionally monosubstituted to tetrasubstituted, identically or differently, by fluorine, chlorine, methyl, trifluoromethyl or ethyl,
or a grouping 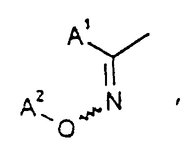 in which
A¹ represents methyl, ethyl, n- or i-propyl, n-, i-, s- or t-butyl, cyclopropyl or cyclobutyl and
A² represents methyl, ethyl, n- or i-propyl, n-, i-, s- or t-butyl, allyl, propargyl, but-2-ene-1-yl, 2-methyl-prop-1-ene-3-yl, cyanomethyl, methoxymethyl, ethoxymethyl, methoxyethyl, ethoxyethyl, methylthiomethyl, ethylthiomethyl, methylthioethyl, ethylthioethyl, dimethylaminomethyl, dimethylaminoethyl, methylaminomethyl, methylamino-ethyl or benzyl.

5. Compounds of the formula (I) according to Claim 1, in which
Ar represents ortho-phenylene, pyridine-2,3-diyl or thiophene-2,3-diyl,
E represents one of the groupings given below: 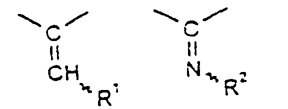 in which
R¹ and R² each represent methoxy,
G represents -C (R⁴)=N-O-CH₂-, wherein
R⁴ represents hydrogen, cyano, methyl, ethyl or cyclopropyl,
Q represents oxygen,
R represents hydrogen, methyl, ethyl, trifluoromethyl, methoxy, ethoxy, amino, methylamino, ethylamino, dimethylamino, diethylamino or N-methyl-N-ethylamino;
Y represents -CO-, or -SO₂- and
Z represents, each optionally monosubstituted to trisubstituted, identically or differently, phenyl, pyridinyl or primidinyl, wherein the possible substituents, are preferably chosen from the following list:
fluorine, chlorine, bromine, cyano, methyl, ethyl, n- or i-propyl, n-. i-, s- or t-butyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, trifluoromethylthio, difluorochloromethylthio, trifluoromethylsulfinyl or trifluoromethylsulfonyl, methoxycarbonyl, ethoxycarbonyl, methoximinomethyl, ethoximinomethyl, methoximinoethyl, ethoximinoethyl, or doubly linked methylenedioxy or ethylenedioxy, each optionally monosubstituted to tetrasubstituted, identically or differently, by fluorine, chlorine, methyl, trifluoromethyl or ethyl.

6. Compounds of the formula (I) according to Claim 1,
in which
Ar represents ortho-phenylene,
E represents one of the groupings given below: 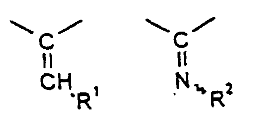 in which
R¹ and R² each represent methoxy,
G represents -T-Ar¹-Q-, wherein
Q represents oxygen or sulfur
Ar¹ represents 1,2,4-thiadiazolediyl, 1,3,4-thiadiazolediyl, 1,2,4-oxadiazolediyl, 1,3,4-oxadiazolediyl or else pyridinediyl, pyrimidinediyl or 1,3,5-triazinediyl, each optionally monosubstituted or disubstituted, identically or differently, by fluorine, chlorine, cyano, methyl, cyclopropyl, methoxy, methylthio, trifluoromethyl, difluoromethoxy, trifluoromethoxy, difluorochloromethoxy,
T represents a single bond or oxygen, sulfur, -CH₂-O-, -CH₂-S-, methylene, ethylene or propylene,
Q represents oxygen,
R represents hydrogen, methyl, ethyl, trifluoromethyl, methoxy, ethoxy, amino, methylamino, ethylamino, dimethylamino, diethylamino or N-methyl-N-ethylamino;
Y represents -CO-, or -SO₂- and
Z represents, phenyl, pyridyl or thienyl, each optionally monosubstituted to trisubstituted, identically or differently, by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy, difluorochloromethoxy, trifluoroethoxy, trifluoromethoxy or doubly linked methylenedioxy or ethylenedioxy, each optionally monosubstituted or polysubstituted, identically or differently, by fluorine, chlorine, methyl, trifluoromethyl or ethyl.

7. A pesticide **characterised by** a concentration of at least one compound of the formula (I) according to Claim 1.

8. A process for controlling pests, **characterised in that** compounds of the formula (I) according to Claim 1 are allowed to act on pests and/or their habitat.

9. A process for preparing compounds of the formula (I) according to Claim 1, **characterised in that**
carboxylic acid amides of the general formula (II) 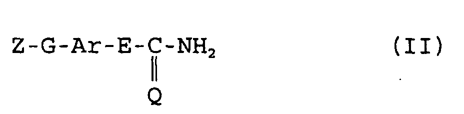 in which
Ar, E, G, Q and Z are defined in the same way as in Claim 1,
are reacted either
a) with a carboxylic acid halide of the formula (III) 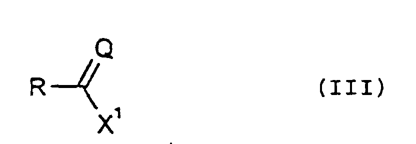 in which
Q and R are defined in the same way as in Claim 1, and
X¹ represents halogen, or
b) with a carboxylic anhydride of the formula (IV), 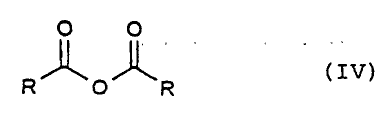 in which
R is defined in the same way as in Claim 1, or
c) with a sulfonic acid halide of the formula (V)
R-SO₂-X² (V)
in which
R is defined in the same way as in Claim 1 and
X² represents halogen,
each optionally in the presence of a diluent and each optionally in the presence of an acidic binder,
or
d) carboxylic acid amides of the general formula (II) are reacted with an isocyanate of the formula (VI),
R⁶-N=C=O (VI)
in which
R⁶ represents alkyl,
optionally in the presence of a diluent and optionally in the presence of a reaction auxiliary agent,
or
e) amidines of the general formula (VII) are hydrolysed 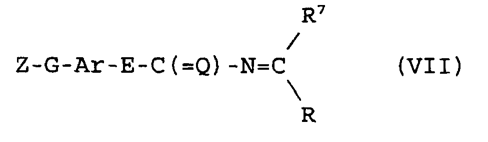 in which
Ar, E, G, Q, R and Z are defined in the same way as in Claim 1 and
R⁷ represents dialkylamino,
optionally in the presence of a diluent and optionally in the presence of an acid.

10. Use of the compounds of the formula (I) according to Claims 1 to 6 for controlling pests.

11. A process for preparing pesticides, **characterised in that** compounds of the formula (I) according to Claims 1 to 6 are blended with extenders and/or surface-active agents.

12. Compounds of the formula (VII) 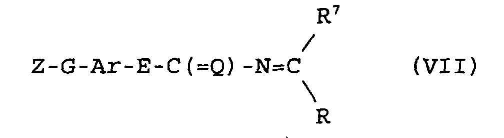 in which
Ar, E, G, Q, R and Z are defined in the same way as in Claim 1 and
R⁷ represents dialkylamino.

## Revendications

1. Composés de formule générale (I) 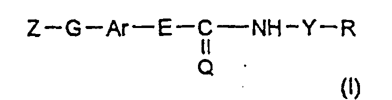 dans laquelle
Ar représente un groupe arylène ou hétéroarylène éventuellement substitué,
E représente un groupement 1-alcène-1, 1-diyle qui contient en position 2 un reste R¹ ou un groupement 2-aza-1-alcène-1,1-diyle qui contient en position 2 un reste R²,
où
R¹ représente l'hydrogène, un halogène, un groupe cyano ou un groupe alkyle, alkoxy, alkylthio, alkylamino ou dialkylamino éventuellement substitué dans chaque cas,
R² représente l'hydrogène, un groupe amino, hydroxy, cyano ou un groupe alkyle, alkoxy, alkylamino ou dialkylamino éventuellement substitué dans chaque cas, et
G représente une liaison simple, l'oxygène, le soufre ou un groupe alcanediyle, alcènediyle, alcynediyle éventuellement substitué dans chaque cas par un radical halogéno, hydroxy, alkyle, halogénalkyle ou cycloalkyle, ou l'un des groupements suivants
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-. -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-. -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH- -C(=N-O-L)-C(R⁴)-O-N=C(CH₃)-, -T-Ar¹- ou -T-Ar¹-Q¹-
où
Ar¹ représente un groupe arylène éventuellement substitué, hétéroarylène, cycloalkylène ou hétérocycloalkylène (c'est-à-dire un noyau aliphatique à deux liaisons, dans lequel un ou plusieurs atomes de carbone sont remplacés par des hétéroatomes, c'est-à-dire des atomes différents du carbone),
n représente les nombres 0, 1 ou 2,
Q¹ est l'oxygène ou le soufre,
R⁴ représente l'hydrogène, un groupe cyano ou un groupe alkyle, alkoxy, alkylthio, alkylamino, dialkylamino ou cycloalkyle dont chacun est éventuellement substitué, et
R⁵ représente l'hydrogène, un groupe hydroxy, cyano ou un groupe alkyle, alkoxy ou cycloalkyle dont chacun est éventuellement substitué, et
L représente l'hydrogène ou un groupe alkyle et
T représente une liaison simple, l'oxygène, le soufre, un groupe -CH₂-O-, -CH₂-S- ou un groupe alcanediyle éventuellement substitué,
Q représente l'oxygène ou le soufre,
R est l'hydrogène, un groupe amino, ou un groupe alkyle, alkoxy, alkylamino ou dialkylamino dont chacun est éventuellement substitué,
Y représente -CO-, -CS- ou -SO₂- et
z représente un groupe alkyle, alcényle, alcynyle, cycloalkyle, aryle ou hétérocyclyle dont chacun est éventuellement substitué, excepté le composé (diméthylhydrazono)-(4-méthoxyphényl)-acétylméthanesulfonamide.

2. Composés de formule (I) suivant la revendication 1, dans lesquels
Ar représente un groupe phénylène ou un groupe naphtylène dont chacun est éventuellement substitué ou bien un groupe hétéroarylène monocyclique ou bicyclique ayant dans chaque cas 5 ou 6 chaînons ou un groupe hétéroarylène condensé au benzène, de 5 ou 6 chaînons dont au moins l'un dans chaque cas est de l'oxygène, du soufre ou de l'azote et, le cas échéant, un ou deux autres chaînons représentent de l'azote, les substituants possibles étant choisis de préférence parmi ceux qui sont énumérés ci-après :
halogéno, cyano, nitro, amino, hydroxy, formyle, carboxy, carbamoyle, thiocarbamoyle ; alkyle, alkoxy, alkylthio, alkylsulfinyle ou alkylsulfonyle ayant chacun 1 à 6 atomes de carbone et étant chacun linéaire ou ramifié ; alcényle, alcényloxy ou alcynyloxy ayant chacun 2 à 6 atomes de carbone et étant chacun linéaire ou ramifié ; halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ; halogénalcényle ou halogénalcényloxy ayant chacun 2 à 6 atomes de carbone et 1 à 11 atomes d'halogènes identiques ou différents et étant chacun linéaire ou ramifié ; alkylamino, dialkylamino, alkylcarbonyle, alkylcarbonyloxy, alkoxycarbonyle, alkylsulfonyloxy, hydroximino-alkyle ou alkoximino-alkyle ayant chacun 1 à 6 atomes de carbone dans les parties alkyle individuelles et étant chacun linéaire ou ramifié ; alkylène ou dioxyalkylène ayant chacun 1 à 6 atomes de carbone et ayant chacun deux liaisons, chacun étant substitué éventuellement une ou plusieurs fois identiques ou différentes par un halogène et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents ;
E représente l'un des groupements suivants : 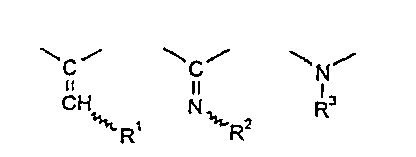 où
R¹ représente l'hydrogène, un halogène, un groupe cyano ou un groupe alkyle, alkoxy, alkylthio, alkylamino ou dialkylamino ayant chacun 1 à 6 atomes de carbone dans les restes alkyle et chacun étant substitué par un radical halogéno, cyano ou alkoxy en C₁ à C₄,
R² représente l'hydrogène, un groupe amino, hydroxy, cyano ou un groupe alkyle, alkoxy, alkylamino ou dialkylamino ayant chacun 1 à 6 atomes de carbone dans les restes alkyle et portant chacun, le cas échéant, un substituant halogéno, cyano ou alkoxy en C₁ à C₄,
un groupe cycloalkyle ou cycloalkylalkyle ayant 3 à 6 atomes de carbone dans les parties cycloalkyle et, le cas échéant, 1 à 4 atomes de carbone dans la partie alkyle et portant, le cas échéant, un substituant halogéno, cyano, alkyle en C₁ à C₄ ou alkoxy en C₁ à C₄ ;
G représente une liaison simple, l'oxygène, le soufre ou un groupe alcanediyle, alcènediyle, alcynediyle ayant chacun jusqu'à 4 atomes de carbone et portant chacun un substituant halogéno, hydroxy, alkyle en C₁ à C₄, halogénalkyle en C₁ à C₄ ou cycloalkyle en C₃ à C₆, ou l'un des groupements suivants :
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)=N-O-CH₂-, -C(=N-O-L)-C(R⁴)-O-N=CH-, -C(=N-O-L)-C(R⁴)-O-N=C(CH₃)-, -T-Ar¹- ou -T-Ar¹-Q¹-
où
n représente les nombres 0, 1 ou 2,
Q¹ est l'oxygène ou le soufre,
R⁴ représente l'hydrogène, un groupe cyano, un groupe alkyle, alkoxy, alkylthio, alkylamino ou dialkylamino ayant chacun 1 à 6 atomes de carbone dans les groupes alkyle et portant chacun, le cas échéant, un substituant halogéno, cyano ou alkoxy en C₁ à C₄, ou un groupe cycloalkyle de 3 à 6 atomes de carbone portant éventuellement un substituant halogéno, cyano, carboxy, alkyle en C₁ à C₄ ou (alkoxy en C₁ à C₄)-carbonyle, et
R⁵ représente l'hydrogène, un groupe hydroxy, cyano ou un groupe alkyle ayant 1 à 6 atomes de carbone portant éventuellement un substituant halogéno, cyano ou alkoxy en C₁ à C₄, ou un groupe cycloalkyle de 3 à 6 atomes de carbone portant éventuellement un substituant halogéno, cyano, carboxy, alkyle en C₁ à C₄ ou (alkoxy en C₁ à C₄)-carbonyle et
L représente l'hydrogène ou un groupe alkyle ayant 1 à 4 atomes de carbone,
Ar¹ représente un groupe phénylène, naphtylène, cycloalkylène portant chacun, le cas échéant, un ou plusieurs substituants identiques ou différents ou un groupe hétéroarylène ou hétérocycloalkylène à noyau de 3 à 7 atomes dont l'un au moins est un atome d'oxygène, de soufre ou d'azote et, le cas échéant, un ou deux autres représentent de l'azote, les substituants possibles étant choisis de préférence dans l'énumération suivante :
halogéno, cyano, nitro, amino, hydroxy, formyle, carboxy, carbamoyle, thiocarbamoyle ;
alkyle, alkoxy, alkylthio, alkylsulfinyle ou alkylsulfonyle ayant chacun 1 à 6 atomes de carbone et étant chacun linéaire ou ramifié ;
alcényle ou alcényloxy ayant chacun 2 à 6 atomes de carbone et étant chacun linéaire ou ramifié ;
halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et étant chacun linéaire ou ramifié ;
halogénalcényle ou halogénalcényloxy ayant chacun 2 à 6 atomes de carbone et 1 à 11 atomes d'halogènes identiques ou différents et étant chacun linéaire ou ramifié ;
alkylamino, dialkylamino, alkylcarbonyle, alkylcarbonyloxy, alkoxycarbonyle, alkylsulfonyloxy, hydroximinoalkyle ou alkoximinoalkyle étant chacun linéaire ou ramifié et ayant chacun 1 à 6 atomes de carbone dans les parties alkyle individuelles, ainsi que :
cycloalkyle ayant 3 à 6 atomes de carbone, et
T représente une liaison simple, l'oxygène, le soufre, un groupe -CH₂-O-, -CH₂-S- ou un groupe alcanediyle ayant 1 à 3 atomes de carbone ;
Q représente l'oxygène ;
R représente l'hydrogène, un groupe amino, alkyle, halogénalkyle, alkoxy, halogénalkoxy, alkylamino ou dialkylamino ayant chacun 1 à 4 atomes de carbone dans chaque chaîne alkyle ;
Y représente -CO- ou -SO₂- ;
Z représente un groupe alkyle ayant 1 à 8 atomes de carbone éventuellement substitué une ou plusieurs fois identiques ou différentes par un radical halogéno, cyano, hydroxy, amino, alkoxy en C₁ à C₄, alkylthio en C₁ à C₄, alkylsulfinyle en C₁ à C₄ ou alkylsulfonyle en C₁ à C₄ (qui peuvent être substitués dans chaque cas, le cas échéant, par un halogène) ;
un groupe alcényle ou alcynyle ayant chacun jusqu'à 8 atomes de carbone et chacun étant éventuellement substitué par un halogène ;
un groupe cycloalkyle ayant 3 à 6 atomes de carbone éventuellement substitué dans chaque cas une ou plusieurs fois identiques ou différentes par un radical halogéno, cyano, carboxy, phényle (qui est substitué, le cas échéant, par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle ;
un groupe phényle ou naphtyle éventuellement substitué dans chaque cas une ou plusieurs fois identiques ou différentes ou un groupe hétérocyclyle de 3 à 7 atomes dont l'un au moins est un atome d'oxygène, de soufre ou d'azote et, le cas échéant, un ou deux autres atomes représentent de l'azote, les substituants possibles étant choisis de préférence dans l'énumération suivante :
halogéno, cyano, nitro, amino, hydroxy, formyle, carboxy, carbamoyle, thiocarbamoyle ;
alkyle, alkoxy, alkylthio, alkylsulfinyle ou alkylsulfonyle ayant chacun 1 à 6 atomes de carbone et
chacun étant linéaire ou ramifié ;
alcényle ou alcényloxy ayant chacun 2 à 6 atomes de carbone et chacun étant linéaire ou ramifié ;
halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et chacun étant linéaire ou ramifié ;
halogénalcényle ou halogénalcényloxy ayant chacun 2 à 6 atomes de carbone et 1 à 11 atomes d'halogènes identiques ou différents et chacun étant linéaire ou ramifié ;
alkylamino, dialkylamino, alkylcarbonyle, alkylcarbonyloxy, alkoxycarbonyle ou alkylsulfonyloxy ayant chacun 1 à 6 atomes de carbone dans les parties alkyle individuelles et chacun étant linéaire ou ramifié ;
alkylène ou dioxyalkylène ayant chacun 1 à 6 atomes de carbone et chacun deux liaisons, chacun étant éventuellement substitué une ou plusieurs fois identiques ou différentes par un halogène ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents,
cycloalkyle ayant 3 à 6 atomes de carbone ;
hétérocyclyle ou hétérocyclyl-méthyle ayant chacun un noyau de 3 à 7 atomes dont, à chaque fois, 1 à 3 sont des hétéroatomes identiques ou différents - notamment azote, oxygène et/ou soufre -
ou un groupement 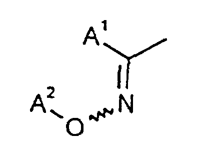 dans lequel
A¹ est un groupe alkyle ayant 1 à 4 atomes de carbone ou un groupe cycloalkyle ayant 1 à 6 atomes de carbone et
A² est un groupe alkyle ayant 1 à 4 atomes de carbone éventuellement substitué par un radical cyano, alkoxy, alkylthio, alkylamino, dialkylamino ou phényle, un groupe alcényle ou alcynyle ayant chacun 2 à 4 atomes de carbone.

3. Composés de formule (I) suivant la revendication 1, dans lesquels
Ar représente un groupe ortho-phénylène, méta-phénylène ou para-phénylène éventuellement substitué dans chaque cas, un groupe furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle (notamment pyridine-2,3-diyle), pyrimidinediyle, pyridazinediyle, pyrazinediyle, 1,3,4-triazinediyle ou 1,2,3-triazinediyle, les substituants possibles étant choisis en particulier dans l'énumération suivante : fluor, chlore, cyano, méthyle, éthyle, cyclopropyle, trifluorométhyle, méthoxy, éthoxy, méthylthio, méthylsulfinyle ou méthylsulfonyle ;
E représente l'un des groupements suivants : 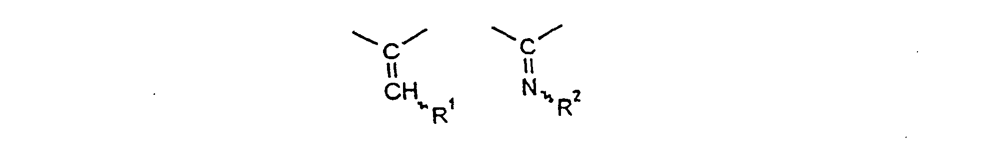 où
R¹ est un groupe méthyle, éthyle ou méthoxy portant chacun, le cas échéant, un substituant fluoro, chloro, cyano, méthoxy ou éthoxy,
R² est un groupe méthyle, éthyle ou méthoxy portant chacun, le cas échéant, un substituant fluoro, chloro, cyano, méthoxy ou éthoxy ;
G représente l'oxygène ou un groupe diméthylène (éthane-1,2-diyle), éthène-1,2-diyle portant chacun, le cas échéant, un substituant fluoro, chloro ou bromo, ou l'un des groupements suivants :
-Q¹-CQ¹-, -CQ¹-Q¹-, -CH₂-Q¹-; -Q¹-CH₂-, -CQ¹-Q¹-CH₂-, -CH₂-Q¹-CQ¹-, -Q¹-CQ¹-CH₂-, -Q¹-CQ¹-Q¹-CH₂-, -N=N-, -S(O)ₙ-, -CH₂-S(O)ₙ-, -CQ¹-, -S(O)ₙ-CH₂-, -C(R⁴)=N-O-, -C(R⁴)=N-O-CH₂-, -N(R⁵)-, -CQ¹-N(R⁵)-, -N(R⁵)-CQ¹-, -Q¹-CQ¹-N(R⁵)-, -N=C(R⁴)-Q¹-CH₂-, -CH₂-O-N=C(R⁴)-, -N(R⁵)-CQ¹-Q¹-, -CQ¹-N(R⁵)-CQ¹-Q¹-, -N(R⁵)-CQ¹-Q¹-CH₂-, -Q¹-C(R⁴)=N-O-CH₂-, -N(R⁵)-C(R⁴)=N-O-CH₂-, -O-CH₂-C(R⁴)=N-O-CH₂-, -N=N-C(R⁴)=N-O-CH₂-, -C(CH₃)-O-N=C(R⁴)-, -C(=N-O-L)-C(R⁴)-O-CH₂)-, -C(=N-O-L)-C(R⁴)-O-N=CH-, -C(-O-L)-C(R⁴)-O-N=C(CH₃)-, -T-Ar¹- ou -T-Ar¹-Q¹-
où
n représente les nombres 0, 1 ou 2,
Q¹ représente l'oxygène ou le soufre,
R⁴ représente l'hydrogène, un groupe cyano, méthyle, éthyle ou cyclopropyle et
R⁵ représente l'hydrogène, un groupe méthyle, éthyle ou cyclopropyle,
L représente l'hydrogène ou le groupe méthyle et
Ar¹ représente un groupe phénylène ou pyridinediyle portant chacun, le cas échéant, un à trois substituants identiques ou différents, un groupe pyrimidinediyle, pyridazinediyle, pyrazinediyle, 1,2,3-triazinediyle, 1,2,4-triazinediyle ou 1,3,5-triazinediyle portant chacun, le cas échéant, un substituant ou un groupe 1,2,4-thiadiazolediyle, 1,3,4-thiadiazolediyle, 1,2,4-oxadiazolediyle, 1,3,4-oxadiazolediyle, les substituants possibles étant choisis de préférence dans l'énumération suivante :
fluor, chlore, brome, cyano, méthyle, éthyle, n-propyle, isopropyle, cyclopropyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle et
T est une liaison simple, l'oxygène, le soufre, un groupe -CH₂-O-, -CH₂-S-, méthylène, éthylène ou propylène ;
Q représente l'oxygène ;
R représente l'hydrogène, un groupe méthyle, éthyle, trifluorométhyle, méthoxy, éthoxy, amino, méthylamino, éthylamino, diméthylamino, diéthylamino ou N-méthyl-N-éthylamino ;
Y représente -CO- ou -SO₂- ;
Z est un groupe phényle, 1,2,4-thiadiazolyle, 1,3,4-thiadiazolyle, 1,2,4-oxadiazolyle, 1,3,4-oxadiazolyle, pyridinyle, pyrimidinyle, pyridazinyle, pyrazinyle, 1,2,3-triazinyle, 1,2,4-triazinyle ou 1,3,5-triazinyle portant chacun, lé cas échéant, un à trois substituants identiques ou différents, les substituants possibles étant choisis de préférence dans l'énumération suivante :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthoxysulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle, trifluorométhylsulfonyle, méthoxycarbonyle, éthoxycarbonyle,
méthylènedioxy, éthylènedioxy ayant chacun deux liaisons et chacun étant éventuellement substitué une à quatre fois identiques ou différentes par un radical fluoro, chloro, méthyle, trifluorométhyle ou éthyle,
ou un groupement 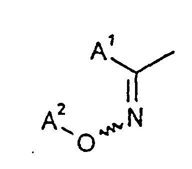 dans lequel
A¹ est un groupe méthyle, éthyle, n-propyle, isopropyle, n-butyle, isobutyle, sec.-butyle, tertio-butyle, cyclopropyle ou cyclobutyle ;
A² représente un groupe méthyle, éthyle, n-propyle, isopropyle, n-butyle, isobutyle, sec.-butyle, tertio-butyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthioéthyle, éthylthioéthyle, diméthylaminométhyle, diméthylaminoéthyle, méthylaminométhyle, méthyl-aminoéthyle ou benzyle.

4. Composés de formule (I) suivant la revendication 1, dans laquelle
Ar est un groupe ortho-phénylène, pyridine-2,3-diyle ou thiophène-2,3-diyle,
E représente l'un des groupements suivants 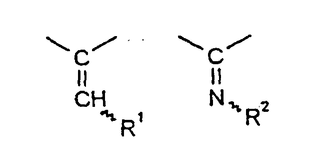 où
R¹ et R² représentent chacun un groupe méthoxy,
G est un groupe -O-CH₂-,
Q est l'oxygène,
R est l'hydrogène, un groupe méthyle, éthyle, trifluorométhyle, méthoxy, éthoxy, amino, méthylamino, éthylamino, diméthylamino, diéthylamino ou N-méthyl-N-éthylamino,
Y est un groupe -CO- ou -SO₂- et
Z représente un groupe phényle portant, le cas échéant, un à trois substituants identiques ou différents, les substituants possibles étant choisis de préférence dans l'énumération suivante :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle, méthoxycarbonyle, éthoxycarbonyle,
méthylènedioxy ou éthylènedioxy ayant chacun deux liaisons et chacun étant éventuellement substitué une à quatre fois identiques ou différentes par du fluor, du chlore, un radical méthyle, trifluorométhyle ou éthyle,
ou un groupement 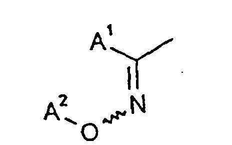 dans lequel
A¹ est un groupe méthyle, éthyle, n-propyle, isopropyle, n-butyle, isobutyle, sec.-butyle, tertio-butyle, cyclopropyle ou cyclobutyle et
A² est un groupe méthyle, éthyle, n-propyle, isopropyle, n-butyle, isobutyle, sec.-butyle, tertio-butyle, allyle, propargyle, but-2-ène-1-yle, 2-méthylprop-1-ène-3-yle, cyanométhyle, méthoxyméthyle, éthoxyméthyle, méthoxyéthyle, éthoxyéthyle, méthylthiométhyle, éthylthiométhyle, méthylthioéthyle, éthylthioéthyle, diméthylaminométhyle, diméthylaminoéthyle, méthylaminométhyle, méthylaminoéthyle ou benzyle.

5. Composés de formule (I) suivant la revendication 1, dans lesquels
Ar est un groupe ortho-phénylène, pyridine-2,3-diyle ou thiophène-2,3-diyle,
E représente l'un des groupements suivants 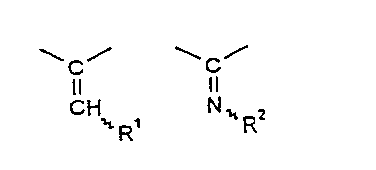 où
R¹ et R² représentent chacun un groupe méthoxy,
G est un groupe -C(R⁴)=N-O-CH₂-, dans lequel
R⁴ est l'hydrogène, un groupe cyano, méthyle, éthyle ou cyclopropyle,
Q est l'oxygène,
R est l'hydrogène, un groupe méthyle, éthyle, trifluorométhyle, méthoxy, éthoxy, amino, méthylamino, éthylamino, diméthylamino, diéthylamino ou N-méthyl-N-éthylamino,
Y représente -CO- ou -SO₂- et
z est un groupe phényle, pyridyle ou pyrimidyle portant chacun, le cas échéant, un à trois substituants identiques ou différents, les substituants possibles étant choisis de préférence dans l'énumération suivante :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, trifluorométhylthio, difluorochlorométhylthio, trifluorométhylsulfinyle ou trifluorométhylsulfonyle, méthoxycarbonyle, éthoxycarbonyle, méthoximinométhyle, éthoximinométhyle, méthoximinoéthyle, éthoximinoéthyle, méthylènedioxy ou éthylènedioxy ayant chacun deux liaisons et portant chacun, le cas échéant, un à quatre substituants fluoro, chloro, méthyle, trifluorométhyle ou éthyle identiques ou différents.

6. Composés de formule (I) suivant la revendication 1, dans laquelle
Ar est un groupe ortho-phénylène,
E représente l'un des groupements suivants : 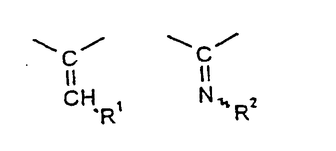 où
R¹ et R² représentent chacun un groupe méthoxy,
G est un groupe -T-Ar¹-Q-, dans lequel
Q est l'oxygène ou le soufre,
Ar¹ est un groupe 1,2,4-thiadiazolylediyle, 1,3,4-thiadiazolediyle, 1,2,4-oxadiazolediyle, 1,3,4-oxadiazolediyle ou un groupe pyridinediyle, pyrimidinediyle ou 1,3,5-triazinediyle portant éventuellement dans chaque cas un ou deux substituants fluoro, chloro, cyano, méthyle, cyclopropyle, méthoxy, méthylthio, trifluorométhyle, difluorométhoxy, trifluorométhoxy ou difluorochlorométhoxy identiques ou différents,
T représente une liaison simple, l'oxygène, le soufre, un groupe -CH₂-O-, -CH₂-S-, méthylène, éthylène ou propylène,
Q représente l'oxygène,
R est l'hydrogène, un groupe méthyle, éthyle, trifluorométhyle, méthoxy, éthoxy, amino, méthylamino, éthylamino, diméthylamino, diéthylamino ou N-méthyl-N-éthylamino,
Y représente -CO- ou -SO₂- et
Z représente un groupe phényle, pyridyle ou thiényle portant chacun, le cas échéant, un à trois substituants, identiques ou différents, fluoro, chloro, bromo, cyano, méthyle, éthyle, n-propyle, isopropyle, n-butyle, isobutyle, sec.-butyle, tertiobutyle, trifluorométhyle, méthoxy, éthoxy, n-propoxy, isopropoxy, difluorométhoxy, difluorochlorométhoxy, trifluoréthoxy, trifluorométhoxy, ou bien méthylènedioxy ou éthylènedioxy ayant chacun deux liaisons et portant chacun, le cas échéant, un ou plusieurs substituants, identiques ou différents, fluoro, chloro, méthyle, trifluorométhyle ou éthyle.

7. Compositions pesticides, **caractérisées par** une teneur en au moins un composé de formule (I) suivant la revendication 1.

8. Procédé pour combattre des parasites, **caractérisé en ce qu'**on fait agir des composés de formule (I) suivant la revendication 1 sur des parasites et/ou sur leur milieu.

9. Procédé de production de composés de formule (I) suivant la revendication 1, **caractérisé en ce qu'**on fait réagir des amides d'acides carboxyliques de formule générale (II) 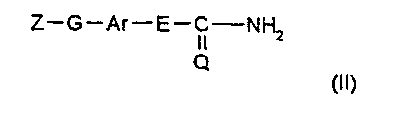 dans laquelle
Ar, E, G, Q et Z ont les définitions indiquées dans la revendication 1,
a) avec un halogénure d'acide carboxylique de formule (III). 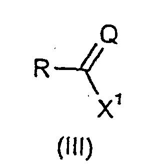 dans laquelle
Q et R ont les définitions indiquées dans la revendication 1, et
X¹ représente un halogène, ou bien
b) avec un anhydride d'acide carboxylique de formule (IV), 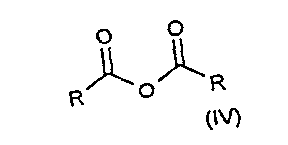 dans laquelle
R a la définition indiquée dans la revendication 1, ou bien
c) avec un halogénure d'acide sulfonique de formule (V) 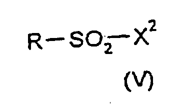 dans laquelle
R a la définition indiquée dans la revendication 1 et
X² représente un halogène,
dans chaque cas éventuellement en présence d'un diluant et, dans chaque cas, en présence éventuelle d'un accepteur d'acide,
ou bien
d) on fait réagir des amides d'acides carboxyliques de formule (II)
avec un isocyanate de formule (VI) 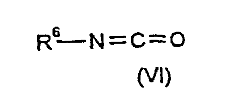 dans laquelle
R⁶ est un groupe alkyle,
en présence éventuelle d'un diluant et, le cas échéant en présence d'un auxiliaire de réaction,
ou bien
e) on hydrolyse des amidines de formule générale (VII) 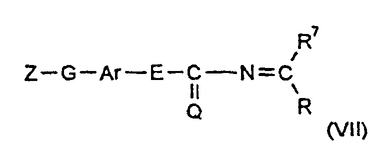 dans laquelle
Ar, E, G, Q, R et Z ont les définitions indiquées dans la revendication 1 et
R⁷ est un groupe dialkylamino,
éventuellement en présence d'un diluant et, le cas échéant en présence d'un acide.

10. Utilisation de composés de formule (I) suivant les revendications 1 à 6 pour combattre des parasites.

11. Procédé de préparation de compositions pesticides, **caractérisé en ce qu'**on mélange des composés de formule (I) suivant les revendications 1 à 6 avec des diluants et/ou des agents tensio-actifs.

12. Composés de formule (VII) 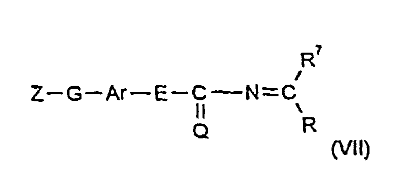 dans laquelle
Ar, E, G, Q, R et Z ont les définitions indiquées dans la revendication 1 et
R⁷ est un groupe dialkylamino.
